# EUROPEAN PATENT APPLICATION

(11) **EP 4 361 141 A1**
(43) Date of publication of application: **01.05.2024**
(21) Application number: 22827559.0
(22) Date of filing: 21.06.2022
(51) Int. Cl.: C07D 401/14, C07D 471/04, C07D 471/14, C07D 403/12, C07D 401/04, A61K 31/4439, A61K 31/536, A61P 35/00, A61P 31/12, A61P 9/00, A61P 7/00, A61P 31/20, A61P 31/22, A61P 31/18, A61P 31/16

(54) **PROTEIN DEGRADATION AGENT**

(30) Priority: 22.06.2021 CN 202110693057
(71) Applicant: Suzhou Kintor Pharmaceuticals, Inc., Jiangsu 215123 (CN)
(72) Inventor: TONG, Youzhi, Suzhou, Jiangsu 215123 (CN); YANG, Zhaohui, Suzhou, Jiangsu 215123 (CN); XU, Ruo, Suzhou, Jiangsu 215123 (CN); MA, Liandong, Suzhou, Jiangsu 215123 (CN); LAI, Luhua, Beijing 100871 (CN)
(74) Representative: HGF
(86) International application number: PCT/CN2022/100078
(87) International publication number: WO 2022/268066

(57) **Abstract**

The present invention relates to a protein degradation agent, and a preparation method therefor and a use thereof. The protein degradation agent can degrade various proteins comprising c-Myc protein, and can therefore be used for the prevention and treatment of diseases related to dysregulation of various proteins comprising c-Myc protein, such as cancer, cardiovascular and cerebrovascular diseases, and viral infection-related diseases.

## Description

### Cross Reference to Related Application

The present application claims the benefit of Chinese Patent Application No. 2021106930574 filed on June 22, 2021, which is hereby incorporated herein by reference in its entirety.

### Technical Field

The present invention belongs to the field of medicine, and in particular relates to a protein degradation agent, and a preparation method therefor and use thereof.

### Background Art

The intrinsically disordered protein c-Myc is a well-known transcription factor that can regulate approximately 15% of human genes. c-Myc can regulate various biological functions, such as cell proliferation, apoptosis, cell cycle progression, cell metabolism and embryonic development, and plays a very important role in the occurrence, development and evolution of diseases.

A large number of studies have shown that c-Myc is closely related to various tumor diseases, including lymphoma, breast cancer, prostate cancer, colon cancer, cervical cancer, multiple myeloma, myeloid leukemia, melanoma, osteosarcoma, malignant glioma, small cell lung cancer and medulloblastoma. c-Myc can promote occurrence and growth of tumors in many aspects (C Yu, et al. Sci Rep 6.1, 1-11).

In addition to cancer, c-Myc is related to some other diseases. For example, with an increased expression of c-Myc in diabetes, insulin-producing β-islet cells dedifferentiate or undergo apoptosis, and the insulin secreted thereby decreases (Magid R et al. J Biol Chem, 2003, 278: 32994). According to the "monoclonal hypothesis" of atherosclerosis, an increased expression of c-Myc in vivo is related to the generation of aortic and carotid plaques. Indeed, it was found in the early coronary artery lesions in Watanabe heritable hyperlipidemic rabbit models and the early lipid accumulation within coronary artery walls of hypercholesterolemic pigs that the c-Myc-dependent signaling pathway was activated, and more importantly, antioxidants down-regulated the overexpression of c-Myc in a manner similar to that observed in tumor cells (Prasad KN et al. Biochem Cell Biol 68, 1250-55).

c-Myc protein has become one of the most potentially attractive anti-tumor targets. There are currently two main methods reported to inhibit the function of c-Myc protein. The first method involves directly inhibiting the function of c-Myc protein, comprising inhibiting the binding of Myc/Max dimers and Myc/Max/E-box trimers; and the second method involves eliminating or degrading c-Myc protein.

GSPT1 (G1 to S phase transition 1) mediates the recognition of stop codons and promotes the release of transcribed peptide chains from ribosomes. In addition to playing a role in the transcription termination process, GSPT1 is also closely associated with a variety of important cellular activities, such as cell cycle regulation, cytoskeleton formation and apoptosis. GSPT1 is considered to be an oncogenic factor for various tumors, including breast cancer, liver cancer, gastric cancer and prostate cancer (Cui, Jian, et al. International journal of oncology, 2020, 56(4): 867-878).

Casein kinase 1α (CK1α) can phosphorylate p53 protein. As a tumor suppressor protein, p53 protein participates in various signal transductions within cells and plays an important role in processes such as cell cycle regulation and apoptosis. Phosphorylated p53 can bind to murine double minute 2 (MDM2) and then be degraded by ubiquitination (Huart, Anne-Sophie, et al. Journal of Biological Chemistry 284.47 (2009): 32384-32394). Therefore, blocking CK1α activity can stabilize p53, thereby exerting its tumor suppressor activity.

The zinc finger transcription factors IKZF1/2/3 (Aiolos, Helios and Ikaros) belong to the Ikaros zinc-finger (IKZF) family, and are essential for the survival of lymphoid cells. For example, Aiolos can bind to the enhancer of B-cell lymphoma-2 (Bcl-2), thereby upregulating the effect of Bcl-2 protein on cell survival. Moreover, abnormally activated Helios and Ikaros can upregulate the expression of Bcl-XL and drive the occurrence and development of various hematological tumors.

Current methods generally have the disadvantage of poor activity. In order to satisfy this unmet clinical need, the applicant has developed a series of compounds that can efficiently clear c-Myc protein, which can be used to treat diseases related to high expression of various proteins comprising c-Myc.

### Summary of the Invention

The object of the present invention is to provide a c-Myc protein degradation agent, and a preparation method therefor and use thereof. The compounds of the present invention can be used to degrade various proteins comprising c-Myc protein, such as N-myc, GSPT1, CK1α, IKZF (1/2/3), AR and AR-V7, thereby preventing, alleviating or treating diseases related to dysregulation of these proteins.

In one aspect, the present invention provides a compound represented by formula (I), and a pharmaceutically acceptable salt, solvent compound, stereoisomer, isotope and prodrug thereof: 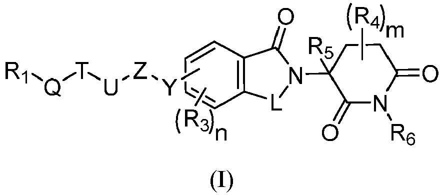 wherein,
R₁ is selected from RₐC(=O)-, RₐCH₂C(=O)-, RₐCH₂CH₂C(=O)-, Rₐ-, RₐCH₂-, RₐCH₂CH₂-, RₐNHC(=O)-, RₐCH₂NHC(=O)-, RₐCH₂CH₂NHC(=O)-, RₐOC(=O)-, RₐCH₂OC(=O)-, RₐCH₂CH₂OC(=O)-, RₐS(=O)₂-, RₐCH₂S(=O)₂- or RₐCH₂CH₂S(=O)₂-, wherein the "CH₂" is optionally substituted with one or more C₁-C₄ alkyl, or is optionally substituted with -CH₂CH₂-, and the "NH" is optionally substituted with R_{b}-, R_{b}C(=O)- or R_{b}S(=O)₂-;
Q is selected from -NR₂- or -O-;
R₂ is selected from hydrogen, R_{b}-, R_{b}C(=O)- or R_{b}S(=O)₂-;
Rₐ and R_{b} are each independently selected from C₁-C₈ alkyl, C₃-C₁₀ cycloalkyl, a C₃-C₁₀ bridged cyclic group, -NR₁₁R₁₂, C₃-C₁₀ heterocyclyl optionally containing O, S, SO₂, N or NHC(=O)R₂₂, aryl, heteroaryl, fused arylcycloalkyl, fused heteroarylheterocyclyl, arylcycloalkyl, aryl-heterocyclyl, cycloalkyl-heterocyclyl or heterocyclyl-heterocyclyl, wherein a group selected from one of -O-, -S-, -C(=O)-, -S(=O)-, -S(=O)₂-, -NH-, - NHC(=O)-, -NHC(=O)NH- or -NHS(=O)₂- may be inserted between any two C-C of the C₁-C₈ alkyl, and Rₐ or R_{b} may be optionally substituted with one or more R₉;
T, U and Z are each independently selected from a chemical bond, carbonyl, C₁-C₆ alkylene, C₃-C₁₀ cycloalkylene, arylene, heteroarylene or heterocyclylene, wherein the alkylene, cycloalkylene, arylene, heteroarylene or heterocyclylene may be optionally substituted with one or more R₉;
Y is selected from a chemical bond, -NHC(=O)-, -C(=O)NH-, -C(=O)NHCH₂-, - NHC(=O)CH₂-, -O-, -OCH₂-, -OCH₂CH₂-, -NH-, -NHCH₂- or -NHCH₂CH₂-, wherein the "CH₂" is optionally substituted with one or more C₁-C₄ alkyl, or is optionally substituted with -CH₂CH₂-, and the "NH" is optionally substituted with R_{b}-, R_{b}C(=O)- or R_{b}S(=O)₂-;
L is selected from -(CR₇R₈)ₒ- or -C(=O)-;
R₃-R₅ and R₇-R₉ are each independently selected from hydrogen, halogen, hydroxyl, cyano, amino, nitro, -R₂₁NHC(=O)R₂₂, -R₂₁C(=O)OR₂₂, C₁-C₆ alkyl, C₁-C₆ alkoxy, C₁-C₆ alkylthio, C₁-C₆ alkylamino, C₃-C₈ cycloalkyl, C₂-C₈ alkenyl, C₂-C₈ alkynyl, aryl, 5- to 6-membered heteroaryl containing 1-3 heteroatoms or 3- to 10-membered heterocyclyl containing 1-3 heteroatoms, wherein the alkyl, alkoxy, alkylamino, alkylthio, cycloalkyl, aryl, heteroaryl or heterocyclyl is optionally substituted with 1-3 groups each selected from halogen, cyano, C₁-C₃ alkyl or C₁-C₃ alkoxy; when there are multiple R₃-R₅ and R₇-R₉, any two adjacent ones may be combined to form a ring;
R₆ is selected from hydrogen, C₁-C₆ alkyl, C₃-C₈ cycloalkyl, C₂-C₈ alkenyl, C₂-C₈ alkynyl, aryl, 5- to 6-membered heteroaryl containing 1-3 heteroatoms or 3- to 10-membered heterocyclyl containing 1-3 heteroatoms, wherein the alkyl, cycloalkyl, aryl, heteroaryl or heterocyclyl is optionally substituted with 1-3 groups each selected from halogen, hydroxyl, cyano, amino, nitro, -C(=O)OR₂₃, -OC(=O)R₂₃, -NHC(=O)R₂₃, - C(=O)NHR₂₃, C₁-C₃ alkyl, C₁-C₃ alkoxy or -OP(=O)(OM)₂;
M is independently selected from hydrogen or C₁-C₄ alkyl;
R₁₁ and R₁₂ are each independently selected from hydrogen, C₁-C₄ alkyl or aryl;
R₂₁ is selected from C₁-C₄ alkyl;
R₂₂ is selected from hydrogen, amino, C₁-C₄ alkyl, C₁-C₄ alkoxy, allyl or benzyl, wherein the C₁-C₄ alkyl, C₁-C₄ alkoxy, allyl or benzyl is optionally substituted with aryl or -C(=O)OR₂₁;
R₂₃ is selected from hydrogen or C₁-C₄ alkyl, wherein the C₁-C₄ alkyl is optionally substituted with 1-3 groups each selected from halogen, hydroxyl or amino;
n is selected from 0, 1, 2 or 3;
m is selected from 0, 1, 2, 3 or 4;
o is selected from 1 or 2.

Preferably, the present invention provides a compound represented by formula (I), and a pharmaceutically acceptable salt, solvent compound, stereoisomer, isotope and prodrug thereof: 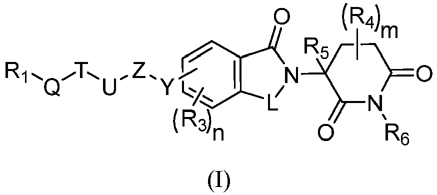 wherein,
R₁ is selected from RₐC(=O)-, RₐCH₂C(=O)-, RₐCH₂CH₂C(=O)-, Rₐ-, RₐCH₂-, RₐCH₂CH₂-, RₐNHC(=O)-, RₐCH₂NHC(=O)-, RₐCH₂CH₂NHC(=O)-, RₐOC(=O)-, RₐCH₂OC(=O)-, RₐCH₂CH₂OC(=O)-, RaS(=O)2-, RₐCH₂S(=O)₂- or RₐCH₂CH₂S(=O)₂-, wherein the "CH₂" is optionally substituted with one or more C₁-C₄ alkyl, or is optionally substituted with -CH₂CH₂-, and the "NH" is optionally substituted with C₁-C₄ alkyl;
Q is selected from -NR₂- or -O-;
R₂ is selected from hydrogen, R_{b}-, R_{b}C(=O)- or R_{b}S(=O)₂-;
Rₐ and R_{b} are each independently selected from C₁-C₈ alkyl, C₃-C₁₀ cycloalkyl, a C₃-C₁₀ bridged cyclic group, -NR₁₁R₁₂, C₃-C₁₀ heterocyclyl optionally containing O, S, SO₂, N or NHC(=O)R₂₂, aryl, heteroaryl, fused arylcycloalkyl, fused heteroarylheterocyclyl, arylcycloalkyl, aryl-heterocyclyl, cycloalkyl-heterocyclyl or heterocyclyl-heterocyclyl, and Rₐ or R_{b} may be optionally substituted with one or more R₉;
T, U and Z are each independently selected from a chemical bond, carbonyl, C₁-C₆ alkylene, C₃-C₁₀ cycloalkylene, arylene, heteroarylene or heterocyclylene, wherein the alkylene, cycloalkylene, arylene, heteroarylene or heterocyclylene may be optionally substituted with one or more R₉;
Y is selected from a chemical bond, -NHC(=O)-, -C(=O)NH-, -C(=O)NHCH₂-, - NHC(=O)CH₂-, -O-, -OCH₂-, -OCH₂CH₂-, -NH-, -NHCH₂- or -NHCH₂CH₂-, wherein the "CH₂" is optionally substituted with one or more C₁-C₄ alkyl, or is optionally substituted with -CH₂CH₂-, and the "NH" is optionally substituted with C₁-C₄ alkyl;
L is selected from -(CR₇R₈)ₒ- or -C(=O)-;
R₃-R₅ and R₇-R₉ are each independently selected from hydrogen, halogen, hydroxyl, cyano, amino, -R₂₁NHC(=O)R₂₂, -R₂₁C(=O)OR₂₂, C₁-C₆ alkyl, C₁-C₆ alkoxy, C₁-C₆ alkylthio, C₁-C₆ alkylamino, C₃-C₈ cycloalkyl, C₂-C₈ alkenyl, C₂-C₈ alkynyl, aryl, 5- to 6-membered heteroaryl containing 1-3 heteroatoms or 3- to 10-membered heterocyclyl containing 1-3 heteroatoms, wherein the alkyl, alkoxy, alkylamino, alkylthio, cycloalkyl, aryl, heteroaryl or heterocyclyl is optionally substituted with 1-3 groups each selected from halogen, cyano, C₁-C₃ alkyl or C₁-C₃ alkoxy; when there are multiple R₃-R₅ and R₇-R₉, any two adjacent ones may be combined to form a ring;
R₆ is selected from hydrogen, C₁-C₆ alkyl, C₃-C₈ cycloalkyl, C₂-C₈ alkenyl, C₂-C₈ alkynyl, aryl, 5- to 6-membered heteroaryl containing 1-3 heteroatoms or 3- to 10-membered heterocyclyl containing 1-3 heteroatoms, wherein the alkyl, cycloalkyl, aryl, heteroaryl or heterocyclyl is optionally substituted with 1-3 groups each selected from halogen, cyano, C₁-C₃ alkyl or C₁-C₃ alkoxy;
R₁₁ and R₁₂ are each independently selected from hydrogen, C₁-C₄ alkyl or aryl;
R₂₁ is selected from C₁-C₄ alkyl;
R₂₂ is selected from hydrogen, amino, C₁-C₄ alkyl, C₁-C₄ alkoxy, allyl or benzyl, wherein the C₁-C₄ alkyl, C₁-C₄ alkoxy, allyl or benzyl is optionally substituted with aryl or -C(=O)OR₂₁;
n is selected from 0, 1, 2 or 3;
m is selected from 0, 1, 2, 3 or 4;
o is selected from 1 or 2.

In some embodiments, the R₁ is selected from RₐC(=O)-, RₐCH₂C(=O)-, RₐCH₂CH₂C(=O)-, Rₐ-, RₐCH₂-, RₐCH₂CH₂-, RₐNHC(=O)-, RₐCH₂NHC(=O)-, RₐCH₂CH₂NHC(=O)-, RₐOC(=O)- or RₐCH₂OC(=O)- or RₐCH₂CH₂OC(=O)-, wherein the "CH₂" is optionally substituted with one or more C₁-C₄ alkyl, or is optionally substituted with -CH₂CH₂-, the "NH" is optionally substituted with R_{b}-, R_{b}C(=O)- or R_{b}S(=O)₂-, the R_{b} is selected from C₁-C₄ alkyl, the C₁-C₄ alkyl is optionally substituted with one or more R₉, the Rₐ is selected from C₁-C₈ alkyl, -NR₁₁R₁₂, aryl or heteroaryl, and Rₐ may be optionally substituted with one or more R₉.

In some embodiments, the R₁ is selected from RₐC(=O)-, RₐCH₂C(=O)-, RₐCH₂CH₂C(=O)-, Rₐ-, RₐCH₂-, RₐCH₂CH₂-, RₐNHC(=O)-, RₐCH₂NHC(=O)-, RₐCH₂CH₂NHC(=O)-, RₐOC(=O)- or RₐCH₂OC(=O)-, or RₐCH₂CH₂OC(=O)-, wherein the "CH₂" is optionally substituted with one or more C₁-C₄ alkyl, or is optionally substituted with -CH₂CH₂-, the "NH" is optionally substituted with C₁-C₄ alkyl, the Rₐ is selected from C₁-C₈ alkyl, -NR₁₁R₁₂, aryl or heteroaryl, and Rₐ may be optionally substituted with one or more R₉.

In some embodiments, the Rₐ is selected from C₂-C₆ alkyl, carbazolyl, 1-azacarbazolyl, 2-azacarbazolyl, 1,8-diazacarbazolyl, indolyl, phenoxazinyl, naphthyl, fluorenyl, diphenylamino, dibenzylamino, tert-butyl, quinolyl, isoquinolyl, phenyl, 2,3-indolinyl, 7-azaindolyl, 2,3-dihydro-7-azaindolyl, naphthyridinyl, tetrahydronaphthyridinyl, tetrahydroquinolyl, pyrimidyl or triazolyl, and Ra may be optionally substituted with one or more R₉, wherein the R₉ is selected from hydrogen, halogen, C₁-C₆ alkoxy, cyano, C₁-C₆ alkyl, or C₁-C₆ alkyl, phenyl or naphthyl optionally substituted with 1-3 groups selected from halogen or hydroxyl.

In some embodiments, the Rₐ is selected from C₂-C₆ alkyl, carbazolyl, 1-azacarbazolyl, 2-azacarbazolyl, 1,8-diazacarbazolyl, indolyl, phenoxazinyl, naphthyl, fluorenyl, diphenylamino, dibenzylamino, tert-butyl, quinolyl, isoquinolyl or phenyl, and Rₐ may be optionally substituted with one or more R₉, wherein the R₉ is selected from hydrogen, halogen, C₁-C₆ alkoxy, cyano, C₁-C₆ alkyl, or C₁-C₆ alkyl optionally substituted with 1-3 groups selected from halogen.

Preferably, the Rₐ is selected from tert-butyl, carbazol-1-yl, 1-azacarbazol-9-yl, 2-azacarbazol-9-yl, 1,8-diazacarbazol-9-yl, indol-1-yl, phenoxazin-10-yl, naphthalen-1-yl, naphthalen-2-yl, fluoren-9-yl, diphenylamino, dibenzylamino, tert-butyl, quinolin-4-yl, quinolin-5-yl, quinolin-8-yl, isoquinolin-1-yl, isoquinolin-4-yl, isoquinolin-5-yl, isoquinolin-8-yl, phenyl, 2,3-dihydro-indol-1-yl, 7-azaindol-1-yl, 2,3-dihydro-7-azaindol-1-yl, 1,2,3,4-tetrahydro-1,8-naphthyridin-1-yl, 1,2,3,4-tetrahydroquinolin-1-yl, pyrimidin-2-yl, pyrimidin-4-yl, pyrimidin-5-yl, 1,2,3-triazol-1-yl, 1,2,4-triazol-1-yl or 1,3,4-triazol-1-yl, and Rₐ may be optionally substituted with one or more R₉, wherein the R₉ is selected from F, Cl, Br, methoxy, ethoxy, cyano, methyl, ethyl, trifluoromethyl, hydroxymethyl, phenyl, naphthalen-1-yl or naphthalen-2-yl.

Preferably, the Rₐ is selected from tert-butyl, carbazol-1-yl, 1-azacarbazol-9-yl, 2-azacarbazol-9-yl, 1,8-diazacarbazol-9-yl, indol-1-yl, phenoxazin-10-yl, naphthalen-1-yl, naphthalen-2-yl, fluoren-9-yl, diphenylamino, dibenzylamino, tert-butyl, quinolin-4-yl, quinolin-5-yl, quinolin-8-yl, isoquinolin-1-yl, isoquinolin-4-yl, isoquinolin-5-yl, isoquinolin-8-yl or phenyl, and Rₐ may be optionally substituted with one or more R₉, wherein the R₉ is selected from F, Cl, Br, methoxy, ethoxy, cyano, methyl, ethyl or trifluoromethyl.

Preferably, the Rₐ is selected from the following groups:

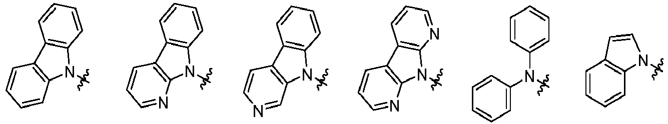

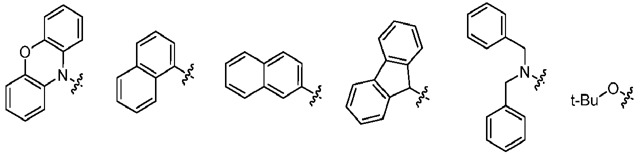

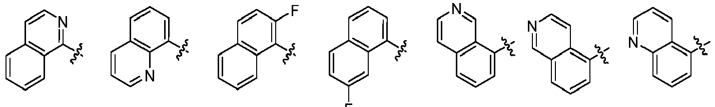

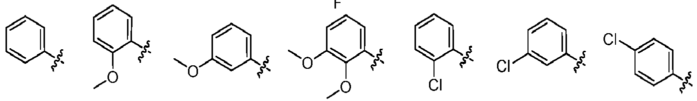

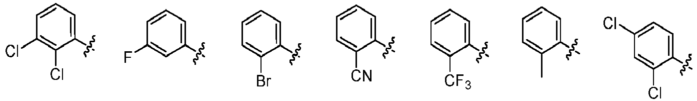

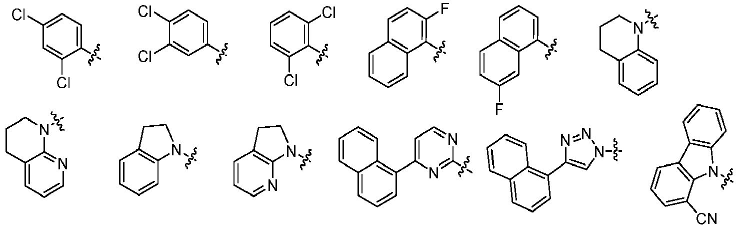

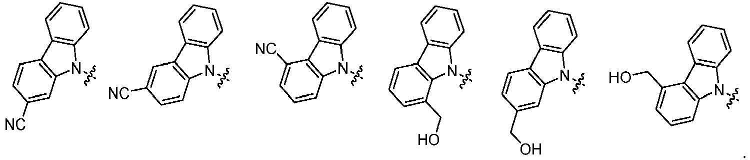

Preferably, the Rₐ is selected from the following groups:

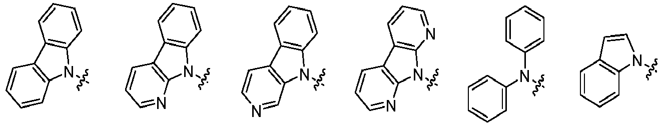

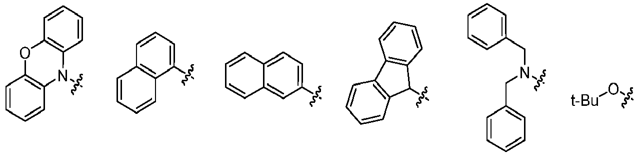

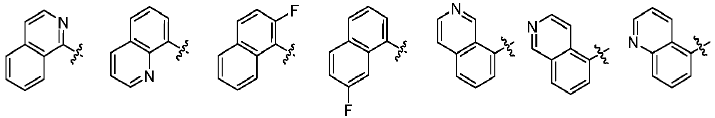

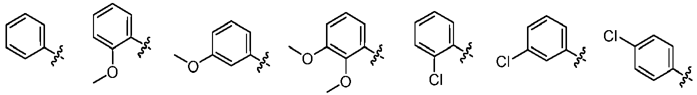

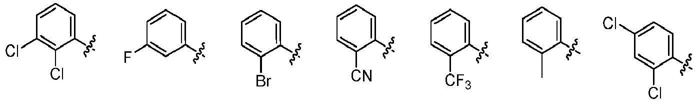

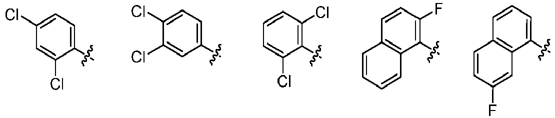

In some embodiments, the Q is selected from -NR₂-; the R₂ is selected from hydrogen, C₁-C₈ alkyl, C₃-C₁₀ cycloalkyl, aryl, C₁-C₈ alkyl C(=O)- or C₁-C₈ alkyl S(=O)₂-, wherein the C₁-C₈ alkyl, C₃-C₁₀ cycloalkyl, aryl and the alkyl portion of the C₁-C₈ alkyl C(=O)- or C₁-C₈ alkyl S(=O)₂- are optionally substituted with one or more R₉, and the R₉ is selected from hydrogen, halogen, hydroxyl, amino, cyano, carboxyl, C₁-C₆ alkyl, C₁-C₆ alkoxy or aryl.

In some embodiments, the Q is selected from -NR₂-; the R₂ is selected from hydrogen, C₁-C₈ alkyl, C₃-C₁₀ cycloalkyl, aryl, C₁-C₈ alkyl C(=O)- or C₁-C₈ alkyl S(=O)₂-, wherein the C₁-C₈ alkyl, C₃-C₁₀ cycloalkyl, aryl and the alkyl portion of the C₁-C₈ alkyl C(=O)- or C₁-C₈ alkyl S(=O)₂- are optionally substituted with one or more R₉, and the R₉ is selected from hydrogen, halogen, cyano, C₁-C₆ alkyl, C₁-C₆ alkoxy or aryl.

In some embodiments, the Q is selected from -NH-, -O-, -N(CH₃)-, - N(SO₂CH₃)-, -N(COCH₃)-, -N(CO-isopropyl)-, -N(CO-cyclopropyl)-, -N(isopropyl)-, - N(cyclopropyl)-, -N(2-methoxyethyl)-, -N(2-cyanoethyl)-, -N(phenyl)-, -N(benzyl)-, -N(1-naphthylmethyl)-, -N(2-naphthylethyl)-, -N(CH₂OH)-, -N(CH₂CH₂OH)-, - N(CH₂CH₂CH₂OH)-, -N(COCH₂OH)-, -N(COCH₂CH₂OH)-, -N(COCH₂CH₂CH₂OH)-, - N(CH₂NH₂)-, -N(CH₂CH₂NH₂)-, -N(COCH₂CH₂CH₂NH₂)-, -N(COCH₂NH₂)-, - N(COCH₂CH₂NH₂)- or -N(COCH₂CH₂CH₂NH₂)-.

In some embodiments, the Q is selected from -NH-, -O-, -N(CH₃)-, - N(SO₂CH₃)-, -N(COCH₃)-, -N(CO-isopropyl)-, -N(CO-cyclopropyl)-, -N(isopropyl)-, - N(cyclopropyl)-, -N(2-methoxyethyl)-, -N(2-cyanoethyl)-, -N(phenyl)- or -N(benzyl)-.

In some embodiments, the T and Z are each independently selected from a chemical bond, carbonyl, C₁-C₆ alkylene or C₃-C₁₀ cycloalkylene, wherein the C₁-C₆ alkylene or C₃-C₁₀ cycloalkylene may be optionally substituted with one or more R₉, wherein the R₉ is selected from hydrogen, C₁-C₆ alkyl or C₃-C₈ cycloalkyl.

Preferably, the T and Z are each independently selected from a chemical bond, carbonyl, methylene, 1,2-ethylene, 1,1-cyclopropylene or 2,2-propylene.

In some embodiments, the U is selected from C₁-C₆ alkylene, C₃-C₁₀ cycloalkylene, arylene or heteroarylene, wherein the alkylene, cycloalkylene, arylene or heteroarylene may be optionally substituted with one or more R₉.

Preferably, the U is selected from C₂-C₆ alkylene, C₅-C₆ cycloalkylene, C₆-C₁₀ arylene or 5- to 6-membered monocyclic heteroarylene, wherein the alkylene, cycloalkylene, arylene or heteroarylene may be optionally substituted with one or more R₉.

Preferably, the U is selected from 1,2-ethylene, 1,3-propylene, 1,4-butylene, 1,5-pentylene, 1,6-hexylene, 1,3-cyclopentylene, 1,3-cyclohexylene, 1,4-cyclohexylene, 1,3-phenylene, 1,4-phenylene, 2,5-pyridylene, 2,5-pyrimidinylene, 2,5-thiazolylene or 2,4-thiazolylene, wherein the 1,3-phenylene, 1,4-phenylene, 2,5-pyridylene, 2,5-pyrimidinylene, 2,5-thiazolylene or 2,4-thiazolylene may be optionally substituted with one or more R₉.

Preferably, the R₉ is selected from hydrogen, halogen, C₁-C₆ alkyl or C₁-C₆ alkoxy.

Preferably, the R₉ is selected from F, Cl, Br, methyl, methoxy or trifluoromethyl.

Preferably, the U is selected from the following groups:

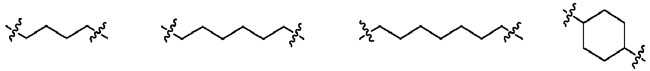

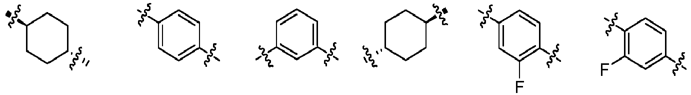

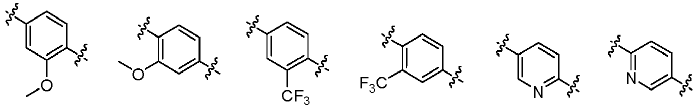

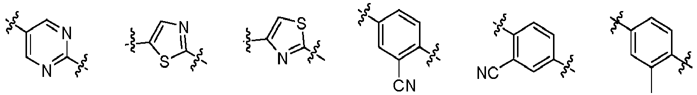

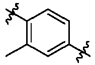

Preferably, the U is selected from the following groups:

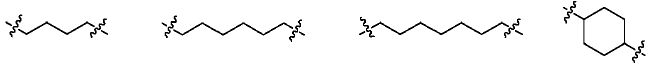

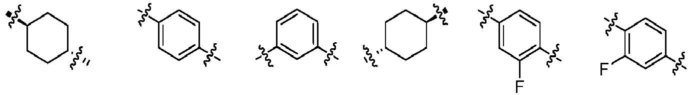

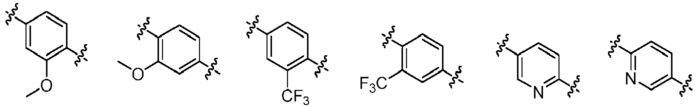

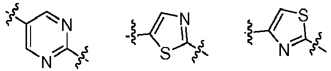

In some embodiments, the -T-U-Z- together form a group selected from:

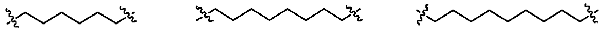

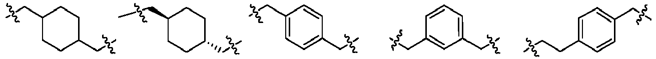

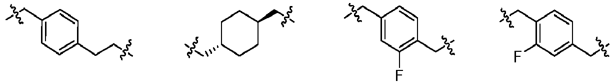

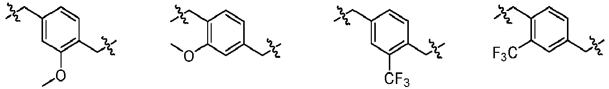

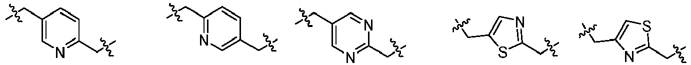

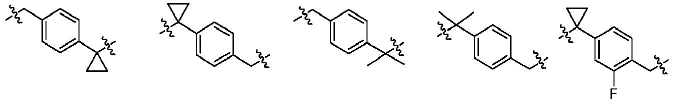

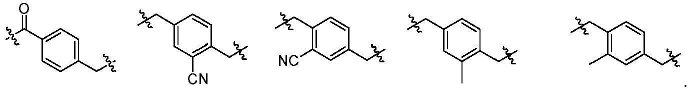

In some embodiments, the -T-U-Z- together form a group selected from:

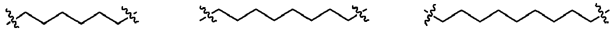

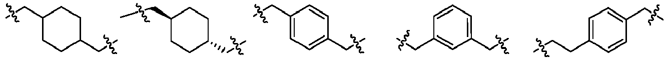

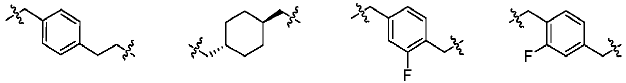

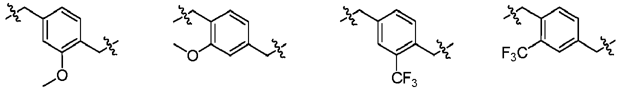

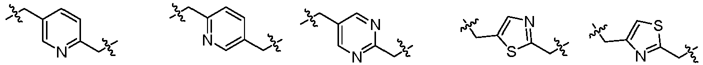

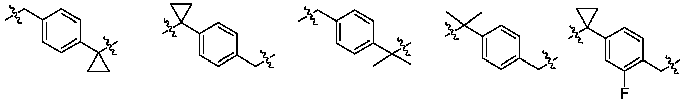

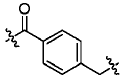

In some embodiments, the Y is selected from a chemical bond, -NHC(=O)-, - C(=O)NH-, -C(=O)NHCH₂-, -NHC(=O)CH₂-, -O-, -OCH₂-, -OCH₂CH₂-, -NH-, -NHCH₂- or -N(CH₃)CH₂-, wherein the "NH" is optionally substituted with R_{b}-, R_{b}C(=O)- or R_{b}S(=O)₂-, the R_{b} is selected from C₁-C₄ alkyl, and the C₁-C₄ alkyl is optionally substituted with one or more R₉.

In some embodiments, the Y is selected from a chemical bond, -NHC(=O)-, - C(=O)NH-, -C(=O)NHCH₂-, -NHC(=O)CH₂-, -O-, -OCH₂-, -OCH₂CH₂-, -NH-, -NHCH₂-, -N(CH₃)CH₂-, -N(CH₂OH)CH₂-, -N(CH₂CH₂OH)CH₂-, -N(CH₂CH₂CH₂OH)CH₂-, - N(COCH₂OH)CH₂-, -N(COCH₂CH₂OH)CH₂-, -N(COCH₂CH₂CH₂OH)CH₂-, - N(CH₂NH₂)CH₂-, -N(CH₂CH₂NH₂)CH₂-, -N(COCH₂CH₂CH₂NH₂)CH₂-, - N(COCH₂NH₂)CH₂-, -N(COCH₂CH₂NH₂)CH₂- or -N(COCH₂CH₂CH₂NH₂)CH₂-.

In some embodiments, the Y is selected from a chemical bond, -NHC(=O)-, - C(=O)NH-, -C(=O)NHCH₂-, -NHC(=O)CH₂-, -O-, -OCH₂-, -OCH₂CH₂-, -NH-, -NHCH₂- or -N(CH₃)CH₂-.

Preferably, the Y is selected from -NHC(=O)-, -C(=O)NH- or -NHCH₂-.

In some embodiments, the L is selected from -CH₂-, -CH₂CH₂- or -C(=O)-.

Preferably, the L is selected from -CH₂- or -C(=O)-.

In some embodiments, the R₃-R₅ are each independently selected from hydrogen, halogen, hydroxyl, cyano, amino, C₁-C₆ alkyl, C₁-C₆ alkoxy, C₁-C₆ alkylthio, C₁-C₆ alkylamino or C₃-C₈ cycloalkyl, wherein the alkyl, alkoxy, alkylamino, alkylthio or cycloalkyl is optionally substituted with 1-3 groups each selected from halogen, C₁-C₃ alkyl or C₁-C₃ alkoxy; when there are multiple R₃-R₅, any two adjacent ones may be combined to form a ring.

Preferably, the R₃-R₅ are each independently selected from hydrogen, halogen, cyano, C₁-C₄ alkyl or C₁-C₄ haloalkyl.

In some embodiments, the R₆ is selected from hydrogen, C₁-C₆ alkyl or C₃-C₈ cycloalkyl, wherein the alkyl or cycloalkyl is optionally substituted with 1-3 groups each selected from halogen, hydroxyl, cyano, amino, -C(=O)OR₂₃, -OC(=O)R₂₃, -NHC(=O)R₂₃, -C(=O)NHR₂₃ or -OP(=O)(OM)₂.

In some embodiments, the R₆ is selected from hydrogen, C₁-C₄ alkyl, C₁-C₄ haloalkyl, -C₁-C₄ alkylene OC(=O)R₂₃, -C₁-C₄ alkylene C(=O)OR₂₃ or -C₁-C₄ alkylene OP(=O)(OH)₂; R₂₃ is selected from hydrogen or C₁-C₄ alkyl, wherein the C₁-C₄ alkyl is optionally substituted with 1-3 groups each selected from hydroxyl or amino.

In some embodiments, the R₆ is selected from hydrogen, C₁-C₄ alkyl, C₁-C₄ haloalkyl, -CH₂OC(=O)R₂₃, -CH₂CH₂OC(=O)R₂₃, -CH₂C(=O)OR₂₃, -CH₂CH₂C(=O)OR₂₃, -CH₂OP(=O)(OH)₂ or -CH₂CH₂OP(=O)(OH)₂; R₂₃ is selected from hydrogen or C₁-C₄ alkyl, wherein the C₁-C₄ alkyl is optionally substituted with 1-3 groups each selected from hydroxyl or amino.

In some embodiments, the R₆ is selected from hydrogen, C₁-C₄ alkyl, C₁-C₄ haloalkyl, -CH₂OC(=O)C₁-C₄ alkyl, -CH₂OC(=O)CH₂OH, -CH₂OC(=O)CH₂NH₂ or - CH₂OP(=O)(OH)₂.

In some embodiments, the R₆ is selected from hydrogen, C₁-C₆ alkyl or C₃-C₈ cycloalkyl, wherein the alkyl or cycloalkyl is optionally substituted with 1-3 groups each selected from halogen, C₁-C₃ alkyl or C₁-C₃ alkoxy.

Preferably, the R₆ is selected from hydrogen, C₁-C₄ alkyl or C₁-C₄ haloalkyl.

In some embodiments, the compound represented by formula (I) is a compound represented by formula (I-1) or formula (I-2): 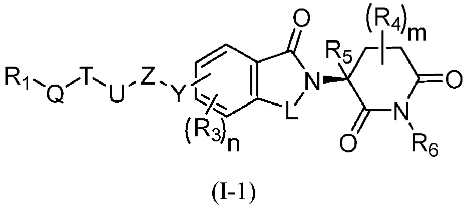 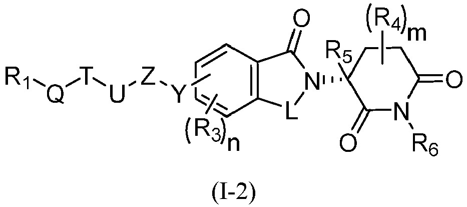 wherein R₁, R₃-R₆, Q, T, U, Z, Y, L, n and m are defined as above.

In some embodiments, the compound is a compound represented by formula (II) or formula (III), and a pharmaceutically acceptable salt, solvent compound, stereoisomer, isotope and prodrug thereof: 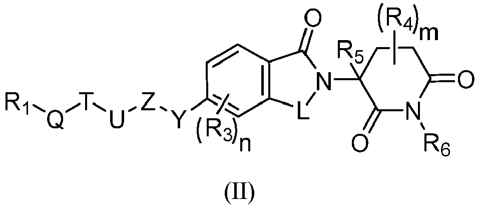 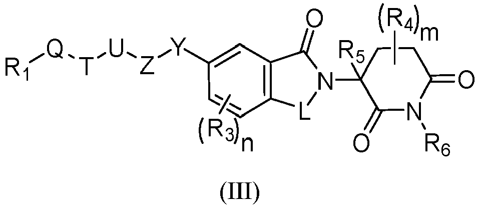 wherein R₁, R₃-R₆, Q, T, U, Z, Y, L, n and m are defined as above.

In some embodiments, the compound is a compound represented by formula (IV) or formula (V), and a pharmaceutically acceptable salt, solvent compound, stereoisomer, isotope and prodrug thereof: 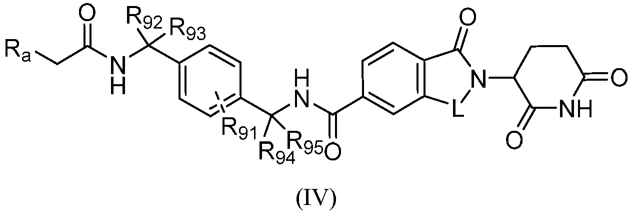 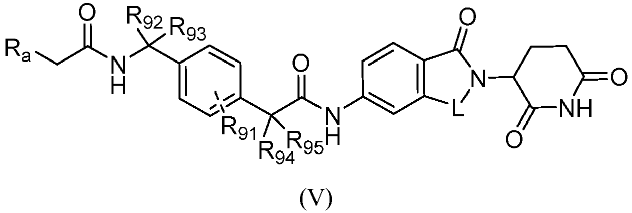
wherein the "NH" in the RₐCH₂C(=O)NH-, -C(R₉₄)(R₉₅)NHC(=O)- or - C(R₉₄)(R₉₅)C(=O)NH- is optionally substituted with R_{b}-, R_{b}C(=O)- or R_{b}S(=O)₂-;
Rₐ, R_{b} and L are defined as above;
R₉₁-R₉₅ are each independently selected from hydrogen, halogen, hydroxyl, cyano, amino, -R₂₁NHC(=O)R₂₂, -R₂₁C(=O)OR₂₂, C₁-C₆ alkyl, C₁-C₆ alkoxy, C₁-C₆ alkylthio, C₁-C₆ alkylamino, C₃-C₈ cycloalkyl, C₂-C₈ alkenyl, C₂-C₈ alkynyl, aryl, 5- to 6-membered heteroaryl containing 1-3 heteroatoms or 3- to 10-membered heterocyclyl containing 1-3 heteroatoms, wherein the alkyl, alkoxy, alkylamino, alkylthio, cycloalkyl, aryl, heteroaryl or heterocyclyl is optionally substituted with 1-3 groups each selected from halogen, cyano, C₁-C₃ alkyl or C₁-C₃ alkoxy; any two adjacent ones of R₉₂-R₉₄ may be combined to form a ring.

Preferably, the "NH" in the RₐCH₂C(=O)NH-, -C(R₉₄)(R₉₅)NHC(=O)- or - C(R₉₄)(R₉₅)C(=O)NH- is unsubstituted. Preferably, the R₉₁ is selected from hydrogen, halogen, hydroxyl, cyano, amino, C₁-C₆ alkyl, C₁-C₆ alkoxy, C₁-C₆ alkylthio, C₁-C₆ alkylamino, C₃-C₈ cycloalkyl, aryl, 5- to 6-membered heteroaryl containing 1-3 heteroatoms or 3- to 10-membered heterocyclyl containing 1-3 heteroatoms, wherein the alkyl, alkoxy, alkylamino, alkylthio, cycloalkyl, aryl, heteroaryl or heterocyclyl is optionally substituted with 1-3 groups each selected from halogen, cyano, C₁-C₃ alkyl or C₁-C₃ alkoxy.

Preferably, the R₉₂-R₉₄ are each independently selected from hydrogen or C₁-C₆ alkyl, or any two adjacent ones of R₉₂-R₉₄ may be combined to form cycloalkyl. Preferably, the R₉₂-R₉₄ are each independently selected from hydrogen, methyl or ethyl, or R₉₂ and R₉₃ together form -CH₂CH₂-, or R₉₄ and R₉₅ together form -CH₂CH₂-.

In some embodiments, the compound is a compound represented by formula (IV') or formula (V'), and a pharmaceutically acceptable salt, solvent compound, stereoisomer, isotope and prodrug thereof: 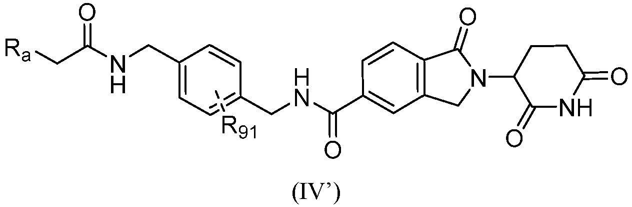 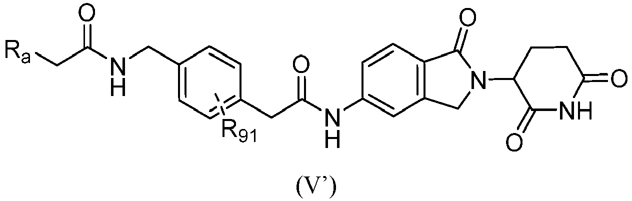
wherein the "NH" in the RₐCH₂C(=O)NH-, -CH₂NHC(=O)- or -CH₂C(=O)NH- is optionally substituted with R_{b}-, R_{b}C(=O)- or R_{b}S(=O)₂-;
Rₐ, R_{b} and R₉₁ are defined as above.

Preferably, the "NH" in the RₐCH₂C(=O)NH-, -CH₂NHC(=O)- or - CH₂C(=O)NH- is unsubstituted. In some embodiments, the compound is a compound represented by formula (VI), and a pharmaceutically acceptable salt, solvent compound, stereoisomer, isotope and prodrug thereof: 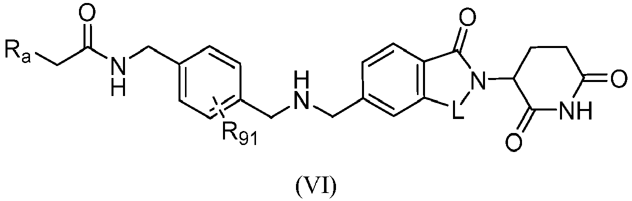
wherein the "NH" in the RₐCH₂C(=O)NH- or -CH₂NHCH₂- is optionally substituted with R_{b}-, R_{b}C(=O)- or R_{b}S(=O)₂-;
Rₐ, R_{b}, R₉₁ and L are defined as above.

Preferably, the "NH" in the RₐCH₂C(=O)NH- or -CH₂NHCH₂- is unsubstituted.

In some embodiments, the compound is a compound represented by formula (VI'), and a pharmaceutically acceptable salt, solvent compound, stereoisomer, isotope and prodrug thereof: 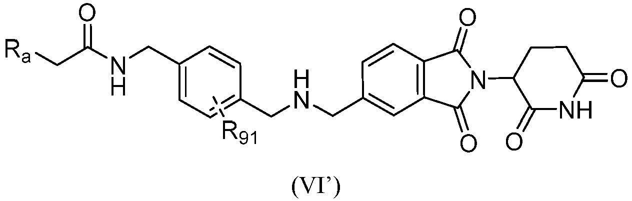
wherein the "NH" in the RₐCH₂C(=O)NH- or -CH₂NHCH₂- is optionally substituted with R_{b}-, R_{b}C(=O)- or R_{b}S(=O)₂-;
Rₐ, R_{b} and R₉₁ are defined as above.

Preferably, the "NH" in the RₐCH₂C(=O)NH- or -CH₂NHCH₂- is unsubstituted.

In some embodiments, the compound is a compound represented by formula (VII), and a pharmaceutically acceptable salt, solvent compound, stereoisomer, isotope and prodrug thereof: 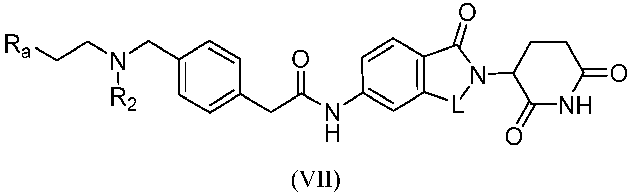
wherein the "NH" in the -CH₂C(=O)NH- is optionally substituted with R_{b}-, R_{b}C(=O)- or R_{b}S(=O)₂-;
Rₐ, R_{b}, R₂ and L are defined as above.

Preferably, the "NH" in the -CH₂C(=O)NH- is unsubstituted.

Preferably, the R₂ is selected from hydrogen, C₁-C₈ alkyl, C₃-C₁₀ cycloalkyl, aryl, C₁-C₈ alkyl C(=O)- or C₁-C₈ alkyl S(=O)₂-, wherein the C₁-C₈ alkyl, C₃-C₁₀ cycloalkyl, aryl and the alkyl portion of the C₁-C₈ alkyl C(=O)- or C₁-C₈ alkyl S(=O)₂- are optionally substituted with one or more R₉, and the R₉ is selected from hydrogen, halogen, hydroxyl, amino, cyano, C₁-C₆ alkyl, C₁-C₆ alkoxy or aryl; preferably, the R₂ is selected from hydrogen, methyl, ethyl, isopropyl, cyclopropyl, -SO₂CH₃, -CO cyclopropyl, 2-methoxyethyl, 2-cyanoethyl, 2-hydroxyethyl, 2-aminoethyl, phenyl, benzyl, 1-naphthylmethyl or 2-naphthylethyl.

Preferably, the R₂ is selected from hydrogen, C₁-C₈ alkyl, C₃-C₁₀ cycloalkyl, aryl, C₁-C₈ alkyl C(=O)- or C₁-C₈ alkyl S(=O)₂-, wherein the C₁-C₈ alkyl, C₃-C₁₀ cycloalkyl, aryl and the alkyl portion of the C₁-C₈ alkyl C(=O)- or C₁-C₈ alkyl S(=O)₂- are optionally substituted with one or more R₉, and the R₉ is selected from hydrogen, halogen, cyano, C₁-C₆ alkyl, C₁-C₆ alkoxy or aryl.

Preferably, the R₂ is selected from hydrogen, methyl, ethyl, isopropyl, cyclopropyl, -SO₂CH₃, -CO cyclopropyl, 2-methoxyethyl, 2-cyanoethyl, phenyl or benzyl.

In some embodiments, the compound is a compound represented by formula (VII'), and a pharmaceutically acceptable salt, solvent compound, stereoisomer, isotope and prodrug thereof: 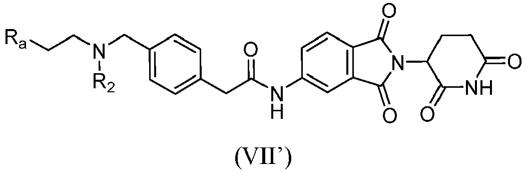
wherein the "NH" in the -CH₂C(=O)NH- is optionally substituted with R_{b}-, R_{b}C(=O)- or R_{b}S(=O)₂-;
Rₐ, R_{b} and R₂ are defined as above.

Preferably, the "NH" in the -CH₂C(=O)NH- is unsubstituted.

Preferably, the R_{b} according to the present invention is selected from C₁-C₄ alkyl, wherein the C₁-C₄ alkyl is optionally substituted with one or more R₉; the R₉ is selected from hydrogen, halogen, hydroxyl, amino, cyano or carboxyl.

The term "pharmaceutically acceptable salt" is used to describe a salt form of one or more compounds described herein, which is provided to increase the solubility of the compounds in the gastric juices of the gastrointestinal tract of a patient, thereby facilitating dissolution and bioavailability of the compounds. Pharmaceutically acceptable salts include, where applicable, salts derived from pharmaceutically acceptable inorganic or organic bases and acids. Suitable salts include those derived from alkali metals (such as potassium and sodium) and alkaline earth metals (such as calcium, magnesium and ammonium salts), as well as salts of many other acids and bases well known in the pharmaceutical field.

Pharmaceutically acceptable salts according to the present invention include hydrochloride, phosphate, hydrogen phosphate, dihydrogen phosphate, sulfate, nitrate, bicarbonate, carbonate, glutarate, hydrobromide, acetate, citrate, lactate, maleate, benzoate, mesylate, oxalate, benzenesulfonate, p-toluenesulfonate, tartrate, malate, succinate, ascorbate, gluconate, lactate, etc.

The term "solvent compound" is selected from hemihydrate, monohydrate, dihydrate, etc. The term "stereoisomer" is selected from enantiomers, diastereomers, etc. The term "prodrug" is a derivative of a compound and contains an additional moiety that is readily removed in the body to yield a parent molecule as a pharmacologically active substance. An example of prodrugs is an ester, which is cleaved in the body to produce a compound of interest. Another example is an N-methyl derivative of a compound, which is susceptible to oxidative metabolic mechanisms leading to N-demethylation. Prodrugs of various compounds as well as materials and methods for producing prodrugs by derivatizing parent compounds are known and are suitable for use in the present invention.

In particular, the present invention provides the following compounds:

| | | | |
|---|---|---|---|
| **A1** | 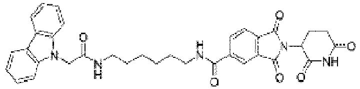 | **A118** | 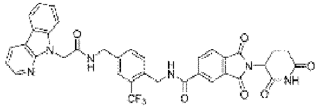 |
| **A2** | 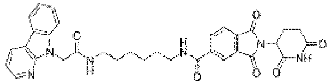 | **A119** | 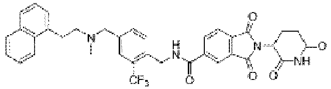 |
| **A3** | 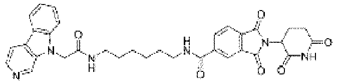 | **A120** | 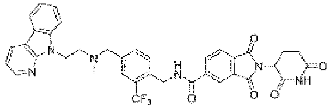 |
| **A4** | 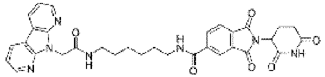 | **A121** | 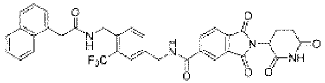 |
| **A5** | 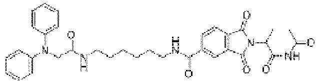 | **A122** | 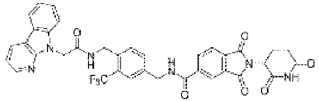 |
| **A6** | 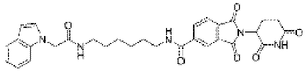 | **A123** | 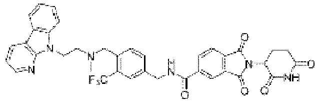 |
| **A7** | 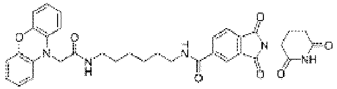 | **A124** | 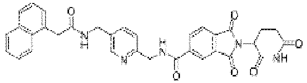 |
| **A8** | 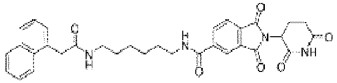 | **A125** | 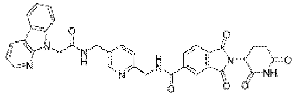 |
| **A9** | 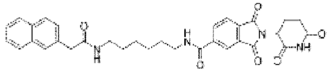 | **A126** | 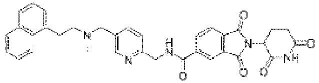 |
| **A10** | 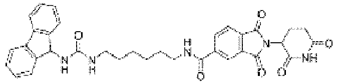 | **A127** | 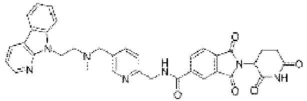 |
| **A11** | 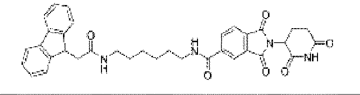 | **A128** | 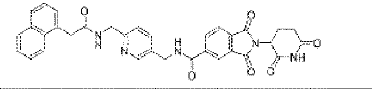 |
| **A12** | 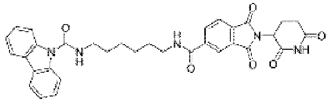 | **A129** | 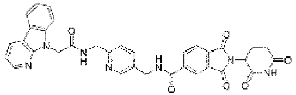 |
| **A13** | 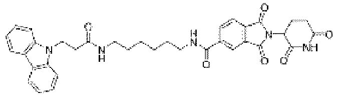 | **A130** | 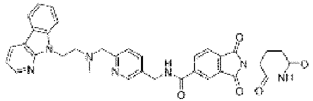 |
| **A14** | 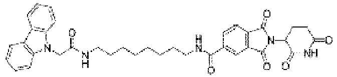 | **A131** | 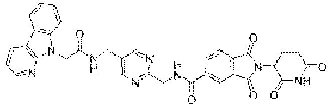 |
| **A15** | 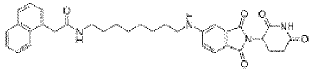 | **A132** | 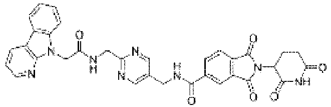 |
| **A16** | 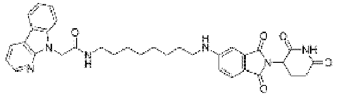 | **A133** | 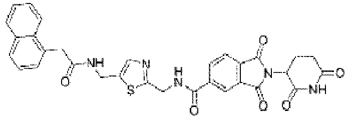 |
| **A17** | 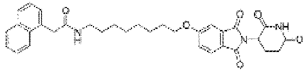 | **A134** | 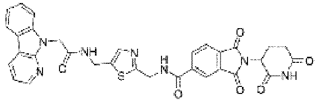 |
| **A18** | 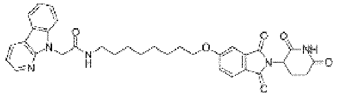 | **A135** | 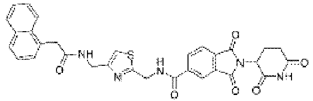 |
| **A19** | 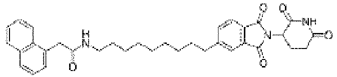 | **A136** | 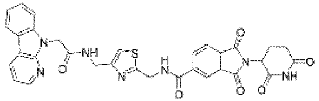 |
| **A20** | 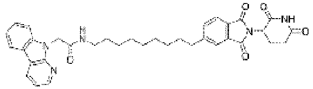 | **A1 37** | 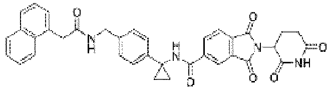 |
| **A21** | 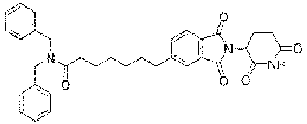 | **A138** | 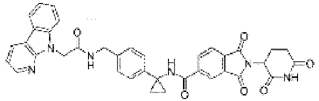 |
| **A22** | 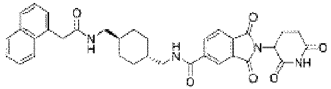 | **A139** | 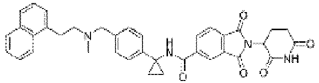 |
| **A23** | 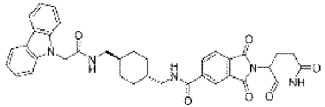 | **A140** | 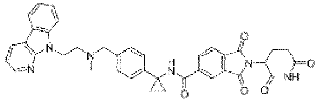 |
| **A24** | 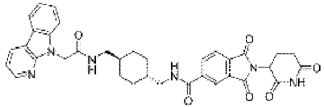 | **A141** | 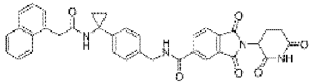 |
| **A25** | 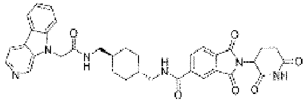 | **A142** | 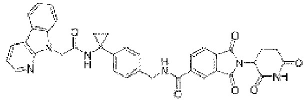 |
| **A26** | 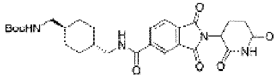 | **A143** | 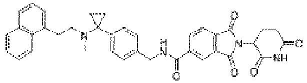 |
| **A27** | 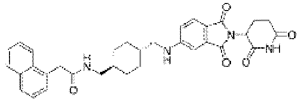 | **A144** | 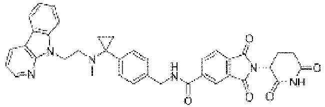 |
| **A28** | 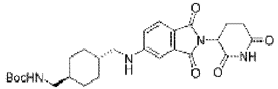 | **A145** | 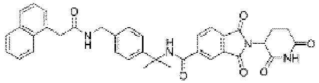 |
| **A29** | 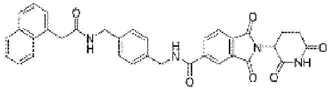 | **A146** | 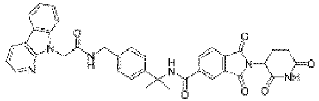 |
| **A30** | 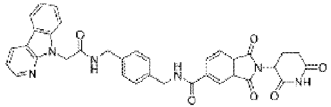 | **A147** | 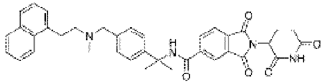 |
| **A31** | 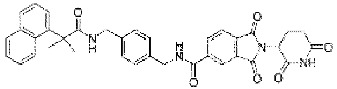 | **A148** | 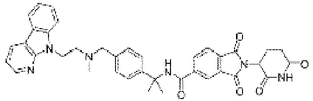 |
| **A32** | 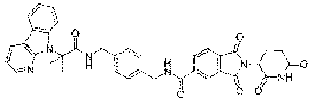 | **A149** | 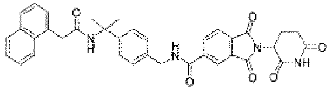 |
| **A33** | 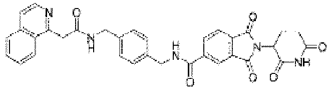 | **A150** | 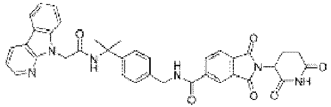 |
| **A34** | 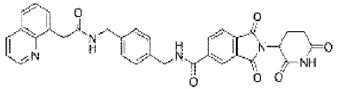 | **A151** | 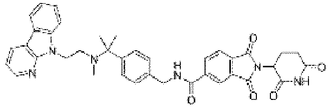 |
| **A35** | 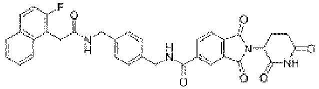 | **A152** | 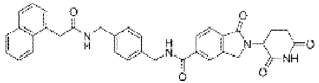 |
| **A36** | 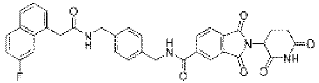 | **A153** | 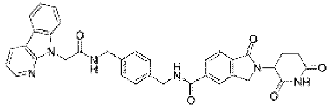 |
| **A37** | 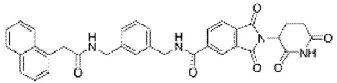 | **A154** | 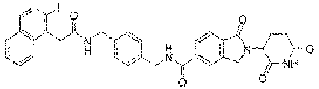 |
| **A38** | 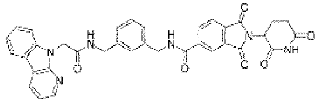 | **A155** | 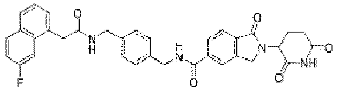 |
| **A39** | 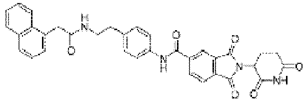 | **A156** | 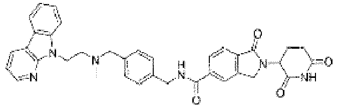 |
| **A40** | 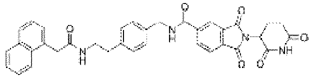 | **A157** | 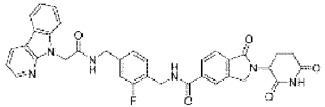 |
| **A41** | 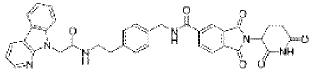 | **A158** | 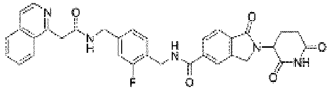 |
| **A42** | 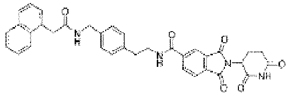 | **A159** | 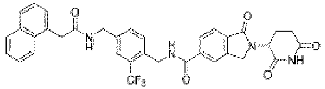 |
| **A43** | 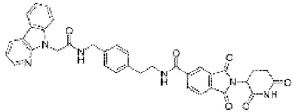 | **A160** | 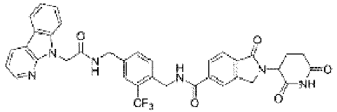 |
| **A44** | 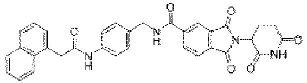 | **A161** | 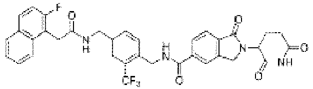 |
| **A45** | 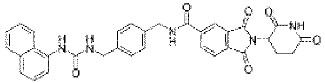 | **A162** | 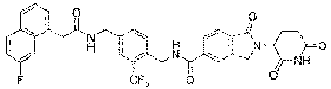 |
| **A46** | 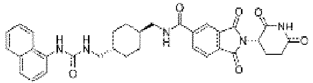 | **A163** | 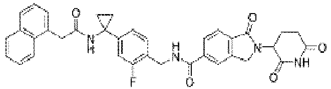 |
| **A47** | 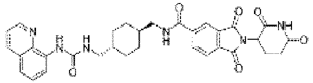 | **A164** | 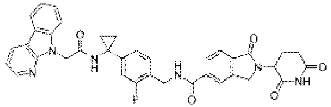 |
| **A48** | 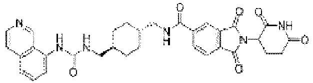 | **A165** | 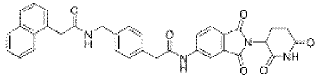 |
| **A49** | 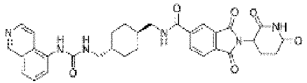 | **A166** | 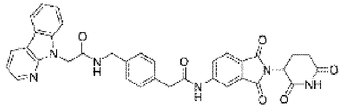 |
| **A50** | 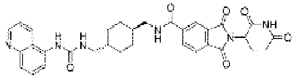 | **A167** | 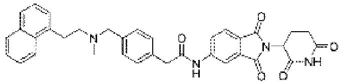 |
| **A51** | 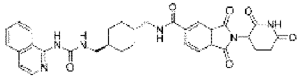 | **A168** | 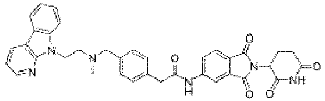 |
| **A52** | 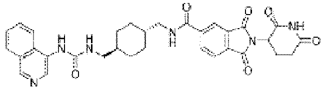 | **A169** | 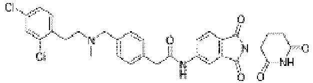 |
| **A53** | 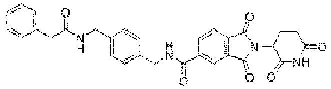 | **A170** | 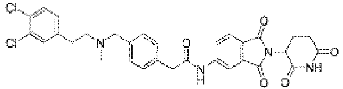 |
| **A54** | 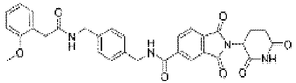 | **A171** | 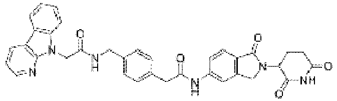 |
| **A55** | 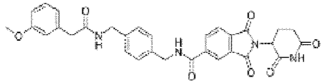 | **A172** | 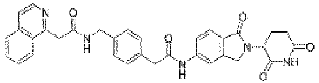 |
| **A56** | 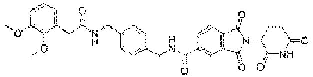 | **A173** | 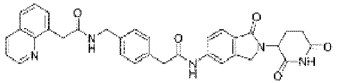 |
| **A57** | 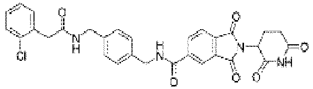 | **A174** | 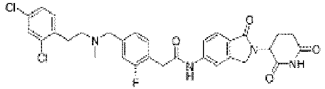 |
| **A58** | 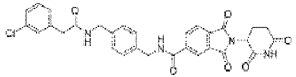 | **A175** | 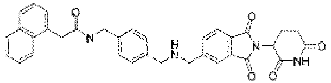 |
| **A59** | 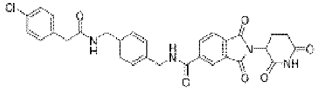 | **A176** | 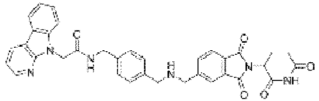 |
| **A60** | 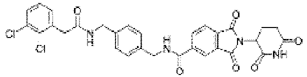 | **A177** | 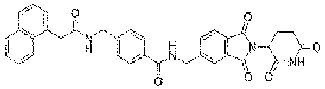 |
| **A61** | 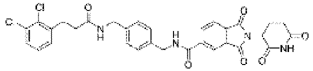 | **A178** | 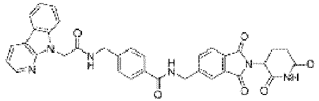 |
| **A62** | 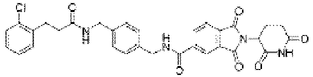 | **A179** | 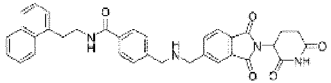 |
| **A63** | 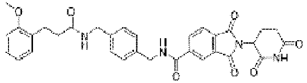 | **A180** | 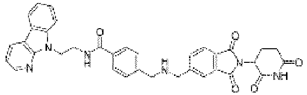 |
| **A64** | 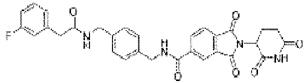 | **A181** | 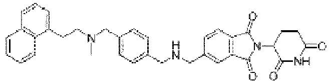 |
| **A65** | 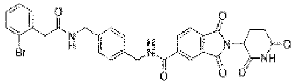 | **A182** | 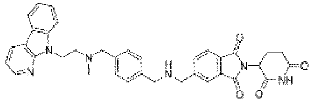 |
| **A66** | 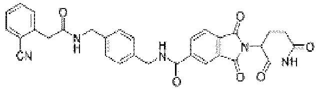 | **A183** | 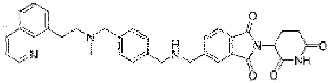 |
| **A67** | 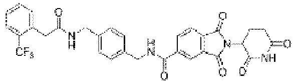 | **A184** | 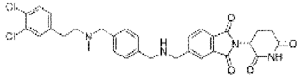 |
| **A68** | 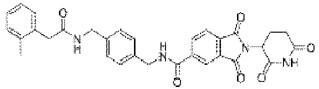 | **A185** | 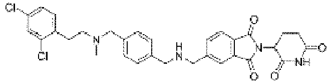 |
| **A69** | 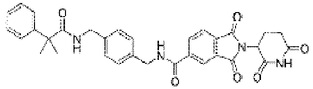 | **A186** | 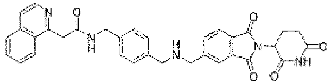 |
| **A70** | 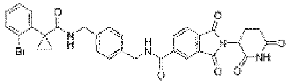 | **A187** | 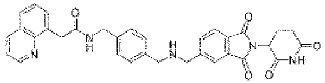 |
| **A71** | 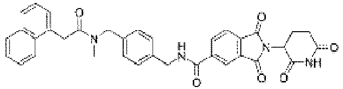 | **A188** | 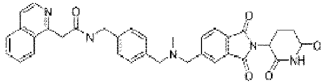 |
| **A72** | 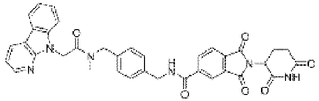 | **A189** | 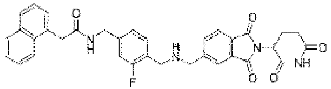 |
| **A73** | 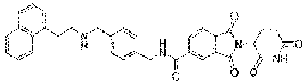 | **A190** | 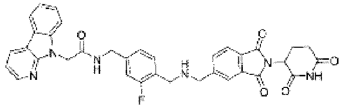 |
| **A74** | 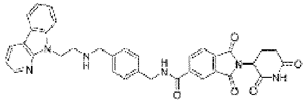 | **A191** | 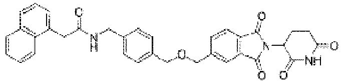 |
| **A75** | 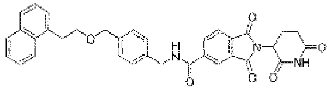 | **A192** | 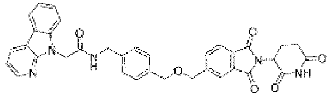 |
| **A76** | 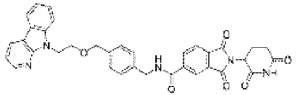 | **A193** | 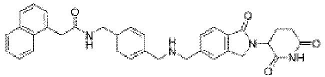 |
| **A77** | 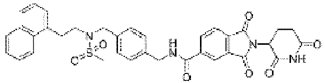 | **A194** | 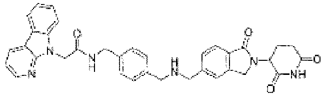 |
| **A78** | 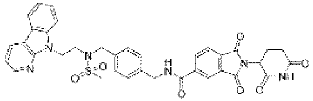 | **A195** | 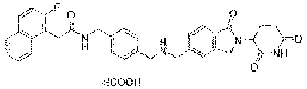 |
| **A79** | 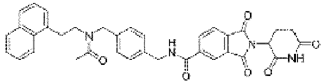 | **A196** | 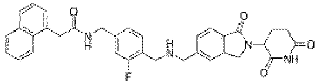 |
| **A80** | 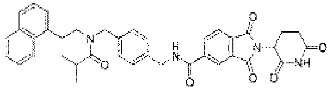 | **A197** | 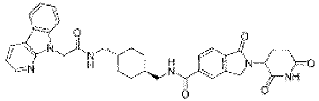 |
| **A81** | 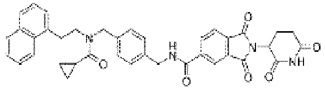 | **A198** | 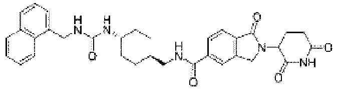 |
| **A82** | 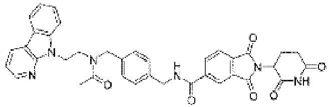 | **A199** | 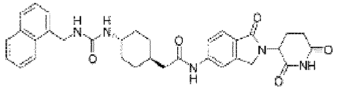 |
| **A83** | 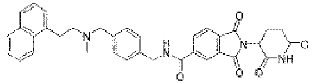 | **A200** | 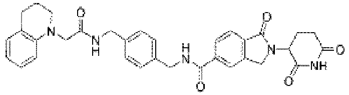 |
| **A84** | 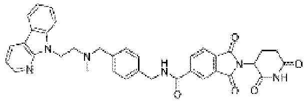 | **A201** | 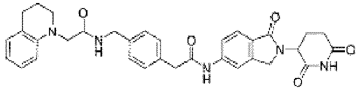 |
| **A85** | 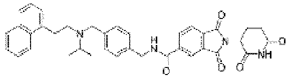 | **A202** | 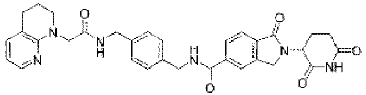 |
| **A86** | 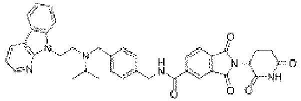 | **A203** | 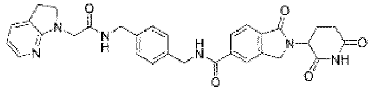 |
| **A87** | 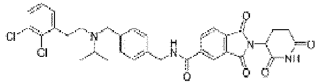 | **A204** | 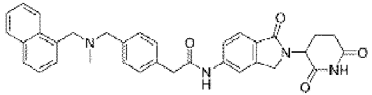 |
| **A88** | 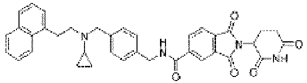 | **A205** | 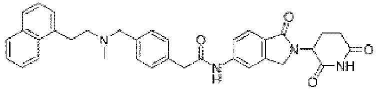 |
| **A89** | 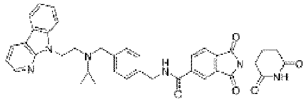 | **A206** | 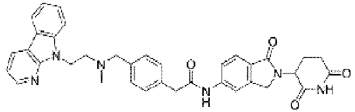 |
| **A90** | 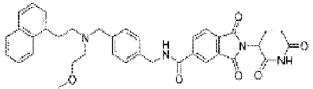 | A**207** | 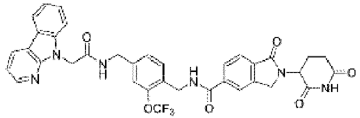 |
| **A91** | 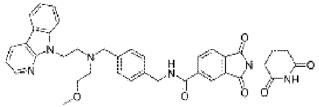 | **A208** | 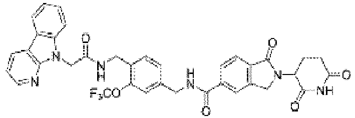 |
| **A92** | 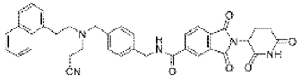 | **A209** | 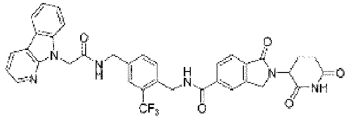 |
| **A93** | 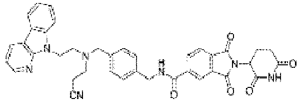 | **A210** | 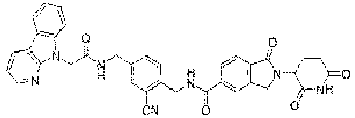 |
| **A94** | 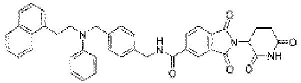 | **A211** | 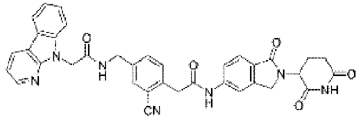 |
| **A95** | 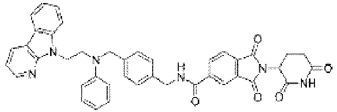 | **A212** | 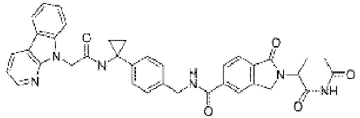 |
| **A96** | 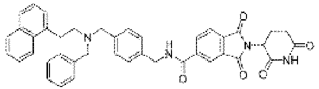 | **A213** | 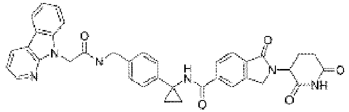 |
| **A97** | 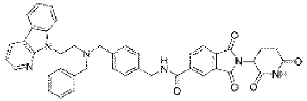 | **A214** | 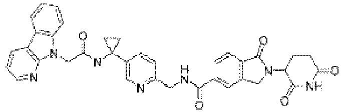 |
| **A98** | 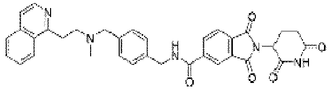 | **A215** | 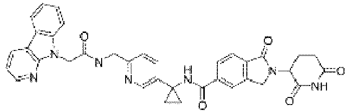 |
| **A99** | 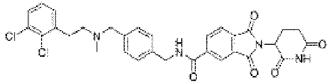 | **A216** | 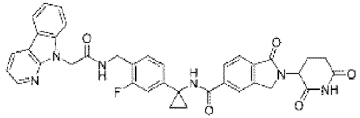 |
| **A100** | 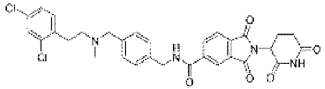 | **A217** | 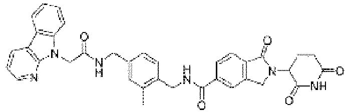 |
| **A101** | 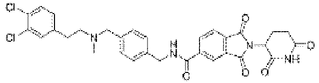 | **A218** | 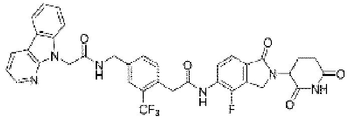 |
| **A102** | 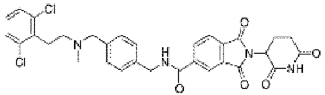 | **A219** | 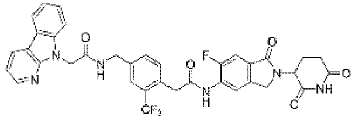 |
| **A103** | 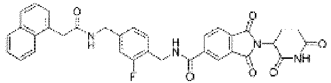 | **A220** | 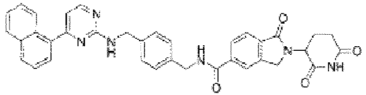 |
| **A104** | 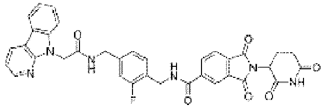 | **A221** | 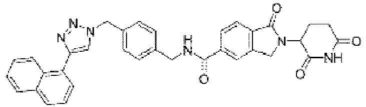 |
| **A105** | 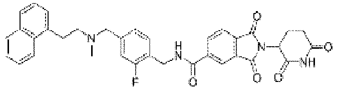 | **A222** | 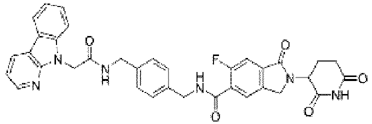 |
| **A106** | 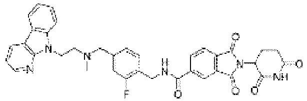 | **A223** | 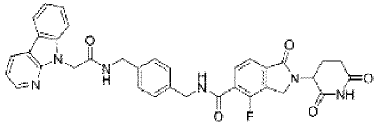 |
| **A107** | 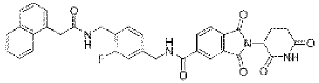 | **A224** | 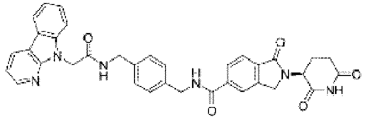 |
| **A108** | 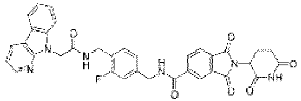 | **A225** | 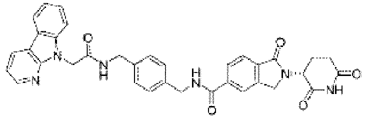 |
| **A109** | 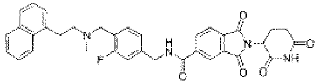 | **A226** | 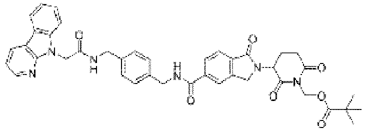 |
| **A110** | 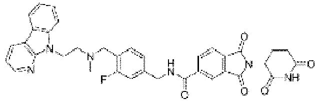 | **A227** | 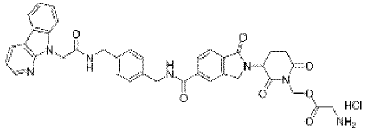 |
| **A111** | 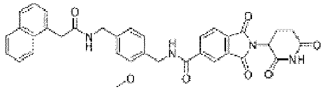 | **A228** | 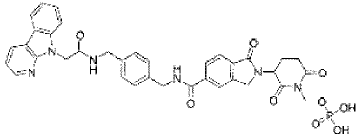 |
| **A112** | 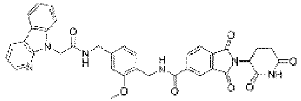 | **A229** | 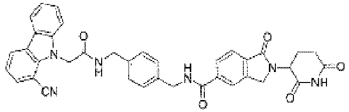 |
| **A113** | 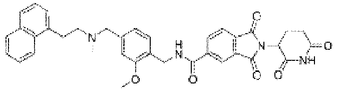 | **A230** | 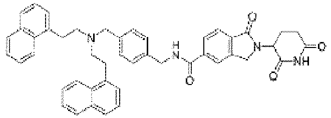 |
| **A114** | 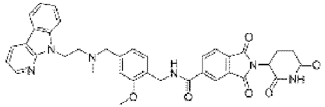 | **A231** | 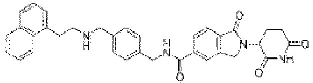 |
| **A115** | 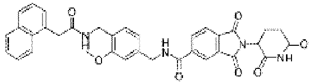 | **A232** | 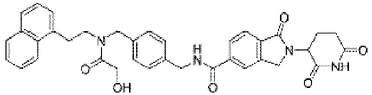 |
| **A116** | 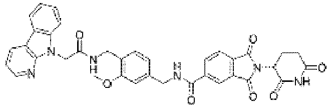 | **A233** | 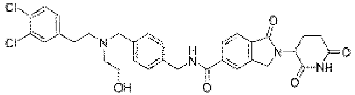 |
| **A117** | 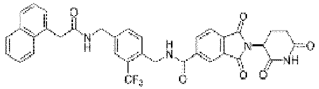 | **A234** | 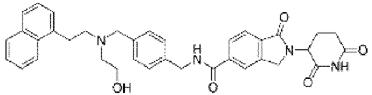 |
| **A235** | 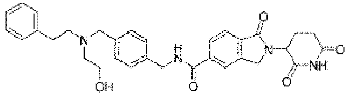 | **A236** | 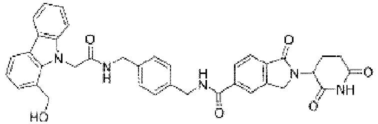 |

Another object of the present invention is to provide a pharmaceutical composition, comprising a pharmaceutically acceptable carrier and the above-mentioned compound and the pharmaceutically acceptable salt, solvent compound, stereoisomer, isotope and prodrug thereof; the pharmaceutical composition may further comprise an MYC inhibitor, a DNA methyltransferase inhibitor or a Bcl-2 selective inhibitor; the MYC inhibitor is any one or more of OMO-103, APTO-253, PLX-51107, DCR-M1711, Oncomyc-NG, INX-3280, PU-27, GSK-3179106, cholesterol butyrate and NSC-165563; the DNA methyltransferase inhibitor is any one or more of 5-azacytidine, RG108, SGI-1027, GSK3685032, CM272, Bobcat339 hydrochloride, Decitabine (NSC 127716), Thioguanine (NSC 752), 2'-Deoxy-5-Fluorocytidine, Procainamide HCl or Zebularine (NSC 309132); the Bcl-2 selective inhibitor is any one or more of Venetoclax (ABT-199), S55746, BDA-366, Obatoclax Mesylate (GX15-070), HA14-1 or APG-2575 (CAS No. 2180923-05-9). Further preferably, the pharmaceutical composition comprises a Bcl-2 selective inhibitor, and the Bcl-2 selective inhibitor is Venetoclax (ABT-199), Obatoclax Mesylate (GX15-070) or APG-2575 (CAS No. 2180923-05-9).

Yet another object of the present invention is to provide the use of the compound and the pharmaceutically acceptable salt, solvent compound, stereoisomer, isotope and prodrug thereof and the pharmaceutical composition according to the present invention in the preparation of a protein degradation agent for any one of or at least two of c-Myc, N-myc, GSPT1, CK1α, IKZF (1/2/3), AR and AR-V7.

Yet another object of the present invention is to provide the use of the compound and the pharmaceutically acceptable salt, solvent compound, stereoisomer, isotope and prodrug thereof and the pharmaceutical composition according to the present invention in the preparation of a drug for treating a disease related to dysregulation of proteins comprising any one of or at least two of c-Myc, N-myc, GSPT1, CK1α, IKZF (1/2/3), AR and AR-V7.

Yet another object of the present invention is to provide the use of the compound and the pharmaceutically acceptable salt, solvent compound, stereoisomer, isotope and prodrug thereof and the pharmaceutical composition according to the present invention in the treatment of a disease related to dysregulation of proteins comprising any one of or at least two of c-Myc, N-myc, GSPT1, CK1α, IKZF (1/2/3), AR and AR-V7.

Preferably, the dysregulation of proteins is selected from an overexpression of proteins.

Preferably, the disease related to dysregulation of proteins is selected from cancer, a cardiovascular and cerebrovascular disease, a viral infection-related disease, etc.

Preferably, the cancer is selected from leukemia, lymphoma, malignant glioma, medulloblastoma, melanoma, multiple myeloma, myelodysplastic syndrome, liver cancer, lung cancer, kidney cancer, pancreatic cancer, oral cancer, gastric cancer, esophageal cancer, laryngeal cancer, nasopharyngeal cancer, skin cancer, breast cancer, colon cancer, rectal cancer, cervical cancer, ovarian cancer, prostate cancer, rhabdomyosarcoma, osteoblastic sarcoma or chondrosarcoma; the viral infection-related disease is selected from HIV, hepatitis B, hepatitis C, hepatitis A, influenza, epidemic encephalitis B, herpes, etc.; the leukemia includes chronic lymphocytic leukemia (CLL), chronic granulocytic leukemia (CML), acute myeloid leukemia (AML), acute non-lymphocytic leukemia (ANLL), etc.

Preferably, the present invention provides the use of the compound and the pharmaceutically acceptable salt, solvent compound, stereoisomer, isotope and prodrug thereof and the pharmaceutical composition according to the present invention in the preparation of a drug for treating acute myeloid leukemia (AML). Further preferably, the pharmaceutical composition comprises a Bcl-2 selective inhibitor, and the Bcl-2 selective inhibitor is Venetoclax (ABT-199), Obatoclax Mesylate (GX15-070) or APG-2575 (CAS No. 2180923-05-9). The compound and the pharmaceutically acceptable salt, solvent compound, stereoisomer, isotope and prodrug thereof according to the present invention are used in combination or in connection with a Bcl-2 selective inhibitor. For example, using compounds A1-A236 of the present invention in combination or in connection with the Bcl-2 selective inhibitor Venetoclax (ABT-199) can solve the drug resistance problem of using the Bcl-2 selective inhibitor Venetoclax (ABT-199) alone to treat acute myeloid leukemia (AML).

Yet another object of the present invention is to provide the use of the compound and the pharmaceutically acceptable salt, solvent compound, stereoisomer, isotope and prodrug thereof according to the present invention in the preparation of a proteolysis targeting chimera (PROTAC) or an antibody-drug conjugate (ADC). Beneficial effects of the present invention

The compounds provided by the present invention have excellent effects on degrading any one or more of proteins comprising c-Myc, N-myc, GSPT1, CK1α, IKZF (1/2/3), AR, AR-V7, etc., and therefore can be used to prevent, alleviate or treat diseases related to high expression of any one or more of the above proteins (e.g., c-Myc), such as cancer, cardiovascular and cerebrovascular diseases and viral infection-related diseases. The methods for synthesizing the compounds are simple and convenient, and the compounds have precise and significant effects on degrading c-Myc, N-myc, GSPT1, CK1α, IKZF (1/2/3), AR and AR-V7 proteins.

### Definitions

Unless defined otherwise, the terms used herein have the same meanings as commonly understood by those of ordinary skill in the art to which the present invention pertains. The terms used in the specification are merely used to describe specific embodiments, and are not intended to limit the present invention.

In the present invention, 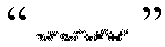 indicates a connection point for connecting moieties.

The term "unsubstituted" shall mean substitution only with a hydrogen atom.

The term "substituted" or "optionally substituted" means the independent presence of one or more substituents, preferably 1-5 substituents, and most preferably 1-3 substituents at any carbon (or nitrogen) position in a molecule, wherein the substituents may be selected from hydroxyl, mercapto, carboxyl, cyano, nitro, halogen (preferably, 1, 2 or 3 halogen, especially on alkyl, especially methyl, such as trifluoromethyl), alkyl (preferably C₁-C₁₀ alkyl, and more preferably C₁-C₆ alkyl), haloalkyl, alkenyl, alkynyl, cycloalkyl, aryl (especially phenyl), heteroaryl, heterocyclyl, alkoxy (preferably C₁-C₆ alkoxy), aryloxy (preferably phenoxy), thioether (C₁-C₆ alkylthio or arylthio such as phenylthio), acyl (preferably C₁-C₆ acyl, or arylcarbonyl such as benzoyl), C₁-C₆ alkylamino, bis(C₁-C₆ alkyl)amido, aminoacyl substituted with one or two C₁-C₆ alkyl (including carbamoyl optionally substituted with one or two C₁-C₆ alkyl), a C₁-C₆ ester group, etc.

The term "alkyl" refers to a linear or branched fully saturated hydrocarbon group or alkyl group which may be optionally substituted, preferably C₁-C₁₀ alkyl, more preferably C₁-C₆ alkyl, or C₁-C₄ alkyl. Examples of alkyl are methyl, ethyl, propyl, isopropyl, n-butyl, sec-butyl, isobutyl, tert-butyl, n-pentyl, n-hexyl, n-heptyl, n-octyl, n-nonyl, n-decyl, etc.

The term "alkylene" refers to a divalent group formed by further removing one hydrogen atom from alkyl.

The term "cycloalkyl" refers to a cyclic hydrocarbon group or alkyl group which may be optionally substituted, preferably C₃-C₂₀ alkyl, more preferably C₃-C₁₅ alkyl, or C₃-C₈ alkyl. Examples of cycloalkyl are cyclopropyl, cyclobutyl, cyclopentyl, cyclohexyl, cyclohexyl, cycloheptyl, etc.

The term "cycloalkylene" refers to a divalent group formed by further removing one hydrogen atom from cycloalkyl.

The term "bridged cyclic group" refers to a cyclic structure formed by two or more cyclic structures which may be optionally substituted and share two non-adjacent carbon atoms with each other, preferably a C₅-C₁₅ bridged cyclic group, more preferably a C₆-C₁₂ bridged cyclic group, or a C₇-C₁₀ bridged cyclic group. Examples of bridged cyclic groups are norbornyl, adamantyl, etc.

The term "alkenyl" refers to a linear chain, branched chain or cyclic C₂-C₁₀ (preferably C₂-C₈) hydrocarbon group containing at least one C=C bond.

The term "alkynyl" refers to a linear chain, branched chain or cyclic C₂-C₁₀ (preferably C₂-C₈) hydrocarbon group containing at least one C=C bond.

The term "aryl" refers to a C6-C16 aromatic group, preferably a C6-C10 aromatic group, which may be optionally substituted and has a single ring (such as phenyl) or a fused ring (such as naphthyl, anthryl, phenanthryl and fluorenyl).

The term "arylene" refers to a divalent group formed by further removing one hydrogen atom from aryl.

The term "heteroaryl" refers to a 5- to 16-membered aromatic group, preferably a 5- to 10-membered aromatic group, which may be optionally substituted and contains one or more heteroatoms selected from N, O, S or P. Examples of heteroaryl include imidazolyl, pyrazolyl, triazolyl, tetrazolyl, pyrrolyl, furyl, thienyl, oxazolyl, isoxazolyl, thiazolyl, isothiazolyl, pyridyl, pyrazinyl, pyrimidyl, pyridazinyl, indolyl, benzoimidazolyl, benzopyrazolyl, benzofuryl, benzothienyl, benzothiazolyl, dibenzofuryl, dibenzothienyl, quinolyl, isoquinolyl, naphthyridinyl, carbazolyl, azacarbazolyl (such as 1-azacarbazolyl, 2-azacarbazolyl and 1,8-diazacarbazolyl), indolazinyl, azaindolizinyl, phenoxazinyl, phenothiazinyl, etc.

The term "heteroarylene" refers to a divalent group formed by further removing one hydrogen atom from heteroaryl.

The term "heterocyclyl" refers to partially unsaturated or fully unsaturated 3-to 20-membered cyclic group, preferably 3- to 10-membered cyclic group, more preferably 3- to 6-membered cyclic group, which may be optionally substituted and contain one or more heteroatoms selected from N, O, S, SO, SO₂ or P; and the heterocyclyl contains 1-19 carbon atoms, preferably 2-10 carbon atoms, more preferably 3-5 carbon atoms. Examples of heterocyclyl include: azacyclopropanyl, oxacyclopropanyl, azetidinyl, oxetanyl, 1,4-benzodioxanyl, 1,3-benzodioxolyl, dihydroimidazolyl, dihydropyranyl, dihydrofuryl, dioxanyl, ethyleneureido, 1,3-dioxacyclopropanyl, 1,3-dioxanyl, 1,4-dioxanyl, imidazolinyl, indolinyl, morpholinyl, pyridone, 2-pyrrolidone, piperazinyl, homopiperazinyl, piperidyl, homopiperidinyl, phthalimidyl, succimidyl, pyrazinyl, pyrazolinyl, pyrrolinyl, tetrahydrofuryl, tetrahydropyranyl, tetrahydroquinolyl, tetrahydrothienyl, oxanyl, oxathiolanyl, thianyl, etc.

The term "heterocyclylene" refers to a divalent group formed by further removing one hydrogen atom from heterocyclyl.

The term "heterocycloalkyl" refers to a fully saturated heterocyclyl group.

The term "fused arylcycloalkyl" refers to a group in which aryl is fused to cycloalkyl as described above.

The term "fused heteroarylheterocyclyl" refers to a group in which heteroaryl is fused to heterocyclyl as described above.

The term "halogen" refers to F, Cl, Br or I.

The term "pharmaceutically acceptable carrier" may mean any and all solvents, dispersion media, coatings, antibacterial agents, etc. that are compatible with drug administration.

Unless otherwise indicated, the term "compound" refers to any specific compound disclosed herein and includes tautomers, regioisomers, geometric isomers, and, where applicable, stereoisomers, including optical isomers (enantiomers) and other stereoisomers (diastereomers), as well as pharmaceutically acceptable salts and derivatives (including prodrug forms) thereof. When used in the context, the term "compound" generally refers not only to a single compound, but may include other compounds, such as stereoisomers, regioisomers and/or optical isomers (including racemic mixtures), as well as specific enantiomers or enantiomer-enriched mixtures of the disclosed compounds. The term in the context also refers to a prodrug form of the compound, which has been modified to facilitate administration and delivery of the compound to an active site.

The term "proteolysis targeting chimera (PROTAC)"is a hybrid bifunctional small molecule compound (Sakamoto KM. et al. Proc Natl Acad Sci U S A, 2001, 98:8554-8559), including a small molecule compound that can bind to a protein of interest (POI) connected via a linking group at an appropriate position thereof to a small molecule compound that can bind to an E3 ubiquitin ligase.

The term "antibody-drug conjugate (ADC)" refers to a product in which a biologically active small molecule drug is connected to a monoclonal antibody via a chemical linker, wherein the monoclonal antibody serves as a carrier to deliver the small molecule drug to the cells of interest.

### Brief Description of the Drawings

FIG. 1 shows degradation results of various proteins in HL60 cells by the compounds of the present invention.
FIG. 2 shows degradation results of c-Myc and n-Myc proteins in H69 cells by the compounds of the present invention.
FIG. 3 shows degradation results of c-Myc protein in Ramos and MM1S cells by the compounds of the present invention.

### Detailed Description of Embodiments

The present invention will be exemplified below in connection with further detailed description. It should be noted that the following description is to exemplify rather than limit the technical solutions of the present invention. The scope of protection of the present invention shall be in accordance with the appended claims.

### Abbreviations of reagents in examples:

PE: petroleum ether; EA: ethyl acetate; THF: tetrahydrofuran; MTBE: methyl tert-butyl ether; TFA: trifluoroacetic acid; DCM: dichloromethane; HATU: 2-(7-azabenzotriazole)-N,N,N',N'-tetramethyluronium hexafluorophosphate; TEA: triethylamine; DMF: N,N-dimethylformamide; LDA: lithium diisopropylamide; DEAD: diethyl azodicarboxylate; DCE: dichloroethane; Selectfluor: selective fluorine reagent; DDQ: 2,3-dichloro-5,6-dicyano-p-benzoquinone; DIPEA: N,N-diisopropylethylamine; DPPA: diphenyl azidophosphate; DBU: 1,8-diazabicycloundec-7-ene; NBS: N-bromosuccinimide; AIBN: azodiisobutyronitrile; BPO: dibenzoyl peroxide; TCFH: N,N,N',N'-tetramethylchloroformamidinium hexafluorophosphate; ACN: acetonitrile; DIBAL: diisobutylaluminum hydride; TMSCH₂N₃: trimethylsilyldiazomethane.

Example 1:

### Synthesis of compound A1

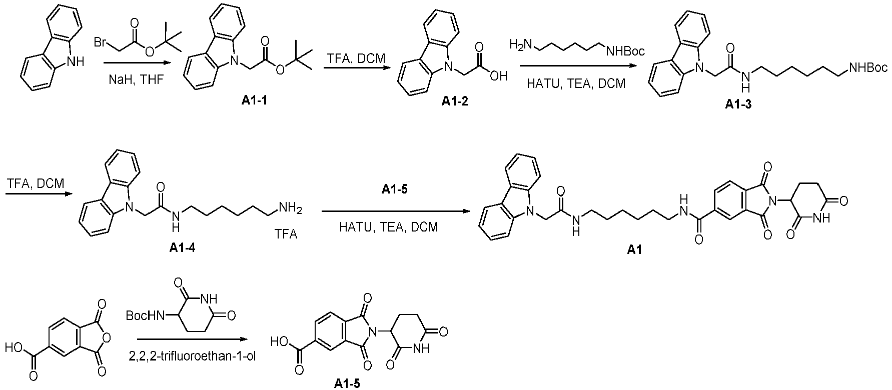

### Synthesis of compound A1-1

Carbazole (3.0 g, 18 mmol) was dissolved in anhydrous THF (40 ml), and NaH (2.8 g, 60%, 72 mmol) was added in batches under ice bath. After the addition was completed, the reaction was stirred at room temperature for 1 h. The compound tert-butyl bromoacetate (3.5 ml, 21.5 mmol) was added, and the reaction was stirred at room temperature for 2 h. Monitoring by TLC showed that the reaction was completed. The reaction was quenched with water and extracted with ethyl acetate (40 ml*3). The organic phases were combined, and the solvent was removed by drying. The solute was separated and purified by column chromatography (PE : EA = 100 : 1-5 : 1) to obtain compound A1-1 (4.7 g, yield 93%).

### Synthesis of compound A1-2

Compound A1-1 (300 mg, 1.06 mmol) was placed in a 25-mL round bottom flask, and 4 mL of (DCM : TFA = 3 : 1) solution was added. The reaction was stirred at room temperature for 2 h. TLC showed that the raw materials were completely reacted. The solvent was removed by drying to obtain compound A1-2 (238 mg, crude), which was directly used in the next reaction. ESI-MS(M-H)⁻: 224.2.

### Synthesis of compound A1-3

Compound A1-2 (238 mg, 1.06 mmol) was dissolved in dichloromethane (3 mL), and then HATU (570 mg, 1.5 mmol), tert-butyl N-(6-aminohexyl)carbamate (260 mg, 1.2 mmol) and triethylamine (303 mg, 3 mmol) were successively added. The reaction was stirred at room temperature for 2 h, and then TLC showed that the raw materials were completely reacted. Water (20 mL) was added, and the mixture was extracted with dichloromethane (20 ml*3). The organic phases were combined, and the solvent was removed by drying. The solute was separated and purified by a thick preparative plate using a developing solvent (dichloromethane : methanol = 20 : 1) to obtain compound A1-3 (134 mg, yield 32%). ESI-MS(M+H)⁺: 424.2.

### Synthesis of compound A1-4

Compound A1-3 (114 mg, 0.27 mmol) was placed in a 25-mL round bottom flask, and 2 mL of (DCM : TFA = 3 : 1) solution was added. The reaction was stirred at room temperature for 2 h, and then TLC showed that the raw materials were completely reacted. The solvent was removed by drying to obtain compound A1-4 (150 mg, crude), which was directly used in the next reaction. ESI-MS(M+H)⁺: 324.2.

### Synthesis of compound A1-5

Trimellitic anhydride (1.9 g, 10 mmol) and 3-*N*-tertbutoxycarbonylamino-2,6-dioxopiperidine (2.2 g, 10 mmol) were dissolved in trifluoroethanol (18 ml), and the mixture was reacted under microwave at 120°C for 2 h, cooled to room temperature and then further cooled to -20°C, with a blue solid precipitated. The precipitated solid was filtered, and the filter cake was washed with ethyl acetate (10 mL) to obtain compound A1-5 (2.8 g, yield 92%).

### Synthesis of compound A1

Compound A1-4 (150 mg, 0.27 mmol) and compound A1-5 (98 mg, 0.32 mmol) were dissolved in dichloromethane (3 mL), and then HATU (153 mg, 0.4 mmol) and triethylamine (82 mg, 0.81 mmol) were added. The reaction was stirred at room temperature for 2 h, and then monitoring by TLC showed that the raw materials were completely reacted. Water (20 mL) was added, and the mixture was extracted with dichloromethane (20 mL *3). The organic phases were combined, and the solvent was removed by drying. The solute was separated and purified by a thick preparative plate using a developing solvent (dichloromethane : methanol = 15 : 1), and then purified by medium-pressure preparative chromatography to obtain compound A1 (19 mg, yield 12%). ESI-MS(M+H)⁺: 608.2.

¹H NMR (400 MHz, DMSO) δ 11.15 (s, 1H), 8.83 (t, *J* = 5.4 Hz, 1H), 8.36 - 8.28 (m, 2H), 8.25 (t, *J* = 5.5 Hz, 1H), 8.13 (d, *J* = 7.7 Hz, 2H), 8.01 (d, *J* = 7.8 Hz, 1H), 7.51 (d, *J* = 8.2 Hz, 2H), 7.42 (t, *J* = 7.6 Hz, 2H), 7.19 (t, *J* = 7.4 Hz, 2H), 5.20 (dd, *J* = 12.9, 5.3 Hz, 1H), 5.00 (s, 2H), 3.31 - 3.25 (m, 2H), 3.11 - 3.05 (m, 2H), 2.91 - 2.89 (m, 1H), 2.68 - 2.53 (m, 2H), 2.12 - 2.08 (m, 1H), 1.55 - 1.51 (m, 2H), 1.48 - 1.39 (m, 2H), 1.35 - 1.26 (m, 4H).

The synthesis methods for compounds A2-A9, A11, A13-A14, A22-A25, A29-A44, A71-A72, A197, A208, A210, A214, A220 and A221 are the same as that for A1.

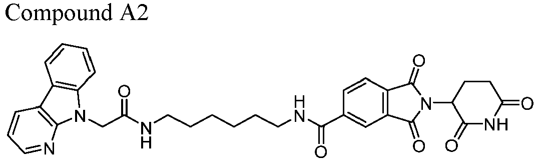

ESI-MS(M+H)⁺: 609.2
¹H NMR (400 MHz, DMSO) δ 11.17 (s, 1H), 8.84 (t, *J* = 5.5 Hz, 1H), 8.53 (dd, *J* = 7.6, 1.4 Hz, 1H), 8.43 (dd, *J* = 4.8, 1.4 Hz, 1H), 8.34 (s, 1H), 8.31 (d, *J* = 7.8 Hz, 1H), 8.25 (t, *J* = 5.5 Hz, 1H), 8.19 (d, *J* = 7.7 Hz, 1H), 8.01 (d, *J* = 7.7 Hz, 1H), 7.56 - 7.45 (m, 2H), 7.31 - 7.19 (m, 2H), 5.20 (dd, *J* = 12.8, 5.4 Hz, 1H), 5.08 (s, 2H), 3.28 (dd, *J* = 12.7, 6.6 Hz, 2H), 3.08 (q, *J* = 6.5 Hz, 2H), 2.95 - 2.82 (m, 1H), 2.69 - 2.52 (m, 2H), 2.13 - 2.03 (m, 1H), 1.59 - 1.48 (m, 2H), 1.48 - 1.38 (m, 2H), 1.36 - 1.25 (m, 4H).

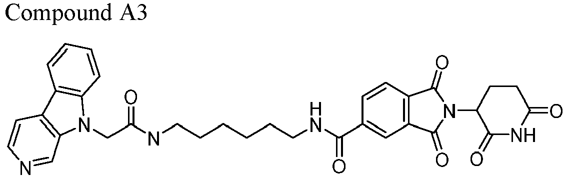

ESI-MS(M+H)+: 609.2
¹H NMR (400 MHz, DMSO) δ 11.14 (s, 1H), 9.06 (s, 1H), 8.84 (t, *J* = 5.5 Hz, 1H), 8.49 - 8.36 (m, 2H), 8.34 - 8.21 (m, 4H), 8.01 (d, *J* = 7.8 Hz, 1H), 7.64 (d, *J* = 3.9 Hz, 2H), 7.34 - 7.29 (m, 1H), 5.22 - 5.14 (m, 3H), 3.30 - 3.24 (m, 4H), 2.94 - 2.86 (m, 1H), 2.64 - 2.54 (m, 2H), 2.10 - 2.03 (m, 1H), 1.56 - 1.50 (m, 2H), 1.48 - 1.40 (m, 2H), 1.35 - 1.27 (m, 4H).

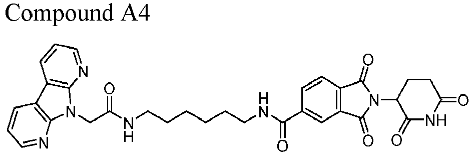

ESI-MS(M+H)⁺: 610.2.

¹H NMR (400 MHz, DMSO) δ 11.15 (s, 1H), 8.82 (t, *J* = 5.5 Hz, 1H), 8.65 - 8.55 (m, 2H), 8.49 (dd, *J* = 4.9, 1.6 Hz, 2H), 8.38 - 8.26 (m, 2H), 8.20 (t, *J* = 5.5 Hz, 1H), 8.00 (d, *J* = 7.7 Hz, 1H), 7.39 - 7.27 (m, 2H), 5.19 (dd, *J* = 12.8, 5.4 Hz, 1H), 5.08 (s, 2H), 3.30 - 3.25 (m, 2H), 3.07 (dd, *J* = 12.5, 6.5 Hz, 2H), 2.95 - 2.86 (m, 1H), 2.67 - 2.52 (m, 2H), 2.12 - 2.06 (m, 1H), 1.58 - 1.50 (m, 2H), 1.47 - 1.37 (m, 2H), 1.36 - 1.26 (m, 4H).

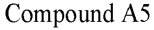

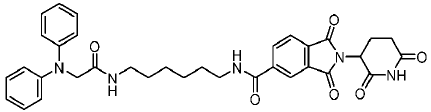

ESI-MS(M+H)⁺: 610.2.

¹H NMR (400 MHz, DMSO) δ 11.13 (s, 1H), 8.88 (t, *J* = 5.5 Hz, 1H), 8.37 - 8.31 (m, 2H), 8.03 - 7.98 (m, 2H), 7.25 (t, *J* = 7.8 Hz, 4H), 6.99 (d, *J* = 8.0 Hz, 4H), 6.92 (t, *J* = 7.3 Hz, 2H), 5.19 (dd, *J* = 12.9, 5.3 Hz, 1H), 4.25 (s, 2H), 3.27 (dd, *J* = 12.9, 6.8 Hz, 2H), 3.07 (q, *J* = 6.5 Hz, 2H), 2.96 - 2.85 (m, 1H), 2.66 - 2.52 (m, 2H), 2.13 - 2.03 (m, 1H), 1.55 - 1.45 (m, 2H), 1.42 - 1.35 (m, 2H), 1.30 - 1.21 (m, 4H).

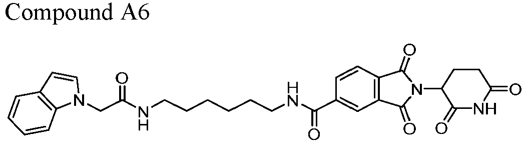

ESI-MS(M+H)⁺: 558.2.

¹H NMR (400 MHz, DMSO) δ 11.16 (s, 1H), 8.86 (t, *J* = 5.5 Hz, 1H), 8.36 - 8.26 (m, 2H), 8.14 (t, *J* = 5.6 Hz, 1H), 8.02 (d, *J* = 7.7 Hz, 1H), 7.53 (d, *J* = 7.8 Hz, 1H), 7.35 - 7.30 (m, 2H), 7.10 (t, *J* = 7.4 Hz, 1H), 7.00 (t, *J=* 7.4 Hz, 1H), 6.42 (d, *J* = 3.0 Hz, 1H), 5.19 (dd, *J* = 12.9, 5.3 Hz, 1H), 4.79 (s, 2H), 3.31 - 3.25 (m, 2H), 3.08 (q, *J* = 6.5 Hz, 2H), 2.95 - 2.85 (m, 1H), 2.69 - 2.52 (m, 2H), 2.13 - 2.05 (m, 1H), 1.59 - 1.49 (m, 2H), 1.48 - 1.36 (m, 2H), 1.35 - 1.26 (m, 4H).

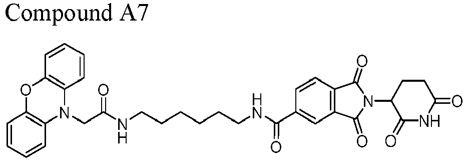

ESI-MS(M+H)⁺: 624.2.

¹H NMR (400 MHz, DMSO) δ 11.14 (s, 1H), 8.86 (t, *J* = 5.5 Hz, 1H), 8.36 - 8.28 (m, 2H), 8.20 (t, *J* = 5.8 Hz, 1H), 8.01 (d, *J* = 7.7 Hz, 1H), 6.83 - 6.74 (m, 2H), 6.67 - 6.65 (m, 4H), 6.48 (d, *J* = 7.9 Hz, 2H), 5.19 (dd, *J* = 12.8, 5.3 Hz, 1H), 4.17 (s, 2H), 3.30 - 3.25 (m, 2H), 3.10 (q, *J* = 6.5 Hz, 2H), 2.94 - 2.85 (m, 1H), 2.69 - 2.52 (m, 2H), 2.14 - 2.04 (m, 1H), 1.59 - 1.49 (m, 2H), 1.48 - 1.38 (m, 2H), 1.35 - 1.24 (m, 4H).

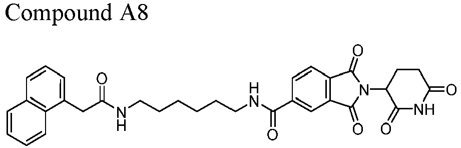

ESI-MS(M+H)⁺: 569.2.

¹H NMR (400 MHz, DMSO) δ 11.17 (s, 1H), 8.84 (t, *J* = 5.5 Hz, 1H), 8.35 (s, 1H), 8.34 - 8.30 (m, 1H), 8.12 (dd, *J* = 10.9, 5.4 Hz, 1H), 8.08 (d, *J* = 7.7 Hz, 1H), 8.02 (d, *J* = 7.8 Hz, 1H), 7.89 (dd, *J* = 8.3, 6.4 Hz, 1H), 7.82 - 7.75 (m, 1H), 7.54 - 7.47 (m, 2H), 7.45 - 7.40 (m, 2H), 5.20 (dd, *J* = 12.9, 5.4 Hz, 1H), 3.88 (s, 2H), 3.27 (dd, *J* = 12.7, 6.6 Hz, 2H), 3.09 - 3.04 (m, 2H), 2.95 - 2.86 (m, 1H), 2.67 - 2.52 (m, 2H), 2.14 - 2.07 (m, 1H), 1.59 - 1.46 (m, 2H), 1.46 - 1.37 (m, 2H), 1.34 - 1.20 (m, 4H).

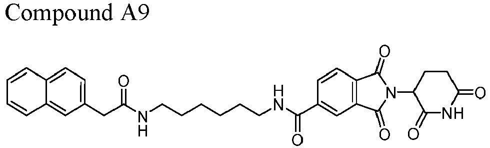

ESI-MS(M+H)⁺: 569.2.

¹H NMR (400 MHz, DMSO) δ 11.14 (s, 1H), 8.84 (t, *J* = 5.4 Hz, 1H), 8.34 - 8.30 (m, 2H), 8.09 (t, *J* = 5.4 Hz, 1H), 8.02 (d, *J* = 7.8 Hz, 1H), 7.89 - 7.79 (m, 3H), 7.73 (s, 1H), 7.50 - 7.38 (m, 3H), 5.19 (dd, *J* = 12.9, 5.4 Hz, 1H), 3.56 (s, 2H), 3.27 (dd, *J* = 13.0, 6.6 Hz, 2H), 3.06 (dd, *J* = 12.6, 6.6 Hz, 2H), 2.95 - 2.83 (m, 1H), 2.69 - 2.53 (m, 2H), 2.13 - 2.03 (m, 1H), 1.59 - 1.46 (m, 2H), 1.46 - 1.37 (m, 2H), 1.35 - 1.26 (m, 4H).

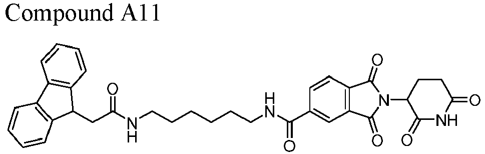

ESI-MS(M+H)⁺: 607.2.

¹H NMR (400 MHz, DMSO) δ 11.16 (s, 1H), 8.89 (t, *J* = 5.4 Hz, 1H), 8.38 - 8.29 (m, 2H), 8.03 (d, *J* = 7.8 Hz, 1H), 7.94 (t, *J* = 5.4 Hz, 1H), 7.86 (d, *J* = 7.5 Hz, 2H), 7.51 (d, *J* = 7.4 Hz, 2H), 7.36 (t, *J* = 7.3 Hz, 2H), 7.28 (t, *J* = 7.4 Hz, 2H), 5.19 (dd, *J* = 12.9, 5.4 Hz, 1H), 4.34 (t, *J* = 7.6 Hz, 1H), 3.31 - 3.29 (m, 2H), 3.17 (q, *J* = 6.6 Hz, 2H), 2.95 - 2.84 (m, 1H), 2.69 - 2.52 (m, 2H), 2.49 - 2.48 (m, 2H), 2.12 - 2.05 (m, 1H), 1.62 - 1.52 (m, 2H), 1.52 - 1.41 (m, 2H), 1.40 - 1.28 (m, 4H).

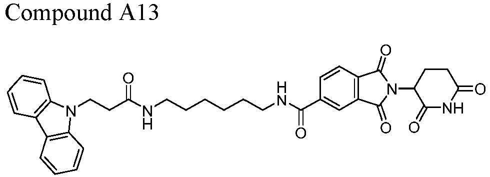

ESI-MS(M+H)⁺: 622.2.

¹H NMR (400 MHz, DMSO) δ 11.16 (s, 1H), 8.85 (t, *J* = 5.5 Hz, 1H), 8.35 - 8.31 (m, 2H), 8.12 (d, *J* = 7.7 Hz, 2H), 8.02 (d, *J* = 7.8 Hz, 1H), 7.83 (t, *J* = 5.5 Hz, 1H), 7.58 (d, *J* = 8.2 Hz, 2H), 7.43 (t, *J* = 7.6 Hz, 2H), 7.17 (t, *J* = 7.4 Hz, 2H), 5.19 (dd, *J* = 12.8, 5.4 Hz, 1H), 4.60 (t, *J* = 6.6 Hz, 2H), 3.25 (dd, *J* = 12.7, 6.6 Hz, 2H), 2.96 - 2.83 (m, 3H), 2.65 - 2.51 (m, 4H), 2.12 - 2.02 (m, 1H), 1.49 - 1.38 (m, 2H), 1.23 - 1.13 (m, 4H), 1.09 - 1.00 (m, 2H).

The corresponding intermediates were prepared as follows:

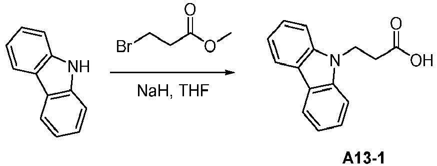

### Synthesis of compound A13-1

Carbazole (200 mg, 1.20 mmol) was dissolved in anhydrous tetrahydrofuran (6 mL), and sodium hydride (69 mg, 60%, 1.73 mmol) was slowly added under ice bath. After the addition, the mixture was reacted at room temperature for 30 min, and then the compound methyl 3-bromopropionate (240 mg, 1.44 mmol) was added. The reaction was stirred at room temperature for 2 h, and then monitoring by TLC showed that the raw materials were completely reacted. The mixture was adjusted to pH 3-4 with dilute hydrochloric acid and extracted with ethyl acetate (25 mL*3). The organic phases were combined, dried over anhydrous sodium sulfate and concentrated. The solute was separated and purified by a thick preparative plate (developing solvent: PE : EA = 2 : 1) to obtain compound A13-1 (240 mg, yield 83%). ESI-MS(M-H)⁻: 238.1.

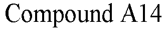

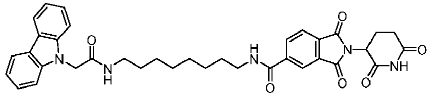

ESI-MS(M+H)⁺: 636.2.

¹H NMR (400 MHz, DMSO) δ 11.14 (s, 1H), 8.88 (t, *J* = 5.4 Hz, 1H), 8.38 - 8.26 (m, 3H), 8.13 (d, *J* = 7.7 Hz, 2H), 8.01 (d, *J* = 7.7 Hz, 1H), 7.51 (d, *J* = 8.2 Hz, 2H), 7.45 - 7.36 (m, 2H), 7.19 (t, *J* = 7.4 Hz, 2H), 5.19 (dd, *J* = 12.9, 5.4 Hz, 1H), 5.00 (s, 2H), 3.31 - 3.25 (m, 2H), 3.08 (dd, *J* = 12.6, 6.6 Hz, 2H), 2.94 - 2.85 (m, 1H), 2.67 - 2.53 (m, 2H), 2.13 - 2.04 (m, 1H), 1.60 - 1.48 (m, 2H), 1.45 - 1.37 (m, 2H), 1.24 - 1.23 (m, 8H).

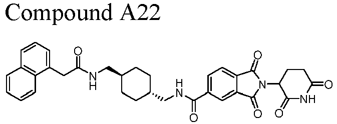

ESI-MS(M+H)⁺: 595.2.

¹H NMR (400 MHz, DMSO) δ 11.16 (s, 1H), 8.87 (t, *J* = 5.5 Hz, 1H), 8.38 - 8.27 (m, 2H), 8.17 (t, *J* = 5.7 Hz, 1H), 8.12 - 8.06 (m, 1H), 8.03 (d, *J* = 7.8 Hz, 1H), 7.95 - 7.87 (m, 1H), 7.80 (d, *J* = 7.4 Hz, 1H), 7.54 - 7.47 (m, 2H), 7.47 - 7.37 (m, 2H), 5.20 (dd, *J* = 12.9, 5.3 Hz, 1H), 3.89 (s, 2H), 3.14 (t, *J* = 6.1 Hz, 2H), 2.97 - 2.83 (m, 3H), 2.68 - 2.52 (m, 2H), 2.14 - 2.03 (m, 1H), 1.83 - 1.65 (m, 4H), 1.56 - 1.29 (m, 2H), 0.97 - 0.75 (m, 4H).

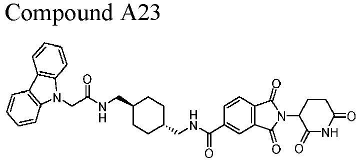

ESI-MS(M+H)⁺: 634.2.

¹H NMR (400 MHz, DMSO) δ 11.14 (s, 1H), 8.85 (t, *J* = 5.6 Hz, 1H), 8.39 - 8.30 (m, 2H), 8.26 (t, *J* = 5.7 Hz, 1H), 8.14 (d, *J* = 7.7 Hz, 2H), 8.03 (d, *J* = 7.8 Hz, 1H), 7.52 (d, *J* = 8.2 Hz, 2H), 7.46 - 7.40 (m, 2H), 7.26 - 7.15 (m, 2H), 5.20 (dd, *J* = 12.9, 5.4 Hz, 1H), 5.02 (s, 2H), 3.16 (t, *J* = 6.2 Hz, 2H), 2.96 (t, *J* = 6.1 Hz, 2H), 2.92 - 2.84 (m, 1H), 2.70 - 2.52 (m, 2H), 2.13 - 2.05 (m, 1H), 1.75 (dd, *J* = 18.4, 10.4 Hz, 4H), 1.59 - 1.46 (m, 1H), 1.42 - 1.32 (m, 1H), 0.97 - 0.79 (m, 4H).

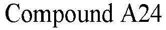

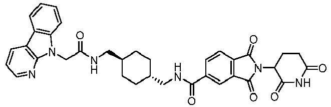

ESI-MS(M+H)⁺: 635.2.

¹H NMR (400 MHz, DMSO) δ 11.17 (s, 1H), 8.87 (t, *J* = 5.6 Hz, 1H), 8.53 (dd, *J* = 7.6, 1.4 Hz, 1H), 8.43 (dd, *J* = 4.8, 1.4 Hz, 1H), 8.38 - 8.30 (m, 2H), 8.25 (t, *J* = 5.7 Hz, 1H), 8.20 (d, *J* = 7.7 Hz, 1H), 8.03 (d, *J* = 7.8 Hz, 1H), 7.56 - 7.46 (m, 2H), 7.30 - 7.21 (m, 2H), 5.20 (dd, *J* = 12.9, 5.3 Hz, 1H), 5.10 (s, 2H), 3.16 (t, *J* = 6.1 Hz, 2H), 2.95 (t, *J* = 6.0 Hz, 2H), 2.93 - 2.84 (m, 1H), 2.68 - 2.52 (m, 2H), 2.12 - 2.04 (m, 1H), 1.76 (t, *J* = 12.7 Hz, 4H), 1.59 - 1.31 (m, 2H), 0.99 - 0.79 (m, 4H).

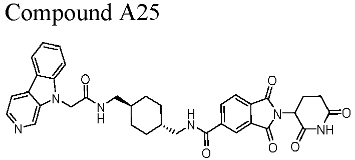

ESI-MS(M+H)⁺: 635.2.

¹H NMR (400 MHz, DMSO) δ 8.96 (s, 1H), 8.88 (t, *J* = 5.5 Hz, 1H), 8.42 (t, *J* = 5.5 Hz, 1H), 8.38 (d, *J* = 5.2 Hz, 1H), 8.36 - 8.24 (m, 3H), 8.13 (d, *J* = 5.1 Hz, 1H), 8.01 (d, *J* = 7.7 Hz, 1H), 7.64 - 7.56 (m, 2H), 7.28 (t, *J* = 6.8 Hz, 1H), 5.15 (s, 2H), 5.05 - 4.95 (m, 1H), 3.18 - 3.12 (m, 2H), 2.96 (t, *J* = 5.8 Hz, 2H), 2.79 - 2.66 (m, 1H), 2.61 - 2.52 (m, 2H), 2.05 - 1.96 (m, 1H), 1.82 - 1.68 (m, 4H), 1.42 - 1.33 (m, 2H), 0.94 - 0.83 (m, 4H).

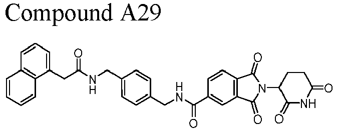

ESI-MS(M+H)⁺: 589.2.

¹H NMR (400 MHz, DMSO) δ 11.16 (s, 1H), 9.43 (t, *J* = 5.9 Hz, 1H), 8.63 (t, *J* = 5.9 Hz, 1H), 8.41 - 8.32 (m, 2H), 8.12 - 8.08 (m, 1H), 8.04 (d, *J* = 7.8 Hz, 1H), 7.94 - 7.89 (m, 1H), 7.82 (dd, *J* = 8.4, 4.5 Hz, 1H), 7.54 - 7.47 (m, 2H), 7.47 - 7.42 (m, 2H), 7.28 (d, *J* = 8.1 Hz, 2H), 7.20 (d, *J* = 8.1 Hz, 2H), 5.20 (dd, *J* = 12.9, 5.4 Hz, 1H), 4.49 (d, *J* = 5.8 Hz, 2H), 4.26 (d, *J* = 5.8 Hz, 2H), 3.95 (s, 2H), 2.95 - 2.85 (m, 1H), 2.68 - 2.52 (m, 2H), 2.12 - 2.05 (m, 1H).

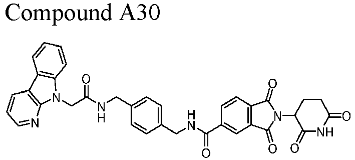

ESI-MS(M+H)+: 629.2.

¹H NMR (400 MHz, DMSO) δ 11.15 (s, 1H), 9.44 (t, *J* = 5.8 Hz, 1H), 8.74 (t, *J* = 6.0 Hz, 1H), 8.54 (dd, *J* = 7.6, 1.6 Hz, 1H), 8.45 (dd, *J* = 4.8, 1.6 Hz, 1H), 8.39 (s, 1H), 8.36 (dd, *J* = 7.8, 1.4 Hz, 1H), 8.21 (d, *J* = 7.7 Hz, 1H), 8.04 (d, *J=* 7.8 Hz, 1H), 7.56 (d, *J* = 8.2 Hz, 1H), 7.53 - 7.46 (m, 1H), 7.32 - 7.24 (m, 6H), 5.20 (dd, *J* = 12.9, 5.4 Hz, 1H), 5.16 (s, 2H), 4.50 (d, *J* = 5.8 Hz, 2H), 4.28 (d, *J* = 5.8 Hz, 2H), 2.95 - 2.84 (m, 1H), 2.66 - 2.52 (m, 2H), 2.13 - 2.04 (m, 1H).

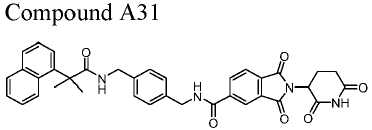

ESI-MS(M+H)+: 617.2.

¹H NMR (400 MHz, DMSO) δ 11.15 (s, 1H), 9.40 (t, *J* = 5.9 Hz, 1H), 8.41 - 8.30 (m, 2H), 8.04 (d, *J* = 7.8 Hz, 1H), 7.89 (t, *J* = 8.1 Hz, 2H), 7.82 (d, *J* = 8.1 Hz, 1H), 7.61 (dd, *J* = 12.3, 6.3 Hz, 2H), 7.51 - 7.44 (m, 1H), 7.46 - 7.40 (m, 1H), 7.38 - 7.33 (m, 1H), 7.13 (d, *J* = 8.1 Hz, 2H), 6.96 (d, *J* = 8.1 Hz, 2H), 5.19 (dd, *J* = 12.8, 5.4 Hz, 1H), 4.43 (d, *J* = 5.7 Hz, 2H), 4.12 (d, *J* = 5.9 Hz, 2H), 2.96 - 2.82 (m, 1H), 2.68 - 2.52 (m, 2H), 2.15 - 2.05 (m, 1H), 1.61 (s, 6H).

The corresponding intermediates were prepared as follows:

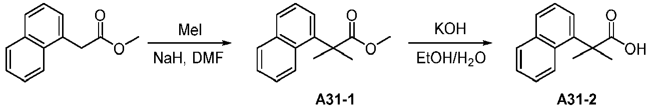

### Synthesis of compound A31-1

NaH (200 mg, 60%, 5.0 mmol) and iodomethane (700 mg, 5.0 mmol) were dissolved in DMF (10 mL). Under ice bath, a solution of the compound methyl naphthalene-1-acetate (200 mg, 1.0 mmol) in DMF (10 mL) was added dropwise. The mixture was reacted overnight at room temperature, and then monitoring by TLC showed that the reaction was completed. Water was added, and then the mixture was extracted with ethyl acetate (30 mL*3). The organic phases were combined, washed with saturated brine (10 mL*2) and then dried by rotary evaporation. The solute was separated and purified by a thick preparative plate to obtain compound A31-1 (160 mg, yield 70%). ¹H NMR (400 MHz, DMSO) δ 7.99 - 7.93 (m, 1H), 7.85 (d, *J* = 8.1 Hz, 1H), 7.70 (d, *J* = 8.0 Hz, 1H), 7.58 (d, *J* = 6.4 Hz, 1H), 7.55 - 7.46 (m, 3H), 3.51 (s, 3H), 1.68 (s, 6H).

### Synthesis of compound A31-2

Compound A31-1 (50 mg, 0.22 mmol) was dissolved in ethanol (9 mL) and water (3 mL), and KOH (50 mg, 0.88 mmol) was added. The mixture was reacted overnight at 100°C and adjusted to pH 3-4 with dilute hydrochloric acid. The aqueous phase was extracted with ethyl acetate (20 mL*3). The organic phases were combined, and the solvent was removed by drying. The solute was separated and purified by a thick preparative plate (developing solvent: DCM : MeOH = 15 : 1) to obtain compound A31-2 (30 mg, yield 64%).

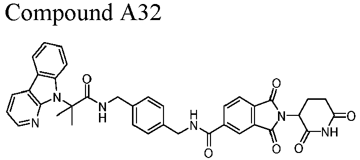

ESI-MS (M+H)⁺: 657.2.

¹H NMR (400 MHz, DMSO) δ 11.15 (s, 1H), 9.41 (t, *J* = 5.9 Hz, 1H), 8.50 (dd, *J* = 7.7, 1.7 Hz, 1H), 8.45 - 8.34 (m, 4H), 8.19 (d, *J* = 7.5 Hz, 1H), 8.04 (d, *J* = 7.8 Hz, 1H), 7.43 - 7.32 (m, 2H), 7.25 - 7.16 (m, 4H), 7.08 (d, *J* = 8.1 Hz, 2H), 5.19 (dd, *J* = 12.9, 5.4 Hz, 1H), 4.46 (d, *J* = 5.8 Hz, 2H), 4.19 (d, *J* = 5.8 Hz, 2H), 2.96 - 2.83 (m, 1H), 2.68 - 2.52 (m, 2H), 2.13 - 2.07 (m, 1H), 2.03 (s, 6H).

The corresponding intermediates were prepared as follows:

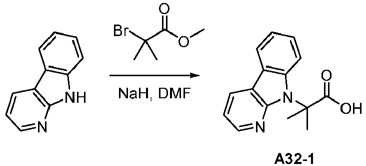

### Synthesis of compound A32-1

Carbazole (100 mg, 0.6 mmol) was dissolved in DMF (4 mL), and sodium hydride (96 mg, 60%, 2.4 mmol) was added in batches under ice bath. After the bubbles completely disappeared, the mixture was reacted at room temperature for 1 h. Methyl 2-bromoisobutyrate (140 mg, 0.77 mmol) was further added. The mixture was reacted at room temperature for 1 h, and then refluxed at 100°C for 2 h. Monitoring by TLC showed that the reaction was completed. The mixture was cooled to room temperature, adjusted to neutral pH with dilute hydrochloric acid and extracted with ethyl acetate (30 mL*3). The organic phases were combined, washed with saturated brine (10 mL*2) and then dried by rotary evaporation. The solute was separated and purified by a thick preparative plate (developing solvent: PE : EA = 2 : 1) to obtain compound A32-1 (100 mg, yield 66%). ESI-MS (M-H)⁻: 253.1.

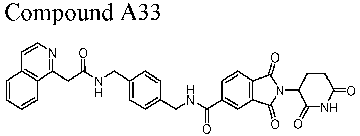

ESI-MS (M+H)⁺: 590.2.

¹H NMR (400 MHz, DMSO) δ 11.13 (s, 1H), 9.43 (t, *J* = 5.9 Hz, 1H), 8.69 (t, *J* = 5.9 Hz, 1H), 8.44 - 8.33 (m, 3H), 8.23 (d, *J* = 8.3 Hz, 1H), 8.04 (d, *J* = 7.8 Hz, 1H), 7.95 (d, *J* = 8.3 Hz, 1H), 7.78 - 7.71 (m, 2H), 7.68 - 7.60 (m, 1H), 7.29 (d, *J* = 8.1 Hz, 2H), 7.24 (d, *J* = 8.1 Hz, 2H), 5.20 (dd, *J* = 12.9, 5.4 Hz, 1H), 4.49 (d, *J* = 5.7 Hz, 2H), 4.28 (d, *J* = 5.8 Hz, 2H), 4.21 (s, 2H), 2.95 - 2.81 (m, 1H), 2.68 - 2.52 (m, 2H), 2.15 - 2.03 (m, 1H).

The corresponding intermediates were prepared as follows:

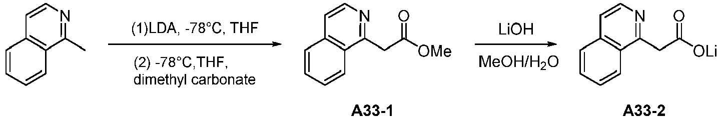

### Synthesis of compound A33-1

1-methylisoquinoline (1.3 g, 9.09 mmol) was dissolved in anhydrous tetrahydrofuran (20 mL). Under N₂ protection, at -78°C, LDA (13.6 ml, 2 M, 27.2 mmol) was added. The mixture was stirred at -78°C for 2 h, and then dimethyl carbonate (980 mg, 10.91 mmol) was added. The mixture was reacted at -78°C for another half an hour. Monitoring by TLC showed that the raw materials were completely reacted. The reaction was quenched with saturated aqueous sodium bicarbonate solution (20 ml) and then extracted with ethyl acetate (20 ml*3). The organic phases were combined, washed with saturated brine (10 ml) and then dried by rotary evaporation. The solute was purified using a chromatographic column (PE : EA = 20 : 1-1 : 1) to obtain compound A33-1 (1.4 g, yield 77%). ¹H NMR (400 MHz, DMSO) δ 8.41 (d, *J* = 5.7 Hz, 1H), 8.17 (d, *J* = 8.4 Hz, 1H), 7.99 (d, *J* = 8.2 Hz, 1H), 7.82 - 7.76 (m, 2H), 7.73 - 7.65 (m, 1H), 4.39 (s, 2H), 3.62 (s, 3H).

### Synthesis of compound A33-2

Compound A33-1 (500 mg, 2.49 mmol) was dissolved in 8 mL of methanol/H₂O (3 : 1), and lithium hydroxide hydrate (209 mg, 4.98 mmol) was added. The mixture was reacted at room temperature for 2 h, and then TLC showed that the reaction was completed. The reaction liquid was filtered, and the filter cake was washed with ethyl acetate (5 mL) to obtain compound A33-2 (400 mg, yield 83%).

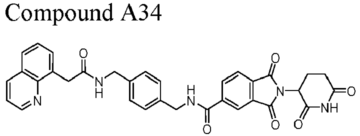

ESI-MS (M+H)⁺: 590.2.

¹H NMR (400 MHz, DMSO) δ 11.14 (s, 1H), 9.42 (t, *J* = 5.8 Hz, 1H), 8.92 (dd, *J* = 4.2, 1.7 Hz, 1H), 8.47 (t, *J* = 5.9 Hz, 1H), 8.39 - 8.35 (m, 3H), 8.04 (d, *J* = 7.8 Hz, 1H), 7.88 (d, *J* = 7.0 Hz, 1H), 7.67 (d, *J* = 7.1 Hz, 1H), 7.59 - 7.47 (m, 2H), 7.26 (dd, *J* = 18.4, 8.2 Hz, 4H), 5.19 (dd, *J* = 12.9, 5.4 Hz, 1H), 4.48 (d, *J* = 5.7 Hz, 2H), 4.26 (d, *J* = 5.9 Hz, 2H), 4.11 (s, 2H), 2.96 - 2.82 (m, 1H), 2.69 - 2.56 (m, 2H), 2.15 - 2.04 (m, 1H).

The corresponding intermediates were prepared as follows:

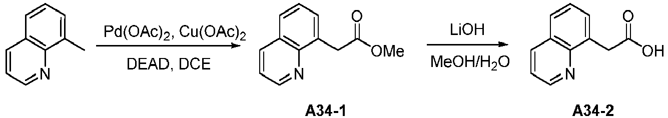

### Synthesis of compound A34-1

8-methylquinoline (1.01 g, 7 mmol) was dissolved in 8 mL of DCE, and palladium acetate (80 mg, 0.35 mmol), copper acetate (130 mg, 0.70 mmol) and DEAD (2.43 g, 14.0 mmol) were successively added. The reaction was stirred overnight at 100°C, and then TLC showed that about half of the raw materials remained. The solution was filtered, and the filtrate was diluted with ethyl acetate (40 mL) and then washed with saturated brine (10 mL*3). The organic phase was dried by rotary evaporation, and then the solute was refined by column chromatography (mobile phase: PE : EA = 100 : 1-5 : 1) to obtain compound A34-1 (190 mg, yield 13%). ¹H NMR (400 MHz, DMSO) δ 8.90 (dd, *J* = 4.2, 1.8 Hz, 1H), 8.38 (dd, *J* = 8.3, 1.7 Hz, 1H), 7.92 (dd, *J* = 8.2, 1.3 Hz, 1H), 7.70 (d, *J* = 6.5 Hz, 1H), 7.60 - 7.53 (m, 2H), 4.21 (s, 2H), 4.07 (q, *J* = 7.1 Hz, 2H), 1.16 (t, *J* = 7.1 Hz, 3H).

### Synthesis of compound A34-2

Compound A34-1 (190 mg, 0.88 mmol) was dissolved in 8 mL of methanol/H₂O (3 : 1), and lithium hydroxide hydrate (54 mg, 1.30 mmol) was added. The mixture was reacted at room temperature for 2 h, and then TLC showed that the reaction was completed. The mixture was adjusted to pH 3-4 with dilute hydrochloric acid, and then the aqueous phase was extracted with ethyl acetate (40 mL*3). The organic phases were combined and dried by rotary evaporation to obtain compound A34-2 (70 mg, yield 43%).

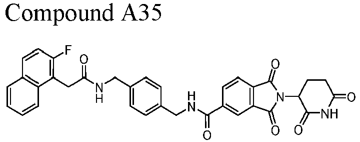

ESI-MS (M+H)⁺: 607.2.

¹H NMR (400 MHz, DMSO) δ 11.15 (s, 1H), 9.43 (t*, J* = 5.9 Hz, 1H), 8.69 (t, *J* = 5.6 Hz, 1H), 8.42 - 8.32 (m, 2H), 8.03 (t, *J* = 7.9 Hz, 2H), 7.98 - 7.88 (m, 2H), 7.56 (t, *J* = 7.3 Hz, 1H), 7.48 (t, *J* = 7.3 Hz, 1H), 7.42 (t, *J* = 9.3 Hz, 1H), 7.29 (d, *J* = 8.1 Hz, 2H), 7.22 (d, *J* = 8.1 Hz, 2H), 5.19 (dd, *J* = 12.8, 5.3 Hz, 1H), 4.49 (d, *J* = 5.7 Hz, 2H), 4.26 (d, *J* = 5.8 Hz, 2H), 3.95 (d, *J* = 27.9 Hz, 2H), 2.96 - 2.81 (m, 1H), 2.70 - 2.53 (m, 2H), 2.14 - 2.07 (m, 1H).

The corresponding intermediates were prepared as follows:

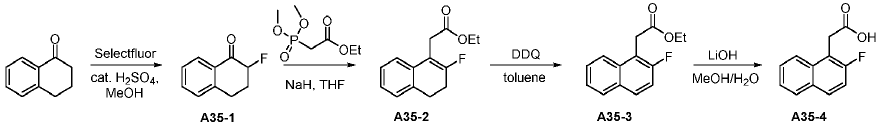

### Synthesis of compound A35-1

The compound 1-tetralone (6.5 mL, 50 mmol) was dissolved in 40 mL of methanol, and selective fluorine reagent (21.2 g, 60 mmol) and concentrated sulfuric acid (0.3 mL, 5.4 mmol) were successively added. The mixture was reacted overnight at 50°C, and then TLC showed that the reaction was completed. The mixture was diluted with ethyl acetate (100 mL). The organic phase was washed with saturated brine (20 mL*3) and then dried by rotary evaporation. The solute was purified by column chromatography (mobile phase: PE : EA = 100 : 1-5 : 1) to obtain compound A35-1 (6.8 g, yield 83%). ¹H NMR (400 MHz, CDCl₃) δ 8.05 (d, *J* = 7.9 Hz, 1H), 7.53 (t, *J* = 7.5 Hz, 1H), 7.35 (t, *J* = 7.6 Hz, 1H), 7.27 (d, *J* = 7.7 Hz, 1H), 5.21 (dd, *J* = 12.8, 5.1 Hz, 1H), 5.09 (dd, *J* = 12.8, 5.1 Hz, 1H), 3.13 (dd, *J* = 9.2, 3.9 Hz, 2H), 2.58 (ddd, *J* = 12.8, 9.6, 4.8 Hz, 1H), 2.35 (ddd, *J* = 9.8, 7.5, 3.7 Hz, 1H).

### Synthesis of compound A35-2

NaH (2.01 g, 60%, 50.0 mmol) was suspended in 120 mL of anhydrous tetrahydrofuran. Under ice bath, the compound dimethyl ethylphosphorylacetate (6.5 g, 33 mmol) was added dropwise. The mixture was reacted under ice bath for 30 min, and then A35-1 (6.8 g, 41.5 mmol) was added. The mixture was warmed to 70°C and reacted overnight, and then TLC showed that the reaction was completed. The reaction was quenched with saturated aqueous sodium bicarbonate solution (30 mL). The aqueous phase was extracted with ethyl acetate (60 mL*3). The organic phases were combined and dried by rotary evaporation, and then the solute was separated and purified by column chromatography (mobile phase: PE : EA = 100 : 1-5 : 1) to obtain compound A35-2 (3.4 g, yield 35%). ¹H NMR (400 MHz, CDCl₃) δ 7.23 - 7.14 (m, 1H), 7.13 - 7.06 (m, 3H), 4.20 - 4.10 (m, 2H), 3.53 (s, 2H), 3.00 (td, *J* = 8.3, 2.7 Hz, 2H), 2.64 - 2.55 (m, 2H), 1.23 (t, *J* = 7.1 Hz, 3H).

### Synthesis of compound A35-3

Compound A35-2 (3.4 g, 14.5 mmol) was dissolved in 120 mL of toluene, and DDQ (3.9 g, 17.4 mmol) was added. The mixture was reacted at 120°C for 2 h, and then TLC showed that the reaction was completed. The reaction liquid was filtered, and the filtrate was diluted with ethyl acetate (200 mL). The organic phase was washed with saturated brine and then dried by rotary evaporation. The solute was purified by column chromatography (mobile phase: PE : EA = 100 : 1-5 : 1) to obtain A35-3 (2.9 g, yield 88%). ¹H NMR (400 MHz, CDCl₃) δ 7.92 (d, *J* = 8.5 Hz, 1H), 7.85 (d, *J* = 8.1 Hz, 1H), 7.79 (dd, *J* = 8.9, 5.7 Hz, 1H), 7.56 (t, *J* = 7.6 Hz, 1H), 7.45 (t, *J* = 7.5 Hz, 1H), 7.29 (t, *J* = 7.2 Hz, 1H), 4.16 (q, *J* = 7.1 Hz, 2H), 4.10 (s, 2H), 1.22 (t, *J* = 7.1 Hz, 3H).

### Synthesis of compound A35-4

Compound A35-3 (100 mg, 0.43 mmol) was dissolved in 4 mL of methanol/H₂O (3 : 1), and lithium hydroxide hydrate (26 mg, 0.65 mmol) was added. The mixture was reacted at room temperature for 2 h, and then TLC showed that the reaction was completed. The mixture was adjusted to pH 3-4 with dilute hydrochloric acid, and then the aqueous phase was extracted with ethyl acetate (40 mL*3). The organic phases were combined and dried by rotary evaporation to obtain compound A35-4 (60 mg, yield 69%).

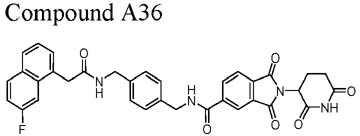

ESI-MS (M+H)⁺: 607.2.

¹H NMR (400 MHz, DMSO) δ 11.14 (s, 1H), 9.43 (t, *J* = 5.8 Hz, 1H), 8.68 (t, *J* = 5.8 Hz, 1H), 8.41 - 8.31 (m, 2H), 8.08 - 7.98 (m, 2H), 7.97 - 7.80 (m, 2H), 7.59 - 7.46 (m, 1H), 7.46 - 7.37 (m, 2H), 7.31 - 7.25 (m, 2H), 7.24 - 7.16 (m, 2H), 5.18 (dd, *J* = 12.9, 5.4 Hz, 1H), 4.48 (d, *J* = 5.8 Hz, 2H), 4.26 (d, *J* = 5.8 Hz, 2H), 3.92 (s, 2H), 2.93 - 2.84 (m, 1H), 2.68 - 2.53 (m, 2H), 2.13 - 2.00 (m, 1H).

The corresponding intermediates were prepared as follows:

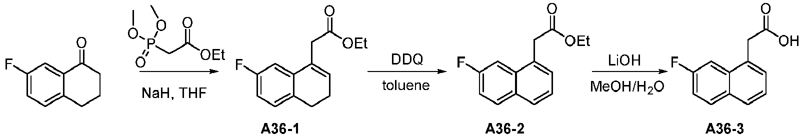

### Synthesis of compound A36-1

NaH (1.58 g, 60%, 39.6 mmol) was suspended in anhydrous tetrahydrofuran (100 mL). Under ice bath, the compound dimethyl ethylphosphorylacetate (8.20 g, 36.6 mmol) was added dropwise. The mixture was reacted under ice bath for 30 min, and then 7-fluoro-3,4-dihydronaphthalene-1(2H)-one (5.0 g, 30.5 mmol) was added. The mixture was reacted overnight at room temperature, and then TLC showed that the reaction was completed. The reaction was quenched with saturated aqueous sodium bicarbonate solution (30 mL). The aqueous phase was extracted with ethyl acetate (50 mL*3). The organic phases were combined and then dried by rotary evaporation. The solute was separated by column chromatography (mobile phase: PE : EA = 100 : 1-5 : 1) to obtain compound A36-1 (4.0 g, yield 56%).

### Synthesis of compound A36-2

Compound A36-1 (4.0 g, 17.1 mmol) was dissolved in 25 mL of toluene, and DDQ (4.6 g, 20.5 mmol) was added. The mixture was reacted at room temperature for 16 h, and then TLC showed that the reaction was completed. The reaction liquid was filtered, and the filtrate was diluted with ethyl acetate (100 mL). The organic phase was washed with saturated brine (20 mL*2) and then dried by rotary evaporation. The solute was purified by column chromatography (mobile phase: PE : EA = 100 : 1-5 : 1) to obtain A36-2 (1.61 g, yield 40%). ¹H NMR (400 MHz, DMSO) δ 8.05 (dd, *J* = 9.0, 6.1 Hz, 1H), 7.91 (d, *J* = 7.9 Hz, 1H), 7.69 (dd, *J* = 11.5, 2.4 Hz, 1H), 7.47 (dt, *J* = 9.5, 7.0 Hz, 3H), 4.15 - 4.05 (m, 4H), 1.17 (t, *J* = 7.2 Hz, 3H).

### Synthesis of compound A36-3

Compound A36-3 (1.6 g, 6.9 mmol) was dissolved in 20 mL of methanol/H₂O (3 : 1), and lithium hydroxide hydrate (580 mg, 13.8 mmol) was added. The mixture was reacted at room temperature for 2 h, and then TLC showed that the reaction was completed. The mixture was adjusted to pH 3-4 with dilute hydrochloric acid. The precipitated solid was filtered, and the filter cake was dried to obtain compound A36-3 (1.0 g, yield 71%). ¹H NMR (400 MHz, DMSO) δ 12.46 (s, 1H), 8.04 (dd, *J* = 9.0, 6.1 Hz, 1H), 7.90 (d, *J* = 7.8 Hz, 1H), 7.69 (dd, *J* = 11.5, 2.3 Hz, 1H), 7.54 - 7.38 (m, 3H), 4.03 (s, 2H).

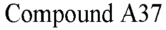

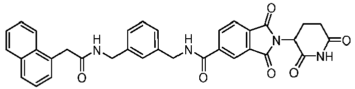

ESI-MS (M+H)⁺: 589.2.

¹H NMR (400 MHz, DMSO) δ 11.15 (s, 1H), 9.45 (t, *J* = 5.8 Hz, 1H), 8.66 (t, *J* = 5.9 Hz, 1H), 8.42 - 8.34 (m, 2H), 8.09 (dd, *J* = 12.8, 6.1 Hz, 1H), 8.04 (d, *J* = 7.8 Hz, 1H), 7.95 - 7.87 (m, 1H), 7.85 - 7.76 (m, 1H), 7.56 - 7.47 (m, 2H), 7.46 - 7.39 (m, 2H), 7.29 - 7.23 (m, 1H), 7.22 (s, 2H), 7.13 (d, *J* = 7.4 Hz, 1H), 5.20 (dd, *J* = 12.9, 5.4 Hz, 1H), 4.48 (d, *J* = 5.7 Hz, 2H), 4.28 (d, *J* = 5.9 Hz, 2H), 3.95 (s, 2H), 2.95 - 2.85 (m, 1H), 2.66 - 2.52 (m, 2H), 2.12 - 2.07 (m, 1H).

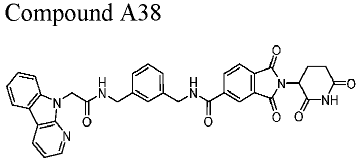

ESI-MS (M+H)⁺: 629.2.

¹H NMR (400 MHz, DMSO) δ 11.15 (s, 1H), 9.46 (t, *J* = 5.9 Hz, 1H), 8.78 (t, *J* = 5.9 Hz, 1H), 8.54 (dd, *J* = 7.6, 1.5 Hz, 1H), 8.44 (dd, *J* = 4.8, 1.5 Hz, 1H), 8.41 (s, 1H), 8.38 (dd, *J* = 7.8, 1.3 Hz, 1H), 8.20 (d, *J* = 7.7 Hz, 1H), 8.04 (d, *J* = 7.8 Hz, 1H), 7.56 (d, *J* = 8.2 Hz, 1H), 7.53 - 7.45 (m, 1H), 7.35 - 7.16 (m, 6H), 5.20 (dd, *J* = 12.5, 5.0 Hz, 1H), 5.16 (s, 2H), 4.51 (d, *J* = 5.7 Hz, 2H), 4.31 (d, *J* = 5.9 Hz, 2H), 2.94 - 2.85 (m, 1H), 2.67 - 2.52 (m, 2H), 2.13 - 2.03 (m, 1H).

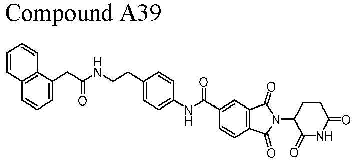

ESI-MS (M+H)⁺: 589.2.

¹H NMR (400 MHz, DMSO) δ 11.18 (s, 1H), 10.56 (s, 1H), 8.49 (s, 1H), 8.43 (d, *J* = 7.8 Hz, 1H), 8.19 (t, *J* = 5.6 Hz, 1H), 8.09 (d, *J* = 7.8 Hz, 1H), 8.04 (d, *J* = 7.3 Hz, 1H), 7.95 - 7.89 (m, 1H), 7.81 (d, *J* = 8.1 Hz, 1H), 7.71 (d, *J* = 8.4 Hz, 2H), 7.57 - 7.47 (m, 2H), 7.47 - 7.36 (m, 2H), 7.18 (d, *J* = 8.5 Hz, 2H), 5.22 (dd, *J* = 12.9, 5.3 Hz, 1H), 3.88 (s, 2H), 3.33 - 3.28 (m, 2H), 2.97 - 2.86 (m, 1H), 2.66 (dd, *J* = 15.5, 8.4 Hz, 2H), 2.65 - 2.53 (m, 2H), 2.15 - 2.07 (m, 1H).

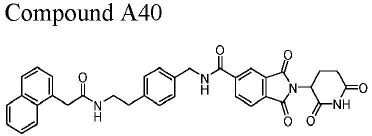

ESI-MS (M+H)⁺: 603.2.

¹H NMR (400 MHz, DMSO) δ 11.14 (s, 1H), 9.43 (t, *J* = 5.9 Hz, 1H), 8.43 - 8.35 (m, 2H), 8.17 (t, *J* = 5.6 Hz, 1H), 8.08 - 8.01 (m, 2H), 7.92 - 7.86 (m, 1H), 7.79 (d, *J* = 8.0 Hz, 1H), 7.53 - 7.45 (m, 2H), 7.44 - 7.36 (m, 2H), 7.24 (d, *J* = 8.0 Hz, 2H), 7.12 (d, *J* = 8.1 Hz, 2H), 5.20 (dd, *J* = 12.9, 5.4 Hz, 1H), 4.49 (d, *J* = 5.8 Hz, 2H), 3.86 (s, 2H), 3.30 - 3.23 (m, 2H), 2.94 - 2.85 (m, 1H), 2.69 (t, *J* = 7.1 Hz, 2H), 2.64 - 2.52 (m, 2H), 2.14 - 2.04 (m, 1H).

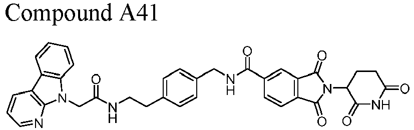

ESI-MS (M+H)⁺: 643.2.

¹H NMR (400 MHz, DMSO) δ 11.14 (s, 1H), 9.43 (t, *J* = 5.6 Hz, 1H), 8.53 (dd, *J* = 7.6, 1.5 Hz, 1H), 8.44 - 8.36 (m, 3H), 8.30 (d, *J* = 5.4 Hz, 1H), 8.19 (d, *J* = 7.8 Hz, 1H), 8.05 (d, *J* = 7.8 Hz, 1H), 7.52 - 7.42 (m, 2H), 7.29 - 7.20 (m, 4H), 7.18 (d, *J* = 8.1 Hz, 2H), 5.19 (dd, *J* = 12.8, 5.4 Hz, 1H), 5.06 (s, 2H), 4.50 (d, *J* = 5.9 Hz, 2H), 3.28 - 3.24 (m, 2H), 2.96 - 2.83 (m, 1H), 2.71 (t, *J* = 7.4 Hz, 2H), 2.67 - 2.54 (m, 2H), 2.13 - 2.04 (m, 1H).

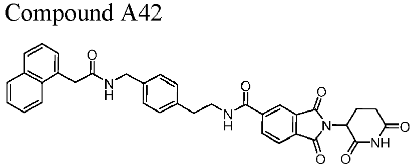

ESI-MS (M+H)⁺: 603.2.

¹H NMR (400 MHz, DMSO) δ 11.14 (s, 1H), 8.97 (t, *J* = 5.4 Hz, 1H), 8.60 (t, *J* = 6.0 Hz, 1H), 8.32 - 8.29 (m, 2H), 8.13 - 8.06 (m, 1H), 8.03 (d, *J* = 7.7 Hz, 1H), 7.94 - 7.89 (m, 1H), 7.84 - 7.77 (m, 1H), 7.54 - 7.48 (m, 2H), 7.46 - 7.41 (m, 2H), 7.22 - 7.12 (m, 4H), 5.19 (dd, *J* = 12.8, 5.4 Hz, 1H), 4.25 (d, *J* = 5.9 Hz, 2H), 3.96 (s, 2H), 3.51 (dd, *J* = 13.0, 7.0 Hz, 2H), 2.96 - 2.82 (m, 3H), 2.69 - 2.53 (m, 2H), 2.13 - 2.03 (m, 1H).

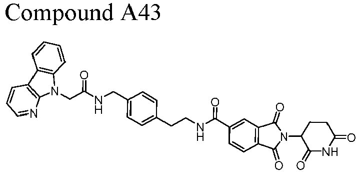

ESI-MS(M+H)⁺: 643.2.

¹H NMR (400 MHz, DMSO) δ 11.14 (s, 1H), 8.99 (t, *J* = 5.0 Hz, 1H), 8.72 (t, *J* = 6.2 Hz, 1H), 8.54 (dd, *J* = 7.6, 1.5 Hz, 1H), 8.44 (dd, *J* = 4.8, 1.5 Hz, 1H), 8.33 - 8.30 (m, 2H), 8.21 (d, *J* = 7.8 Hz, 1H), 8.03 (d, *J* = 7.8 Hz, 1H), 7.57 (d, *J* = 8.2 Hz, 1H), 7.49 (t, *J* = 7.7 Hz, 1H), 7.30 - 7.23 (m, 2H), 7.23 - 7.17 (m, 4H), 5.22 - 5.17 (m, 1H), 5.16 (s, 2H), 4.27 (d, *J* = 5.8 Hz, 2H), 3.54 - 3.49 (m, 2H), 2.95 - 2.84 (m, 3H), 2.68 - 2.53 (m, 2H), 2.13 - 2.03 (m, 1H).

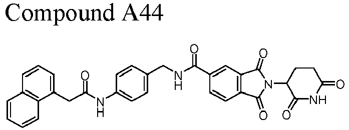

ESI-MS(M+H)⁺: 575.2.

¹H NMR (400 MHz, DMSO) δ 11.14 (s, 1H), 10.32 (s, 1H), 9.40 (t, *J* = 5.8 Hz, 1H), 8.40 - 8.33 (m, 2H), 8.12 (d, *J* = 8.1 Hz, 1H), 8.03 (d, *J* = 7.8 Hz, 1H), 7.93 (d, *J* = 7.4 Hz, 1H), 7.87 - 7.82 (m, 1H), 7.60 - 7.45 (m, 6H), 7.28 (d, *J* = 8.5 Hz, 2H), 5.19 (dd, *J* = 12.9, 5.3 Hz, 1H), 4.46 (d, *J* = 5.8 Hz, 2H), 4.13 (s, 2H), 2.93 - 2.82 (m, 1H), 2.69 - 2.53 (m, 2H), 2.12 - 2.03 (m, 1H).

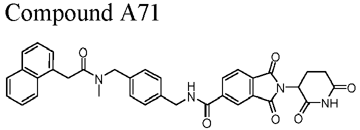

ESI-MS(M+H)⁺: 603.2.

¹H NMR (400 MHz, DMSO) δ 11.15 (s, 1H), 9.49 - 9.42 (m, 1H), 8.42 - 8.34 (m, 2H), 8.05 (d, *J* = 7.8 Hz, 1H), 7.98 - 7.86 (m, 2H), 7.81 (t, *J* = 7.4 Hz, 1H), 7.56 - 7.29 (m, 6H), 7.26 - 7.21 (m, 2H), 5.20 (dd, *J* = 12.9, 5.4 Hz, 1H), 4.75 - 4.47 (m, 4H), 4.20 (d, *J* = 16.8 Hz, 2H), 3.08 - 2.82 (m, 4H), 2.68 - 2.51 (m, 2H), 2.14 - 2.06 (m, 1H).

The corresponding intermediates were prepared as follows:

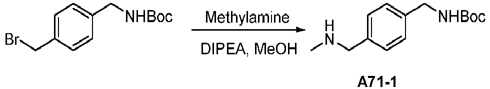

### Synthesis of compound A71-1

The compound tert-butyl 4-(bromomethyl)benzylcarbamate (635 mg, 2.11 mmol) was dissolved in methanol (10 mL), and methylamine alcohol solution (0.5 ml, 4.25 mmol) and DIPEA (544 mg, 4.22 mmol) were added dropwise. The reaction was stirred at room temperature for 1 h, and then monitoring by TLC showed that the reaction was completed. The solvent was removed by drying. Water was added, and then the mixture was extracted with dichloromethane (40 ml*3). The organic phases were combined. The solvent was removed by drying, and the solute was separated by a preparative plate (DCM : MeOH = 10 : 1) to obtain the product (400 mg, yield 76%).

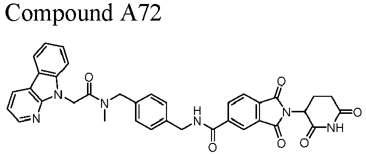

ESI-MS(M+H)⁺: 643.2.

¹H NMR (400 MHz, DMSO) δ 11.15 (s, 1H), 9.51 - 9.42 (m, 1H), 8.57 - 8.49 (m, 1H), 8.47 - 8.33 (m, 3H), 8.20 (t, *J* = 6.9 Hz, 1H), 8.06 - 8.03 (m, 1H), 7.63 - 7.19 (m, 8H), 5.46 (d, *J* = 10.8 Hz, 2H), 5.20 (dd, *J* = 12.9, 5.3 Hz, 1H), 4.86 - 4.46 (m, 4H), 3.21 - 2.77 (m, 4H), 2.67 - 2.52 (m, 2H), 2.14 - 2.06 (m, 1H).

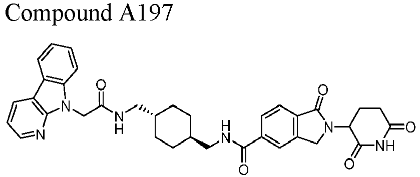

ESI-MS(M+H)⁺: 621.2

¹H NMR (400 MHz, DMSO) δ 10.98 (s, 1H), 8.62 (t, *J* = 5.6 Hz, 1H), 8.58 - 8.51 (m, 1H), 8.43 (dd, *J* = 4.8, 1.2 Hz, 1H), 8.24 - 8.19 (m, 2H), 8.05 (s, 1H), 7.97 (d, *J* = 8.0 Hz, 1H), 7.81 (d, *J* = 7.9 Hz, 1H), 7.57 - 7.45 (m, 2H), 7.32 - 7.20 (m, 2H), 5.19 - 5.04 (m, 3H), 4.46 (dd, *J* = 50.4, 17.6 Hz, 2H), 3.14 (t, *J* = 6.1 Hz, 2H), 2.99 - 2.82 (m, 3H), 2.65 - 2.56 (m, 1H), 2.46 - 2.33 (m, 1H), 2.09 - 1.93 (m, 1H), 1.79 - 1.72 (m, 4H), 1.58 - 1.33 (m, 2H), 1.01 - 0.79 (m, 4H).

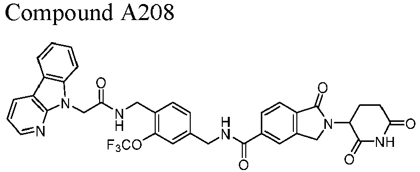

ESI-MS(M+H)⁺: 699.2

¹H NMR (400 MHz, DMSO) δ 11.01 (s, 1H), 9.31 (t, *J* = 5.9 Hz, 1H), 8.78 (t, *J* = 5.9 Hz, 1H), 8.55 (dd, *J* = 7.6, 1.6 Hz, 1H), 8.45 (dd, *J* = 4.8, 1.6 Hz, 1H), 8.21 (d, *J* = 7.7 Hz, 1H), 8.09 (s, 1H), 7.99 (d, *J* = 8.0 Hz, 1H), 7.83 (d, *J* = 7.8 Hz, 1H), 7.58 (d, *J* = 8.2 Hz, 1H), 7.52 - 7.44 (m, 2H), 7.35 (d, *J* = 8.0 Hz, 1H), 7.33 - 7.19 (m, 3H), 5.18 (s, 2H), 5.14 (dd, *J* = 13.3, 5.1 Hz, 1H), 4.58 - 4.37 (m, 4H), 4.33 (d, *J* = 5.8 Hz, 2H), 2.98 - 2.83 (m, 1H), 2.71 - 2.55 (m, 1H), 2.47 - 2.34 (m, 1H), 2.06 - 1.93 (m, 1H).

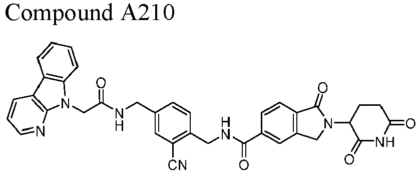

ESI-MS(M+H)⁺: 640.2

¹H NMR (400 MHz, DMSO) δ 11.00 (s, 1H), 9.38 (t, *J* = 5.6 Hz, 1H), 8.82 (t, *J* = 6.0 Hz, 1H), 8.55 (d, *J* = 7.7 Hz, 1H), 8.48 (d, *J* = 3.5 Hz, 1H), 8.21 (d, *J* = 7.7 Hz, 1H), 8.10 (s, 1H), 8.03 (d, *J* = 8.1 Hz, 1H), 7.84 (d, *J* = 8.0 Hz, 1H), 7.72 (s, 1H), 7.62 - 7.52 (m, 2H), 7.54 - 7.44 (m, 2H), 7.35 - 7.23 (m, 2H), 5.24 - 5.07 (m, 3H), 4.64 (d, *J* = 5.5 Hz, 2H), 4.47 (dd, *J* = 51.9, 17.5 Hz, 2H), 4.33 (d, *J* = 5.8 Hz, 2H), 2.98 - 2.85 (m, 1H), 2.70 - 2.58 (m, 1H), 2.42 - 2.36 (m, 1H), 2.06 - 2.00 (m, 1H).

The intermediates were prepared as follows:

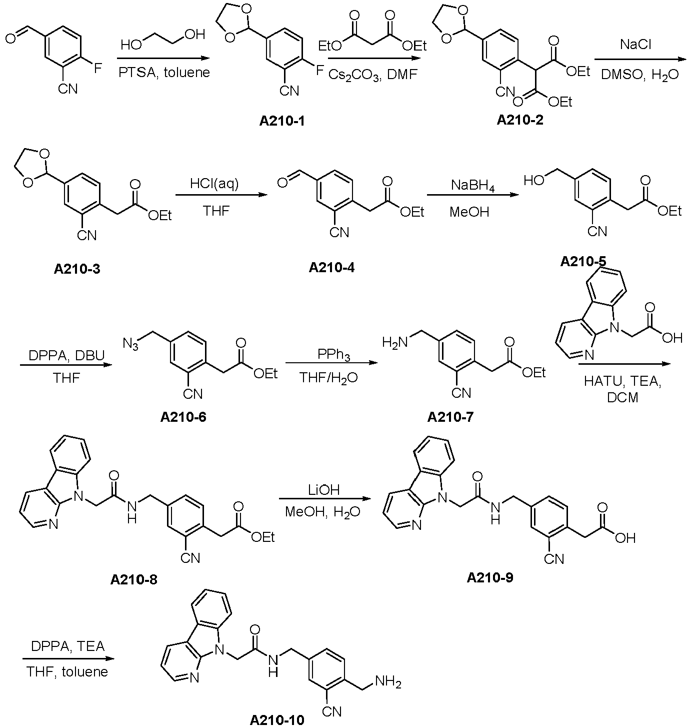

### Synthesis of compound A210-1

2-fluoro-5-formylbenzonitrile (10 g, 67.1 mmol), p-toluenesulfonic acid (6.4 g, 33.5 mmol) and ethylene glycol (41.6 g, 670.1 mmol) were dissolved in 150 mL of toluene. The mixture was reacted at 140°C for 3 h, and then TLC showed that the reaction was completed. The solvent was dried by rotary evaporation, and then 60 mL of water was added. The aqueous phase was extracted with ethyl acetate (80 mL*3). The organic phases were combined, dried over anhydrous sodium sulfate and dried by rotary evaporation. The solute was separated and purified by column chromatography (mobile phase: petroleum ether : ethyl acetate = 5 : 1) to obtain compound A210-1 (12.0 g, yield 93%).

### Synthesis of compound A210-2

A210-1 (12.0 g, 62.1 mmol), diethyl malonate (10.9 g, 68.3 mmol) and cesium carbonate (40.5 g, 124.2 mmol) were dissolved in 90 mL of DMF. The mixture was reacted at 80°C for 3 h, and then TLC showed that the reaction was completed. The mixture was cooled to room temperature and diluted with ethyl acetate (400 mL). The organic phase was washed with saturated brine (50 mL*3), dried over anhydrous sodium sulfate and dried by rotary evaporation. The solute was separated and purified by column chromatography (mobile phase: petroleum ether : ethyl acetate = 5 : 1) to obtain compound A210-2 (13.0 g, yield 63%).

### Synthesis of compound A210-3

A210-2 (13.0 g, 39.0 mmol) was dissolved in DMSO/H₂O (90/15 mL). The mixture was reacted overnight at 120°C, and then TLC showed that the reaction was completed. The mixture was cooled to room temperature and diluted with ethyl acetate (400 mL). The organic phase was washed with saturated brine (50 mL*3), dried over anhydrous sodium sulfate and dried by rotary evaporation. The solute was separated and purified by column chromatography (mobile phase: petroleum ether : ethyl acetate = 5 : 1) to obtain compound A210-3 (5.6 g, yield 55%).

### Synthesis of compound A210-4

A210-3 (5.6 g, 21.4 mmol) and 1 N hydrochloric acid (60 mL) were dissolved in 60 mL of THF. The mixture was reacted at 70°C for 1.5 h, and then TLC showed that the reaction was completed. The mixture was cooled to room temperature and then adjusted to pH 8 with saturated sodium bicarbonate solution. The aqueous phase was extracted with ethyl acetate (80 mL*3). The organic phases were combined, washed with saturated brine (20 mL*3), dried over anhydrous sodium sulfate and dried by rotary evaporation. The solute was separated and purified by column chromatography (mobile phase: petroleum ether : ethyl acetate = 5 : 1) to obtain compound A210-4 (5.0 g, yield 99%).

### Synthesis of compound A210-5

A210-4 (5.0 g, 23.0 mmol) was dissolved in 25 mL of methanol. Under ice bath, sodium borohydride (1.3 g, 34.5 mmol) was added in batches. The mixture was reacted at room temperature for 1 h, and then TLC showed that the reaction was completed. The reaction was quenched with ice water (20 mL). The aqueous phase was extracted with ethyl acetate (20 ml*3). The organic phases were combined, dried over anhydrous sodium sulfate and dried by rotary evaporation. The solute was separated and purified by column chromatography (mobile phase: petroleum ether : ethyl acetate = 3 : 1) to obtain compound A210-5 (4.0 g, yield 80%).

### Synthesis of compound A210-6

A210-5 (500 mg, 2.3 mmol) was dissolved in toluene/THF (15 mL/6 mL), and DPPA (1.26 g, 4.60 mmol) and DBU (700 mg, 4.60 mmol) were added. The mixture was reacted overnight at 60°C, and then TLC showed that the reaction was completed. The mixture was cooled to room temperature and diluted with water (50 mL). The aqueous phase was extracted with ethyl acetate (30 mL*3). The organic phases were combined, dried over anhydrous sodium sulfate and dried by rotary evaporation to obtain compound A210-6 (600 mg, crude).

### Synthesis of compound A210-7

Compound A210-6 (600 mg, crude, 2.3 mmol) was dissolved in THF/H₂O (15/3 mL), and triphenylphosphine (660 mg, 2.5 mmol) was added. The mixture was reacted at 50°C for 2 h, and then TLC showed that the reaction was completed. The mixture was cooled to room temperature and adjusted to pH 4 with 2 N HCl. The aqueous phase was extracted with ethyl acetate (10 mL). The aqueous phase was further adjusted to pH 8 with saturated sodium carbonate solution, and then extracted with dichloromethane/methanol (10 : 1, 60 ml*3). The dichloromethane phases were combined, dried over anhydrous sodium sulfate and dried by rotary evaporation to obtain A207-7 (500 mg, crude).

### Synthesis of compound A210-8

Compound A207-7 (500 mg, 2.3 mmol) and 2-(9H-pyrido[2,3-b]indole)acetic acid (620 mg, 2.7 mmol) were dissolved in dichloromethane (25 mL), and HATU (1.3 g, 3.4 mmol) and triethylamine (930 mg, 9.2 mmol) were added. The mixture was reacted at room temperature for 2 h, and then monitoring by TLC showed that the reaction was completed. Water (20 ml) was added. The aqueous phase was extracted with dichloromethane (20 mL*3). The organic phases were combined, washed with saturated brine (25 ml), dried over anhydrous sodium sulfate and dried by rotary evaporation. The solute was separated and purified by column chromatography (mobile phase: dichloromethane : methanol = 10 : 1) to obtain compound A210-8 (600 mg, yield 61%). Synthesis of compound A210-9

Compound A210-8 (600 mg, 1.4 mmol) was dissolved in 27 mL of methanol/H₂O (2 : 1), and lithium hydroxide hydrate (120 mg, 2.8 mmol) was added. The mixture was reacted at room temperature for 2 h, and then TLC showed that the reaction was completed. The mixture was adjusted to pH 3-4 with dilute hydrochloric acid, and then the precipitated solid was subjected to suction filtration and dried to obtain compound A210-9 (500 mg, yield 89%).

### Synthesis of compound A210-10

A210-9 (200 mg, 0.50 mmol) was dissolved in toluene/THF (14/3 mL), and triethylamine (100 mg, 1.0 mmol) and DPPA (210 mg, 0.8 mmol) were added. The mixture was reacted at 80°C for 4 h, and then TLC showed that the reaction was completed. The reaction was cooled to room temperature, quenched with 2 N hydrochloric acid (2 mL) and then diluted with water (50 mL). The aqueous phase was extracted with ethyl acetate (30 mL*3), and then the organic phases were combined, dried over anhydrous sodium sulfate and dried by rotary evaporation. The solute was separated and purified by column chromatography (mobile phase: dichloromethane : methanol = 10 : 1) to obtain compound A210-10 (100 mg, yield 40%).

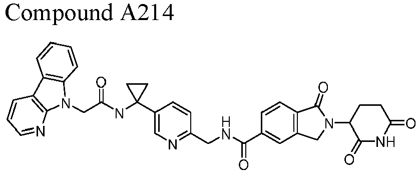

ESI-MS(M+H)⁺: 642.2
¹H NMR (400 MHz, DMSO) δ 11.01 (s, 1H), 9.24 (t, *J* = 5.8 Hz, 1H), 9.15 (s, 1H), 8.54 (dd, *J* = 7.6, 1.4 Hz, 1H), 8.45 (dd, *J* = 4.8, 1.4 Hz, 1H), 8.40 (d, *J* = 2.2 Hz, 1H), 8.20 (d, *J* = 7.8 Hz, 1H), 8.10 (s, 1H), 8.02 (d, *J* = 8.0 Hz, 1H), 7.83 (d, *J* = 7.9 Hz, 1H), 7.64 - 7.44 (m, 3H), 7.33 - 7.16 (m, 3H), 5.21 - 5.12 (m, 3H), 4.59 - 4.35 (m, 4H), 2.99 - 2.82 (m, 1H), 2.66 - 2.55 (m, 1H), 2.46 - 2.34 (m, 1H), 2.08 - 1.99 (m, 1H), 1.30 - 1.20 (m, 4H).

The intermediates were prepared as follows:

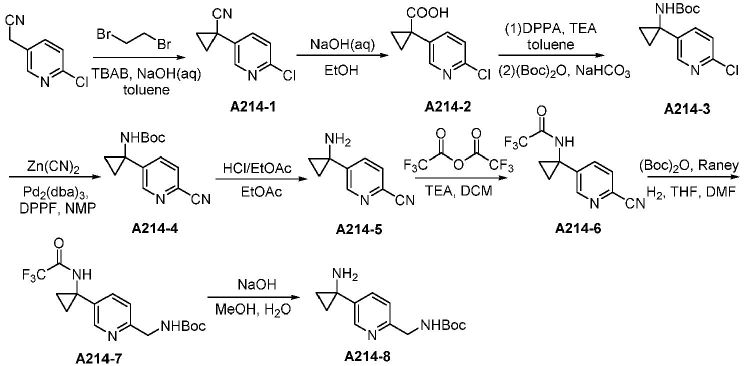

### Synthesis of compound A214-1

2-chloro-5-pyridineacetonitrile (88.5 g, 580.0 mmol) and 1,2-bromoethane (16.6 g, 881.6 mmol) were dissolved in 350 mL of toluene. Under ice bath, 50% aqueous sodium hydroxide solution (350 mL) and TBAB (39.3 g, 121.8 mmol) were added. The mixture was reacted at room temperature for 3 h, and then TLC showed that the reaction was completed. The aqueous phase was extracted with ethyl acetate (200 mL*3). The organic phases were combined, dried over anhydrous sodium sulfate and dried by rotary evaporation. The solute was separated and purified by column chromatography (mobile phase: petroleum ether : ethyl acetate = 4 : 1) to obtain compound A214-1 (72.2 g, yield 70%).

### Synthesis of compound A214-2

A214-1 (72.2 g, 404.2 mmol) was dissolved in 620 mL of ethanol. Under ice bath, 25% aqueous sodium hydroxide solution (320 mL) was added. The mixture was reacted at 100°C for 4 h, and then TLC showed that the reaction was completed. The mixture was cooled to room temperature and adjusted to pH 4 with 6 N hydrochloric acid. The aqueous phase was extracted with dichloromethane (300 mL*3). The organic phases were combined, dried over anhydrous sodium sulfate and dried by rotary evaporation to obtain compound A214-2 (73.6 g, yield 92%).

### Synthesis of compound A214-3

A214-2 (22.6 g, 114.4 mmol) was dissolved in toluene (225 mL), and triethylamine (11.57 g, 114.4 mmol) and DPPA (31.5 g, 114.4 mmol) were added. The mixture was reacted at 100°C for 1 h. 12 N hydrochloric acid (38 mL) was then added, and the mixture was reacted for another 1 h. The reaction was cooled to room temperature, quenched with ice water (50 mL) and then adjusted to pH 8 with sodium bicarbonate solid. (Boc)₂O (37.42 g, 171.54 mmol) was added. The mixture was reacted overnight at room temperature, and then 400 mL of water was added. The aqueous phase was extracted with ethyl acetate (200 mL*2). The organic phases were combined, dried over anhydrous sodium sulfate and dried by rotary evaporation. The solute was separated and purified by column chromatography (mobile phase: petroleum ether : ethyl acetate = 4 : 1) to obtain compound A214-3 (20.5 g, yield 69%).

### Synthesis of compound A214-4

A214-3 (20.5 g, 76.3 mmol) was dissolved in 310 mL of NMP, and zinc cyanide (5.37 g, 45.8 mmol), DPPF (5.46 g, 15.3 mmol) and Pd₂(dba)₃ (6.99 g, 7.63 mmol) were successively added. The mixture was reacted overnight at 110°C under nitrogen protection, and then TLC showed that the reaction was completed. The reaction liquid was cooled to room temperature and filtered, and the filtrate was diluted with 300 mL of water. The aqueous phase was extracted with ethyl acetate (500 mL*2). The organic phases were combined, washed with saturated brine, dried over anhydrous sodium sulfate (250 mL*2) and dried by rotary evaporation. The mixture was slurried with 40 mL of methyl tert-butyl ether at room temperature to obtain compound A214-4 (10.5 g, yield 53%).

### Synthesis of compound A214-5

A214-4 (10.5 g, 40.5 mmol) was dissolved in 50 mL of ethyl acetate, and HCl/EtOAc (4 M, 160 mL) was added. The mixture was reacted at room temperature for half an hour, and then TLC showed that the reaction was completed. The solvent was dried by rotary evaporation to obtain compound A214-5 (10.3 g, crude).

### Synthesis of compound A214-6

A214-5 (6.95 g, 38.6 mmol) and triethylamine (11.71 g, 115.7 mmol) were dissolved in 70 mL of dichloromethane. Under ice bath, trifluoroacetic anhydride (16.2 g, 77.1 mmol) was added dropwise. The mixture was reacted at room temperature for 1 h, and then TLC showed that the reaction was completed. The reaction was quenched with 50 mL of ice water. The aqueous phase was extracted with dichloromethane (50 mL*2). The organic phases were combined, dried over anhydrous sodium sulfate and dried by rotary evaporation. The solute was separated and purified by column chromatography (mobile phase: petroleum ether : ethyl acetate = 1 : 1) to obtain compound A214-6 (9.1 g, yield 92%).

### Synthesis of compound A214-7

A214-6 (9.1 g, 35.7 mmol) was dissolved in DMF/THF (50 mL/50 mL). (Boc)₂O (19.5 g, 89.2 mmol) and Raney Ni (9.0 g) were added. The mixture was reacted overnight at room temperature under hydrogen atmosphere, and then TLC showed that the reaction was completed. The reaction liquid was filtered, and the filtrate was dried by rotary evaporation. The solute was separated and purified by column chromatography (mobile phase: petroleum ether : ethyl acetate = 3 : 1) to obtain compound A214-7 (9.0 g, yield 70%).

### Synthesis of compound A214-8

A214-7 (9.0 g, 25.0 mmol) was dissolved in methanol/water (36 mL/36 mL). Under ice bath, sodium hydroxide (6.81 g, 170.3 mmol) was added. The mixture was reacted overnight at 50°C. TLC showed that the reaction was completed. The reaction liquid was extracted with dichloromethane/methanol (10 : 1, 50 ml*3). The organic phases were combined, dried over anhydrous sodium sulfate and dried by rotary evaporation. The solute was separated and purified by column chromatography (mobile phase: petroleum ether : ethyl acetate = 1 : 1) to obtain compound A214-8 (5.85 g, yield 88%).

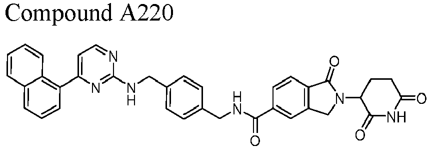

ESI-MS(M+H)⁺: 611.2
¹H NMR (400 MHz, DMSO) δ 11.00 (s, 1H), 9.21 (t, J= 5.7 Hz, 1H), 8.41 (d, *J* = 5.0 Hz, 1H), 8.20 - 7.92 (m, 5H), 7.88 (t, *J* = 6.3 Hz, 1H), 7.81 (d, *J* = 7.9 Hz, 1H), 7.65 - 7.40 (m, 3H), 7.30 (s, 4H), 6.84 (d, *J* = 5.0 Hz, 1H), 5.14 (dd, *J* = 13.3, 5.0 Hz, 1H), 4.61 - 4.33 (m, 6H), 3.01 - 2.85 (m, 1H), 2.69 - 2.53 (m, 1H), 2.46 - 2.29 (m, 1H), 2.06 - 1.96 (m, 1H).

The intermediates were prepared as follows:

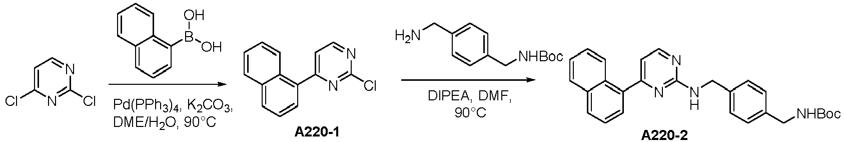

### Synthesis of compound A220-1

2,4-dichloropyrimidine (1.49 g, 10.0 mmol), 2-naphthaleneboronic acid (1.72 g, 10.0 mmol) and potassium carbonate (1.38 g, 10.0 mmol) were dissolved in DME/water (18 mL/3 mL), and Pd (PPh₃)₄ (1.15 g, 0.10 mmol) was added. The mixture was subjected to nitrogen replacement three times and then reacted at 90°C for 4 h. TLC showed that the reaction was completed. The mixture was cooled to room temperature. The reaction liquid was filtered, and the filtrate was diluted with ethyl acetate (20 mL). The organic phase was washed with saturated brine (10 ml*3), dried over anhydrous sodium sulfate and dried by rotary evaporation. The solute was separated and purified by column chromatography (mobile phase: petroleum ether : ethyl acetate = 10 : 1-7 : 1) to obtain compound A12-3 (2.27 g, yield 94%).

### Synthesis of compound A220-2

A220-1 (120 mg, 0.5 mmol) and 1-(N-Boc-aminomethyl)-4-(aminomethyl)benzene (236 mg, 1.0 mmol) were dissolved in 10 mL of DMF, and DIPEA (194 mg, 1.5 mmol) was added. The mixture was reacted overnight at 90°C. TLC showed that the reaction was completed. The mixture was cooled to room temperature and diluted with water (100 mL). The aqueous phase was extracted with ethyl acetate (50 mL*3) and then washed with saturated brine (20 mL*2). The organic phases were combined, dried over anhydrous sodium sulfate and dried by rotary evaporation. The solute was separated and purified by column chromatography (mobile phase: dichloromethane : methanol = 50 : 1) to obtain A220-2 (180 mg, yield 82%).

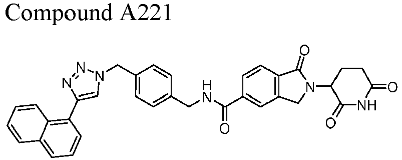

ESI-MS(M+H)⁺: 585.2
¹H NMR (400 MHz, DMSO) δ 11.00 (s, 1H), 9.23 (t, *J* = 5.9 Hz, 1H), 8.67 (s, 1H), 8.48 - 8.46 (m, 1H), 8.08 (s, 1H), 8.03 - 7.91 (m, 3H), 7.81 (d, *J* = 7.9 Hz, 1H), 7.75 (d, *J* = 7.1 Hz, 1H), 7.62 - 7.50 (m, 3H), 7.39 (q, *J* = 8.3 Hz, 4H), 5.70 (s, 2H), 5.13 (dd, *J* = 13.2, 5.1 Hz, 1H), 4.57 - 4.33 (m, 4H), 2.98 - 2.84 (m, 1H), 2.69 - 2.53 (m, 1H), 2.47 - 2.28 (m, 1H), 2.07 - 1.95 (m, 1H).

The intermediates were prepared as follows:

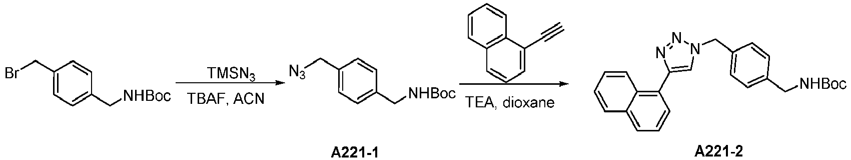

### Synthesis of compound A221-1

Tert-butyl 4-(bromomethyl)benzylcarbamate (300 mg, 1 mmol) was dissolved in 5 ml of acetonitrile, and TMSN₃ (172 mg, 1.5 mmol) and TBAF (1.5 ml, 1.5 mmol) were added. The mixture was reacted at 50°C for 2 h, and then TLC showed that the reaction was completed. The mixture was cooled to room temperature. The reaction liquid was diluted with water (20 mL). The aqueous phase was extracted with ethyl acetate (30 ml* 3). The organic phases were combined, dried over anhydrous sodium sulfate and dried by rotary evaporation. The solute was separated and purified by column chromatography (mobile phase: petroleum ether : ethyl acetate = 10 : 1) to obtain compound A221-1 (200 mg, yield 89%).

### Synthesis of compound A221-2

A221-1 (200 mg, 0.79 mmol) was dissolved in 5 mL of dioxane, and 1-ethynylnaphthalene (200 mg, 0.79 mmol) and triethylamine (160 mg, 1.58 mmol) were added. The mixture was reacted overnight at room temperature. TLC showed that the reaction was completed. The reaction liquid was diluted with water (20 mL). The aqueous phase was extracted with ethyl acetate (30 ml* 3). The organic phases were combined, dried over anhydrous sodium sulfate and dried by rotary evaporation. The solute was separated and purified by column chromatography (mobile phase: petroleum ether : ethyl acetate = 1 : 1) to obtain compound A221-2 (60 mg, yield 18%).

### Example 2:

### Synthesis of compound A53

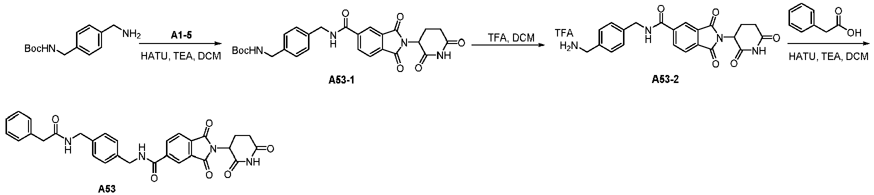

### Synthesis of compound A53-1

Compound A1-5 (100 mg, 0.33 mmol) and the compound 1-(*N*-Boc-aminomethyl)-4-(aminomethyl)benzene (85 mg, 0.36 mmol) were dissolved in dichloromethane (4 mL), and HATU (188 mg, 0.49 mmol) and triethylamine (67 mg, 0.66 mmol) were added. The mixture was reacted at room temperature for 2 h, and then monitoring by TLC showed that the raw materials were completely reacted. The mixture was diluted with water (20 ml) and extracted with dichloromethane (20 mL*3). The organic phases were combined, washed with saturated brine (15 mL), dried over anhydrous sodium sulfate and concentrated. The solute was purified by a preparative plate (developing solvent: DCM : MeOH = 15 : 1) to obtain compound A53-1 (100 mg, yield 58%). ESI-MS(M+H)⁺: 521.2.

### Synthesis of compound A53-2

Compound A53-1 (100 mg, 0.19 mmol) was dissolved in dichloromethane (4 mL), and trifluoroacetic acid (1.5 mL) was added with stirring. The mixture was reacted at room temperature for 2 h, and then monitoring by TLC showed that the raw materials were completely reacted. The solvent was removed by drying to obtain compound A53-2 (180 mg, crude) as an oil, which was directly used in the next reaction. ESI-MS(M+H)⁺: 421.2.

### Synthesis of compound A53

Compound A53-2 (180 mg, 0.19 mmol) and phenylacetic acid (25 mg, 0.18 mmol) were dissolved in dichloromethane (2 mL), and HATU (84 mg, 0.22 mmol) and triethylamine (56 uL, 0.4 mmol) were added. The mixture was reacted at room temperature for 2 h, and then monitoring by TLC showed that the raw materials were completely reacted. Water (20 ml) was added, and the mixture was extracted with dichloromethane (20 mL*3). The organic phases were combined, washed with saturated brine (25 ml), dried over anhydrous sodium sulfate and concentrated. The solute was purified by a preparative plate (developing solvent: DCM : MeOH = 10 : 1), and then purified by medium-pressure preparative chromatography to obtain compound A53 (30 mg, yield 55%). ESI-MS(M+H)⁺: 539.2.

¹H NMR (400 MHz, DMSO) δ 11.15 (s, 1H), 9.42 (t, *J* = 5.8 Hz, 1H), 8.52 (t, *J* = 5.8 Hz, 1H), 8.41 - 8.29 (m, 2H), 8.03 (d, *J* = 7.8 Hz, 1H), 7.33 - 7.24 (m, 6H), 7.24 - 7.14 (m, 3H), 5.19 (dd, *J* = 12.9, 5.4 Hz, 1H), 4.48 (d, *J* = 5.7 Hz, 2H), 4.24 (d, *J* = 5.8 Hz, 2H), 3.45 (s, 2H), 2.96 - 2.85 (m, 1H), 2.67 - 2.51 (m, 2H), 2.13 - 2.06 (m, 1H).

The synthesis methods for compounds A26, A54-A70, A103-A104, A107-A108, A111-A112, A115-A118, A121-A122, A124-A125, A128-A129, A131-A138, A141-A142, A145-A146, A149-A150, A152-A155, A157-A164, A200, A202, A203, , A207, A209, A212, A213, A215-A217, A222, A223, A229 and A236 are the same as that for A53.

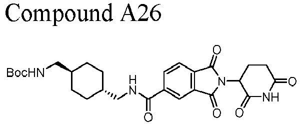

ESI-MS(M+H)⁺: 527.2.

¹H NMR (400 MHz, DMSO) δ 11.15 (s, 1H), 8.85 (t, *J* = 5.6 Hz, 1H), 8.38 - 8.25 (m, 2H), 8.02 (d, *J* = 7.8 Hz, 1H), 6.80 (t, *J* = 5.7 Hz, 1H), 5.19 (dd, *J* = 12.9, 5.3 Hz, 1H), 3.14 (t, *J* = 6.2 Hz, 2H), 2.98 - 2.83 (m, 1H), 2.76 (t, *J* = 6.2 Hz, 2H), 2.65 - 2.52 (m, 2H), 2.14 - 2.04 (m, 1H), 1.73 (dd, *J* = 28.6, 12.1 Hz, 4H), 1.57 - 1.45 (m, 1H), 1.36 (s, 9H), 1.33 - 1.25 (m, 1H), 0.97 - 0.76 (m, 4H).

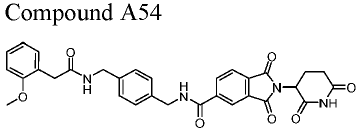

ESI-MS(M+H)⁺: 569.2.

¹H NMR (400 MHz, DMSO) δ 11.14 (s, 1H), 9.43 (t, *J* = 5.8 Hz, 1H), 8.39 - 8.34 (m, 2H), 8.28 (t, *J* = 5.9 Hz, 1H), 8.04 (d, *J* = 7.8 Hz, 1H), 7.30 (d, *J* = 8.1 Hz, 2H), 7.23 - 7.14 (m, 4H), 6.94 (d, *J* = 8.1 Hz, 1H), 6.86 (t, *J* = 7.4 Hz, 1H), 5.19 (dd, *J* = 12.9, 5.4 Hz, 1H), 4.49 (d, *J* = 5.8 Hz, 2H), 4.25 (d, *J* = 5.9 Hz, 2H), 3.74 (s, 3H), 3.44 (s, 2H), 2.95 - 2.85 (m, 1H), 2.67 - 2.52 (m, 2H), 2.13 - 2.05 (m, 1H).

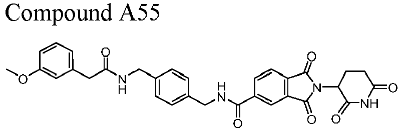

ESI-MS(M+H)⁺: 569.2.

¹H NMR (400 MHz, DMSO) δ 11.14 (s, 1H), 9.42 (t, *J* = 5.8 Hz, 1H), 8.50 (t, *J* = 5.8 Hz, 1H), 8.38 - 8.34 (m, 2H), 8.04 (d, *J* = 7.8 Hz, 1H), 7.28 (d, *J* = 8.0 Hz, 2H), 7.24 - 7.13 (m, 3H), 6.84 - 6.76 (m, 3H), 5.19 (dd, *J* = 12.9, 5.4 Hz, 1H), 4.48 (d, *J* = 5.8 Hz, 2H), 4.24 (d, *J* = 5.8 Hz, 2H), 3.71 (s, 3H), 3.42 (s, 2H), 2.94 - 2.85 (m, 1H), 2.66 - 2.51 (m, 2H), 2.13 - 2.07 (m, 1H).

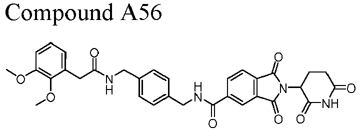

ESI-MS(M+H)⁺: 599.2.

¹H NMR (400 MHz, DMSO) δ 11.14 (s, 1H), 9.43 (t, *J* = 5.9 Hz, 1H), 8.44 - 8.32 (m, 3H), 8.04 (d, *J* = 7.8 Hz, 1H), 7.30 (d, *J* = 8.1 Hz, 2H), 7.26 - 7.19 (m, 2H), 7.01 - 6.89 (m, 2H), 6.80 (dd, *J* = 7.5, 1.6 Hz, 1H), 5.19 (dd, *J* = 12.9, 5.4 Hz, 1H), 4.49 (d, *J* = 5.8 Hz, 2H), 4.25 (d, *J* = 5.9 Hz, 2H), 3.77 (s, 3H), 3.66 (s, 3H), 3.46 (s, 2H), 2.94 - 2.85 (m, 1H), 2.68 - 2.53 (m, 2H), 2.16 - 2.02 (m, 1H).

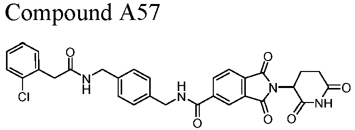

ESI-MS(M+H)⁺: 573.1.

¹H NMR (400 MHz, DMSO) δ 11.14 (s, 1H), 9.43 (t, *J* = 5.8 Hz, 1H), 8.52 (t, *J* = 5.8 Hz, 1H), 8.39 - 8.34 (m, 2H), 8.04 (d, *J* = 7.8 Hz, 1H), 7.43 - 7.39 (m, 1H), 7.37 - 7.35 (m, 1H), 7.32 - 7.20 (m, 6H), 5.19 (dd, *J* = 12.8, 5.4 Hz, 1H), 4.49 (d, *J* = 5.7 Hz, 2H), 4.27 (d, *J* = 5.8 Hz, 2H), 3.64 (s, 2H), 2.94 - 2.85 (m, 1H), 2.69 - 2.52 (m, 2H), 2.14 - 2.05 (m, 1H).

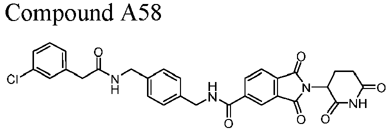

ESI-MS(M+H)⁺: 573.1.

¹H NMR (400 MHz, DMSO) δ 11.14 (s, 1H), 9.43 (t, *J* = 5.9 Hz, 1H), 8.56 (t, *J* = 5.8 Hz, 1H), 8.41 - 8.31 (m, 2H), 8.04 (d, *J* = 7.8 Hz, 1H), 7.35 - 7.27 (m, 5H), 7.24 - 7.10 (m, 3H), 5.19 (dd, *J* = 12.9, 5.4 Hz, 1H), 4.49 (d, *J* = 5.8 Hz, 2H), 4.24 (d, *J* = 5.8 Hz, 2H), 3.48 (s, 2H), 2.96 - 2.84 (m, 1H), 2.66 - 2.52 (m, 2H), 2.13 - 2.04 (m, 1H).

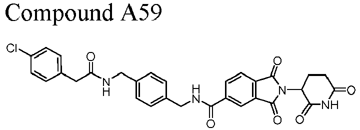

ESI-MS(M+H)⁺: 573.1.

¹H NMR (400 MHz, DMSO) δ 11.14 (s, 1H), 9.42 (t, *J* = 5.9 Hz, 1H), 8.54 (t, *J* = 5.7 Hz, 1H), 8.41 - 8.31 (m, 2H), 8.03 (d, *J* = 7.8 Hz, 1H), 7.37 - 7.32 (m, 2H), 7.31 - 7.24 (m, 4H), 7.19 (d, *J* = 8.1 Hz, 2H), 5.19 (dd, *J* = 12.8, 5.4 Hz, 1H), 4.48 (d, *J* = 5.8 Hz, 2H), 4.23 (d, *J* = 5.8 Hz, 2H), 3.46 (s, 2H), 2.95 - 2.84 (m, 1H), 2.67 - 2.52 (m, 2H), 2.14 - 2.05 (m, 1H).

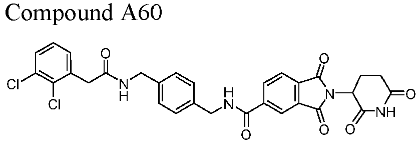

ESI-MS(M+H)⁺: 607.1.

¹H NMR (400 MHz, DMSO) δ 11.14 (s, 1H), 9.43 (t, *J* = 5.8 Hz, 1H), 8.56 (t, *J* = 5.8 Hz, 1H), 8.42 - 8.33 (m, 2H), 8.04 (d, *J* = 7.8 Hz, 1H), 7.53 (dd, *J* = 7.8, 1.6 Hz, 1H), 7.36 - 7.18 (m, 6H), 5.19 (dd, *J* = 12.8, 5.4 Hz, 1H), 4.47 (d, *J* = 5.6 Hz, 2H), 4.27 (d, *J* = 5.8 Hz, 2H), 3.71 (s, 2H), 2.97 - 2.82 (m, 1H), 2.68 - 2.53 (m, 2H), 2.13 - 2.03 (m, 1H).

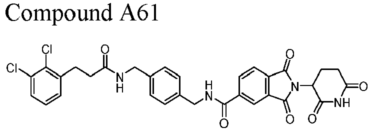

ESI-MS(M+H)⁺: 621.1.

¹H NMR (400 MHz, DMSO) δ 11.14 (s, 1H), 9.43 (t, *J* = 5.8 Hz, 1H), 8.43 - 8.31 (m, 3H), 8.04 (d, *J* = 7.8 Hz, 1H), 7.47 (dd, *J* = 10.0, 5.0 Hz, 1H), 7.33 - 7.21 (m, 4H), 7.12 (d, *J* = 8.1 Hz, 2H), 5.19 (dd, *J* = 12.9, 5.4 Hz, 1H), 4.48 (d, *J* = 5.8 Hz, 2H), 4.22 (d, *J* = 5.8 Hz, 2H), 3.00 (t, *J* = 7.6 Hz, 2H), 2.94 - 2.85 (m, 1H), 2.65 - 2.52 (m, 2H), 2.49 - 2.44 (m, 2H), 2.12 - 2.06 (m, 1H).

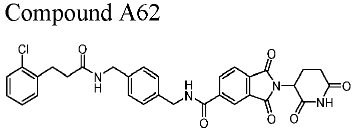

ESI-MS(M+H)⁺: 587.2.

¹H NMR (400 MHz, DMSO) δ 11.14 (s, 1H), 9.43 (t, *J* = 5.8 Hz, 1H), 8.42 - 8.30 (m, 3H), 8.04 (d, *J* = 7.8 Hz, 1H), 7.42 - 7.37 (m, 1H), 7.32 - 7.20 (m, 5H), 7.14 (d, *J* = 8.1 Hz, 2H), 5.19 (dd, *J* = 12.8, 5.4 Hz, 1H), 4.48 (d, *J* = 5.6 Hz, 2H), 4.22 (d, *J* = 5.8 Hz, 2H), 2.96 - 2.84 (m, 3H), 2.69 - 2.53 (m, 2H), 2.47 - 2.42 (m, 2H), 2.12 - 2.04 (m, 1H).

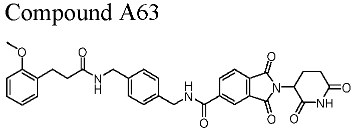

ESI-MS(M+H)⁺: 583.2.

¹H NMR (400 MHz, DMSO) δ 11.14 (s, 1H), 9.43 (t, *J* = 5.9 Hz, 1H), 8.42 - 8.32 (m, 2H), 8.27 (t, *J* = 5.9 Hz, 1H), 8.04 (d, *J* = 7.8 Hz, 1H), 7.27 (d, *J* = 8.1 Hz, 2H), 7.20 - 7.08 (m, 4H), 6.93 (d, *J* = 8.0 Hz, 1H), 6.83 (t, *J* = 7.1 Hz, 1H), 5.19 (dd, *J* = 12.9, 5.4 Hz, 1H), 4.48 (d, *J* = 5.7 Hz, 2H), 4.21 (d, *J* = 5.6 Hz, 2H), 3.77 (s, 3H), 2.94 - 2.85 (m, 1H), 2.83 - 2.76 (m, 2H), 2.66 - 2.52 (m, 2H), 2.41 - 2.34 (m, 2H), 2.13 - 2.06 (m, 1H).

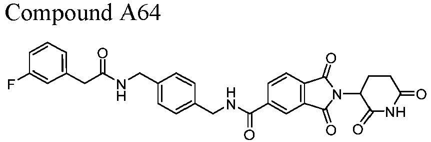

ESI-MS(M+H)⁺: 557.2.

¹H NMR (400 MHz, DMSO) δ 11.15 (s, 1H), 9.43 (t, *J* = 5.9 Hz, 1H), 8.55 (t, *J* = 5.8 Hz, 1H), 8.43 - 8.30 (m, 2H), 8.03 (d, *J* = 7.8 Hz, 1H), 7.37 - 7.25 (m, 4H), 7.24 - 7.19 (m, 2H), 7.16 - 7.10 (m, 2H), 5.19 (dd, *J=* 12.9, 5.4 Hz, 1H), 4.49 (d, *J=* 5.8 Hz, 2H), 4.26 (d, *J=* 5.9 Hz, 2H), 3.53 (s, 2H), 2.96 - 2.83 (m, 1H), 2.66 - 2.52 (m, 2H), 2.13 - 2.07 (m, 1H).

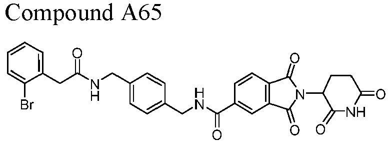

ESI-MS(M+H)⁺: 617.1.

¹H NMR (400 MHz, DMSO) δ 11.14 (s, 1H), 9.43 (t, *J=* 5.8 Hz, 1H), 8.52 (t, *J* = 5.8 Hz, 1H), 8.39 - 8.35 (m, 2H), 8.04 (d, *J=* 7.8 Hz, 1H), 7.43 - 7.33 (m, 2H), 7.33 - 7.21 (m, 6H), 5.19 (dd, *J=* 12.8, 5.4 Hz, 1H), 4.49 (d, *J=* 5.7 Hz, 2H), 4.27 (d, *J=* 5.8 Hz, 2H), 3.64 (s, 2H), 2.96 - 2.85 (m, 1H), 2.69 - 2.52 (m, 2H), 2.13 - 2.03 (m, 1H).

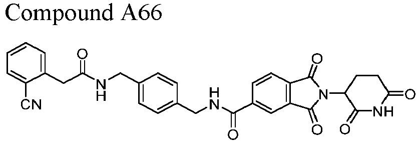

ESI-MS(M+H)⁺: 564.2.

¹H NMR (400 MHz, DMSO) δ 11.14 (s, 1H), 9.43 (t, *J=* 5.8 Hz, 1H), 8.66 (t, *J* = 5.8 Hz, 1H), 8.38 - 8.34 (m, 2H), 8.03 (d, *J* = 7.8 Hz, 1H), 7.84 - 7.74 (m, 1H), 7.64 (td, *J* = 7.7, 1.2 Hz, 1H), 7.52 - 7.40 (m, 2H), 7.29 (d, *J* = 8.1 Hz, 2H), 7.24 (d, *J* = 8.1 Hz, 2H), 5.18 (dd, *J* = 12.8, 5.4 Hz, 1H), 4.49 (d, *J* = 5.7 Hz, 2H), 4.27 (d, *J* = 5.8 Hz, 2H), 3.74 (s, 2H), 2.95 - 2.82 (m, 1H), 2.69 - 2.52 (m, 2H), 2.15 - 2.04 (m, 1H).

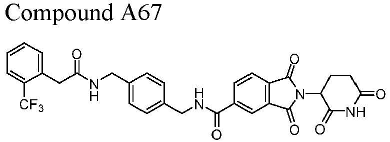

ESI-MS(M+H)⁺: 607.2.

¹H NMR (400 MHz, DMSO) δ 11.15 (s, 1H), 9.43 (t, *J* = 5.9 Hz, 1H), 8.53 (t, *J* = 5.9 Hz, 1H), 8.44 - 8.30 (m, 2H), 8.03 (d, *J* = 7.8 Hz, 1H), 7.67 (d, *J* = 7.8 Hz, 1H), 7.60 (t, *J* = 7.5 Hz, 1H), 7.45 (t, *J* = 8.4 Hz, 2H), 7.30 (d, *J* = 8.1 Hz, 2H), 7.22 (d, *J* = 8.1 Hz, 2H), 5.19 (dd, *J* = 12.8, 5.4 Hz, 1H), 4.49 (d, *J* = 5.8 Hz, 2H), 4.26 (d, *J* = 5.8 Hz, 2H), 3.71 (s, 2H), 2.94 - 2.83 (m, 1H), 2.67 - 2.51 (m, 2H), 2.13 - 2.07 (m, 1H).

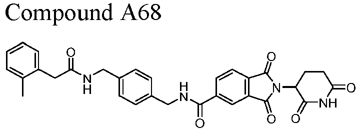

ESI-MS(M+H)⁺: 553.2.

¹H NMR (400 MHz, DMSO) δ 11.14 (s, 1H), 9.43 (t, *J* = 5.8 Hz, 1H), 8.44 (t, *J* = 5.8 Hz, 1H), 8.40 - 8.30 (m, 2H), 8.03 (d, *J* = 7.7 Hz, 1H), 7.29 (d, *J* = 8.0 Hz, 2H), 7.24 - 7.15 (m, 3H), 7.15 - 7.05 (m, 3H), 5.19 (dd, *J* = 12.8, 5.4 Hz, 1H), 4.48 (d, *J* = 5.7 Hz, 2H), 4.24 (d, *J* = 5.8 Hz, 2H), 3.48 (s, 2H), 2.97 - 2.82 (m, 1H), 2.69 - 2.53 (m, 2H), 2.24 (s, 3H), 2.15 - 2.04 (m, 1H).

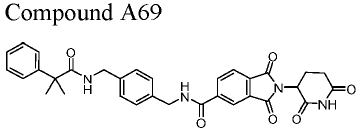

ESI-MS(M+H)⁺: 567.2.

¹H NMR (400 MHz, DMSO) δ 11.15 (s, 1H), 9.42 (t, *J* = 5.8 Hz, 1H), 8.39 - 8.34 (m, 2H), 8.04 (d, *J* = 7.8 Hz, 1H), 7.88 (t, *J* = 6.0 Hz, 1H), 7.35 - 7.28 (m, 4H), 7.27 - 7.16 (m, 3H), 7.09 (d, *J* = 8.0 Hz, 2H), 5.20 (dd, *J* = 12.9, 5.3 Hz, 1H), 4.48 (d, *J* = 5.7 Hz, 2H), 4.21 (d, *J* = 5.9 Hz, 2H), 2.95 - 2.85 (m, 1H), 2.67 - 2.52 (m, 2H), 2.14 - 2.07 (m, 1H), 1.47 (s, 6H).

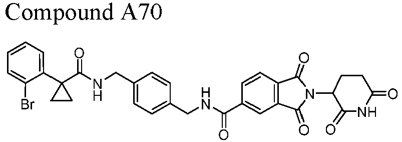

ESI-MS(M+H)⁺: 643.2.

¹H NMR (400 MHz, DMSO) δ 11.14 (s, 1H), 9.40 (t, *J* = 5.8 Hz, 1H), 8.40 - 8.30 (m, 2H), 8.03 (d, *J* = 7.8 Hz, 1H), 7.63 (dd, *J* = 7.9, 1.1 Hz, 1H), 7.45 (dd, *J* = 7.6, 1.7 Hz, 1H), 7.39 - 7.35 (m, 1H), 7.27 - 7.23 (m, 4H), 7.16 (d, *J* = 8.1 Hz, 2H), 5.19 (dd, *J* = 12.9, 5.4 Hz, 1H), 4.47 (d, *J* = 5.7 Hz, 2H), 4.17 (d, *J* = 6.0 Hz, 2H), 2.94 - 2.84 (m, 1H), 2.66 - 2.52 (m, 2H), 2.14 - 2.02 (m, 1H), 1.54 - 1.47 (m, 2H), 1.02 - 0.95 (m, 2H).

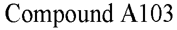

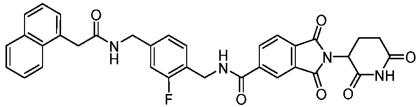

ESI-MS(M+H)⁺: 607.2.

¹H NMR (400 MHz, DMSO) δ 11.15 (s, 1H), 9.40 (t, *J* = 5.7 Hz, 1H), 8.66 (t, *J* = 5.9 Hz, 1H), 8.42 - 8.31 (m, 2H), 8.13 - 8.06 (m, 1H), 8.04 (d, *J* = 7.8 Hz, 1H), 7.96 - 7.87 (m, 1H), 7.85 - 7.78 (m, 1H), 7.56 - 7.47 (m, 2H), 7.44 (d, *J* = 8.4 Hz, 2H), 7.33 (t, *J* = 8.0 Hz, 1H), 7.03 (t, *J* = 8.0 Hz, 2H), 5.20 (dd, *J* = 12.9, 5.4 Hz, 1H), 4.51 (d, *J* = 5.5 Hz, 2H), 4.28 (d, *J* = 5.9 Hz, 2H), 3.97 (s, 2H), 2.95 - 2.85 (m, 1H), 2.67 - 2.53 (m, 2H), 2.15 - 2.02 (m, 1H).

The corresponding intermediates were prepared as follows:

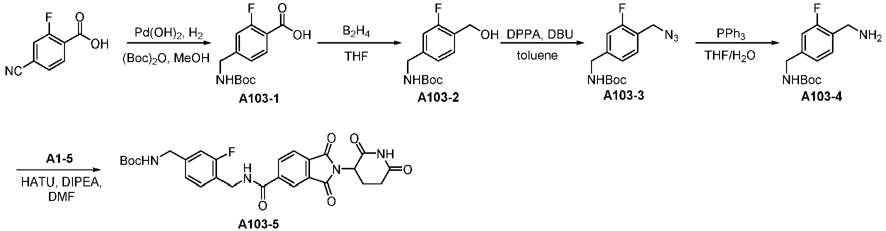

### Synthesis of compound A103-1

4-cyano-2-fluorobenzoic acid (5.0 g, 30.3 mmol) was dissolved in MeOH (100 ml), and (Boc)₂O (9.9 g, 45.5 mmol) and Pd (OH)₂ (1.5 g, 5% in carbon) were added. The mixture was stirred at room temperature under hydrogen atmosphere for 3 h, and then TLC showed that the reaction was completed. The reaction liquid was filtered, and then the filter cake was rinsed with methanol. The filtrate was dried by rotary evaporation. The solute was separated by column chromatography (mobile phase: MeOH : DCM = 1 : 9) to obtain compound A103-1 (2.98 g, yield 37%).

### Synthesis of compound A103-2

A103-1 (2.0 g, 11.8 mmol) was dissolved in 20 mL of anhydrous tetrahydrofuran. Under ice bath, borane/THF solution (35.5 mL, 1 M, 35.5 mmol) was added dropwise. The mixture was reacted at 0°C for 1 h, and then TLC showed that the reaction was completed. The reaction was quenched with ice water (20 mL). The aqueous phase was extracted with ethyl acetate (20 mL*3). The organic phases were combined and dried by rotary evaporation. The solute was separated by column chromatography (mobile phase: MeOH : DCM = 1 : 20) to obtain compound A103-2 (1.10 g, yield 36%).

### Synthesis of compound A103-3

A103-2 (1.10 g, 4.31 mmol) was dissolved in 20 mL of toluene, and DPPA (1.86 g, 6.77 mmol) and DBU (1.04 g, 6.85 mmol) were added. The mixture was reacted at 60°C for 2 h, and then TLC showed that the reaction was completed. The mixture was cooled to room temperature and diluted with ethyl acetate (30 mL). The organic phase was washed with 0.5 M hydrochloric acid (10 mL) and then dried by rotary evaporation. The solute was separated by column chromatography (mobile phase: petroleum ether : ethyl acetate = 3 : 1) to obtain compound A103-3 (620 mg, yield 51%).

### Synthesis of compound A103-4

A103-3 (570 mg, 2.03 mmol) was dissolved in 18 mL of THF/H₂O (8 : 1), and triphenylphosphine (680 mg, 2.23 mmol) was added. The mixture was reacted at 60°C for 2 h, and then TLC showed that the reaction was completed. The mixture was cooled to room temperature and diluted with ethyl acetate (50 mL). The organic phase was washed with saturated brine (10 mL) and then dried by rotary evaporation. The solute was separated by column chromatography (mobile phase: petroleum ether : ethyl acetate = 1 : 1) to obtain compound A103-4 (360 mg, yield 70%).

### Synthesis of compound A103-5

A1-5 (430 mg, 1.42 mmol) was dissolved in 5 mL of DMF, and HATU (650 mg, 1.70 mmol) was added. The mixture was reacted at room temperature for 30 min, and then A103-4 (360 mg, 1.42 mmol) and DIPEA (275 mg, 2.13 mmol) were added. The mixture was reacted at room temperature for 1 h, and then TLC showed that the reaction was completed. The mixture was diluted with ethyl acetate (30 mL). The organic phase was washed with saturated brine (10 mL*3) and then dried by rotary evaporation. The solute was separated by column chromatography (mobile phase: petroleum ether : ethyl acetate = 1 : 1) to obtain compound A103-5 (490 mg, yield 64%). ¹H NMR (400 MHz, DMSO) δ 11.15 (s, 1H), 9.40 (t, *J* = 5.2 Hz, 1H), 8.43 - 8.30 (m, 2H), 8.03 (d, *J* = 7.7 Hz, 1H), 7.40 (d, *J* = 18.8 Hz, 1H), 7.35 (t, *J* = 8.1 Hz, 1H), 7.09 - 6.98 (m, 2H), 5.18 (dd, *J* = 12.8, 5.3 Hz, 1H), 4.52 (d, *J* = 5.4 Hz, 2H), 4.12 (d, *J* = 5.6 Hz, 2H), 2.89 (t, *J* = 12.9 Hz, 1H), 2.68 - 2.53 (m, 2H), 2.14 - 2.05 (m, 1H), 1.38 (s, 9H).

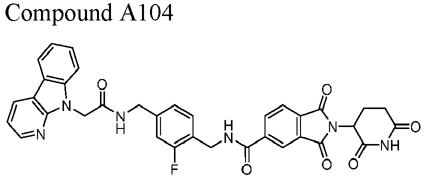

ESI-MS(M+H)⁺: 647.2.

¹H NMR (400 MHz, DMSO) δ 11.14 (s, 1H), 9.41 (t, *J* = 5.6 Hz, 1H), 8.78 (t, *J* = 6.0 Hz, 1H), 8.54 (dd, *J* = 7.6, 1.6 Hz, 1H), 8.45 (dd, *J* = 4.8, 1.6 Hz, 1H), 8.42 - 8.33 (m, 2H), 8.21 (d, *J* = 7.7 Hz, 1H), 8.04 (d, *J* = 7.8 Hz, 1H), 7.59 (d, *J* = 8.2 Hz, 1H), 7.53 - 7.45 (m, 1H), 7.36 (t, *J* = 7.9 Hz, 1H), 7.31 - 7.22 (m, 2H), 7.11 (dd, *J* = 13.8, 9.6 Hz, 2H), 5.28 - 5.08 (m, 3H), 4.53 (d, *J* = 5.5 Hz, 2H), 4.30 (d, *J* = 5.9 Hz, 2H), 2.95 - 2.83 (m, 1H), 2.64 - 2.52 (m, 2H), 2.15 - 2.04 (m, 1H).

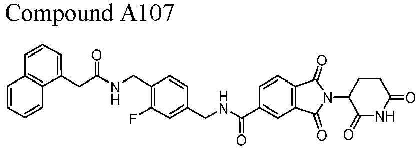

ESI-MS(M+H)⁺: 607.2.

¹H NMR (400 MHz, DMSO) δ 11.15 (s, 1H), 9.45 (t, *J* = 5.9 Hz, 1H), 8.63 (t, *J* = 5.8 Hz, 1H), 8.44 - 8.32 (m, 2H), 8.12 - 8.03 (m, 2H), 7.93 - 7.88 (m, 1H), 7.84 - 7.77 (m, 1H), 7.54 - 7.40 (m, 4H), 7.24 (t, *J* = 7.9 Hz, 1H), 7.12 (dd, *J* = 13.2, 9.5 Hz, 2H), 5.20 (dd, *J* = 12.9, 5.4 Hz, 1H), 4.49 (d, *J* = 5.7 Hz, 2H), 4.29 (d, *J* = 5.7 Hz, 2H), 3.95 (s, 2H), 2.96 - 2.84 (m, 1H), 2.68 - 2.52 (m, 2H), 2.13 - 2.06 (m, 1H).

The corresponding intermediates were prepared as follows:

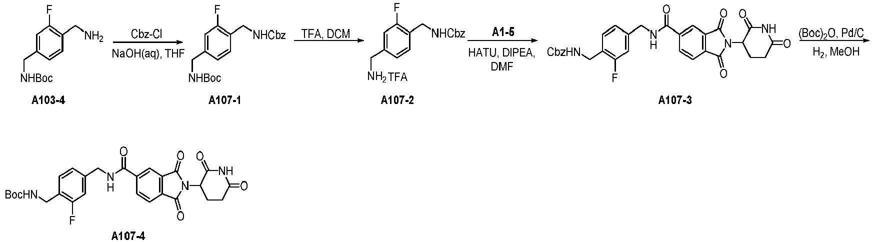

### Synthesis of compound A107-1

Compound A103-4 (1.30 g, 5.12 mmol) was dissolved in 25 mL of tetrahydrofuran, and aqueous sodium hydroxide solution (2 M, 5.2 mL) was added. Under ice bath, Cbz-Cl (960 mg, 5.63 mmol) was added dropwise. The mixture was reacted at room temperature for 3 h, and then TLC showed that the reaction was completed. The mixture was diluted with water (20 mL). The aqueous phase was extracted with ethyl acetate (10 mL*3). The organic phases were combined and dried by rotary evaporation to obtain compound A107-1 (1.30 g, yield 65%).

### Synthesis of compound A107-2

Compound A107-1 (1.30 g, 3.35 mmol) was dissolved in 10 mL of dichloromethane, and 5 mL of trifluoroacetic acid was added. The mixture was reacted at room temperature for 1 h, and then TLC showed that the reaction was completed. The solvent was dried by rotary evaporation to obtain compound A107-2 (1.5 g, crude).

### Synthesis of compound A107-3

Compound A107-2 (1.90 g, crude, 3.35 mmol), compound A1-5 (1.13 g, 3.74 mmol) and DIPEA (2.42 g, 18.7 mmol) were dissolved in 20 mL of DMF, and HATU (1.43 g, 3.75 mmol) was added. The mixture was reacted at room temperature for 1 h, and then TLC showed that the reaction was completed. The mixture was diluted with ethyl acetate (100 mL). The organic phase was washed with saturated brine (20 mL*3) and then dried by rotary evaporation. The solute was separated by column chromatography (mobile phase: petroleum ether : ethyl acetate = 1 : 1) to obtain compound A107-3 (1.22 g, yield 68%).

### Synthesis of compound A107-4

A107-3 (1.20 g, 2.11 mmol) was dissolved in 20 mL of methanol, and (Boc)₂O (690 mg, 3.15 mmol) and Pd/C (200 mg) were added. The mixture was reacted at room temperature under hydrogen atmosphere for 2 h, and then TLC showed that the reaction was completed. The reaction liquid was filtered, and the filtrate was dried by rotary evaporation. The solute was separated by column chromatography (mobile phase: MeOH : DCM = 1 : 10) to obtain compound A107-4 (500 mg, yield 48%). ¹H NMR (400 MHz, DMSO) δ 11.11 (s, 1H), 9.46 (t, *J* = 5.7 Hz, 1H), 8.42 - 8.31 (m, 2H), 8.04 (d, *J* = 7.8 Hz, 1H), 7.37 (s, 1H), 7.25 (t, *J* = 7.7 Hz, 1H), 7.13 (t, *J* = 10.0 Hz, 2H), 5.19 (dd, *J* = 12.8, 5.4 Hz, 1H), 4.50 (d, *J* = 5.6 Hz, 2H), 4.13 (d, *J* = 6.0 Hz, 2H), 2.96 - 2.83 (m, 1H), 2.68 - 2.52 (m, 2H), 2.12 - 2.04 (m, 1H), 1.38 (s, 9H).

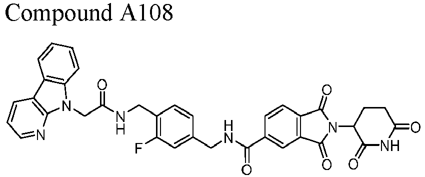

ESI-MS(M+H)⁺: 647.2.

¹H NMR (400 MHz, DMSO) δ 11.15 (s, 1H), 9.46 (t, *J* = 5.9 Hz, 1H), 8.75 (t, *J* = 5.8 Hz, 1H), 8.54 (dd, *J* = 7.6, 1.6 Hz, 1H), 8.44 (dd, *J* = 4.8, 1.6 Hz, 1H), 8.42 - 8.33 (m, 2H), 8.20 (d, *J* = 7.7 Hz, 1H), 8.05 (d, *J* = 7.8 Hz, 1H), 7.56 (d, *J* = 8.2 Hz, 1H), 7.51 - 7.44 (m, 1H), 7.35 (t, *J* = 8.0 Hz, 1H), 7.30 - 7.22 (m, 2H), 7.18 - 7.13 (m, 2H), 5.23 - 5.10 (m, 3H), 4.51 (d, *J* = 5.8 Hz, 2H), 4.31 (d, *J* = 5.7 Hz, 2H), 2.95 - 2.85 (m, 1H), 2.69 - 2.53 (m, 2H), 2.14 - 2.07 (m, 1H).

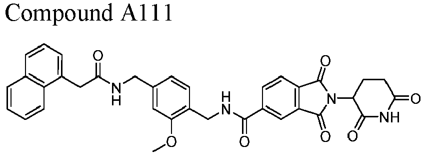

ESI-MS(M+H)⁺: 619.2.

¹H NMR (400 MHz, DMSO) δ 11.15 (s, 1H), 9.23 (t, *J* = 5.7 Hz, 1H), 8.63 (t, *J* = 5.9 Hz, 1H), 8.43 - 8.33 (m, 2H), 8.18 - 8.10 (m, 1H), 8.04 (d, *J* = 7.8 Hz, 1H), 7.96 - 7.89 (m, 1H), 7.85 - 7.79 (m, 1H), 7.55 - 7.41 (m, 4H), 7.14 (d, *J* = 7.5 Hz, 1H), 6.80 - 6.77 (m, 2H), 5.20 (dd, *J* = 12.9, 5.4 Hz, 1H), 4.44 (d, *J* = 5.6 Hz, 2H), 4.28 (d, *J* = 5.9 Hz, 2H), 3.97 (s, 2H), 3.66 (s, 3H), 2.97 - 2.84 (m, 1H), 2.70 - 2.53 (m, 2H), 2.15 - 2.08 (m, 1H).

The corresponding intermediates were prepared as follows:

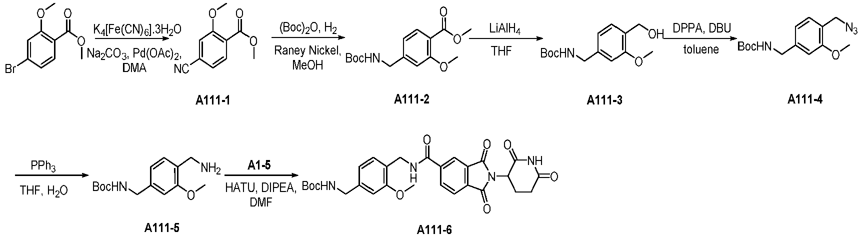

### Synthesis of compound A111-1

Methyl 4-bromo-2-methoxybenzoate (24 g, 98.0 mmol), K₄[Fe (CN)₆].3H₂O (29.4 g, 70.5 mmol), sodium carbonate (20.8 g, 196.0 mmol) and Pd (OAc)₂ (2.1 g, 9.79 mmol) were added to 250 mL of DMAC. The mixture was reacted overnight at 80°C under nitrogen atmosphere, and then TLC showed that the reaction was completed. The reaction liquid was filtered, and the filtrate was diluted with water (200 mL). The aqueous phase was then extracted with ethyl acetate (50 mL*3). The organic phases were combined, washed with saturated brine (20 mL*3) and then dried by rotary evaporation. The solute was separated by column chromatography (mobile phase: petroleum ether : ethyl acetate = 10 : 1) to obtain compound A111-1 (6.43 g, yield 36%).

### Synthesis of compound A111-2

Compound A111-1 (4.0 g, 11.9 mmol) was dissolved in 180 mL of methanol, and (Boc)₂O (4.0 g, 11.9 mmol) and Raney Ni (4.0 g) were added. The mixture was reacted overnight at room temperature under hydrogen atmosphere, and then TLC showed that the reaction was completed. The reaction liquid was filtered, and the filtrate was dried by rotary evaporation. The solute was separated by column chromatography (mobile phase: petroleum ether : ethyl acetate = 5 : 1) to obtain compound A111-2 (4.7 g, yield 58%).

### Synthesis of compound A111-3

Lithium aluminum hydride (1.08 g, 28.5 mmol) was dissolved in 30 mL of anhydrous tetrahydrofuran. Under ice bath, a solution of compound A111-2 (4.0 g, 13.6 mmol) in tetrahydrofuran (10 mL) was added dropwise. After the dropwise addition was completed, the mixture was reacted under ice bath for 1 h, and then TLC showed that the reaction was completed. The reaction was quenched by successively adding 1 mL of water, 1 mL of 15% aqueous NaOH solution and 3 mL of water. The reaction liquid was filtered, and the filtrate was dried by rotary evaporation to obtain compound A111-3 (3.39 g, yield 89%).

### Synthesis of compound A111-4

A111-3 (3.39 g, 12.7 mmol) was dissolved in 40 mL of toluene, and DPPA (7.3 g, 26.3 mmol) and DBU (4.0 g, 26.3 mmol) were added. The mixture was reacted overnight at 60°C, and then TLC showed that the reaction was completed. The mixture was diluted with water (50 mL). The aqueous phase was extracted with ethyl acetate (20 mL*2). The organic phases were combined and dried by rotary evaporation. The solute was separated by column chromatography (mobile phase: petroleum ether : ethyl acetate = 9 : 1) to obtain compound A111-4 (3.28 g, yield 88%).

### Synthesis of compound A111-5

A111-4 (1.64 g, 5.61 mmol) was dissolved in 45 mL of THF/H₂O (9 : 1), and triphenylphosphine (1.80 g, 6.17 mmol) was added. The mixture was reacted at 60°C for 3 h, and then TLC showed that the reaction was completed. The solvent was dried by rotary evaporation. The solute was separated by column chromatography (mobile phase: MeOH : DCM = 1 : 15) to obtain compound A111-5 (1.03 g, yield 69%).

### Synthesis of compound A111-6

A1-5 (1.17 g, 3.89 mmol) was dissolved in 30 mL of DMF, and HATU (1.77 g, 4.67 mmol) was added. The mixture was reacted at room temperature for 30 min, and then compound A111-5 (1.03 g, 3.89 mmol) and DIPEA (760 mg, 5.84 mmol) were added. The mixture was reacted at room temperature for 1 h, and then TLC showed that the reaction was completed. The mixture was diluted with water (50 mL). The aqueous phase was then extracted with ethyl acetate (20 mL*3). The organic phases were combined, washed with saturated brine (10 mL*3) and then dried by rotary evaporation. The solute was separated by column chromatography (mobile phase: petroleum ether : ethyl acetate = 1 : 3) to obtain compound A111-6 (1.60 g, yield 76%). ¹H NMR (400 MHz, DMSO) δ 11.15 (s, 1H), 9.24 (t, *J* = 5.6 Hz, 1H), 8.45 - 8.33 (m, 2H), 8.03 (d, *J* = 7.8 Hz, 1H), 7.36 (t, *J* = 6.0 Hz, 1H), 7.16 (d, *J* = 7.7 Hz, 1H), 6.89 (s, 1H), 6.78 (d, *J* = 7.6 Hz, 1H), 5.19 (dd, *J* = 12.9, 5.4 Hz, 1H), 4.45 (d, *J* = 5.4 Hz, 2H), 4.10 (d, *J* = 6.0 Hz, 2H), 3.81 (s, 3H), 2.98 - 2.83 (m, 1H), 2.65 - 2.57 (m, 2H), 2.15 - 2.04 (m, 1H), 1.38 (s, 9H).

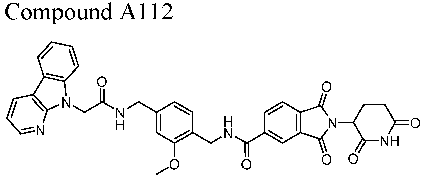

ESI-MS(M+H)⁺: 659.2.

¹H NMR (400 MHz, DMSO) δ 11.14 (s, 1H), 9.24 (t, *J* = 5.8 Hz, 1H), 8.73 (t, *J* = 5.9 Hz, 1H), 8.55 (dd, *J=* 7.6, 1.5 Hz, 1H), 8.45 - 8.39 (m, 2H), 8.37 (dd, *J=* 7.8, 1.4 Hz, 1H), 8.21 (d, *J* = 7.7 Hz, 1H), 8.04 (d, *J* = 7.8 Hz, 1H), 7.58 (d, *J* = 8.2 Hz, 1H), 7.49 (t, *J* = 7.7 Hz, 1H), 7.32 - 7.22 (m, 2H), 7.17 (d, *J* = 7.7 Hz, 1H), 6.89 (s, 1H), 6.84 (d, *J* = 7.6 Hz, 1H), 5.24 - 5.13 (m, 3H), 4.46 (d, *J=* 5.6 Hz, 2H), 4.29 (d, *J=* 5.8 Hz, 2H), 3.79 (s, 3H), 2.96 - 2.82 (m, 1H), 2.68 - 2.52 (m, 2H), 2.15 - 2.02 (m, 1H).

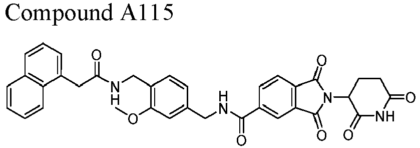

ESI-MS(M+H)⁺: 619.2.

¹H NMR (400 MHz, DMSO) δ 11.14 (s, 1H), 9.41 (t, *J=* 5.8 Hz, 1H), 8.43 - 8.31 (m, 3H), 8.13 - 8.07 (m, 1H), 8.04 (d, *J* = 7.8 Hz, 1H), 7.94 - 7.89 (m, 1H), 7.85 - 7.76 (m, 1H), 7.55 - 7.46 (m, 2H), 7.46 - 7.40 (m, 2H), 7.08 (d, *J* = 7.7 Hz, 1H), 6.97 (s, 1H), 6.84 (d, *J* = 7.7 Hz, 1H), 5.19 (dd, *J* = 12.9, 5.4 Hz, 1H), 4.48 (d, *J* = 5.7 Hz, 2H), 4.21 (d, *J=* 5.7 Hz, 2H), 3.96 (s, 2H), 3.76 (s, 3H), 2.96 - 2.84 (m, 1H), 2.68 - 2.52 (m, 2H), 2.14 - 2.04 (m, 1H).

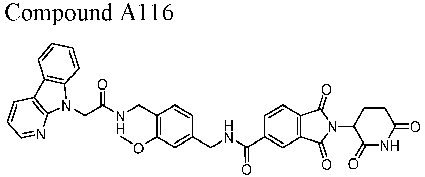

ESI-MS(M+H)⁺: 659.2.

¹H NMR (400 MHz, DMSO) δ 11.15 (s, 1H), 9.42 (t, *J* = 5.9 Hz, 1H), 8.56 - 8.51 (m, 2H), 8.45 (dd, *J=* 4.8, 1.6 Hz, 1H), 8.42 - 8.33 (m, 2H), 8.20 (d, *J* = 7.7 Hz, 1H), 8.05 (d, *J* = 7.9 Hz, 1H), 7.57 (d, *J* = 8.2 Hz, 1H), 7.54 - 7.46 (m, 1H), 7.31 - 7.23 (m, 2H), 7.20 (d, *J* = 7.7 Hz, 1H), 6.98 (s, 1H), 6.90 (d, *J* = 7.7 Hz, 1H), 5.23 - 5.13 (m, 3H), 4.50 (d, *J* = 5.7 Hz, 2H), 4.23 (d, *J* = 5.7 Hz, 2H), 3.77 (s, 3H), 2.97 - 2.83 (m, 1H), 2.70 - 2.52 (m, 2H), 2.13 - 2.03 (m, 1H).

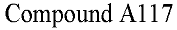

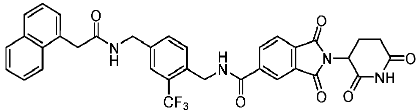

ESI-MS(M+H)⁺: 657.2.

¹H NMR (400 MHz, DMSO) δ 11.15 (s, 1H), 9.48 (t, *J* = 5.5 Hz, 1H), 8.72 (t, *J* = 6.1 Hz, 1H), 8.42 (s, 1H), 8.38 (dd, *J* = 7.8, 1.4 Hz, 1H), 8.10 - 8.04 (m, 2H), 7.93 - 7.88 (m, 1H), 7.85 - 7.78 (m, 1H), 7.58 (s, 1H), 7.53 - 7.42 (m, 6H), 5.20 (dd, *J* = 12.9, 5.4 Hz, 1H), 4.65 (d, *J* = 5.1 Hz, 2H), 4.34 (d, *J* = 5.9 Hz, 2H), 3.97 (s, 2H), 2.97 - 2.82 (m, 1H), 2.70 - 2.54 (m, 2H), 2.14 - 2.05 (m, 1H).

The corresponding intermediates were prepared as follows:

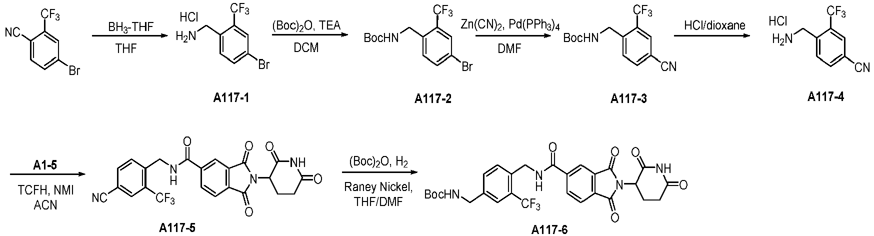

### Synthesis of compound A117-1

4-bromo-2-(trifluoromethyl)benzonitrile (2.5 g, 10.0 mmol) was dissolved in 10 mL of anhydrous tetrahydrofuran. Under ice bath, borane/THF solution (30 mL, 1 M, 30.0 mmol) was added dropwise. After the dropwise addition was completed, the mixture was warmed to 80°C and reacted for 3 h, and then TLC showed that the reaction was completed. The reaction was cooled to room temperature and then quenched by successively adding methanol (2 mL) and HCl/ethyl acetate (5 mL, 4 M) to the reaction liquid. The precipitated solid was filtered, and the filter cake was rinsed with ethyl acetate to obtain compound A117-1 (2.54 g, crude).

### Synthesis of compound A117-2

A117-1 (2.54 g, 10.0 mmol) was dissolved in 15 mL of dichloromethane, and triethylamine (2.03 g, 20.0 mmol) and (Boc)₂O (2.41 g, 11.0 mmol) were added. The mixture was reacted at room temperature for 1 h, and then TLC showed that the reaction was completed. The solvent was dried by rotary evaporation. The solute was separated by column chromatography (mobile phase: petroleum ether : ethyl acetate = 20 : 1) to obtain compound A117-2 (2.02 g, yield 57%).

### Synthesis of compound A117-3

Compound A117-2 (2.02 g, 5.70 mmol) was dissolved in 10 mL of DMF, and zinc cyanide (403 mg, 3.42 mmol) and tetrakis(triphenylphosphine)palladium (397 mg, 0.034 mmol) were successively added. The mixture was reacted overnight at 90°C under nitrogen protection, and then TLC showed that the reaction was completed. The reaction liquid was filtered, and the filtrate was diluted with 30 mL of water. The aqueous phase was extracted with ethyl acetate (10 mL*3). The organic phases were combined, washed with saturated brine (10 mL) and then dried by rotary evaporation. The solute was separated by column chromatography (mobile phase: petroleum ether : ethyl acetate = 6 : 1) to obtain compound A117-3 (1.36 g, yield 79%).

### Synthesis of compound A117-4

Compound A117-3 (600 mg, 2.00 mmol) was dissolved in HCl/dioxane (6 mL, 4 M). The mixture was reacted at room temperature for 1 h, and then TLC showed that the reaction was completed. The solvent was dried by rotary evaporation to obtain compound A117-4 (600 mg, crude).

### Synthesis of compound A117-5

Compound A117-4 (600 mg, 2.00 mmol), compound A1-5 (540 mg, 1.80 mmol) and N-methylimidazole (455 mg, 5.60 mmol) were dissolved in 5 mL of acetonitrile, and TCFH (602 mg, 2.17 mmol) was added. The mixture was reacted at room temperature for 1 h, and then TLC showed that the reaction was completed. 20 mL of water was added to the reaction liquid, and the precipitated solid was subjected to suction filtration. The filter cake was dried and then separated by column chromatography (mobile phase: dichloromethane : methanol = 20 : 1) to obtain compound A117-5 (770 mg, yield 88%).

### Synthesis of compound A117-6

Compound A117-5 (740 mg, 1.52 mmol) was dissolved in 10 mL of THF/DMF (1 : 1), and (Boc)₂O (830 mg, 3.81 mmol) and Raney Ni (1.0 g) were added. The mixture was reacted overnight at room temperature under hydrogen atmosphere, and then TLC showed that the reaction was completed. The reaction liquid was filtered, and the filtrate was dried by rotary evaporation. The solute was separated by column chromatography (mobile phase: dichloromethane : methanol = 20 : 1) to obtain compound A117-6 (463 mg, yield 59%). ¹H NMR (400 MHz, DMSO) δ 11.15 (s, 1H), 9.48 (t, *J* = 5.6 Hz, 1H), 8.49 - 8.35 (m, 2H), 8.06 (d, *J* = 7.8 Hz, 1H), 7.60 (s, 1H), 7.52 (dd, *J* = 15.6, 7.2 Hz, 3H), 5.20 (dd, *J* = 12.9, 5.4 Hz, 1H), 4.67 (d, *J* = 4.8 Hz, 2H), 4.18 (d, *J* = 6.0 Hz, 2H), 2.96 - 2.83 (m, 1H), 2.68 - 2.57 (m, 2H), 2.15 - 2.04 (m, 1H), 1.35 (s, 9H).

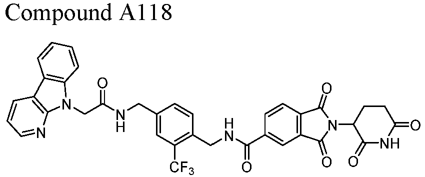

ESI-MS(M+H)⁺: 697.2.

¹H NMR (400 MHz, DMSO) δ 11.15 (s, 1H), 9.49 (t, *J* = 5.6 Hz, 1H), 8.82 (t, *J* = 5.9 Hz, 1H), 8.54 (dd, *J* = 7.6, 1.5 Hz, 1H), 8.46 - 8.40 (m, 2H), 8.38 (dd, *J* = 7.8, 1.3 Hz, 1H), 8.21 (d, *J* = 7.7 Hz, 1H), 8.07 (d, *J* = 7.8 Hz, 1H), 7.63 (s, 1H), 7.59 - 7.44 (m, 4H), 7.32 - 7.22 (m, 2H), 5.26 - 5.11 (m, 3H), 4.67 (d, *J=* 5.2 Hz, 2H), 4.36 (d, *J* = 5.9 Hz, 2H), 2.99 - 2.83 (m, 1H), 2.67 - 2.53 (m, 2H), 2.13 - 2.04 (m, 1H).

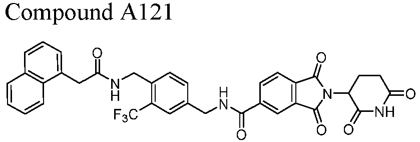

ESI-MS(M+H)⁺: 657.2.

¹H NMR (400 MHz, DMSO) δ 11.15 (s, 1H), 9.50 (t, *J=* 5.8 Hz, 1H), 8.68 (t, *J* = 5.7 Hz, 1H), 8.41 - 8.32 (m, 2H), 8.10 (d, *J* = 7.7 Hz, 1H), 8.05 (d, *J* = 7.8 Hz, 1H), 7.95 - 7.88 (m, 1H), 7.85 - 7.79 (m, 1H), 7.67 (s, 1H), 7.58 - 7.49 (m, 3H), 7.47 - 7.39 (m, 3H), 5.20 (dd, *J* = 12.9, 5.4 Hz, 1H), 4.55 (d, *J* = 5.7 Hz, 2H), 4.43 (d, *J* = 5.4 Hz, 2H), 4.01 (s, 2H), 2.96 - 2.83 (m, 1H), 2.68 - 2.52 (m, 2H), 2.14 - 2.04 (m, 1H).
The corresponding intermediates were prepared as follows:

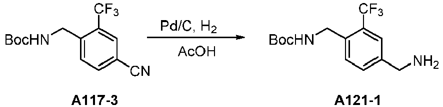

### Synthesis of compound A121-1

Compound A117-3 (600 mg, 1.92 mmol) was dissolved in 6 mL of glacial acetic acid, and Pd/C (115 mg, 5%) was added. The mixture was reacted at 55°C under hydrogen atmosphere for 1 h, and then TLC showed that the reaction was completed. The reaction liquid was filtered, and then the filtrate was dried by rotary evaporation. 50 mL of dichloromethane/methanol (5 : 1) was added. The organic phase was washed with saturated aqueous sodium bicarbonate solution (5 mL) and then dried by rotary evaporation. The solute was separated by column chromatography (mobile phase: dichloromethane : methanol = 20 : 1) to obtain compound A121-1 (364 mg, yield 31%).

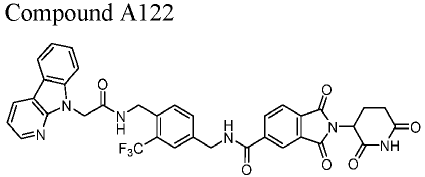

ESI-MS(M+H)⁺: 697.2.

¹H NMR (400 MHz, DMSO) δ 11.15 (s, 1H), 9.51 (t, *J* = 5.8 Hz, 1H), 8.83 (t, *J* = 5.8 Hz, 1H), 8.56 - 8.53 (m, 1H), 8.50 - 8.42 (m, 1H), 8.39 - 8.35 (m, 2H), 8.21 (d, *J* = 3.6 Hz, 1H), 8.05 (d, *J* = 7.8 Hz, 1H), 7.70 - 7.56 (m, 4H), 7.54 - 7.49 (m, 1H), 7.33 - 7.23 (m, 2H), 5.26 - 5.12 (m, 3H), 4.57 (d, *J* = 5.6 Hz, 2H), 4.45 (d, *J* = 5.3 Hz, 2H), 2.97 - 2.83 (m, 1H), 2.67 - 2.53 (m, 2H), 2.14 - 2.04 (m, 1H).

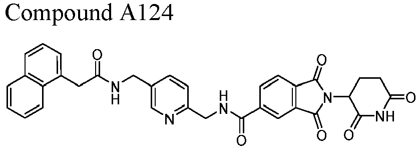

ESI-MS(M+H)⁺: 590.2.

¹H NMR (400 MHz, DMSO) δ 11.15 (s, 1H), 9.52 (t, *J* = 5.6 Hz, 1H), 8.68 (t, *J* = 5.8 Hz, 1H), 8.45 - 8.34 (m, 3H), 8.05 (d, *J* = 7.6 Hz, 2H), 7.94 - 7.88 (m, 1H), 7.84 - 7.79 (m, 1H), 7.60 (dd, *J* = 8.1, 1.9 Hz, 1H), 7.52 - 7.47 (m, 2H), 7.45 - 7.41 (m, 2H), 7.31 (d, *J* = 7.8 Hz, 1H), 5.20 (dd, *J* = 12.9, 5.3 Hz, 1H), 4.58 (d, *J* = 5.7 Hz, 2H), 4.29 (d, *J* = 5.8 Hz, 2H), 3.95 (s, 2H), 2.96 - 2.83 (m, 1H), 2.70 - 2.53 (m, 2H), 2.13 - 2.06 (m, 1H). The corresponding intermediates were prepared as follows:

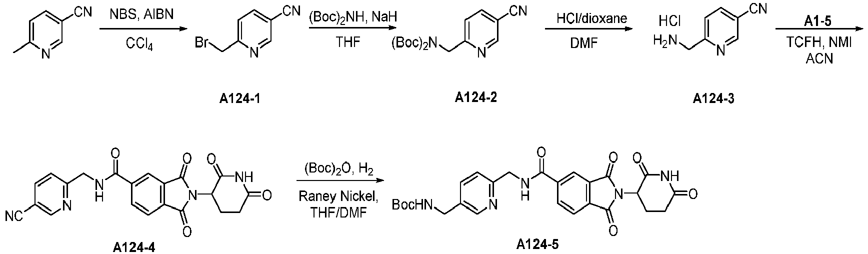

### Synthesis of compound A124-1

5-cyano-2-methylpyridine (10.0 g, 84.7 mmol) was dissolved in 100 mL of carbon tetrachloride, and NBS (18.0 g, 101.7 mmol) and AIBN (4.17 g, 25.4 mmol) were added. The mixture was reacted at 80°C for 1 h, and then TLC showed that the reaction was completed. The mixture was diluted with 130 mL of dichloromethane. The organic phase was washed with saturated aqueous sodium bicarbonate solution (30 mL*3) and dried by rotary evaporation. The solute was separated by column chromatography (mobile phase: petroleum ether : ethyl acetate =9 : 1) to obtain compound A124-1 (6.88 g, yield 42%).

### Synthesis of compound A124-2

Sodium hydride (1.68 g, 60%, 41.9 mmol) was suspended in 40 mL of anhydrous tetrahydrofuran. Under ice bath, a solution of (Boc)₂NH (8.34 g, 38.4 mmol) in anhydrous tetrahydrofuran (20 mL) was added dropwise. The mixture was stirred for 30 min, and then a solution of compound A124-1 (1.0 g, 41.9 mmol) in tetrahydrofuran (20 mL) was added. The mixture was stirred overnight at room temperature, and then TLC showed that the reaction was completed. The reaction was quenched with 20 mL of saturated sodium bicarbonate solution. The aqueous phase was extracted with ethyl acetate (20 mL*3). The organic phases were combined and dried by rotary evaporation, and then the solute was separated by column chromatography (mobile phase: petroleum ether : ethyl acetate = 15 : 1) to obtain compound A124-2 (10.8 g, yield 93%).

### Synthesis of compound A124-3

A124-2 (4.69 g, 14.0 mmol) was dissolved in 25 mL of HCl/dioxane (4 M). The mixture was reacted at room temperature for 1 h, and then TLC showed that the reaction was completed. The solvent was dried by rotary evaporation to obtain compound A124-3 (2.50 g, crude).

### Synthesis of compound A124-4

A124-3 (1.72 g, 12.9 mmol), A1-5 (3.06 g, 12.9 mmol) and *N-*methylimidazole (2.58 g, 80.2 mmol) were dissolved in 30 mL of acetonitrile, and TCFH (3.42 g, 20.7 mmol) was added. The mixture was reacted at room temperature for 1 h, and then TLC showed that the reaction was completed. 20 mL of ethyl acetate/water (1 : 1) was added to the reaction liquid. The precipitated solid was subjected to suction filtration to obtain compound A124-4 (1.55 g, yield 32%).

### Synthesis of compound A124-5

Compound A124-4 (800 mg, 1.92 mmol) was dissolved in 20 mL of THF/DMF (1 : 1), and (Boc)₂O (1.05 g, 4.80 mmol) and Raney Ni (2.5 g) were added. The mixture was reacted overnight at room temperature under hydrogen atmosphere, and then TLC showed that the reaction was completed. The reaction liquid was filtered, and the filtrate was dried by rotary evaporation. The solute was separated by column chromatography (mobile phase: dichloromethane : methanol = 20 : 1) to obtain compound A124-5 (513 mg, yield 51%). ¹H NMR (400 MHz, DMSO) δ 11.15 (s, 1H), 9.52 (t, *J* = 5.8 Hz, 1H), 8.39 (dd, *J* = 14.5, 6.8 Hz, 3H), 8.05 (d, *J* = 7.8 Hz, 1H), 7.61 (dd, *J* = 8.0, 2.0 Hz, 1H), 7.44 (t, *J* = 5.8 Hz, 1H), 7.33 (d, *J* = 8.0 Hz, 1H), 5.20 (dd, *J* = 12.9, 5.4 Hz, 1H), 4.59 (d, *J* = 5.7 Hz, 2H), 4.10 (t, *J* = 15.0 Hz, 2H), 2.96 - 2.84 (m, 1H), 2.67 - 2.52 (m, 2H), 2.15 - 2.04 (m, 1H), 1.39 (s, 9H).

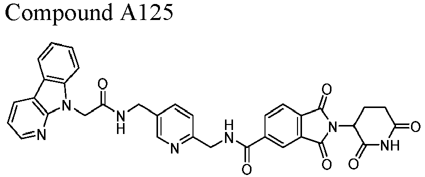

ESI-MS(M+H)⁺: 630.2.

¹H NMR (400 MHz, DMSO) δ 11.16 (s, 1H), 9.53 (t, *J* = 5.9 Hz, 1H), 8.79 (t, *J* = 5.9 Hz, 1H), 8.54 (dd, *J* = 7.6, 1.6 Hz, 1H), 8.48 - 8.40 (m, 3H), 8.38 (dd, *J* = 7.8, 1.4 Hz, 1H), 8.21 (d, *J=* 7.7 Hz, 1H), 8.06 (d, *J=* 7.8 Hz, 1H), 7.66 (dd, *J=* 8.1, 2.2 Hz, 1H), 7.56 (d, *J* = 8.2 Hz, 1H), 7.53 - 7.44 (m, 1H), 7.34 (d, *J* = 8.0 Hz, 1H), 7.29 - 7.24 (m, 2H), 5.21 (dd, *J* = 12.9, 5.4 Hz, 1H), 5.16 (s, 2H), 4.59 (d, *J* = 5.8 Hz, 2H), 4.31 (d, *J* = 5.8 Hz, 2H), 2.97 - 2.85 (m, 1H), 2.68 - 2.53 (m, 2H), 2.15 - 2.08 (m, 1H).

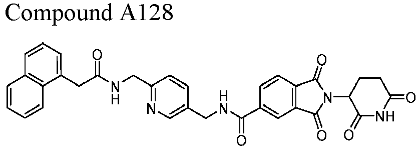

ESI-MS(M+H)⁺: 590.2.

¹H NMR (400 MHz, DMSO) δ 11.15 (s, 1H), 9.49 (t, *J=* 5.7 Hz, 1H), 8.73 (t, *J* = 5.8 Hz, 1H), 8.50 (d, *J* = 1.5 Hz, 1H), 8.39 - 8.34 (m, 2H), 8.11 (d, *J* = 7.6 Hz, 1H), 8.04 (d, *J* = 7.8 Hz, 1H), 7.94 - 7.87 (m, 1H), 7.85 - 7.79 (m, 1H), 7.69 (dd, *J* = 8.0, 2.1 Hz, 1H), 7.55 - 7.42 (m, 4H), 7.22 (d, *J* = 8.0 Hz, 1H), 5.20 (dd, *J* = 12.9, 5.4 Hz, 1H), 4.51 (d, *J* = 5.6 Hz, 2H), 4.36 (d, *J* = 5.9 Hz, 2H), 4.00 (s, 2H), 2.95 - 2.85 (m, 1H), 2.69 - 2.52 (m, 2H), 2.15 - 2.03 (m, 1H).
The corresponding intermediates were prepared as follows:

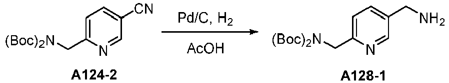

### Synthesis of compound A128-1

A124-2 (5.0 g, 15.0 mmol) was dissolved in 15 mL of glacial acetic acid, and Pd/C (1 g, 5%) was added. The mixture was reacted at 55°C under hydrogen atmosphere for 1 h, and then TLC showed that the reaction was completed. The reaction liquid was filtered, and then the filtrate was dried by rotary evaporation. 50 mL of dichloromethane/methanol (5 : 1) was added. The organic phase was washed with saturated aqueous sodium bicarbonate solution (5 mL) and then dried by rotary evaporation. The solute was separated by column chromatography (mobile phase: dichloromethane : methanol = 15 : 1) to obtain compound A128-1 (1.19 g, yield 24%).

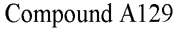

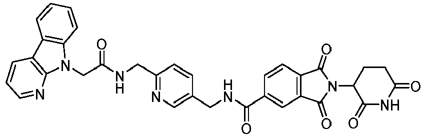

ESI-MS(M+H)⁺: 630.2.

¹H NMR (400 MHz, DMSO) δ 11.15 (s, 1H), 9.47 (t, *J=* 5.7 Hz, 1H), 8.84 (t, *J=* 5.9 Hz, 1H), 8.59 - 8.49 (m, 2H), 8.45 (dd, *J* = 4.8, 1.5 Hz, 1H), 8.41 - 8.30 (m, 2H), 8.20 (d, *J=* 7.7 Hz, 1H), 8.04 (d, *J=* 7.8 Hz, 1H), 7.75 (dd, *J=* 8.0, 2.2 Hz, 1H), 7.60 (d, *J* = 8.2 Hz, 1H), 7.54 - 7.45 (m, 1H), 7.34 (d, *J* = 8.0 Hz, 1H), 7.30 - 7.21 (m, 2H), 5.25 - 5.15 (m, 3H), 4.52 (d, *J=* 5.6 Hz, 2H), 4.38 (d, *J=* 5.9 Hz, 2H), 2.97 - 2.85 (m, 1H), 2.69 - 2.52 (m, 2H), 2.14 - 2.01 (m, 1H).

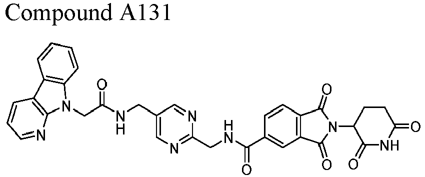

ESI-MS(M+H)⁺: 631.2.

¹H NMR (400 MHz, DMSO) δ 11.17 (s, 1H), 9.54 (t, *J* = 5.9 Hz, 1H), 8.82 (t, *J* = 5.8 Hz, 1H), 8.67 (s, 2H), 8.54 (dd, *J* = 7.7, 1.4 Hz, 1H), 8.44 - 8.40 (m, 2H), 8.38 (d, *J* = 7.8 Hz, 1H), 8.21 (d, *J* = 7.7 Hz, 1H), 8.07 (d, *J* = 7.8 Hz, 1H), 7.59 (d, *J* = 8.1 Hz, 1H), 7.49 (t, *J* = 7.7 Hz, 1H), 7.33 - 7.21 (m, 2H), 5.21 (dd, *J* = 12.9, 5.5 Hz, 1H), 5.17 (s, 2H), 4.69 (d, *J* = 5.9 Hz, 2H), 4.32 (d, *J* = 5.7 Hz, 2H), 2.96 - 2.83 (m, 1H), 2.67 - 2.53 (m, 2H), 2.17 - 2.05 (m, 1H).

The corresponding intermediates were prepared as follows:

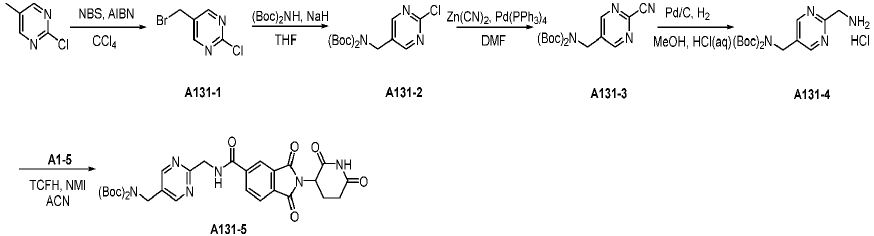

### Synthesis of compound A131-1

2-chloro-5-methylpyrimidine (6.0 g, 46.8 mmol) was dissolved in 60 mL of carbon tetrachloride, and NBS (10.0 g, 56.4 mmol) and AIBN (2.0 g, 12.1 mmol) were added. The mixture was reacted at 80°C for 1 h, and then TLC showed that the reaction was completed. The mixture was diluted with 150 mL of dichloromethane. The organic phase was washed with saturated aqueous sodium bicarbonate solution (30 mL*3) and dried by rotary evaporation. The solute was separated by column chromatography (mobile phase: petroleum ether : ethyl acetate = 9 : 1) to obtain compound A131-1 (6.64 g, yield 69%).

### Synthesis of compound A131-2

Sodium hydride (1.54 g, 60%, 38.5 mmol) was suspended in 20 mL of anhydrous tetrahydrofuran. Under ice bath, a solution of (Boc)₂NH (7.66 g, 35.3 mmol) in anhydrous tetrahydrofuran (20 mL) was added dropwise. The mixture was stirred for 30 min, and then a solution of A131-1 (6.64 g, 32.2 mmol) in tetrahydrofuran (20 mL) was added. The mixture was stirred overnight at room temperature, and then TLC showed that the reaction was completed. The reaction was quenched with 50 mL of saturated sodium bicarbonate solution. The aqueous phase was extracted with ethyl acetate (50 mL*3). The organic phases were combined and dried by rotary evaporation, and then the solute was separated by column chromatography (mobile phase: petroleum ether : ethyl acetate = 6 : 1) to obtain compound A131-2 (6.46 g, yield 58%).

### Synthesis of compound A131-3

A131-2 (6.46 g, 18.8 mmol) was dissolved in 50 mL of DMF, and zinc cyanide (1.77 g, 15.1 mmol) and tetrakis(triphenylphosphine)palladium (2.18 g, 1.88 mmol) were successively added. The mixture was reacted overnight at 90°C under nitrogen protection, and then TLC showed that the reaction was completed. The reaction liquid was filtered, and the filtrate was diluted with 130 mL of water. The aqueous phase was extracted with ethyl acetate (50 mL*3). The organic phases were combined, washed with saturated brine (20 mL*3) and then dried by rotary evaporation. The solute was separated by column chromatography (mobile phase: petroleum ether : ethyl acetate = 6 : 1) to obtain compound A131-3 (5.08 g, yield 80%).

### Synthesis of compound A131-4

A131-3 (1.0 g, 2.99 mmol) was dissolved in 10 mL of methanol, and dilute hydrochloric acid (6 mL, 1 M) and Pd/C (200 mg) were added. The mixture was reacted at room temperature under hydrogen atmosphere for 1 h, and then TLC showed that the reaction was completed. The reaction liquid was filtered, and the filtrate was dried by rotary evaporation to obtain compound A131-4 (1.0 g, crude).

### Synthesis of compound A131-5

Compound A131-4 (1.0 g, 2.95 mmol), compound A1-5 (893 mg, 2.95 mmol) and N-methylimidazole (995 mg, 12.1 mmol) were dissolved in 20 mL of acetonitrile, and TCFH (994 mg, 3.55 mmol) was added. The mixture was reacted at room temperature for 1 h, and then TLC showed that the reaction was completed. 20 mL of water was added to the reaction liquid, and the precipitated solid was subjected to suction filtration. The filter cake was dried and then separated by column chromatography (mobile phase: petroleum ether : ethyl acetate = 1 : 1) to obtain compound A131-5 (510 mg, yield 28%). ¹H NMR (400 MHz, DMSO) δ 11.16 (s, 1H), 9.54 (t, *J* = 5.8 Hz, 1H), 8.66 (s, 2H), 8.43 - 8.33 (m, 2H), 8.05 (t, *J* = 6.9 Hz, 1H), 5.20 (dd, *J* = 12.8, 5.3 Hz, 1H), 4.70 (d, *J* = 5.4 Hz, 4H), 2.91 (dd, *J* = 22.7, 8.5 Hz, 1H), 2.70 - 2.53 (m, 2H), 2.17 - 2.04 (m, 1H), 1.42 (s, 18H).

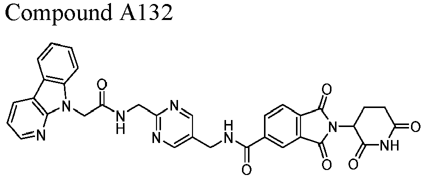

ESI-MS(M+H)⁺: 631.2.

¹H NMR (400 MHz, DMSO) δ 11.17 (s, 1H), 9.51 (t, *J* = 5.6 Hz, 1H), 8.90 (t, *J* = 5.8 Hz, 1H), 8.79 (s, 2H), 8.54 (dd, *J* = 7.6, 1.5 Hz, 1H), 8.44 (dd, *J* = 4.8, 1.5 Hz, 1H), 8.39 - 8.34 (m, 2H), 8.19 (d, *J* = 7.7 Hz, 1H), 8.05 (d, *J* = 7.8 Hz, 1H), 7.59 (d, *J* = 8.2 Hz, 1H), 7.49 (t, *J* = 7.4 Hz, 1H), 7.30 - 7.16 (m, 2H), 5.23 - 5.17 (m, 3H), 4.54 (d, *J* = 5.5 Hz, 2H), 4.51 (d, *J* = 5.8 Hz, 2H), 2.95 - 2.83 (m, 1H), 2.68 - 2.52 (m, 2H), 2.13 - 2.04 (m, 1H).

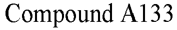

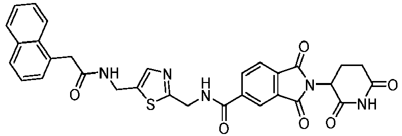

ESI-MS(M+H)⁺: 596.2.

¹H NMR (400 MHz, DMSO) δ 11.17 (s, 1H), 9.59 (t, *J* = 5.8 Hz, 1H), 9.02 (t, *J* = 6.0 Hz, 1H), 8.39 - 8.33 (m, 2H), 8.05 (dd, *J* = 17.2, 8.3 Hz, 2H), 7.87 (d, *J* = 7.9 Hz, 1H), 7.77 (dd, *J* = 8.6, 7.0 Hz, 1H), 7.62 (s, 1H), 7.49 - 7.35 (m, 4H), 5.21 (dd, *J* = 13.0, 5.3 Hz, 1H), 4.65 (d, *J* = 5.6 Hz, 2H), 4.50 (d, *J* = 6.0 Hz, 2H), 3.96 (s, 2H), 2.97 - 2.84 (m, 1H), 2.67 - 2.52 (m, 2H), 2.15 - 2.03 (m, 1H).

The corresponding intermediates were prepared as follows:

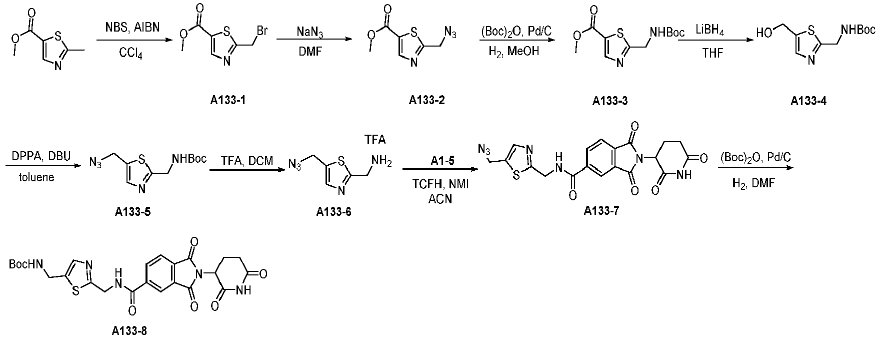

### Synthesis of compound A133-1

Methyl 2-methylthiazole-5-carboxylate (9.0 g, 52.6 mmol) was dissolved in 90 mL of carbon tetrachloride, and NBS (11.2 g, 63.2 mmol) and AIBN (2.59 g, 15.8 mmol) were added. The mixture was reacted at 80°C for 1 h, and then TLC showed that the reaction was completed. The mixture was diluted with 150 mL of dichloromethane. The organic phase was washed with saturated aqueous sodium bicarbonate solution (30 mL*3) and dried by rotary evaporation. The solute was separated by column chromatography (mobile phase: petroleum ether : ethyl acetate =1 : 3) to obtain compound A133-1 (5.05 g, yield 41%).

### Synthesis of compound A133-2

A133-1 (5.0 g, 21.2 mmol) was dissolved in 50 mL of DMF, and sodium azide (4.82 g, 74.1 mmol) was added. The mixture was reacted at room temperature for 1 h, and then TLC showed that the reaction was completed. The mixture was diluted with water (100 mL). The aqueous phase was extracted with ethyl acetate (150 mL*2). The organic phases were combined, washed with saturated brine (100 mL) and then dried by rotary evaporation to obtain compound A133-2 (4.5 g, crude).

### Synthesis of compound A133-3

Compound A133-2 (4.5 g, 21.2 mmol) was dissolved in 90 mL of methanol, and (Boc)₂O (7.43 g, 34.1 mmol) and Pd/C (7.43 g, 5%) were added. The mixture was reacted overnight at room temperature under hydrogen atmosphere, and then TLC showed that the reaction was completed. The reaction liquid was filtered, and the filtrate was dried by rotary evaporation. The solute was separated by column chromatography (mobile phase: petroleum ether : ethyl acetate = 1 : 1) to obtain compound A133-3 (4.31 g, yield 70%).

### Synthesis of compound A133-4

Compound A133-3 (4.3 g, 15.8 mmol) was dissolved in 80 mL of anhydrous tetrahydrofuran. Under ice bath, lithium borohydride/THF (7.9 mL, 2 M, 15.8 mmol) was added dropwise. After the dropwise addition was completed, the mixture was reacted at room temperature for 2 h, and then TLC showed that the reaction was completed. Under ice bath, the reaction was quenched with water (34 mL) and dilute hydrochloric acid (40 mL, 2 M). The aqueous phase was extracted with ethyl acetate (100 mL*4). The organic phases were combined and dried by rotary evaporation to obtain compound A133-4 (2.7 g, yield 70%).

### Synthesis of compound A133-5

A133-4 (2.70 g, 11.0 mmol) was dissolved in 30 mL of toluene, and DPPA (6.36 g, 23.1 mmol) and DBU (3.49 g, 22.9 mmol) were added. The mixture was reacted at 60°C for 1 h, and then TLC showed that the reaction was completed. The mixture was diluted with water (50 mL). The aqueous phase was extracted with ethyl acetate (30 mL*2). The organic phases were combined and dried by rotary evaporation. The solute was separated by column chromatography (mobile phase: petroleum ether : ethyl acetate = 1 : 3) to obtain compound A133-5 (1.21 g, yield 65%).

### Synthesis of compound A133-6

Compound A133-5 (1.21 g, 7.16 mmol) was dissolved in dichloromethane (12 mL), and trifluoroacetic acid (6 mL) was added with stirring. The mixture was reacted at room temperature for 1 h, and then monitoring by TLC showed that the raw materials were completely reacted. The solvent was dried by rotary evaporation to obtain crude compound A133-6 (1.52 g) as an oil.

### Synthesis of compound A133-7

Compound A133-6 (1.52 g, 7.16 mmol), compound A1-5 (1.36 g, 4.49 mmol) and N-methylimidazole (2.21 g, 26.9 mmol) were dissolved in 20 mL of acetonitrile, and TCFH (1.51 g, 5.39 mmol) was added. The mixture was reacted at room temperature for 1 h, and then TLC showed that the reaction was completed. 20 mL of water was added to the reaction liquid, and the precipitated solid was subjected to suction filtration. The filter cake was dried to obtain compound A133-7 (1.74 g, yield 85%).

### Synthesis of compound A133-8

Compound A133-7 (1.74 g, 3.40 mmol) was dissolved in 15 mL of DMF, and (Boc)₂O (2.23 g, 10.2 mmol) and Pd/C (462 mg, 5%) were added. The mixture was reacted at room temperature under hydrogen atmosphere for 3 h, and then TLC showed that the reaction was completed. The reaction liquid was filtered, and the filtrate was dried by rotary evaporation. The solute was separated by column chromatography (mobile phase: dichloromethane : methanol = 15 : 1) to obtain compound A133-8 (227 mg, yield 11%). ¹H NMR (400 MHz, DMSO) δ 11.15 (s, 1H), 9.56 (s, 1H), 8.34 (s, 2H), 8.05 (s, 1H), 7.71 (s, 1H), 7.60 (s, 1H), 5.19 (d, *J* = 7.9 Hz, 1H), 4.65 (s, 2H), 4.31 (s, 2H), 2.88 (d, *J* = 11.9 Hz, 1H), 2.73 - 2.56 (m, 2H), 2.08 (s, 1H), 1.38 (s, 9H).

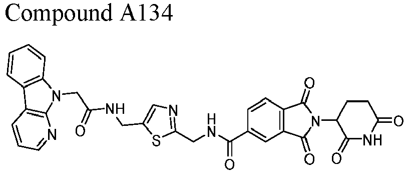

ESI-MS(M+H)⁺: 636.2.

¹H NMR (400 MHz, DMSO) δ 11.17 (s, 1H), 9.61 (t, *J* = 5.7 Hz, 1H), 9.11 (t, *J* = 6.0 Hz, 1H), 8.52 (dd, *J* = 7.6, 1.4 Hz, 1H), 8.40 - 8.36 (m, 3H), 8.18 (d, *J* = 7.7 Hz, 1H), 8.08 (d, *J* = 8.4 Hz, 1H), 7.62 (s, 1H), 7.52 (d, *J* = 8.2 Hz, 1H), 7.42 (t, *J* = 7.4 Hz, 1H), 7.26 - 7.19 (m, 2H), 5.26 - 5.18 (m, 1H), 5.17 (s, 2H), 4.67 (d, *J* = 5.6 Hz, 2H), 4.51 (d, *J* = 5.9 Hz, 2H), 2.95 - 2.84 (m, 1H), 2.69 - 2.53 (m, 2H), 2.15 - 2.05 (m, 1H).

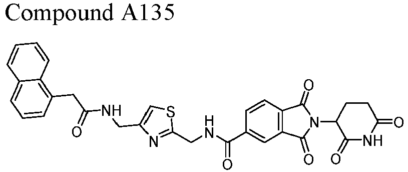

ESI-MS(M+H)⁺: 596.2.

¹H NMR (400 MHz, DMSO) δ 11.17 (s, 1H), 9.83 (t, *J* = 5.8 Hz, 1H), 8.71 (t, *J* = 5.7 Hz, 1H), 8.38 (dd, *J* = 10.6, 2.7 Hz, 2H), 8.08 (t, *J* = 8.5 Hz, 2H), 7.95 - 7.88 (m, 1H), 7.81 (t, *J* = 4.7 Hz, 1H), 7.56 - 7.48 (m, 2H), 7.47 - 7.42 (m, 2H), 7.23 (s, 1H), 5.21 (dd, *J* = 12.8, 5.3 Hz, 1H), 4.77 (d, *J* = 5.8 Hz, 2H), 4.36 (d, *J* = 5.7 Hz, 2H), 3.97 (s, 2H), 2.98 - 2.82 (m, 1H), 2.67 - 2.52 (m, 2H), 2.13 - 2.04 (m, 1H).

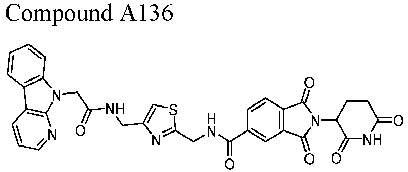

ESI-MS(M+H)⁺: 636.2.

¹H NMR (400 MHz, DMSO) δ 11.17 (s, 1H), 9.85 - 9.82 (m, 1H), 8.84 (t, *J* = 5.6 Hz, 1H), 8.55 (dd, *J* = 7.6, 1.4 Hz, 1H), 8.45 (dd, *J* = 4.8, 1.4 Hz, 1H), 8.42 - 8.35 (m, 2H), 8.21 (d, *J* = 7.7 Hz, 1H), 8.07 (d, *J* = 7.8 Hz, 1H), 7.59 (d, *J* = 8.2 Hz, 1H), 7.50 (t, *J* = 7.7 Hz, 1H), 7.37 (s, 1H), 7.32 - 7.20 (m, 2H), 5.24 - 5.13 (m, 3H), 4.77 (d, *J* = 5.8 Hz, 2H), 4.38 (d, *J* = 5.6 Hz, 2H), 2.95 - 2.87 (m, 1H), 2.69 - 2.52 (m, 2H), 2.13 - 2.03 (m, 1H).

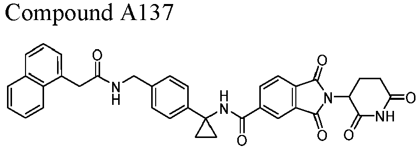

ESI-MS(M+H)⁺: 615.2.

¹H NMR (400 MHz, DMSO) δ 11.13 (s, 1H), 9.56 (s, 1H), 8.58 (t, *J* = 5.8 Hz, 1H), 8.40 (s, 1H), 8.34 (dd, *J* = 7.8, 1.4 Hz, 1H), 8.11 - 8.06 (m, 1H), 8.04 (d, *J* = 8.1 Hz, 1H), 7.95 - 7.88 (m, 1H), 7.83 - 7.77 (m, 1H), 7.53 - 7.46 (m, 2H), 7.46 - 7.40 (m, 2H), 7.20 - 7.10 (m, 4H), 5.20 (dd, *J* = 12.9, 5.4 Hz, 1H), 4.23 (d, *J* = 5.8 Hz, 2H), 3.94 (s, 2H), 2.96 - 2.84 (m, 1H), 2.68 - 2.53 (m, 2H), 2.16 - 2.05 (m, 1H), 1.33 - 1.19 (m, 4H).

The corresponding intermediates were prepared as follows:

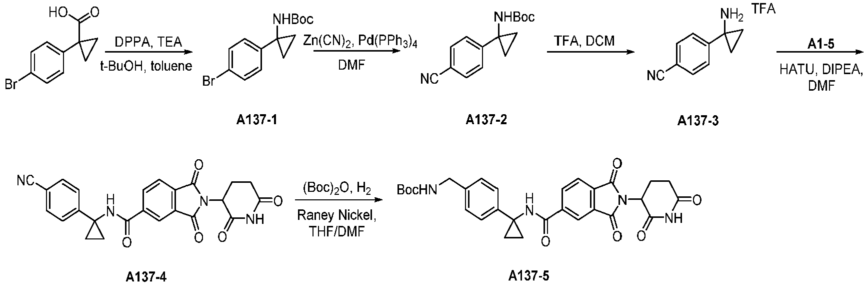

### Synthesis of compound A137-1

1-(4-bromophenyl)cyclopropanecarboxylic acid (15.0 g, 62.2 mmol) was dissolved in 100 mL of toluene, and triethylamine (12.6 g, 124.4 mmol), DPPA (18.8 g, 68.4 mmol) and tert-butanol(23.4 g, 311.1 mmol) were added. The mixture was reacted overnight at 80°C, and then TLC showed that the reaction was completed. The reaction was quenched with water (50 mL). The aqueous phase was extracted with ethyl acetate (30 mL*3). The organic phases were combined and dried by rotary evaporation. The solute was separated by column chromatography (mobile phase: petroleum ether : ethyl acetate = 6 : 1) to obtain compound A137-1 (10.45 g, yield 54%).

### Synthesis of compound A137-2

A137-1 (9.5 g, 30.4 mmol) was dissolved in 50 mL of DMF, and zinc cyanide (2.86 g, 24.3 mmol) and tetrakis(triphenylphosphine)palladium (3.52 g, 3.04 mmol) were successively added. The mixture was reacted overnight at 80°C under nitrogen protection, and then TLC showed that the reaction was completed. The reaction liquid was filtered, and the filtrate was diluted with 100 mL of water. The aqueous phase was extracted with ethyl acetate (20 mL*3). The organic phases were combined, washed with saturated brine (20 mL*2) and then dried by rotary evaporation. The solute was separated by column chromatography (mobile phase: petroleum ether : ethyl acetate = 6 : 1) to obtain compound A137-2 (4.43 g, yield 56%).

### Synthesis of compound A137-3

Compound A137-2 (1.0 g, 3.87 mmol) was dissolved in dichloromethane (10 mL), and trifluoroacetic acid (3 mL) was added with stirring. The mixture was reacted at room temperature for 1 h, and then monitoring by TLC showed that the raw materials were completely reacted. The solvent was dried by rotary evaporation to obtain crude compound A137-3 (1.1 g) as an oil.

### Synthesis of compound A137-4

Compound A137-3 (1.1 g, 3.87 mmol) and compound A1-5 (1.17 g, 3.87 mmol) were dissolved in DMF (10 mL), and HATU (2.88 g, 7.58 mmol) and DIPEA (4.90 g, 37.9 mmol) were added. The mixture was reacted at room temperature for 1 h, and then monitoring by TLC showed that the raw materials were completely reacted. Water (50 mL) was added, and then the aqueous phase was extracted with ethyl acetate (20 mL*3). The organic phases were combined, washed with saturated brine (20 mL*2) and then dried by rotary evaporation. The solute was separated by column chromatography (mobile phase: petroleum ether : ethyl acetate = 1 : 3) to obtain compound A137-4 (950 mg, yield 65%).

### Synthesis of compound A137-5

Compound A137-4 (950 mg, 2.15 mmol) was dissolved in 20 mL of THF/DMF (1 : 1), and (Boc)₂O (700 g, 3.24 mmol) and Raney Ni (1.0 g) were added. The mixture was reacted overnight at room temperature under hydrogen atmosphere, and then TLC showed that the reaction was completed. The reaction liquid was filtered, and the filtrate was dried by rotary evaporation. The solute was separated by column chromatography (mobile phase: petroleum ether : ethyl acetate =1 : 6) to obtain compound A137-5 (390 mg, yield 33%).

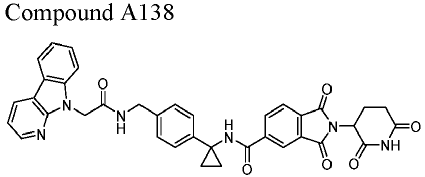

ESI-MS(M+H)⁺: 655.2.

¹H NMR (400 MHz, DMSO) δ 11.15 (s, 1H), 9.57 (s, 1H), 8.71 (t, *J* = 5.8 Hz, 1H), 8.54 (dd, *J* = 7.6, 1.6 Hz, 1H), 8.44 (dd, *J* = 4.8, 1.6 Hz, 1H), 8.41 (s, 1H), 8.34 (dd, *J* = 7.8, 1.4 Hz, 1H), 8.20 (d, *J* = 7.8 Hz, 1H), 8.04 (d, *J* = 7.8 Hz, 1H), 7.55 (d, *J* = 8.2 Hz, 1H), 7.53 - 7.44 (m, 1H), 7.30 - 7.22 (m, 2H), 7.19 (s, 4H), 5.20 (dd, *J* = 12.9, 5.4 Hz, 1H), 5.15 (s, 2H), 4.25 (d, *J* = 5.8 Hz, 2H), 2.97 - 2.84 (m, 1H), 2.69 - 2.53 (m, 2H), 2.14 - 2.05 (m, 1H), 1.34 - 1.21 (m, 4H).

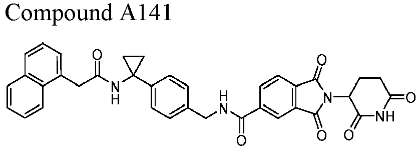

ESI-MS(M+H)⁺: 615.2.

¹H NMR (400 MHz, DMSO) δ 11.15 (s, 1H), 9.37 (t, *J* = 5.8 Hz, 1H), 8.92 (s, 1H), 8.42 - 8.29 (m, 2H), 8.09 (d, *J* = 7.8 Hz, 1H), 8.03 (d, *J* = 7.8 Hz, 1H), 7.95 - 7.86 (m, 1H), 7.81 (d, *J* = 8.6 Hz, 1H), 7.56 - 7.39 (m, 4H), 7.17 (d, *J* = 8.3 Hz, 2H), 7.11 - 7.04 (m, 2H), 5.19 (dd, *J* = 12.9, 5.4 Hz, 1H), 4.43 (d, *J* = 5.7 Hz, 2H), 3.92 (s, 2H), 2.96 - 2.84 (m, 1H), 2.67 - 2.52 (m, 2H), 2.13 - 2.06 (m, 1H), 1.17 - 1.06 (m, 4H).

The corresponding intermediates were prepared as follows:

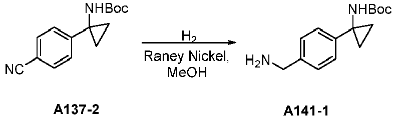

### Synthesis of compound A141-1

Compound A137-2 (2.0 g, 7.75 mmol) was dissolved in 20 mL of methanol, and Raney Ni (1.0 g) was added. The mixture was reacted at room temperature under hydrogen atmosphere for 1 h, and then TLC showed that the reaction was completed. The reaction liquid was filtered, and the filtrate was dried by rotary evaporation. The solute was separated by column chromatography (mobile phase: petroleum ether : ethyl acetate = 1 : 1) to obtain compound A141-1 (420 mg, yield 21%).

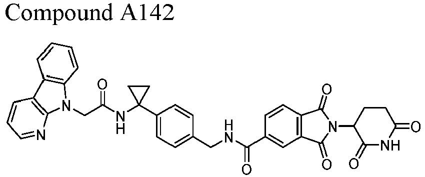

ESI-MS(M+H)⁺: 655.2.

¹H NMR (400 MHz, DMSO) δ 11.14 (s, 1H), 9.39 (t, *J* = 5.8 Hz, 1H), 9.08 (s, 1H), 8.53 (dd, *J* = 7.6, 1.5 Hz, 1H), 8.46 (dd, *J* = 4.8, 1.5 Hz, 1H), 8.38 - 8.33 (m, 2H), 8.19 (d, *J* = 7.7 Hz, 1H), 8.03 (d, *J* = 7.8 Hz, 1H), 7.57 (d, *J* = 8.2 Hz, 1H), 7.50 (t, *J* = 7.2 Hz, 1H), 7.31 - 7.11 (m, 6H), 5.19 (dd, *J* = 12.9, 5.4 Hz, 1H), 5.14 (s, 2H), 4.45 (d, *J* = 5.7 Hz, 2H), 2.95 - 2.84 (m, 1H), 2.69 - 2.53 (m, 2H), 2.13 - 2.03 (m, 1H), 1.18 - 1.11 (m, 4H).

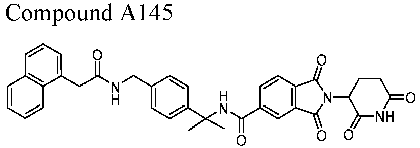

ESI-MS(M+H)⁺: 617.2.

¹H NMR (400 MHz, DMSO) δ 11.15 (s, 1H), 8.86 (s, 1H), 8.62 (t, *J* = 5.7 Hz, 1H), 8.40 (s, 1H), 8.27 (dd, *J* = 7.8, 1.4 Hz, 1H), 8.13 - 8.05 (m, 1H), 8.01 (d, *J* = 7.8 Hz, 1H), 7.94 - 7.88 (m, 1H), 7.85 - 7.73 (m, 1H), 7.52 - 7.46 (m, 2H), 7.45 - 7.40 (m, 2H), 7.32 (d, *J* = 8.3 Hz, 2H), 7.16 (d, *J* = 8.3 Hz, 2H), 5.20 (dd, *J* = 13.0, 5.4 Hz, 1H), 4.25 (d, *J* = 5.8 Hz, 2H), 3.95 (s, 2H), 3.00 - 2.82 (m, 1H), 2.71 - 2.52 (m, 2H), 2.15 - 2.05 (m, 1H), 1.68 (s, 6H).

The corresponding intermediates were prepared according to the route for the intermediates of compound A137.

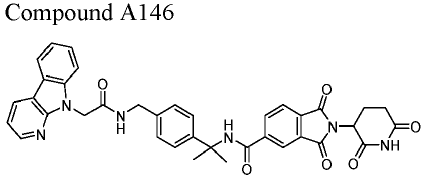

ESI-MS(M+H)⁺: 657.2.

¹H NMR (400 MHz, DMSO) δ 11.16 (s, 1H), 8.87 (s, 1H), 8.74 (t, *J* = 5.7 Hz, 1H), 8.54 (dd, *J* = 7.6, 1.4 Hz, 1H), 8.48 - 8.38 (m, 2H), 8.28 (dd, *J* = 7.8, 1.2 Hz, 1H), 8.20 (d, *J* = 7.7 Hz, 1H), 8.01 (d, *J* = 7.8 Hz, 1H), 7.57 (d, *J* = 8.2 Hz, 1H), 7.49 (t, *J* = 7.3 Hz, 1H), 7.35 (d, *J* = 8.2 Hz, 2H), 7.30 - 7.23 (m, 2H), 7.21 (d, *J* = 8.2 Hz, 2H), 5.26 - 5.12 (m, 3H), 4.27 (d, *J* = 5.7 Hz, 2H), 2.98 - 2.82 (m, 1H), 2.68 - 2.54 (m, 2H), 2.16 - 2.04 (m, 1H), 1.69 (s, 6H).

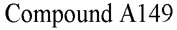

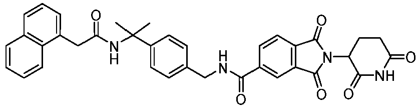

ESI-MS(M+H)⁺: 617.2.

¹H NMR (400 MHz, DMSO) δ 11.15 (s, 1H), 9.39 (t, *J* = 5.8 Hz, 1H), 8.43 (s, 1H), 8.41 - 8.30 (m, 2H), 8.11 (d, *J* = 8.4 Hz, 1H), 8.03 (d, *J* = 7.8 Hz, 1H), 7.89 (d, *J* = 7.6 Hz, 1H), 7.78 (d, *J* = 7.5 Hz, 1H), 7.57 - 7.37 (m, 4H), 7.29 (d, *J* = 8.3 Hz, 2H), 7.19 (d, *J* = 8.3 Hz, 2H), 5.20 (dd, *J* = 12.9, 5.4 Hz, 1H), 4.44 (d, *J* = 5.7 Hz, 2H), 3.94 (s, 2H), 2.97 - 2.83 (m, 1H), 2.69 - 2.53 (m, 2H), 2.14 - 2.04 (m, 1H), 1.56 (s, 6H).

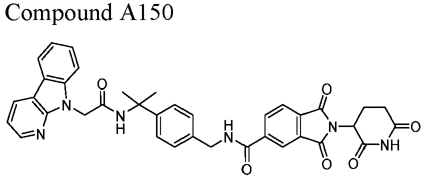

ESI-MS(M+H)⁺: 657.2.

¹H NMR (400 MHz, DMSO) δ 11.15 (s, 1H), 9.39 (t, *J* = 5.7 Hz, 1H), 8.61 (s, 1H), 8.51 (dd, *J* = 7.6, 1.5 Hz, 1H), 8.45 (dd, *J* = 4.8, 1.5 Hz, 1H), 8.38 - 8.34 (m, 2H), 8.16 (d, *J* = 7.8 Hz, 1H), 8.03 (d, *J* = 7.8 Hz, 1H), 7.54 (d, *J =* 8.2 Hz, 1H), 7.48 (t, *J* = 7.2 Hz, 1H), 7.37 (d, *J* = 8.3 Hz, 2H), 7.27 - 7.18 (m, 4H), 5.25 - 5.14 (m, 3H), 4.45 (d, *J* = 5.7 Hz, 2H), 2.96 - 2.82 (m, 1H), 2.68 - 2.52 (m, 2H), 2.14 - 2.04 (m, 1H), 1.57 (s, 6H).

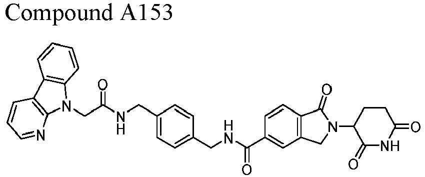

ESI-MS(M+H)⁺: 615.2.

¹H NMR (400 MHz, DMSO) δ 11.00 (s, 1H), 9.21 (t, *J* = 5.9 Hz, 1H), 8.74 (t, *J* = 6.0 Hz, 1H), 8.54 (dd, *J* = 7.6, 1.6 Hz, 1H), 8.45 (dd, *J* = 4.8, 1.6 Hz, 1H), 8.21 (d, *J* = 7.7 Hz, 1H), 8.09 (s, 1H), 8.01 (d, *J* = 8.9 Hz, 1H), 7.82 (d, *J* = 8.0 Hz, 1H), 7.56 (d, *J* = 8.2 Hz, 1H), 7.53 - 7.45 (m, 1H), 7.31 - 7.23 (m, 6H), 5.20 - 5.09 (m, 3H), 4.55 - 4.37 (m, 4H), 4.28 (d, *J* = 5.9 Hz, 2H), 2.98 - 2.85 (m, 1H), 2.69 - 2.55 (m, 1H), 2.46 - 2.30 (m, 1H), 2.10 - 1.95 (m, 1H).

Preparation was performed as follows:

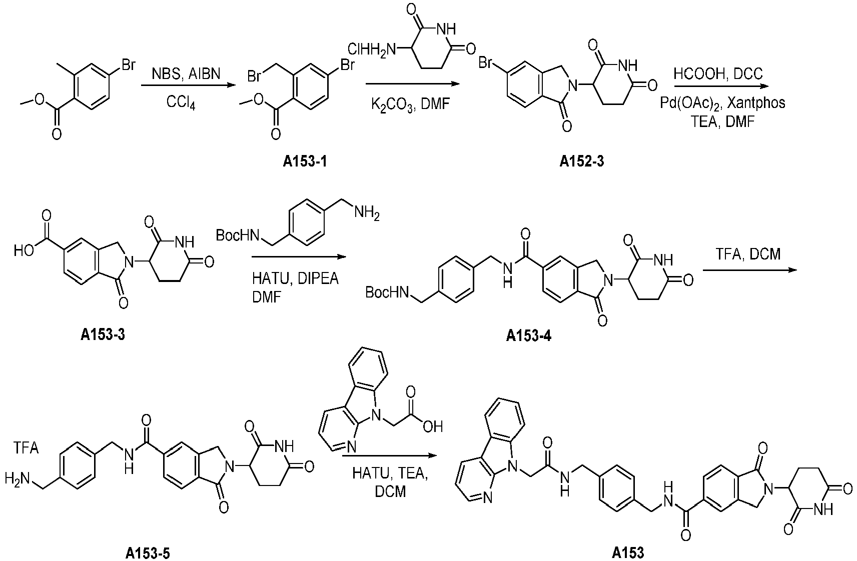

### Synthesis of compound A153-1

Methyl 4-bromo-2-methylbenzoate (30.0 g, 131 mmol) was dissolved in 300 mL of carbon tetrachloride, and NBS (28 g, 131 mmol) and AIBN (6.4 g, 40 mmol) were added. The mixture was reacted at 80°C for 1 h, and then TLC showed that the reaction was completed. The mixture was diluted with 150 mL of MTBE and then filtered. The filtrate was washed with saturated aqueous sodium bicarbonate solution (30 mL*3). The organic phase was dried by rotary evaporation to obtain compound A153-1 (48 g, crude).

### Synthesis of compound A153-2

A153-1 (48 g, 131 mmol) was dissolved in 450 mL of DMF, and 3-aminopiperidine-2,6-dione hydrochloride (28 g, 172 mmol) and potassium carbonate (65 g, 469 mmol) were added. The mixture was reacted at 70°C for 1 h, and then TLC showed that the reaction was completed. The reaction liquid was filtered, and the filtrate was subjected to rotary evaporation to remove most of the solvent. The mixture was slurried with 200 mL of dichloromethane/water (1 : 1) and then subjected to suction filtration. The filter cake was dried to obtain compound A152-2 (19 g, yield 38%).

### Synthesis of compound A153-3

Compound A153-2 (10 g, 31 mmol) was dissolved in 80 mL of DMF, and formic acid (5 g, 109 mmol), palladium acetate (209 mg, 0.93 mmol), DCC (1.3 g, 6.2 mmol), triethylamine (6.3 g, 62 mmol) and Xantphos (539 mg, 0.93 mmol) were successively added. The mixture was reacted at 100°C under nitrogen protection for 3 h, and then TLC showed that the reaction was completed. The reaction liquid was filtered, and then the filtrate was subjected to rotary evaporation to remove most of the solvent. The mixture was slurried with 50 mL of dichloromethane and then subjected to suction filtration. The filter cake was dried to obtain compound A152-3 (7.8 g, yield 88%).

¹H NMR (400 MHz, DMSO) δ 11.02 (s, 1H), 8.17 (s, 1H), 8.00 (d, *J* = 7.9 Hz, 1H), 7.96 (s, 1H), 7.84 (d, *J* = 7.9 Hz, 1H), 5.14 (dd, *J* = 13.3, 5.1 Hz, 1H), 4.55 - 4.37 (m, 2H), 2.99 - 2.84 (m, 1H), 2.69 - 2.58 (m, 1H), 2.46 - 2.38 (m, 1H), 2.07 - 1.95 (m, 1H). Synthesis of compound A153-4

Compound A153-3 (730 mg, 2.53 mmol), 1-(N-Boc-aminomethyl)-4-(aminomethyl)benzene (600 mg, 2.53 mmol) and DIPEA (1.96 g, 15.18 mmol) were successively added to 2 mL of DMF. Under ice bath, HATU (1.15 g, 3.04 mmol) was added. The mixture was reacted at room temperature for 1 h, and then TLC showed that the reaction was completed. 120 mL of water/ethyl acetate (1 : 1) was added, and the mixture was slurried for 1 h and then filtered. The filter cake was washed with ethyl acetate and then dried to obtain compound A153-4 (280 mg, 22%).

¹H NMR (400 MHz, DMSO) δ 11.01 (s, 1H), 9.21 (t, *J* = 5.8 Hz, 1H), 8.09 (s, 1H), 8.01 (d, *J* = 8.3 Hz, 1H), 7.82 (d, *J* = 8.0 Hz, 1H), 7.36 (s, 1H), 7.28 (d, *J* = 7.8 Hz, 2H), 7.19 (d, *J* = 7.9 Hz, 2H), 5.14 (dd, *J* = 13.3, 5.0 Hz, 1H), 4.56 - 4.36 (m, 4H), 4.09 (d, *J* = 5.9 Hz, 2H), 2.97 - 2.85 (m, 1H), 2.69 - 2.57 (m, 1H), 2.46 - 2.37 (m, 1H), 2.08 - 1.98 (m, 1H), 1.38 (s, 9H).

### Synthesis of compound A153-5

Compound A153-4 (50 mg, 0.10 mmol) was dissolved in dichloromethane (4 mL), and TFA (1 mL) was added. The mixture was reacted at room temperature for 1 h, and then monitoring by TLC showed that the raw materials were completely reacted. The solvent was dried by rotary evaporation to obtain compound A153-5 (70 mg, crude) as an oil, which was directly used in the next reaction.

### Synthesis of compound A153

Compound A153-5 (70 mg, 0.10 mmol) and 2-(9H-pyrido[2,3-b]indole)acetic acid (20 mg, 0.09 mmol) were dissolved in dichloromethane (6 mL), and HATU (30 mg, 0.10 mmol) and triethylamine (16 mg, 0.20 mmol) were added. The mixture was reacted at room temperature for 2 h, and then monitoring by TLC showed that the raw materials were completely reacted. Water (20 ml) was added, and the mixture was extracted with dichloromethane (20 mL*3). The organic phases were combined, washed with saturated brine (25 ml), dried over anhydrous sodium sulfate and concentrated. The solute was purified by a preparative plate (developing solvent: DCM : MeOH= 10 : 1), and then purified by medium-pressure preparative chromatography to obtain compound A153 (4 mg, yield 6%).

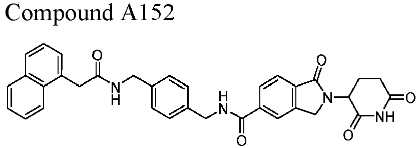

ESI-MS(M+H)⁺: 575.2.

¹H NMR (400 MHz, DMSO) δ 10.99 (s, 1H), 9.19 (t, *J* = 6.0 Hz, 1H), 8.61 (t, *J* = 5.8 Hz, 1H), 8.14 - 8.04 (m, 2H), 8.00 (d, *J* = 7.9 Hz, 1H), 7.94 - 7.86 (m, 1H), 7.84 - 7.78 (m, 2H), 7.55 - 7.46 (m, 2H), 7.46 - 7.40 (m, 2H), 7.26 (d, *J* = 8.1 Hz, 2H), 7.19 (d, *J* = 8.1 Hz, 2H), 5.14 (dd, *J* = 13.3, 5.1 Hz, 1H), 4.55 - 4.37 (m, 4H), 4.26 (d, *J* = 5.8 Hz, 2H), 3.95 (s, 2H), 2.99 - 2.84 (m, 1H), 2.69 - 2.58 (m, 1H), 2.46 - 2.38 (m, 1H), 2.07 - 1.95 (m, 1H).

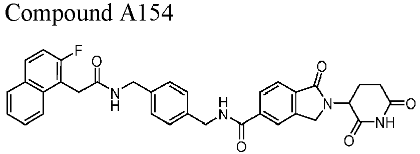

ESI-MS(M+H)⁺: 593.2.

¹H NMR (400 MHz, DMSO) δ 11.03 (s, 1H), 9.40 (t, *J* = 5.9 Hz, 1H), 8.90 (t, *J* = 5.9 Hz, 1H), 8.14 (s, 1H), 8.04 (d, *J* = 8.3 Hz, 2H), 7.98 - 7.87 (m, 2H), 7.81 (d, *J* = 7.9 Hz, 1H), 7.55 (t, *J* = 7.5 Hz, 1H), 7.49 - 7.39 (m, 2H), 7.28 (d, *J* = 8.0 Hz, 2H), 7.21 (d, *J* = 8.0 Hz, 2H), 5.14 (dd, *J* = 13.2, 5.1 Hz, 1H), 4.54 - 4.36 (m, 4H), 4.24 (d, *J* = 6.0 Hz, 2H), 4.00 (s, 2H), 2.98 - 2.84 (m, 1H), 2.67 - 2.55 (m, 1H), 2.46 - 2.32 (m, 1H), 2.08 - 1.94 (m, 1H).

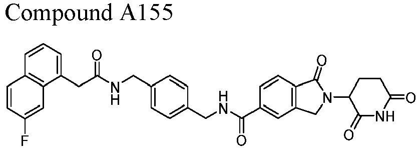

ESI-MS(M+H)⁺: 593.2.

¹H NMR (400 MHz, DMSO) δ 11.02 (s, 1H), 9.22 (t, *J* = 5.8 Hz, 1H), 8.70 (t, *J* = 5.7 Hz, 1H), 8.08 (s, 1H), 8.03 - 7.97 (m, 2H), 7.90 - 7.87 (m, 3H), 7.49 (d, *J* = 6.9 Hz, 1H), 7.45 - 7.36 (m, 2H), 7.26 (d, *J* = 8.0 Hz, 2H), 7.20 (d, *J* = 8.0 Hz, 2H), 5.15 (dd, *J* = 13.3, 5.0 Hz, 1H), 4.57 - 4.33 (m, 4H), 4.26 (d, *J* = 5.7 Hz, 2H), 3.92 (s, 2H), 2.98 - 2.85 (m, 1H), 2.66 - 2.55 (m, 1H), 2.44 - 2.34 (m, 1H), 2.07 - 1.93 (m, 1H).

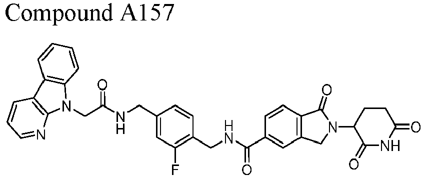

ESI-MS(M+H)⁺: 633.2.

¹H NMR (400 MHz, DMSO) δ 11.03 (s, 1H), 9.21 (t, *J* = 5.6 Hz, 1H), 8.80 (t, *J* = 6.0 Hz, 1H), 8.55 (dd, *J* = 7.6, 1.4 Hz, 1H), 8.48 - 8.43 (m, 1H), 8.22 (d, *J* = 7.8 Hz, 1H), 8.10 (s, 1H), 8.01 (d, *J* = 8.0 Hz, 1H), 7.83 (d, *J* = 8.0 Hz, 1H), 7.60 (d, *J* = 8.3 Hz, 1H), 7.50 (t, *J* = 7.8 Hz, 1H), 7.33 (t, *J* = 7.9 Hz, 1H), 7.30 - 7.23 (m, 2H), 7.10 (dd, *J* = 16.3, 9.7 Hz, 2H), 5.23 - 5.10 (m, 3H), 4.58 - 4.36 (m, 4H), 4.30 (d, *J* = 6.0 Hz, 2H), 2.98 - 2.85 (m, 1H), 2.72 - 2.54 (m, 1H), 2.45 - 2.32 (m, 1H), 2.05 - 1.94 (m, 1H).

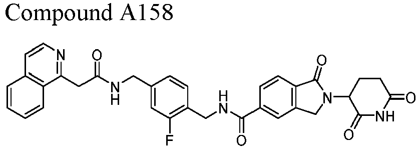

ESI-MS(M+H)⁺: 594.2
¹H NMR (400 MHz, DMSO) δ 11.03 (s, 1H), 9.20 (t, *J* = 5.9 Hz, 1H), 8.76 (t, *J* = 5.9 Hz, 1H), 8.41 (d, *J* = 5.6 Hz, 1H), 8.24 (d, *J* = 8.9 Hz, 1H), 8.09 (s, 1H), 7.99 (dd, *J* = 18.2, 8.1 Hz, 2H), 7.82 (d, *J* = 7.9 Hz, 1H), 7.77 - 7.73 (m, 2H), 7.64 (t, *J* = 7.6 Hz, 1H), 7.32 (t, *J* = 7.8 Hz, 1H), 7.10 (dd, *J* = 18.2, 9.2 Hz, 2H), 5.15 (d, *J =* 8.4 Hz, 1H), 4.54 - 4.36 (m, 4H), 4.29 (d, *J* = 6.0 Hz, 2H), 4.23 (s, 2H), 2.98 - 2.85 (m, 1H), 2.68 - 2.58 (m, 1H), 2.44 - 2.35 (m, 1H), 2.07 - 1.98 (m, 1H).

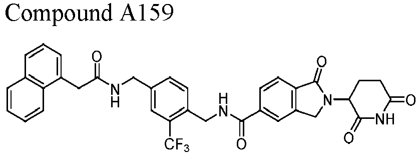

ESI-MS(M+H)⁺: 643.2
¹H NMR (400 MHz, DMSO) δ 11.04 (s, 1H), 9.30 (t, *J* = 5.6 Hz, 1H), 8.75 (t, *J* = 5.9 Hz, 1H), 8.12 (s, 1H), 8.09 - 8.01 (m, 2H), 7.95 - 7.90 (m, 1H), 7.89 - 7.79 (m, 2H), 7.58 (s, 1H), 7.54 - 7.40 (m, 6H), 5.16 (dd, *J* = 13.3, 5.0 Hz, 1H), 4.66 (d, *J* = 4.4 Hz, 2H), 4.56 - 4.37 (m, 2H), 4.34 (d, *J* = 5.8 Hz, 2H), 3.97 (s, 2H), 3.01 - 2.89 (m, 1H), 2.67 - 2.58 (m, 1H), 2.47 - 2.34 (m, 1H), 2.08 - 1.96 (m, 1H).

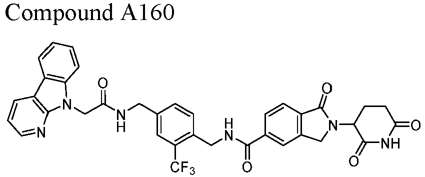

ESI-MS(M+H)⁺: 683.2
¹H NMR (400 MHz, DMSO) δ 11.03 (s, 1H), 9.32 (t, *J* = 5.8 Hz, 1H), 8.87 (t, *J* = 6.0 Hz, 1H), 8.58 (dd, *J* = 7.6, 1.4 Hz, 1H), 8.44 (dd, *J* = 4.9, 1.4 Hz, 1H), 8.22 (d, *J* = 7.7 Hz, 1H), 8.14 (s, 1H), 8.05 (d, *J* = 8.1 Hz, 1H), 7.85 (d, *J* = 7.9 Hz, 1H), 7.63 (s, 1H), 7.60 - 7.46 (m, 4H), 7.32 - 7.25 (m, 2H), 5.24 - 5.11 (m, 3H), 4.66 (d, *J* = 5.1 Hz, 2H), 4.48 (dd, *J* = 52.5, 17.7 Hz, 2H), 4.36 (d, *J* = 5.9 Hz, 2H), 2.99 - 2.85 (m, 1H), 2.68 - 2.56 (m, 1H), 2.47 - 2.32 (m, 1H), 2.09 - 2.00 (m, 1H).

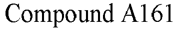

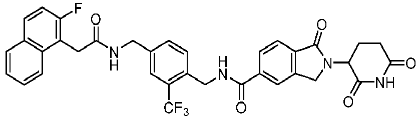

ESI-MS(M+H)⁺: 661.2
¹H NMR (400 MHz, DMSO) δ 11.04 (s, 1H), 9.31 (t, *J* = 5.6 Hz, 1H), 8.83 (t, *J* = 5.8 Hz, 1H), 8.14 (s, 1H), 8.04 (dd, *J* = 13.7, 8.2 Hz, 2H), 8.00 - 7.90 (m, 2H), 7.86 (d, *J* = 7.9 Hz, 1H), 7.61 (s, 1H), 7.58 - 7.38 (m, 5H), 5.17 (dd, *J* = 13.2, 5.0 Hz, 1H), 4.66 (d, *J* = 4.9 Hz, 2H), 4.57 - 4.39 (m, 2H), 4.35 (d, *J* = 5.8 Hz, 2H), 4.02 (s, 2H), 3.02 - 2.87 (m, 1H), 2.68 - 2.55 (m, 1H), 2.47 - 2.36 (m, 1H), 2.08 - 1.93 (m, 1H).

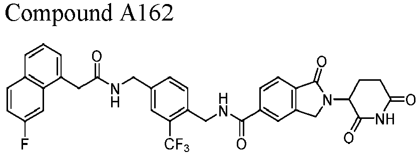

ESI-MS(M+H)⁺: 661.2
¹H NMR (400 MHz, DMSO) δ 11.03 (s, 1H), 9.28 (t, *J* = 5.6 Hz, 1H), 8.79 (t, *J* = 5.9 Hz, 1H), 8.12 (s, 1H), 8.07 - 7.98 (m, 2H), 7.90 - 7.78 (m, 3H), 7.54 (s, 1H), 7.52 - 7.36 (m, 5H), 5.16 (dd, *J* = 13.3, 5.1 Hz, 1H), 4.63 (d, *J* = 5.1 Hz, 2H), 4.47 (dd, *J* = 52.7, 17.6 Hz, 2H), 4.34 (d, *J* = 5.8 Hz, 2H), 3.93 (s, 2H), 3.00 - 2.85 (m, 1H), 2.69 - 2.56 (m, 1H), 2.45 - 2.34 (m, 1H), 2.09 - 1.98 (m, 1H).

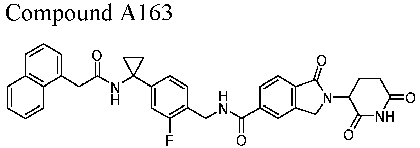

ESI-MS(M+H)⁺: 619.2
¹H NMR (400 MHz, DMSO) δ 11.02 (s, 1H), 9.15 (t, *J* = 5.4 Hz, 1H), 9.00 (s, 1H), 8.17 - 8.04 (m, 2H), 7.98 (d, *J* = 7.9 Hz, 1H), 7.92 (d, *J* = 7.3 Hz, 1H), 7.81 (d, *J* = 7.9 Hz, 2H), 7.57 - 7.39 (m, 4H), 7.20 (t, *J* = 8.0 Hz, 1H), 6.94 - 6.81 (m, 2H), 5.14 (dd, *J* = 13.2, 5.0 Hz, 1H), 4.53 - 4.28 (m, 4H), 3.94 (s, 2H), 2.97 - 2.77 (m, 1H), 2.66 - 2.56 (m, 1H), 2.45 - 2.34 (m, 1H), 2.07 - 1.91 (m, 1H), 1.23 - 1.14 (m, 4H).

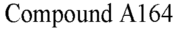

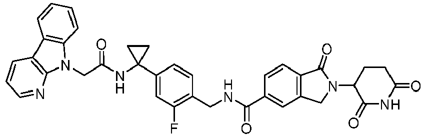

ESI-MS(M+H)⁺: 659.2
¹H NMR (400 MHz, DMSO) δ 11.03 (s, 1H), 9.22 - 9.07 (m, 2H), 8.54 (dd, *J* = 7.6, 1.3 Hz, 1H), 8.47 (dd, *J* = 4.8, 1.3 Hz, 1H), 8.20 (d, *J* = 7.8 Hz, 1H), 8.07 (s, 1H), 7.98 (d, *J* = 7.8 Hz, 1H), 7.81 (d, *J* = 7.9 Hz, 1H), 7.61 (d, *J* = 8.2 Hz, 1H), 7.50 (t, *J* = 7.7 Hz, 1H), 7.32 - 7.22 (m, 3H), 7.11 (dd, *J* = 12.0, 1.3 Hz, 1H), 6.93 (dd, *J* = 8.1, 1.4 Hz, 1H), 5.20 - 5.07 (m, 3H), 4.53 - 4.35 (m, 4H), 2.98 - 2.88 (m, 1H), 2.65 - 2.55 (m, 1H), 2.46 - 2.37 (m, 1H), 2.05 - 1.96 (m, 1H), 1.23 - 1.14 (m, 4H).

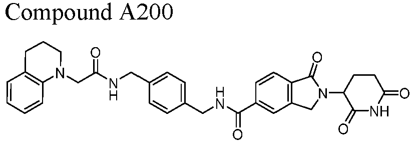

ESI-MS(M+H)⁺: 580.2
¹H NMR (400 MHz, DMSO) δ 11.01 (s, 1H), 9.21 (d, *J* = 5.8 Hz, 1H), 8.37 (d, *J* = 5.7 Hz, 1H), 8.10 (s, 1H), 8.01 (d, J = 8 Hz, 1H), 7.82 (d, *J* = 7.8 Hz, 1H), 7.27 (d, *J* = 7.7 Hz, 2H), 7.19 (d, *J* = 7.9 Hz, 2H), 6.99 - 6.82 (m, 2H), 6.50 (t, *J* = 7.3 Hz, 1H), 6.37 (d, *J* = 8.0 Hz, 1H), 5.15 (dd, *J* = 13.2, 4.8 Hz, 1H), 4.60 - 4.35 (m, 4H), 4.27 (d, *J* = 5.8 Hz, 2H), 3.84 (s, 2H), 3.35 (s, 2H), 2.99 - 2.88 (m, 1H), 2.70 (t, *J* = 6.0 Hz, 2H), 2.62 (d, *J* = 17.3 Hz, 1H), 2.46 - 2.37 (m, 1H), 2.11 - 1.99 (m, 1H), 1.98 - 1.85 (m, 2H).

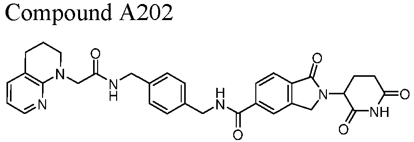

ESI-MS(M+H)⁺: 581.2
¹H NMR (400 MHz, DMSO) δ 11.02 (s, 1H), 9.24 (t, *J* = 5.9 Hz, 1H), 8.27 (s, 1H), 8.10 (s, 1H), 8.02 (d, *J* = 7.9 Hz, 1H), 7.84-7.81 (m, 2H), 7.27 (d, *J* = 8.1 Hz, 2H), 7.22-7.17 (m, 3H), 6.52 - 6.43 (m, 1H), 5.15 (dd, *J=* 13.3, 5.1 Hz, 1H), 4.55-4.38 (m, 4H), 4.25 (d, *J* = 6.0 Hz, 2H), 4.14 (s, 2H), 3.49 - 3.40 (m, 2H), 2.95 - 2.86 (m, 1H), 2.72 (t, *J=* 6.2 Hz, 2H), 2.61 (d, *J* = 16.6 Hz, 1H), 2.43 (dd, *J* = 13.1, 4.5 Hz, 1H), 2.06-1.99 (m, 1H), 1.94 - 1.85 (m, 2H).

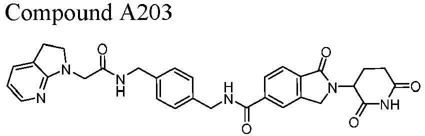

ESI-MS(M+H)⁺: 567.2
¹H NMR (400 MHz, DMSO) δ 11.02 (s, 1H), 9.21 (t, *J* = 5.9 Hz, 1H), 8.48 (t, *J* = 6.0 Hz, 1H), 8.09 (s, 1H), 8.01 (d, *J* = 8.0 Hz, 1H), 7.82 (d, *J* = 7.9 Hz, 1H), 7.74 (d, *J* = 4.0 Hz, 1H), 7.30 - 7.20 (m, 5H), 6.46 (dd, *J* = 6.9, 5.3 Hz, 1H), 5.14 (dd, *J* = 13.3, 5.1 Hz, 1H), 4.54 - 4.36 (m, 4H), 4.26 (d, *J* = 6.0 Hz, 2H), 3.93 (s, 2H), 3.53 (t, *J* = 8.4 Hz, 2H), 3.02 - 2.82 (m, 3H), 2.67 - 2.58 (m, 1H), 2.45 - 2.29 (m, 1H), 2.06 - 1.96 (m, 1H).

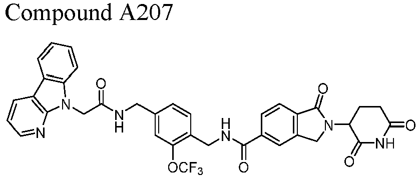

ESI-MS(M+H)⁺: 699.2
¹H NMR (400 MHz, DMSO) δ 11.02 (s, 1H), 9.22 (t, *J* = 5.8 Hz, 1H), 8.82 (t, *J* = 6.0 Hz, 1H), 8.55 (dd, *J* = 7.6, 1.6 Hz, 1H), 8.43 (dd, *J* = 4.8, 1.6 Hz, 1H), 8.21 (d, *J* = 7.7 Hz, 1H), 8.10 (s, 1H), 8.02 (d, *J* = 9.0 Hz, 1H), 7.83 (d, *J* = 8.1 Hz, 1H), 7.56 (d, *J* = 8.2 Hz, 1H), 7.52 - 7.44 (m, 1H), 7.41 (d, *J* = 7.9 Hz, 1H), 7.33 - 7.22 (m, 4H), 5.17 (s, 2H), 5.16 - 5.11 (m, 1H), 4.58 - 4.36 (m, 4H), 4.33 (d, *J* = 5.9 Hz, 2H), 3.00 - 2.86 (m, 1H), 2.70 - 2.59 (m, 1H), 2.45 - 2.35 (m, 1H), 2.07 - 2.01 (m, 1H).

The intermediates were prepared as follows:

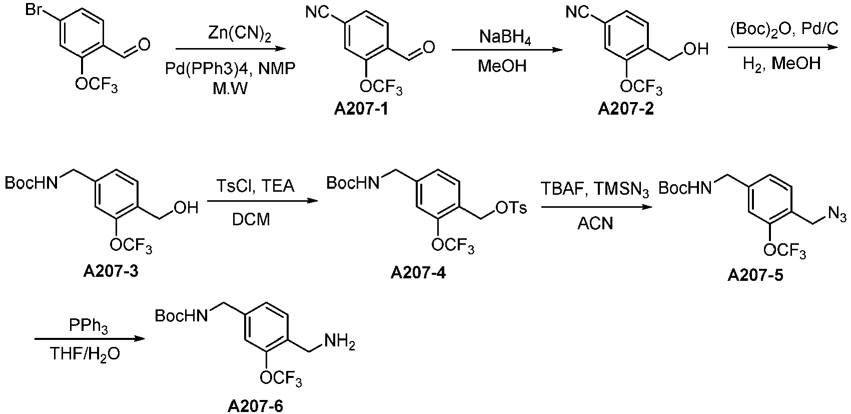

### Synthesis of compound A207-1

4-bromo-2-(trifluoromethoxy)benzaldehyde (10 g, 36.2 mmol) was dissolved in 18 mL of NMP, and zinc cyanide(3.0 g, 26.2 mmol) and tetrakis(triphenylphosphine)palladium (2.2 g, 1.8 mmol) were successively added. The mixture was reacted under microwave at 200°C under nitrogen protection for 5 min, and then TLC showed that the reaction was completed. The mixture was cooled to room temperature. The reaction liquid was filtered, and the filtrate was diluted with 60 mL of water and extracted with ethyl acetate (60 mL*3). The organic phases were combined, washed with saturated brine (20 mL), dried over anhydrous sodium sulfate and dried by rotary evaporation. The solute was separated and purified by column chromatography (mobile phase: petroleum ether : ethyl acetate = 10 : 1) to obtain compound A207-1 (300 mg, yield 4%).

### Synthesis of compound A207-2

A207-1 (300 mg, 1.4 mmol) was dissolved in 12 mL of methanol. Under ice bath, sodium borohydride (65 mg, 1.7 mmol) was added. The mixture was reacted at room temperature for 2 h, and then TLC showed that the reaction was completed. The reaction was quenched with ice water (20 mL). The aqueous phase was extracted with ethyl acetate (80 ml*3). The organic phases were combined, dried over anhydrous sodium sulfate and dried by rotary evaporation. The solute was separated and purified by column chromatography (mobile phase: petroleum ether : ethyl acetate = 3 : 1) to obtain compound A207-2 (230 mg, yield 76%).

### Synthesis of compound A207-3

Compound A207-2 (230 mg, 1.1 mmol) was dissolved in 15 mL of methanol, and (Boc)₂O (350 mg, 1.6 mmol) and Pd/C (30 mg, 5%) were added. The mixture was reacted overnight at room temperature under hydrogen atmosphere, and then TLC showed that the reaction was completed. The reaction liquid was filtered, and the filtrate was dried by rotary evaporation. The solute was separated and purified by column chromatography (mobile phase: petroleum ether : ethyl acetate = 2 : 1) to obtain compound A207-3 (270 mg, yield 79%).

### Synthesis of compound A207-4

Compound A207-3 (90 mg, 0.30 mmol) was dissolved in dichloromethane (15 ml). Under ice bath, p-toluenesulfonyl chloride (80 mg, 0.40 mmol) and triethylamine (60 mg, 0.60 mmol) were added. The reaction was stirred at room temperature for 3 h, and then monitoring by TLC showed that the reaction was completed. The reaction was quenched with water (20 mL). The aqueous phase was extracted with dichloromethane (30 ml*3). The organic phases were combined, dried over anhydrous sodium sulfate and dried by rotary evaporation. The solute was separated and purified by column chromatography (mobile phase: petroleum ether : ethyl acetate = 3 : 1) to obtain A207-4 (75 mg, yield 56%).

### Synthesis of compound A207-5

Compound A207-4 (75 mg, 0.16 mmol) was dissolved in dichloromethane (12 ml), and TMSN₃ (37 mg, 0.3 mmol) and TBAF (87 mg, 0.3 mmol) were added. The mixture was reacted overnight at 60°C, and then TLC showed that the reaction was completed. The mixture was cooled to room temperature. The reaction was quenched with water (20 mL). The aqueous phase was extracted with ethyl acetate (30 ml*3). The organic phases were combined, dried over anhydrous sodium sulfate and dried by rotary evaporation to obtain A207-5 (60 mg, crude).

### Synthesis of compound A207-6

Compound A207-5 (60 mg, crude, 0.16 mmol) was dissolved in THF/H₂O (10/2 mL), and triphenylphosphine (50 mg, 0.2 mmol) was added. The mixture was reacted at 50°C for 2 h, and then TLC showed that the reaction was completed. The mixture was cooled to room temperature and adjusted to pH 4 with 2 N HCl. The aqueous phase was extracted with ethyl acetate (10 mL). The aqueous phase remained, was adjusted to pH 8 with saturated sodium carbonate solution and then extracted with dichloromethane/methanol (10 : 1, 30 ml*3). The organic phases were combined, dried over anhydrous sodium sulfate and dried by rotary evaporation to obtain A207-6 (60 mg, crude).

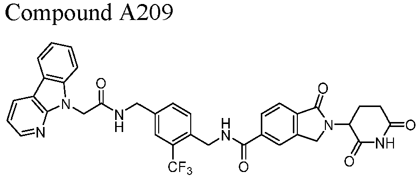

ESI-MS(M+H)⁺: 683.2
¹H NMR (400 MHz, DMSO) δ 11.03 (s, 1H), 9.30 (s, 1H), 8.84 (s, 1H), 8.55 (d, *J* = 7.6 Hz, 1H), 8.43 (d, *J* = 4.1 Hz, 1H), 8.21 (d, *J* = 7.6 Hz, 1H), 8.13 (s, 1H), 8.05 (d, *J* = 8.1 Hz, 1H), 7.86 (d, *J* = 7.9 Hz, 1H), 7.63 (s, 1H), 7.52 (dt, *J* = 17.2, 8.3 Hz, 4H), 7.27 (dd, *J* = 13.0, 5.8 Hz, 2H), 5.25 - 5.07 (m, 3H), 4.65 (s, 2H), 4.59 - 4.28 (m, 4H), 3.00 - 2.85 (m, 1H), 2.61 (d, *J* = 17.4 Hz, 1H), 2.44 (d, *J* = 14.1 Hz, 1H), 2.02 (d, *J* = 24.0 Hz, 1H).

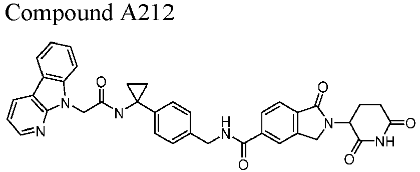

ESI-MS(M+H)⁺: 641.2
¹H NMR (400 MHz, DMSO) δ 11.00 (s, 1H), 9.16 (t, *J* = 5.8 Hz, 1H), 9.07 (s, 1H), 8.53 (dd, *J* = 7.6, 1.5 Hz, 1H), 8.46 (dd, *J* = 4.8, 1.5 Hz, 1H), 8.20 (d, *J* = 7.8 Hz, 1H), 8.07 (s, 1H), 7.99 (d, *J* = 8.3 Hz, 1H), 7.81 (d, *J* = 7.9 Hz, 1H), 7.57 (d, *J* = 8.2 Hz, 1H), 7.50 (t, *J* = 7.2 Hz, 1H), 7.31 - 7.14 (m, 6H), 5.17 - 5.09 (m, 3H), 4.53 - 4.36 (m, 4H), 2.97

- 2.85 (m, 1H), 2.69 - 2.56 (m, 1H), 2.45 - 2.31 (m, 1H), 2.07 - 1.94 (m, 1H), 1.19 - 1.10 (m, 4H).

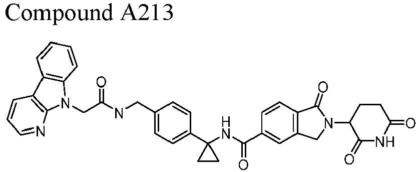

ESI-MS(M+H)⁺: 641.2
¹H NMR (400 MHz, DMSO) δ 11.01 (s, 1H), 9.37 (s, 1H), 8.72 (t, *J* = 5.8 Hz, 1H), 8.54 (dd, *J* = 7.6, 1.5 Hz, 1H), 8.44 (dd, *J* = 4.8, 1.5 Hz, 1H), 8.20 (d, *J* = 7.7 Hz, 1H), 8.09 (s, 1H), 8.01 (d, *J* = 8.0 Hz, 1H), 7.82 (d, *J* = 7.9 Hz, 1H), 7.56 (d, *J* = 8.2 Hz, 1H), 7.49 (t, *J* = 7.2 Hz, 1H), 7.32 - 7.22 (m, 2H), 7.22 - 7.13 (m, 4H), 5.20 - 5.11 (m, 3H), 4.47 (dd, *J* = 49.8, 17.6 Hz, 2H), 4.25 (d, *J* = 5.8 Hz, 2H), 2.99 - 2.83 (m, 1H), 2.66 - 2.57 (m, 1H), 2.47 - 2.33 (m, 1H), 2.10 - 1.99 (m, 1H), 1.35 - 1.24 (m, 4H).

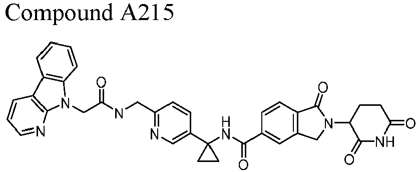

ESI-MS(M+H)⁺: 642.2
¹H NMR (400 MHz, DMSO) δ 11.01 (s, 1H), 9.44 (s, 1H), 8.82 (t, *J* = 5.8 Hz, 1H), 8.54 (dd, *J* = 7.6, 1.5 Hz, 1H), 8.45 (dd, *J* = 4.8, 1.5 Hz, 1H), 8.40 (d, *J* = 2.1 Hz, 1H), 8.20 (d, *J* = 7.7 Hz, 1H), 8.08 (s, 1H), 8.00 (d, *J* = 8.1 Hz, 1H), 7.82 (d, *J* = 7.9 Hz, 1H), 7.64 - 7.56 (m, 2H), 7.49 (t, *J* = 7.2 Hz, 1H), 7.30 - 7.23 (m, 3H), 5.21 - 5.08 (m, 3H), 4.46 (dd, *J* = 49.7, 17.7 Hz, 2H), 4.35 (d, *J* = 5.8 Hz, 2H), 2.98 - 2.84 (m, 1H), 2.68 - 2.55 (m, 1H), 2.46 - 2.35 (m, 1H), 2.08 - 1.99 (m, 1H), 1.32 (d, *J* = 4.2 Hz, 4H).

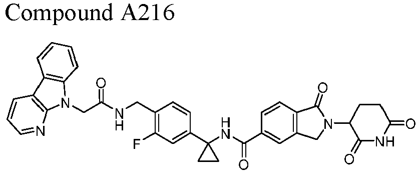

ESI-MS(M+H)⁺: 659.2
¹H NMR (400 MHz, DMSO) δ 11.01 (s, 1H), 9.40 (s, 1H), 8.74 (t, *J* = 5.6 Hz, 1H), 8.55 (d, *J* = 6.9 Hz, 1H), 8.45 (d, *J* = 4.6 Hz, 1H), 8.20 (d, *J* = 7.7 Hz, 1H), 8.10 (s, 1H), 8.01 (d, *J* = 8.1 Hz, 1H), 7.83 (d, *J* = 7.9 Hz, 1H), 7.56 (d, *J* = 8.1 Hz, 1H), 7.49 (t, *J* = 7.6 Hz, 1H), 7.33 - 7.18 (m, 3H), 7.00 (t, *J* = 9.9 Hz, 2H), 5.21 - 5.09 (m, 3H), 4.47 (dd, *J* = 49.6, 17.6 Hz, 2H), 4.28 (d, *J* = 5.5 Hz, 2H), 3.00 - 2.83 (m, 1H), 2.70 - 2.54 (m, 1H), 2.48 - 2.34 (m, 1H), 2.08 - 1.96 (m, 1H), 1.31 (s, 4H).

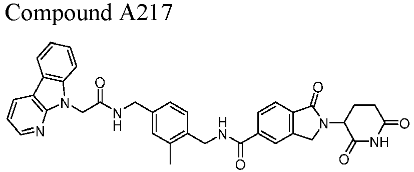

ESI-MS(M+H)⁺: 629.2
¹H NMR (400 MHz, DMSO) δ 11.01 (s, 1H), 9.07 (t, *J* = 5.5 Hz, 1H), 8.71 (t, *J* = 5.8 Hz, 1H), 8.55 (dd, *J* = 7.6, 1.4 Hz, 1H), 8.45 (dd, *J* = 4.8, 1.4 Hz, 1H), 8.21 (d, *J* = 7.7 Hz, 1H), 8.10 (s, 1H), 8.02 (d, *J* = 7.9 Hz, 1H), 7.82 (d, *J* = 7.9 Hz, 1H), 7.56 (t, *J* = 12.2 Hz, 1H), 7.49 (dd, *J* = 17.4, 10.0 Hz, 1H), 7.30 - 7.15 (m, 3H), 7.12 - 7.02 (m, 2H), 5.21 - 5.11 (m, 3H), 4.55 - 4.37 (m, 4H), 4.25 (d, *J* = 5.8 Hz, 2H), 2.98 - 2.84 (m, 1H), 2.70 - 2.54 (m, 1H), 2.46 - 2.35 (m, 1H), 2.32 (s, 3H), 2.08 - 1.97 (m, 1H).

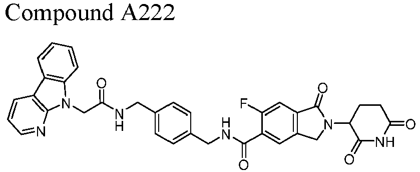

ESI-MS(M+H)⁺: 633.2
¹H NMR (400 MHz, DMSO) δ 11.02 (s, 1H), 9.09 (t, *J* = 5.7 Hz, 1H), 8.81 (t, *J* = 6.0 Hz, 1H), 8.56 (dd, *J* = 7.6, 1.6 Hz, 1H), 8.46 (dd, *J* = 4.8, 1.6 Hz, 1H), 8.22 (d, *J* = 7.7 Hz, 1H), 7.85 (d, *J* = 5.9 Hz, 1H), 7.64 (d, *J* = 8.9 Hz, 1H), 7.59 (d, *J* = 8.2 Hz, 1H), 7.55 - 7.48 (m, 1H), 7.35 - 7.24 (m, 6H), 5.21 - 5.11 (m, 3H), 4.53 - 4.45 (m, 3H), 4.40-4.28 (m, 3H), 2.97 - 2.90 (m, 1H), 2.61 (d, *J* = 15.6 Hz, 1H), 2.41 (dd, *J* = 13.0, 4.7 Hz, 1H), 2.08 - 2.01 (m, 1H).

The intermediates were prepared as follows:

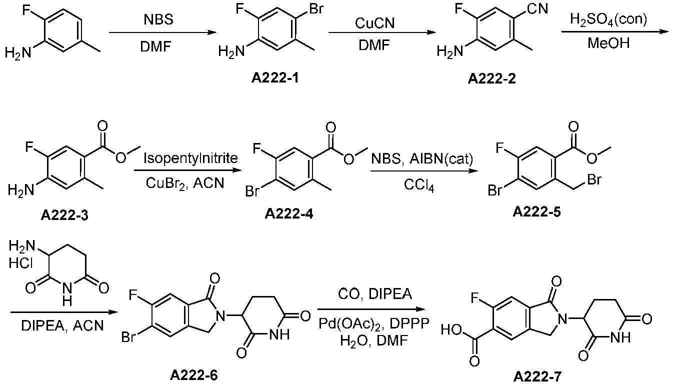

### Synthesis of compound A222-1

The compound 2-fluoro-5-methylaniline (24.34 g, 0.195 mol) was dissolved in DMF (240 ml), and NBS (34.62 g, 0.195 mol) was added in batches under ice bath. After the addition was completed, the mixture was reacted at room temperature for half an hour, and then TLC showed that the reaction was completed. The reaction was quenched with 450 mL of ice water. The aqueous phase was extracted with ethyl acetate (500 ml*3). The organic phases were combined, washed with saturated brine (100 mL*3), dried over anhydrous sodium sulfate and dried by rotary evaporation. The mixture was slurried with methyl tert-butyl ether to obtain A222-1 (32.11 g, yield 81%).

### Synthesis of compound A222-2

Compound A222-1 (32.11 g, 0.157 mol) and cuprous cyanide (18.33 g, 0.205 mol) were dissolved in DMF (320 ml). The mixture was reacted at 200°C under nitrogen protection for 1 h, and then TLC showed that the reaction was completed. The mixture was cooled to room temperature. 500 mL of saturated ammonium chloride solution was added to the reaction liquid, and the mixture was filtered. The filtrate was extracted with ethyl acetate (600 mL*2). The organic phases were combined, washed with saturated brine (200 mL*2), dried over anhydrous sodium sulfate and dried by rotary evaporation. The solute was separated and purified by column chromatography (mobile phase: petroleum ether : ethyl acetate = 7 : 1) to obtain compound A222-2 (18.17 g, yield 77%).

### Synthesis of compound A222-3

Compound A222-2 (18.0 g, 0.120 mol) and concentrated sulfuric acid (18 M, 45 mL) were dissolved in 180 mL of methanol. The mixture was reacted overnight at 75°C, and then TLC showed that the reaction was completed. The mixture was cooled to room temperature and adjusted to pH 8 with saturated sodium carbonate solution. The aqueous phase was extracted with ethyl acetate (250 mL*3). The organic phases were combined, dried over anhydrous sodium sulfate and dried by rotary evaporation. The solute was separated and purified by column chromatography (mobile phase: petroleum ether : ethyl acetate = 7 : 1) to obtain compound A222-3 (13.7 g, yield 62%).

### Synthesis of compound A222-4

Compound A222-3 (13.63 g, 0.094 mol) was dissolved in 260 mL of acetonitrile. Under ice bath, isoamyl nitrite (12.2 g, 0.104 mol) was added dropwise. The mixture was reacted overnight at room temperature, and then TLC showed that the reaction was completed. Most of acetonitrile was dried by rotary evaporation, and then the mixture was adjusted to pH 3-4 with 1 N hydrochloric acid. The aqueous phase was extracted with ethyl acetate (100 mL*3). The organic phases were combined, dried over anhydrous sodium sulfate and dried by rotary evaporation. The solute was separated and purified by column chromatography (mobile phase: petroleum ether : ethyl acetate = 30 : 1) to obtain compound A222-4 (17.1 g, yield 93%).

### Synthesis of compound A222-5

Compound A222-4 (17.1 g, 0.069 mol), NBS (14.8 g, 0.083 mol) and AIBN (3.4 g, 0.020 mol) were dissolved in 170 mL of carbon tetrachloride. The mixture was reacted at 80°C for 2 h, and then TLC showed that the reaction was completed. The mixture was cooled to room temperature. The solvent was dried by rotary evaporation. The mixture was slurried with methyl tert-butyl ether (100 mL), filtered and dried to obtain compound A222-5 (26.57 g, crude).

### Synthesis of compound A222-6

Compound A222-5 (26.57 g, crude, 0.069 mol), 3-amino-2,6-piperidinedione hydrochloride (12.84 g, 0.078 mol) and DIPEA (14.8 g, 0.083 mol) were dissolved in 250 mL of acetonitrile. The mixture was reacted overnight at 80°C, and then TLC showed that the reaction was completed. The mixture was cooled to room temperature. The solvent was dried by rotary evaporation. The mixture was slurried with methyl tert-butyl ether/water (100 mL/100 mL) and then filtered. The filter cake was dried to obtain compound A222-6 (6.6 g, yield 20%).

### Synthesis of compound A222-7

Compound A222-6 (2 g, 0.006 mol), palladium acetate (395 mg, 0.002 mol), water (633 mg, 0.035 mol) and DIPEA (3.79 g, 0.029 mol) were dissolved in 20 mL of DMF, and DPPP (726 mg, 0.002 mol) was added. The mixture was reacted at 90°C under carbon monoxide gas atmosphere for 2 days. TLC showed that the reaction was completed. The reaction was cooled to room temperature and quenched with 40 mL of ice water. The mixture was filtered. The filtrate was extracted with ethyl acetate (50 mL*4). The aqueous phase was adjusted to pH 2 with 1 N hydrochloric acid. The aqueous phase was further extracted with ethyl acetate (50 mL*3). The organic phases were combined, washed with saturated brine (50 mL*2), dried over anhydrous sodium sulfate and dried by rotary evaporation to obtain compound A222-7 (400 mg, yield 65%).

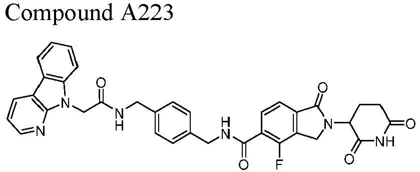

ESI-MS(M+H)⁺: 633.2
¹H NMR (400 MHz, DMSO) δ 11.01 (s, 1H), 9.06 (t, *J* = 6.1 Hz, 1H), 8.74 (t, *J* = 5.8 Hz, 1H), 8.55 (dd, *J* = 7.6, 1.5 Hz, 1H), 8.45 (dd, *J* = 4.8, 1.5 Hz, 1H), 8.21 (d, *J* = 7.7 Hz, 1H), 7.79 - 7.72 (m, 1H), 7.65 (d, *J* = 7.7 Hz, 1H), 7.58 (d, *J* = 8.2 Hz, 1H), 7.52 - 7.48 (m, 1H), 7.32 - 7.25 (m, 6H), 5.21 - 5.07 (m, 3H), 4.66 - 4.40 (m, 4H), 4.29 (d, *J* = 5.9 Hz, 2H), 3.00 - 2.82 (m, 1H), 2.68 - 2.57 (m, 1H), 2.47 - 2.38 (m, 1H), 2.10 - 1.95 (m, 1H).

The intermediates were prepared as follows:

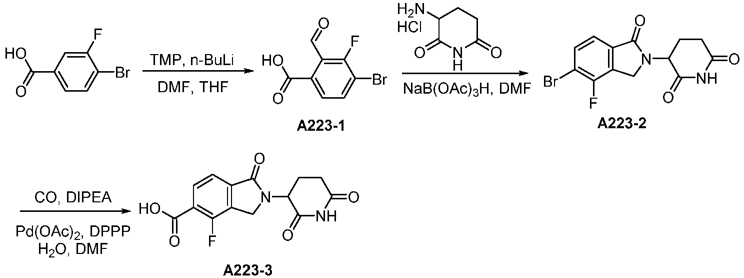

### Synthesis of compound A223-1

Tetramethylpiperidine (27.73 mL, 0.163 mol) was dissolved in anhydrous THF (110 ml). At 0°C, n-butyllithium solution (2.5 M, 63.1 mL, 0.157 mol) was added dropwise. The mixture was reacted for 30 min and then cooled to -45°C. A solution of 4-bromo-3-fluorobenzoic acid (14.33 g, 0.065 mol) in THF (50 mL) was added dropwise to the reaction liquid. The mixture was reacted for 2.5 h, and DMF (7.6 mL) was further added dropwise. After the addition was completed, the mixture was warmed to room temperature and reacted for another 1 h. The reaction was completed and then quenched by dropwise adding 3 N hydrochloric acid (200 mL) to the reaction liquid under ice bath. The aqueous phase was extracted with dichloromethane (100 mL*3). The organic phases were combined, dried over anhydrous sodium sulfate and dried by rotary evaporation. The mixture was slurried with ethyl acetate (80 mL) and then filtered, and the filter cake was dried to obtain compound A223-1 (16.6 g, yield 100%).

### Synthesis of compound A223-2

A223-1 (16.6 g, 0.065 mol) and 3-amino-2,6-piperidinedione hydrochloride (16.65 g, 0.101 mol) were dissolved in DMF (165 mL). Under ice bath, sodium triacetoxyborohydride (35.73 g, 0.168 mol) was added in batches. After the addition was completed, the mixture was reacted at room temperature for another 2 h, and then TLC showed that the reaction was completed. Under ice bath, the reaction was quenched with ice water (400 mL). The mixture was filtered, and the filter cake was rinsed with methyl tert-butyl ether and then dried to obtain compound A223-2 (9.8 g, yield 43%).

### Synthesis of compound A223-3

Compound A222-2 (6 g, 0.018 mol), palladium acetate (1.18 g, 0.005 mol), water (950 mg, 0.053 mol) and DIPEA (11.3 g, 0.087 mol) were dissolved in 60 mL of DMF, and DPPP (2.18 g, 0.005 mol) was added. The mixture was reacted at 90°C under carbon monoxide gas atmosphere for 2 days, and then TLC showed that the reaction was completed. The reaction was cooled to room temperature and quenched with 120 mL of ice water. The mixture was filtered. The filtrate was extracted with ethyl acetate (150 mL*3). The aqueous phase was adjusted to pH 2 with 1 N hydrochloric acid. The aqueous phase was further extracted with ethyl acetate (150 mL*3). The organic phases were combined, washed with saturated brine (50 mL*2), dried over anhydrous sodium sulfate and dried by rotary evaporation to obtain compound A223-3 (1.14 g, yield 21%).

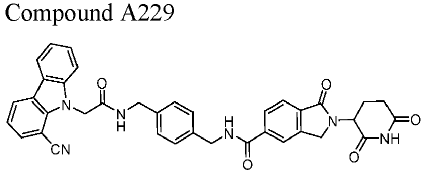

ESI-MS(M+H)⁺: 639.2
¹H NMR (400 MHz, DMSO) δ 11.01 (s, 1H), 9.22 (t, *J* = 5.9 Hz, 1H), 8.83 (t, *J* = 5.8 Hz, 1H), 8.53 (dd, *J* = 7.7, 0.9 Hz, 1H), 8.27 (d, *J* = 7.8 Hz, 1H), 8.08 (s, 1H), 8.00 (d, *J* = 8.0 Hz, 1H), 7.89 - 7.77 (m, 2H), 7.62 (d, *J* = 8.3 Hz, 1H), 7.54 (t, *J* = 7.3 Hz, 1H), 7.39 - 7.31 (m, 2H), 7.31 - 7.22 (m, 4H), 5.42 (s, 2H), 5.14 (dd, *J* = 13.3, 5.1 Hz, 1H), 4.55 - 4.37 (m, 4H), 4.31 (d, *J* = 5.8 Hz, 2H), 2.98 - 2.83 (m, 1H), 2.67 - 2.56 (m, 1H), 2.46 - 2.33 (m, 1H), 2.08 - 1.97 (m, 1H).

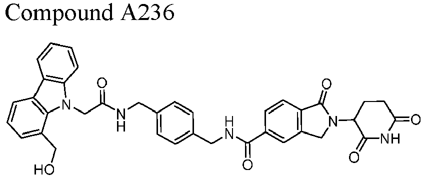

ESI-MS(M+H)⁺: 644.2
¹H NMR (400 MHz, DMSO) δ 11.01 (s, 1H), 9.22 (t, *J* = 5.9 Hz, 1H), 8.71 (t, *J* = 5.9 Hz, 1H), 8.18 - 8.10 (m, 2H), 8.08 (s, 1H), 8.00 (d, *J* = 8.0 Hz, 1H), 7.82 (d, *J* = 7.9 Hz, 1H), 7.48 (d, *J* = 8.2 Hz, 1H), 7.45 - 7.40 (m, 1H), 7.33 (d, *J* = 6.6 Hz, 1H), 7.28 (d, *J* = 8.1 Hz, 2H), 7.24 - 7.18 (m, 3H), 7.17 - 7.12 (m, 1H), 5.55 (t, *J* = 5.3 Hz, 1H), 5.41 (s, 2H), 5.13 (dd, *J* = 13.3, 5.1 Hz, 1H), 4.77 (d, *J* = 5.3 Hz, 2H), 4.55 - 4.37 (m, 4H), 4.28 (d, *J* = 5.8 Hz, 2H), 2.99 - 2.85 (m, 1H), 2.68 - 2.56 (m, 1H), 2.46 - 2.32 (m, 1H), 2.12 - 1.99 (m, 1H).

### Example 3:

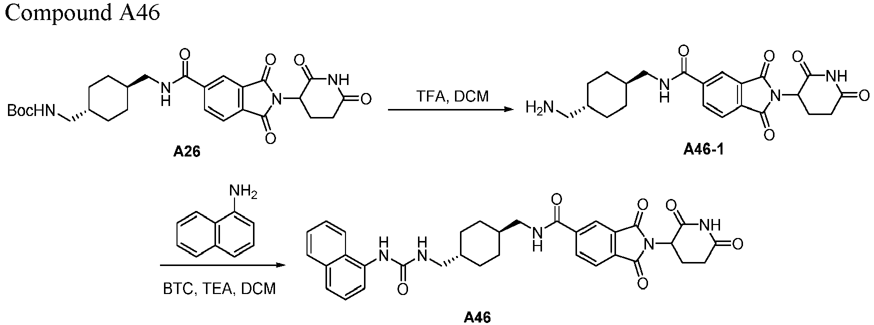

### Synthesis of compound A46-1

Compound A26 (230 mg, 0.46 mmol) was dissolved in dichloromethane (6 mL), and TFA (1.5 mL) was added. The mixture was reacted at room temperature for 1 h, and then monitoring by TLC showed that the raw materials were completely reacted. The solvent was removed by drying to obtain crude compound A46-1 (230 mg) as an oil, which was directly used in the next reaction.

### Synthesis of compound A46

The compounds 1-naphthylamine (70 mg, 0.5 mmol) and triethylamine (100 mg, 1 mmol) were dissolved in dichloromethane (5 mL) and slowly added dropwise under ice salt bath to dichloromethane (5 mL) in which triphosgene (60 mg, 0.2 mmol) was dissolved. The mixture was reacted under ice salt bath for 0.5 h, and then a solution of compound A46-1 (230 mg, 0.44 mmol, crude) and triethylamine (100 mg, 1 mmol) in dichloromethane (5 ml) was added. The mixture was reacted at room temperature for 2 h, and then monitoring by TLC showed that the raw materials were completely reacted. Water (20 mL) was added, and the mixture was extracted with ethyl acetate (20 mL*3). The organic phases were combined, washed with saturated brine (15 mL) and dried over anhydrous sodium sulfate. The solute was purified by a thick preparative plate (developing solvent: DCM : MeOH= 10 : 1), and then purified by medium-pressure preparative chromatography to obtain compound A46 (7 mg, yield 2.4%) as a white solid. ESI-MS (M+H)⁺: 596.2.

¹H NMR (400 MHz, DMSO) δ 11.15 (s, 1H), 8.87 (t, *J* = 5.5 Hz, 1H), 8.46 (s, 1H), 8.33 (dd, *J* = 11.8, 4.0 Hz, 2H), 8.07 (d, *J* = 8.2 Hz, 1H), 8.01 (t, *J* = 7.1 Hz, 2H), 7.92 - 7.85 (m, 1H), 7.56 - 7.48 (m, 3H), 7.40 (t, *J* = 7.9 Hz, 1H), 6.62 (t, *J* = 5.6 Hz, 1H), 5.19 (dd, *J* = 12.8, 5.4 Hz, 1H), 3.17 (t, *J* = 6.1 Hz, 2H), 3.02 (t, *J* = 6.0 Hz, 2H), 2.94 - 2.85 (m, 1H), 2.67 - 2.53 (m, 2H), 2.14 - 2.04 (m, 1H), 1.89 - 1.74 (m, 4H), 1.62 - 1.34 (m, 2H), 1.03 - 0.90 (m, 4H).

The synthesis methods for compounds A45, A47-A52, A198 and A199 are the same as that for A46.

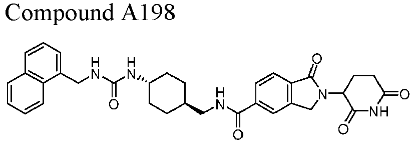

ESI-MS(M+H)⁺: 582.2
¹H NMR (400 MHz, DMSO) δ 11.00 (s, 1H), 8.63 (t, *J* = 5.6 Hz, 1H), 8.09 (d, *J* = 7.8 Hz, 1H), 8.05 (s, 1H), 7.94 (dd, *J* = 13.8, 5.5 Hz, 2H), 7.81 (t, *J* = 9.0 Hz, 2H), 7.59 - 7.49 (m, 2H), 7.49 - 7.37 (m, 2H), 6.18 (t, *J* = 5.6 Hz, 1H), 5.78 (d, *J* = 7.9 Hz, 1H), 5.14 (dd, *J* = 13.2, 5.1 Hz, 1H), 4.66 (d, *J* = 5.6 Hz, 2H), 4.45 (dd, *J* = 50.8, 17.5 Hz, 2H), 3.14 (t, *J* = 6.1 Hz, 2H), 3.00 - 2.83 (m, 2H), 2.67 - 2.56 (m, 1H), 2.47 - 2.31 (m, 1H), 2.08 - 1.96 (m, 1H), 1.90 - 1.81 (m, 2H), 1.80 - 1.69 (m, 2H), 1.58 - 1.44 (m, 1H), 1.10 - 0.95 (m, 4H).

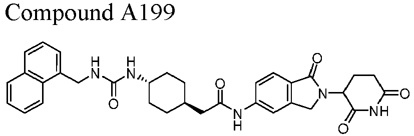

ESI-MS(M+H)⁺: 582.2
¹H NMR (400 MHz, DMSO) δ 10.97 (s, 1H), 10.21 (s, 1H), 8.09 (d, *J* = 7.5 Hz, 1H), 7.98 (s, 1H), 7.96 - 7.90 (m, 1H), 7.82 (d, *J* = 8.0 Hz, 1H), 7.64 (d, *J* = 8.3 Hz, 1H), 7.60 - 7.49 (m, 3H), 7.49 - 7.38 (m, 2H), 6.19 (t, *J* = 5.7 Hz, 1H), 5.79 (d, *J* = 8.0 Hz, 1H), 5.08 (dd, *J* = 13.3, 5.1 Hz, 1H), 4.65 (d, *J* = 5.7 Hz, 2H), 4.35 (dd, *J* = 56.7, 17.3 Hz, 2H), 2.97 - 2.82 (m, 1H), 2.69 - 2.54 (m, 2H), 2.43 - 2.29 (m, 1H), 2.25 (d, *J* = 6.6 Hz, 2H), 2.03 - 1.94 (m, 1H), 1.91 - 1.64 (m, 5H), 1.16 - 0.96 (m, 4H).

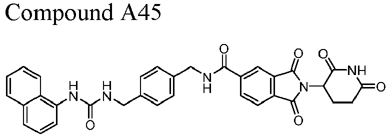

ESI-MS(M+H)⁺: 590.2.

¹H NMR (400 MHz, DMSO) δ 11.15 (s, 1H), 9.45 (t, *J* = 5.8 Hz, 1H), 8.60 (s, 1H), 8.44 - 8.32 (m, 2H), 8.12 - 7.97 (m, 3H), 7.93 - 7.86 (m, 1H), 7.58 - 7.49 (m, 3H), 7.42 (t, *J* = 7.9 Hz, 1H), 7.38 - 7.30 (m, 4H), 7.02 (t, *J* = 5.8 Hz, 1H), 5.20 (dd, *J* = 12.8, 5.4 Hz, 1H), 4.52 (d, *J* = 5.8 Hz, 2H), 4.35 (d, *J* = 5.7 Hz, 2H), 2.95 - 2.83 (m, 1H), 2.70 - 2.53 (m, 2H), 2.13 - 2.05 (m, 1H).

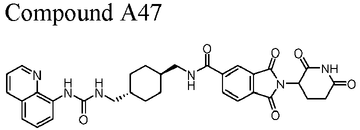

ESI-MS(M+H)⁺: 597.2.

¹H NMR (400 MHz, DMSO) δ 11.13 (s, 1H), 9.34 (s, 1H), 8.85 (d, *J=* 2.6 Hz, 2H), 8.47 (dd, *J=* 7.2, 1.4 Hz, 1H), 8.41 - 8.26 (m, 3H), 8.01 (d, *J=* 7.7 Hz, 1H), 7.58 (dd, *J=* 8.2, 4.2 Hz, 1H), 7.50 - 7.40 (m, 3H), 5.17 (dd, *J=* 12.7, 5.3 Hz, 1H), 3.16 (t, *J=* 5.9 Hz, 2H), 3.00 (t, *J=* 5.8 Hz, 2H), 2.95 - 2.83 (m, 1H), 2.67 - 2.52 (m, 2H), 2.14 - 2.03 (m, 1H), 1.88 - 1.72 (m, 4H), 1.62 - 1.35 (m, 2H), 1.01 - 0.88 (m, 4H).

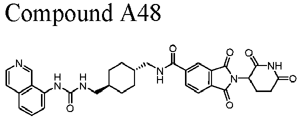

ESI-MS(M+H)⁺: 597.2.

¹H NMR (400 MHz, DMSO) δ 11.15 (s, 1H), 9.48 (s, 1H), 8.87 (s, 2H), 8.47 (d, *J* = 5.7 Hz, 1H), 8.36 - 8.26 (m, 2H), 8.13 (d, *J* = 7.8 Hz, 1H), 8.02 (d, *J* = 7.8 Hz, 1H), 7.77 (d, *J* **=** 5.7 Hz, 1H), 7.66 (t, *J* = 8.0 Hz, 1H), 7.54 (d, *J* = 8.2 Hz, 1H), 6.67 (t, *J* = 5.6 Hz, 1H), 5.18 (dd, *J* = 12.8, 5.5 Hz, 1H), 3.17 (t, *J* = 5.6 Hz, 2H), 3.03 (t, *J* = 6.0 Hz, 2H), 2.95 - 2.82 (m, 1H), 2.69 - 2.51 (m, 2H), 2.13 - 2.04 (m, 1H), 1.85 - 1.74 (m, 4H), 1.62 - 1.36 (m, 2H), 1.02 - 0.88 (m, 4H).

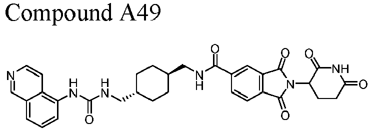

ESI-MS(M+H)⁺: 597.2.

¹H NMR (400 MHz, DMSO) δ 11.14 (s, 1H), 9.25 (s, 1H), 8.87 (t, *J* = 5.7 Hz, 1H), 8.64 (s, 1H), 8.52 (d, *J* = 6.1 Hz, 1H), 8.37 - 8.24 (m, 3H), 8.01 (d, *J* = 7.8 Hz, 1H), 7.94 (d, *J* = 6.1 Hz, 1H), 7.71 (d, *J* = 8.1 Hz, 1H), 7.58 (t, *J* = 7.9 Hz, 1H), 6.71 (t, *J* = 5.7 Hz, 1H), 5.18 (dd, *J* = 12.8, 5.4 Hz, 1H), 3.17 (t, *J* = 5.0 Hz, 2H), 3.02 (t, *J* = 6.0 Hz, 2H), 2.94 - 2.82 (m, 1H), 2.70 - 2.52 (m, 2H), 2.13 - 2.04 (m, 1H), 1.86 - 1.73 (m, 4H), 1.63 - 1.34 (m, 2H), 0.96 (s, 4H).

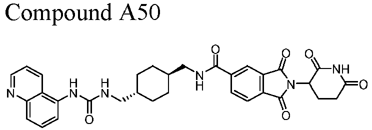

ESI-MS(M+H)⁺: 597.2.

¹H NMR (400 MHz, DMSO) δ 11.15 (s, 1H), 8.96 - 8.78 (m, 3H), 8.64 (d, *J* = 8.5 Hz, 1H), 8.38 - 8.29 (m, 2H), 8.09 (d, *J* = 5.8 Hz, 1H), 8.02 (d, *J* = 7.8 Hz, 1H), 7.73 - 7.61 (m, 2H), 7.58 (dd, *J* = 8.6, 4.2 Hz, 1H), 6.81 (s, 1H), 5.19 (dd, *J* = 12.9, 5.3 Hz, 1H), 3.17 (t, *J* = 6.0 Hz, 2H), 3.02 (t, *J* = 5.9 Hz, 2H), 2.93 - 2.85 (m, 1H), 2.66 - 2.51 (m, 2H), 2.13 - 2.03 (m, 1H), 1.89 - 1.74 (m, 4H), 1.62 - 1.36 (m, 2H), 0.95 (dd, *J* = 21.1, 11.4 Hz, 4H).

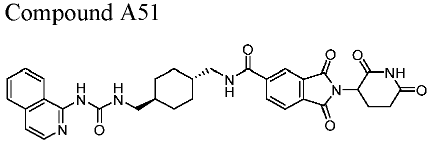

ESI-MS(M+H)⁺: 597.2.

¹H NMR (400 MHz, DMSO) δ 11.14 (s, 1H), 10.12 (s, 1H), 9.56 (s, 1H), 8.85 (t, *J* = 5.5 Hz, 1H), 8.62 (d, *J* = 8.5 Hz, 1H), 8.39 - 8.26 (m, 2H), 8.04 (dd, *J* = 15.2, 6.8 Hz, 2H), 7.88 (d, J= 7.9 Hz, 1H), 7.76 (t, *J* = 7.5 Hz, 1H), 7.61 (t, *J* = 7.6 Hz, 1H), 7.38 (d, *J* = 5.8 Hz, 1H), 5.19 (dd, *J* = 12.9, 5.3 Hz, 1H), 3.21 - 3.15 (m, 4H), 2.96 - 2.83 (m, 1H), 2.66 - 2.52 (m, 2H), 2.12 - 2.03 (m, 1H), 1.88 - 1.73 (m, 4H), 1.62 - 1.41 (m, 2H), 1.07 - 0.92 (m, 4H).

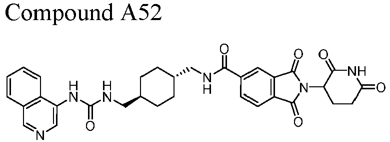

ESI-MS(M+H)⁺: 597.2.

¹H NMR (400 MHz, DMSO) δ 11.13 (s, 1H), 9.02 (s, 1H), 8.95 (s, 1H), 8.86 (t, *J* = 5.6 Hz, 1H), 8.51 (s, 1H), 8.37 - 8.30 (m, 2H), 8.09 (dd, *J* = 8.1, 3.8 Hz, 2H), 8.02 (d, *J* = 7.8 Hz, 1H), 7.82 (t, *J* = 7.7 Hz, 1H), 7.69 (t, *J* = 7.2 Hz, 1H), 6.66 (t, *J* = 6.8 Hz, 1H), 5.19 (dd, *J* = 12.8, 5.2 Hz, 1H), 3.17 (t, *J* = 6.1 Hz, 2H), 3.03 (t, *J* = 6.1 Hz, 2H), 2.96 - 2.84 (m, 1H), 2.69 - 2.53 (m, 2H), 2.13 - 2.04 (m, 1H), 1.88 - 1.75 (m, 4H), 1.64 - 1.50 (m, 1H), 1.47 - 1.39 (m, 1H), 1.05 - 0.90 (m, 4H).

### Example 4:

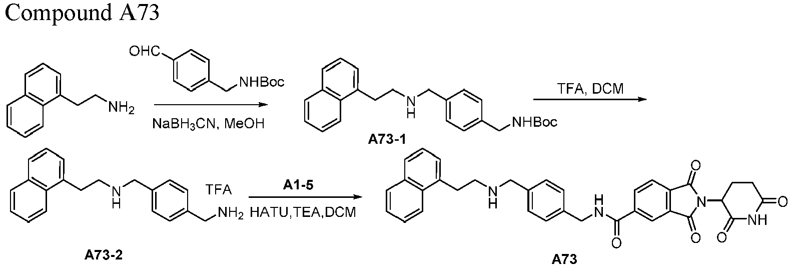

### Synthesis of compound A73-1

The compound 2-naphthylethylamine (85 mg, 0.5 mmol) and the compound tert-butyl 4-formylbenzylcarbamate (118 mg, 0.5 mmol) were dissolved in methanol (3 mL). The mixture was stirred at room temperature for 1 h, and then sodium cyanoborohydride (63 mg, 1 mmol) was added. The mixture was reacted at room temperature for 16 h, and then TLC showed that the raw materials were completely reacted. The solvent was removed by drying. Water (20 mL) was added, and the mixture was extracted with dichloromethane (25 mL*3). The organic phases were combined and then dried over anhydrous sodium sulfate. The solvent was removed by drying, and the solute was purified by a thick preparative plate (developing solvent: DCM : MeOH = 15 : 1) to obtain compound A73-1 (65 mg, yield 33.3%) as a yellow solid.

### Synthesis of compound A73-2

Compound A73-1 (65 mg, 0.17 mmol) was dissolved in dichloromethane (3 mL), and trifluoroacetic acid (0.8 mL) was added with stirring. The mixture was reacted at room temperature for 2 h, and then TLC showed that the raw materials were completely reacted. The solvent was removed by drying to obtain white crude compound A73-2 (70 mg), which was directly used in the next reaction.

### Synthesis of compound A73

Compound A73-2 (70 mg, 0.17 mmol, crude) and compound A1-5 (41 mg, 0.14 mmol) were dissolved in dichloromethane (6 mL), and triethylamine (71 uL, 0.51 mmol) and HATU (52 mg, 0.14 mmol) were added. The mixture was reacted at room temperature for 1 h, and then TLC showed that the raw materials were completely reacted. Water (20 mL) was added, and the mixture was extracted with dichloromethane (25 mL*3). The organic phases were combined and then dried over anhydrous sodium sulfate. The solvent was removed by drying. The solute was separated and purified by medium-pressure preparative chromatography to obtain compound A73 (13 mg, yield 17%) as a white solid. ESI-MS(M+H)⁺: 575.2.

¹H NMR (400 MHz, DMSO) δ 11.15 (s, 1H), 9.51 (t, *J* = 5.9 Hz, 1H), 8.86 (br s, 1H), 8.45 - 8.32 (m, 2H), 8.07 (t, *J* = 7.7 Hz, 2H), 7.96 (d, *J* = 7.8 Hz, 1H), 7.86 (d, *J* = 8.1 Hz, 1H), 7.63 - 7.51 (m, 2H), 7.49 - 7.40 (m, 6H), 5.20 (dd, *J* = 12.8, 5.3 Hz, 1H), 4.55 (d, *J* = 5.8 Hz, 2H), 4.22 (s, 2H), 3.45 - 3.38 (m, 2H), 3.25 - 3.20 (m, 2H), 2.97 - 2.83 (m, 1H), 2.69 - 2.52 (m, 2H), 2.15 - 2.03 (m, 1H).

The synthesis methods for compounds A74 and A231 are the same as that for A73.

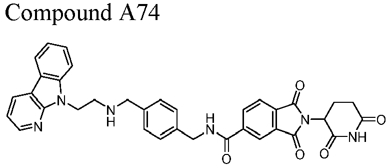

ESI-MS(M+H)⁺: 615.2.

¹H NMR (400 MHz, DMSO) δ 11.14 (s, 1H), 9.49 (t, *J* = 5.7 Hz, 1H), 8.57 (dd, *J* = 7.6, 1.5 Hz, 1H), 8.47 (dd, *J* = 4.9, 1.5 Hz, 1H), 8.40 - 8.33 (m, 2H), 8.23 (d, *J* = 7.7 Hz, 1H), 8.05 (d, *J* = 7.8 Hz, 1H), 7.77 (d, *J* = 8.2 Hz, 1H), 7.56 (t, *J* = 7.7 Hz, 1H), 7.41 (dd, *J* = 21.0, 7.9 Hz, 4H), 7.34 - 7.26 (m, 2H), 5.19 (dd, *J* = 12.8, 5.4 Hz, 1H), 4.79 (t, *J* = 6.3 Hz, 2H), 4.52 (d, *J* = 5.8 Hz, 2H), 4.22 - 4.13 (m, 2H), 3.43 - 3.37 (m, 2H), 2.97 - 2.85 (m, 1H), 2.69 - 2.52 (m, 2H), 2.15 - 2.03 (m, 1H).
The corresponding intermediates were prepared as follows:

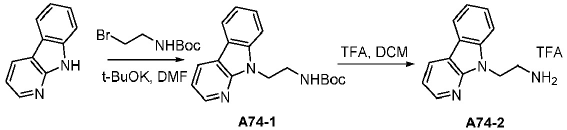

### Synthesis of compound A74-1

The compound 9H-pyrido[2,3-B]indole (200 mg, 1.2 mmol) and the compound *N*-Boc-bromoethylamine (320 mg, 1.4 mmol) were dissolved in DMF (8 mL), and potassium tert-butoxide (270 mg, 2.4 mmol) was added. The mixture was reacted at 100°C for 16 h, and then monitoring by TLC showed that the raw materials were completely reacted. Water (30 mL) was added, and the mixture was extracted with ethyl acetate (30 mL*4). The organic phases were combined, and the solvent was removed by drying. The solute was separated and purified by a thick preparative plate (developing solvent: PE : EA = 5 : 1) to obtain compound A74-1 (230 mg, yield 62%).

### Synthesis of compound A74-2

Compound A74-1 (230 mg, 0.74 mmol) was dissolved in dichloromethane (4 mL), and TFA (1 mL) was added. The mixture was reacted at room temperature for 1 h, and then monitoring by TLC showed that the raw materials were completely reacted. The solvent was dried by rotary evaporation to obtain compound A74-2 (280 mg, crude) as an oil, which was directly used in the next reaction.

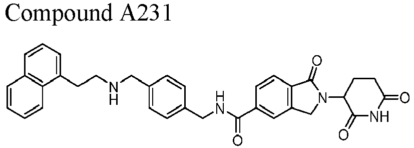

ESI-MS(M+H)⁺: 561.2.

¹H NMR (400 MHz, DMSO) δ 11.01 (s, 1H), 9.23 (t, *J* = 5.9 Hz, 1H), 8.12 - 7.97 (m, 3H), 7.94 - 7.87 (m, 1H), 7.82 (d, *J* = 7.9 Hz, 1H), 7.78 - 7.75 (m, 1H), 7.54 - 7.25 (m, 8H), 5.14 (dd, *J* = 13.3, 5.1 Hz, 1H), 4.59 - 4.34 (m, 4H), 3.83 (s, 2H), 3.25 - 3.20 (m, 2H), 2.92 - 2.83 (m, 3H), 2.69 - 2.55 (m, 1H), 2.46 - 2.34 (m, 1H), 2.07 - 1.90 (m, 1H).

### Example 5:

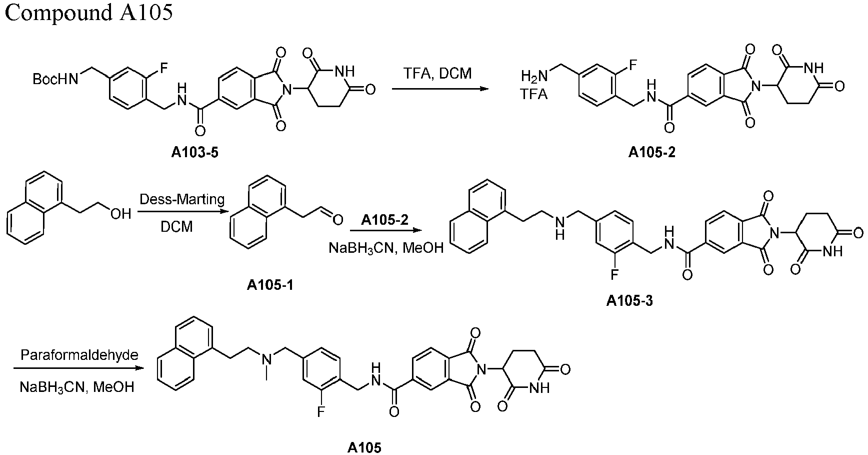

### Synthesis of compound A105-1

The compound naphthaleneethanol (500 mg, 2.91 mmol) was dissolved in 30 mL of dichloromethane, and Dess-Martin reagent (1.85 g, 4.36 mmol) was added. The mixture was reacted at room temperature for 2 h, and then TLC showed that the reaction was completed. The reaction was quenched with water (30 mL). The aqueous phase was extracted with dichloromethane (10 mL*3). The organic phases were combined and dried by rotary evaporation. The solute was separated by column chromatography (mobile phase: petroleum ether : ethyl acetate = 3 : 1) to obtain compound A105-1 (40 mg, yield 81%).

### Synthesis of compound A105-2

Compound A103-5 (108 mg, 0.20 mmol) was dissolved in 6 mL of dichloromethane, and 2 mL of trifluoroacetic acid was added. The mixture was reacted at room temperature for 3 h, and then TLC showed that the reaction was completed. The solvent was dried by rotary evaporation to obtain compound A105-2 (180 mg, crude).

### Synthesis of compound A105-3

Compound A105-1 (28 mg, 0.16 mmol) was dissolved in 6 mL of methanol, and A105-2 (180 mg, 0.20 mmol) and sodium cyanoborohydride (15 mg, 0.23 mmol) were added. The mixture was reacted at room temperature for 5 h, and then TLC showed that the reaction was completed. The reaction was quenched with water (30 mL). The aqueous phase was extracted with dichloromethane (20 mL*3). The organic phases were combined and dried by rotary evaporation. The solute was purified by a preparative plate (mobile phase: dichloromethane : methanol = 20 : 1) to obtain compound A105-3 (45 mg, yield 47%).

### Synthesis of compound A105

Compound A105-3 (45 mg, 0.076 mmol) was dissolved in 5 mL of methanol, and paraformaldehyde (7 mg, 0.23 mmol) and sodium cyanoborohydride (15 mg, 0.23 mmol) were added. The mixture was reacted at room temperature for 5 h, and then TLC showed that the reaction was completed. The reaction was quenched with water (30 mL). The aqueous phase was extracted with dichloromethane (20 mL*3). The organic phases were combined and dried by rotary evaporation. The solute was purified by a preparative plate (mobile phase: dichloromethane : methanol = 20 : 1) and then purified by medium-pressure preparative chromatography to obtain compound A105 (10 mg, yield 22%). ESI-MS(M+H)⁺: 607.2.

¹H NMR (400 MHz, DMSO) δ 11.14 (s, 1H), 9.40 (t, *J* = 5.6 Hz, 1H), 8.44 - 8.32 (m, 2H), 8.05 (d, *J* = 7.7 Hz, 1H), 7.94 (d, *J* = 7.4 Hz, 1H), 7.91 - 7.84 (m, 1H), 7.75 (d, *J* = 7.4 Hz, 1H), 7.51 - 7.29 (m, 5H), 7.14 - 7.04 (m, 2H), 5.20 (dd, *J* = 12.9, 5.4 Hz, 1H), 4.53 (d, *J* = 5.5 Hz, 2H), 3.58 (s, 2H), 3.26 - 3.19 (m, 2H), 2.96 - 2.83 (m, 1H), 2.71 - 2.51 (m, 4H), 2.30 (s, 3H), 2.13 - 2.05 (m, 1H).

The synthesis methods for compounds A83-A84, A98-A102, A106, A109-A110, A113-A114, A119-A120, A123, A126-A127, A130, A139-A140, A143-A144, A147-A148, A151 and A156 are the same as that for A105.

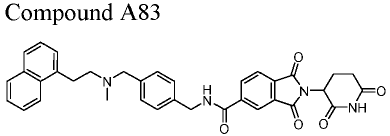

ESI-MS (M+H)⁺: 589.2.

¹H NMR (400 MHz, DMSO) δ 11.15 (s, 1H), 9.43 (t, *J* = 5.9 Hz, 1H), 8.42 - 8.33 (m, 2H), 8.05 (d, *J* = 7.8 Hz, 1H), 7.95 - 7.84 (m, 2H), 7.74 (d, *J* = 7.7 Hz, 1H), 7.49 - 7.34 (m, 4H), 7.30 - 7.23 (m, 4H), 5.19 (dd, *J* = 12.9, 5.4 Hz, 1H), 4.50 (d, *J* = 5.8 Hz, 2H), 3.56 (s, 2H), 3.26 - 3.17 (m, 2H), 2.95 - 2.85 (m, 1H), 2.69 - 2.52 (m, 4H), 2.29 (s, 3H), 2.13 - 2.05 (m, 1H).

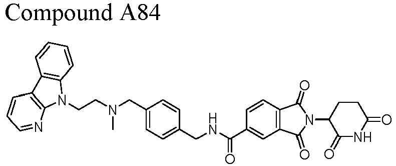

ESI-MS(M+H)⁺: 629.2.

¹H NMR (400 MHz, DMSO) δ 11.14 (s, 1H), 9.37 (t, *J* = 5.8 Hz, 1H), 8.52 (dd, *J* = 7.6, 1.6 Hz, 1H), 8.46 - 8.32 (m, 3H), 8.19 (d, *J* = 7.7 Hz, 1H), 8.05 (d, *J* **=** 7.8 Hz, 1H), 7.57 (d, *J* = 8.2 Hz, 1H), 7.51 - 7.40 (m, 1H), 7.28 - 7.15 (m, 2H), 7.09 (d, *J* = 8.0 Hz, 2H), 6.94 (d, *J* = 8.0 Hz, 2H), 5.20 (dd, *J* = 12.9, 5.4 Hz, 1H), 4.60 (t, *J* = 6.6 Hz, 2H), 4.44 (d, *J* = 5.7 Hz, 2H), 3.48 (s, 2H), 2.95 - 2.85 (m, 1H), 2.77 (t, *J* = 6.5 Hz, 2H), 2.68 - 2.52 (m, 2H), 2.24 (s, 3H), 2.13 - 2.03 (m, 1H).

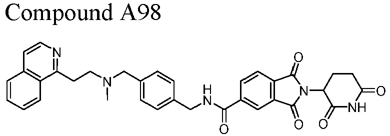

ESI-MS(M+H)⁺: 590.2.

¹H NMR (400 MHz, DMSO) δ 11.15 (s, 1H), 9.42 (t, *J* = 5.9 Hz, 1H), 8.37 (dd, *J* = 15.2, 6.1 Hz, 3H), 8.17 (d, *J* = 8.4 Hz, 1H), 8.04 (d, *J* = 7.8 Hz, 1H), 7.92 (d, *J* = 8.1 Hz, 1H), 7.71 (t, *J* = 7.1 Hz, 1H), 7.67 - 7.57 (m, 2H), 7.28 - 7.20 (m, 4H), 5.20 (dd, *J* = 12.9, 5.4 Hz, 1H), 4.49 (d, *J* = 5.6 Hz, 2H), 3.57 (s, 2H), 3.49 - 3.42 (m, 2H), 2.96 - 2.80 (m, 3H), 2.68 - 2.52 (m, 2H), 2.29 (s, 3H), 2.12 - 2.06 (m, 1H).
The corresponding intermediates were prepared as follows:

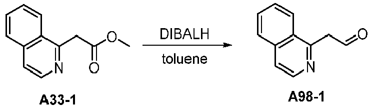

### Synthesis of compound A98-1

Compound A33-1 (100 mg, 0.50 mmol) was dissolved in 5 mL of toluene. At -78°C, DIBALH (0.6 mL, 1 M, 0.60 mmol) was added dropwise. The mixture was reacted at -78°C for 0.5 h, and then TLC showed that the reaction was completed. The reaction was quenched with saturated aqueous sodium bicarbonate solution (20 mL). The aqueous phase was extracted with ethyl acetate (10 mL*3). The organic phases were combined and dried by rotary evaporation. The solute was separated by column chromatography (mobile phase: petroleum ether : ethyl acetate = 3 : 1) to obtain compound A98-1 (80 mg, yield 93%). ¹H NMR (400 MHz, DMSO) δ 8.91 (d, *J* = 3.4 Hz, 1H), 8.25 (d, *J* = 8.3 Hz, 1H), 7.81 - 7.66 (m, 4H), 7.60 - 7.56 (m, 1H), 7.00 (d, *J* = 6.8 Hz, 1H), 6.10 (d, *J* = 3.4 Hz, 1H).

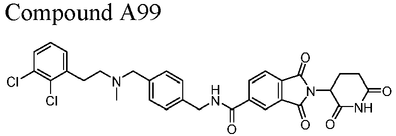

ESI-MS(M+H)⁺: 607.2.

¹H NMR (400 MHz, DMSO) δ 11.14 (s, 1H), 9.42 (t, *J* = 5.8 Hz, 1H), 8.44 - 8.33 (m, 2H), 8.04 (d, *J* = 7.8 Hz, 1H), 7.47 (dd, *J* = 7.8, 1.6 Hz, 1H), 7.35 - 7.24 (m, 4H), 7.18 (d, *J* = 8.0 Hz, 2H), 5.19 (dd, *J* = 12.9, 5.4 Hz, 1H), 4.50 (d, *J* = 5.8 Hz, 2H), 3.51 (s, 2H), 2.98 - 2.84 (m, 3H), 2.67 - 2.53 (m, 4H), 2.21 (s, 3H), 2.13 - 2.06 (m, 1H).
The corresponding intermediates were prepared as follows:

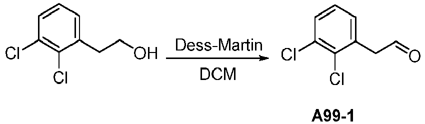

### Synthesis of compound A99-1

The compound 2,3-dichlorophenylethanol (100 mg, 0.52 mmol) was dissolved in 5 mL of dichloromethane, and Dess-Martin reagent (296 mg, 0.70 mmol) was added. The mixture was reacted at room temperature for 2 h, and then TLC showed that the reaction was completed. The reaction was quenched with saturated aqueous sodium bicarbonate solution (20 mL). The aqueous phase was extracted with dichloromethane (10 mL*3). The organic phases were combined and dried by rotary evaporation. The solute was separated by column chromatography (mobile phase: petroleum ether : ethyl acetate = 5 : 1) to obtain compound A99-1 (90 mg, yield 92%).

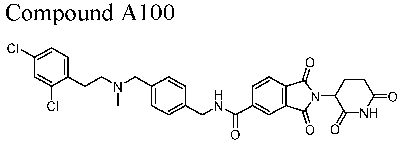

ESI-MS(M+H)⁺: 607.2.

¹H NMR (400 MHz, DMSO) δ 11.14 (s, 1H), 9.42 (t, *J* = 5.9 Hz, 1H), 8.40 - 8.35 (m, 2H), 8.04 (d, *J* = 7.8 Hz, 1H), 7.51 (d, *J* = 1.9 Hz, 1H), 7.39 - 7.30 (m, 2H), 7.22 (dd, *J* = 31.4, 8.0 Hz, 4H), 5.19 (dd, *J* = 12.9, 5.4 Hz, 1H), 4.50 (d, *J* = 5.7 Hz, 2H), 3.50 (s, 2H), 2.95 - 2.88 (m, 1H), 2.86 (t, *J* = 7.4 Hz, 2H), 2.66 - 2.55 (m, 2H), 2.55 - 2.51 (m, 2H), 2.19 (s, 3H), 2.12 - 2.03 (m, 1H).

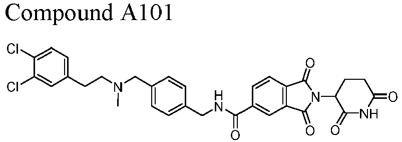

ESI-MS(M+H)⁺: 607.2.

¹H NMR (400 MHz, DMSO) δ 11.14 (s, 1H), 9.42 (t, *J* = 5.8 Hz, 1H), 8.44 - 8.30 (m, 2H), 8.04 (d, *J* = 7.8 Hz, 1H), 7.52 - 7.46 (m, 2H), 7.26 (d, *J* = 8.1 Hz, 2H), 7.22 - 7.13 (m, 3H), 5.19 (dd, *J* = 12.9, 5.4 Hz, 1H), 4.50 (d, *J* = 5.8 Hz, 2H), 3.48 (s, 2H), 2.95 - 2.85 (m, 1H), 2.76 (t, *J* = 7.2 Hz, 2H), 2.68 - 2.52 (m, 4H), 2.15 (s, 3H), 2.12 - 2.06 (m, 1H).

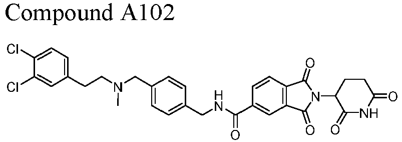

ESI-MS(M+H)⁺: 607.2. [0539] ¹H NMR (400 MHz, DMSO) δ 11.14 (s, 1H), 9.42 (t, *J* = 5.9 Hz, 1H), 8.44 - 8.32 (m, 2H), 8.04 (d, *J* = 7.7 Hz, 1H), 7.40 (d, *J* = 8.0 Hz, 2H), 7.30 - 7.17 (m, 5H), 5.19 (dd, *J* = 12.9, 5.3 Hz, 1H), 4.50 (d, *J* = 5.8 Hz, 2H), 3.54 (s, 2H), 3.09 - 3.01 (m, 2H), 2.95 - 2.83 (m, 1H), 2.67 - 2.51 (m, 4H), 2.27 (s, 3H), 2.13 - 2.06 (m, 1H).

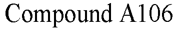

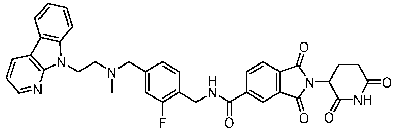

ESI-MS(M+H)⁺: 647.2.

¹H NMR (400 MHz, DMSO) δ 11.15 (s, 1H), 9.34 (s, 1H), 8.52 (d, *J* = 7.5 Hz, 1H), 8.41 - 8.35 (m, 3H), 8.19 (d, *J* = 7.7 Hz, 1H), 8.05 (d, *J* = 7.8 Hz, 1H), 7.61 (d, *J* = 8.1 Hz, 1H), 7.47 (t, *J* = 7.4 Hz, 1H), 7.31 - 7.16 (m, 2H), 7.12 (s, 1H), 6.73 (dd, *J* = 27.0, 9.0 Hz, 2H), 5.20 (dd, *J* = 12.9, 5.4 Hz, 1H), 4.56 (d, *J* = 44.1 Hz, 2H), 4.46 (d, *J* = 4.9 Hz, 2H), 3.49 (s, 2H), 2.97 - 2.72 (m, 3H), 2.69 - 2.53 (m, 2H), 2.24 (s, 3H), 2.13 - 2.04 (m, 1H).
The corresponding intermediates were prepared as follows:

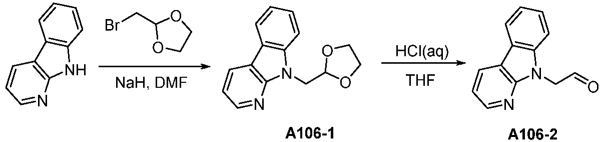

### Synthesis of compound A106-1

The compound 9H-pyrido[2,3-B]indole (2.5 g, 15 mmol) was dissolved in anhydrous DMF (20 mL), and sodium hydride (2.1 g, 53 mmol) was added in batches under ice bath. The mixture was reacted at room temperature for 1 h, and then a solution of the compound 2-bromomethyl-1,3-dioxolane (4.15 g, 25 mmol) in anhydrous DMF (10 mL) was added. The mixture was reacted at room temperature for another 25 h, and then monitoring by TLC showed that the raw materials were completely reacted. Under ice bath, the reaction was quenched by slowly adding ice water (60 mL) and extracted with ethyl acetate (75 mL*3). The organic phases were combined, washed with saturated brine (90 mL), dried over anhydrous sodium sulfate and concentrated. The solute was purified by column chromatography (developing solvent: PE : EA= 80 : 1-5 : 1) to obtain compound A106-1 (1.3 g, yield 34%).

### Synthesis of compound A106-2

Compound A106-1 (180 mg, 0.7 mmol) was dissolved in tetrahydrofuran (3 mL), and hydrochloric acid solution (6 N, 1.5 mL) was added. The mixture was heated and reacted at 75°C for 18 h, and then monitoring by TLC showed that the raw materials were completely reacted. The solvent was removed by drying. The mixture was adjusted to pH 4-5 with saturated sodium bicarbonate solution, with a white solid precipitated, filtered and washed with a small amount of water. The filter cake was dried to obtain compound A106-2 (145 mg, yield 98.6%).

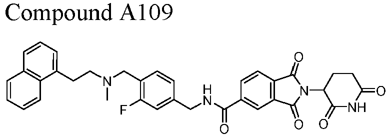

ESI-MS(M+H)⁺: 607.2.

¹H NMR (400 MHz, DMSO) δ 11.15 (s, 1H), 9.45 (t, *J* = 5.8 Hz, 1H), 8.46 - 8.33 (m, 2H), 8.05 (d, *J* = 7.7 Hz, 1H), 7.96 (d, *J* = 7.8 Hz, 1H), 7.93 - 7.85 (m, 1H), 7.75 (d, *J* = 7.0 Hz, 1H), 7.51 - 7.30 (m, 5H), 7.16 - 7.09 (m, 2H), 5.20 (dd, *J* = 12.9, 5.4 Hz, 1H), 4.51 (d, *J* = 5.8 Hz, 2H), 3.61 (s, 2H), 3.26 - 3.17 (m, 2H), 2.95 - 2.85 (m, 1H), 2.71 - 2.51 (m, 4H), 2.30 (s, 3H), 2.14 - 2.03 (m, 1H).

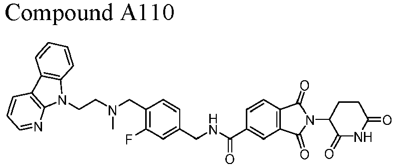

ESI-MS(M+H)⁺: 647.2.

¹H NMR (400 MHz, DMSO) δ 11.14 (s, 1H), 9.40 (t, *J* = 5.9 Hz, 1H), 8.51 (dd, *J* = 7.6, 1.6 Hz, 1H), 8.44 - 8.34 (m, 3H), 8.19 (d, *J* = 7.7 Hz, 1H), 8.05 (d, *J* = 7.7 Hz, 1H), 7.60 (d, *J* = 8.2 Hz, 1H), 7.52 - 7.42 (m, 1H), 7.26 - 7.17 (m, 2H), 7.03 (d, *J* = 10.4 Hz, 1H), 6.95 - 6.82 (m, 2H), 5.20 (dd, *J* = 12.9, 5.4 Hz, 1H), 4.60 (t, *J* = 6.6 Hz, 2H), 4.45 (d, *J* = 5.8 Hz, 2H), 3.55 (s, 2H), 2.96 - 2.84 (m, 1H), 2.80 (t, *J* = 6.6 Hz, 2H), 2.68 - 2.52 (m, 2H), 2.27 (s, 3H), 2.15 - 2.04 (m, 1H).

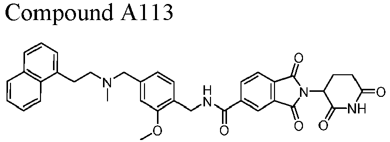

ESI-MS(M+H)⁺: 619.2.

¹H NMR (400 MHz, DMSO) δ 11.15 (s, 1H), 9.24 (t, *J* = 5.7 Hz, 1H), 8.44 - 8.33 (m, 2H), 8.04 (d, *J* = 8.1 Hz, 1H), 7.98 - 7.91 (m, 1H), 7.91 - 7.84 (m, 1H), 7.78 - 7.68 (m, 1H), 7.51 - 7.35 (m, 4H), 7.14 (d, *J* = 7.6 Hz, 1H), 6.93 (s, 1H), 6.84 (d, *J* = 7.7 Hz, 1H), 5.20 (dd, *J* = 12.9, 5.4 Hz, 1H), 4.47 (d, *J* = 5.6 Hz, 2H), 3.77 (s, 3H), 3.57 (s, 2H), 3.23 (t, *J* = 8.0 Hz, 2H), 2.96 - 2.84 (m, 1H), 2.72 - 2.51 (m, 4H), 2.32 (s, 3H), 2.13 - 2.05 (m, 1H).

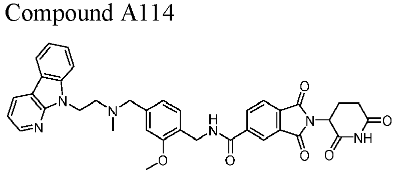

ESI-MS(M+H)⁺: 659.2.

¹H NMR (400 MHz, DMSO) δ 11.14 (s, 1H), 9.18 (t, *J* = 5.7 Hz, 1H), 8.51 (dd, *J* = 7.6, 1.6 Hz, 1H), 8.44 - 8.34 (m, 3H), 8.19 (d, *J* = 7.7 Hz, 1H), 8.04 (d, *J* = 7.8 Hz, 1H), 7.60 (d, *J* = 8.2 Hz, 1H), 7.53 - 7.43 (m, 1H), 7.27 - 7.16 (m, 2H), 6.98 (d, *J* = 7.6 Hz, 1H), 6.63 - 6.52 (m, 2H), 5.18 (dd, *J* = 12.9, 5.4 Hz, 1H), 4.62 (t, *J* = 6.4 Hz, 2H), 4.40 (d, *J* = 5.5 Hz, 2H), 3.57 (s, 3H), 3.49 (s, 2H), 2.96 - 2.78 (m, 3H), 2.68 - 2.52 (m, 2H), 2.25 (s, 3H), 2.13 - 2.06 (m, 1H).

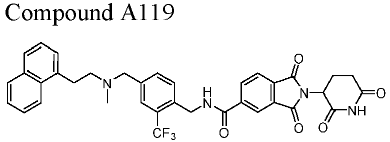

ESI-MS(M+H)⁺: 657.2.

¹H NMR (400 MHz, DMSO) δ 11.15 (s, 1H), 9.50 (s, 1H), 8.45 - 8.33 (m, 2H), 8.06 (d, *J* = 7.7 Hz, 1H), 7.90 (dd, *J* = 20.2, 7.4 Hz, 2H), 7.75 (d, *J* = 7.4 Hz, 1H), 7.68 - 7.61 (m, 2H), 7.52 (d, *J* = 8.0 Hz, 1H), 7.48 - 7.31 (m, 4H), 5.20 (dd, *J* = 12.8, 5.2 Hz, 1H), 4.56 (d, *J* = 5.4 Hz, 2H), 3.68 (s, 2H), 3.27 - 3.20 (m, 2H), 2.96 - 2.84 (m, 1H), 2.76 - 2.54 (m, 4H), 2.34 (s, 3H), 2.15 - 2.04 (m, 1H).

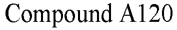

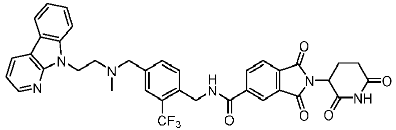

ESI-MS(M+H)⁺: 697.2.

¹H NMR (400 MHz, DMSO) δ 11.15 (s, 1H), 9.42 (s, 1H), 8.50 (d, *J* = 7.5 Hz, 1H), 8.45 - 8.33 (m, 3H), 8.18 (d, *J* = 7.7 Hz, 1H), 8.08 (d, *J* = 7.8 Hz, 1H), 7.60 (d, *J* = 8.2 Hz, 1H), 7.46 (t, *J* = 7.6 Hz, 1H), 7.39 - 7.10 (m, 5H), 5.21 (dd, *J* = 12.9, 5.3 Hz, 1H), 4.62 (d, *J* = 4.7 Hz, 4H), 3.58 (s, 2H), 3.01 - 2.74 (m, 3H), 2.69 - 2.55 (m, 2H), 2.24 (s, 3H), 2.14 - 2.04 (m, 1H).

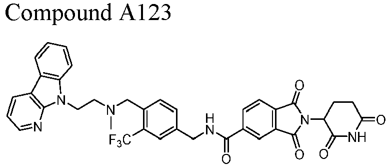

ESI-MS(M+H)⁺: 697.2.

¹H NMR (400 MHz, DMSO) δ 11.15 (s, 1H), 9.43 (s, 1H), 8.51 (d, *J* = 7.4 Hz, 1H), 8.45 - 8.30 (m, 3H), 8.19 (d, *J* = 7.8 Hz, 1H), 8.07 (d, *J* = 7.7 Hz, 1H), 7.58 (d, *J* = 8.2 Hz, 1H), 7.53 (s, 1H), 7.44 (t, *J* = 7.6 Hz, 1H), 7.26 - 7.12 (m, 2H), 7.05 (d, *J* = 7.8 Hz, 1H), 6.92 (d, *J* = 8.0 Hz, 1H), 5.20 (dd, *J* = 12.8, 5.1 Hz, 1H), 4.62 (t, *J* = 5.9 Hz, 2H), 4.46 (d, *J* = 5.3 Hz, 2H), 3.58 (s, 2H), 2.97 - 2.84 (m, 1H), 2.80 (t, *J* = 5.8 Hz, 2H), 2.69 - 2.54 (m, 2H), 2.31 (s, 3H), 2.17 - 2.04 (m, 1H).

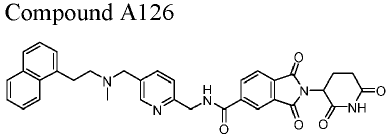

ESI-MS(M+H)⁺: 590.2.

¹H NMR (400 MHz, DMSO) δ 11.13 (s, 1H), 9.52 (t, *J* = 5.8 Hz, 1H), 8.47 - 8.36 (m, 3H), 8.06 (d, *J* = 7.8 Hz, 1H), 7.97 - 7.91 (m, 1H), 7.90 - 7.86 (m, 1H), 7.75 (d, *J* = 7.6 Hz, 1H), 7.64 (dd, *J* = 8.0, 2.1 Hz, 1H), 7.50 - 7.43 (m, 2H), 7.43 - 7.35 (m, 2H), 7.30 (d, *J* = 8.0 Hz, 1H), 5.20 (dd, *J* = 12.9, 5.4 Hz, 1H), 4.58 (d, *J* = 5.6 Hz, 2H), 3.60 (s, 2H), 3.27 - 3.19 (m, 2H), 2.96 - 2.82 (m, 1H), 2.69 - 2.52 (m, 4H), 2.29 (s, 3H), 2.14 - 2.04 (m, 1H).

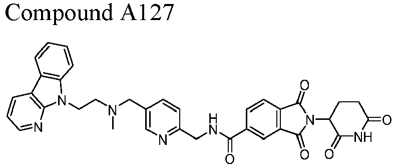

ESI-MS(M+H)⁺: 630.2.

¹H NMR (400 MHz, DMSO) δ 11.16 (s, 1H), 9.46 (t, *J* = 5.7 Hz, 1H), 8.51 (dd, *J* = 7.6, 1.4 Hz, 1H), 8.45 - 8.36 (m, 3H), 8.19 (d, *J* = 7.1 Hz, 2H), 8.06 (d, *J* = 7.8 Hz, 1H), 7.60 (d, *J* = 8.2 Hz, 1H), 7.47 (t, *J* = 7.4 Hz, 1H), 7.29 - 7.16 (m, 3H), 7.06 (d, *J* = 7.9 Hz, 1H), 5.21 (dd, *J* = 12.9, 5.3 Hz, 1H), 4.68 - 4.47 (m, 4H), 3.52 (s, 2H), 2.96 - 2.84 (m, 1H), 2.80 (t, *J* = 6.3 Hz, 2H), 2.68 - 2.53 (m, 2H), 2.23 (s, 3H), 2.14 - 2.04 (m, 1H).

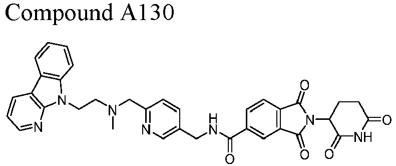

ESI-MS(M+H)⁺: 630.2.

¹H NMR (400 MHz, DMSO) δ 11.15 (s, 1H), 9.41 (t, *J* = 5.8 Hz, 1H), 8.51 (dd, *J* = 7.6, 1.6 Hz, 1H), 8.44 - 8.29 (m, 4H), 8.19 (d, *J* = 7.7 Hz, 1H), 8.06 (d, *J* = 7.8 Hz, 1H), 7.61 (d, *J* = 8.2 Hz, 1H), 7.47 - 7.43 (m, 1H), 7.33 (dd, *J* = 8.0, 2.1 Hz, 1H), 7.26 - 7.14 (m, 2H), 6.74 (d, *J* = 8.0 Hz, 1H), 5.20 (dd, *J* = 12.9, 5.4 Hz, 1H), 4.61 (t, *J* = 6.4 Hz, 2H), 4.44 (d, *J* = 5.7 Hz, 2H), 3.61 (s, 2H), 2.96 - 2.85 (m, 1H), 2.81 (t, *J* = 6.4 Hz, 2H), 2.66 - 2.52 (m, 2H), 2.31 (s, 3H), 2.13 - 2.04 (m, 1H).

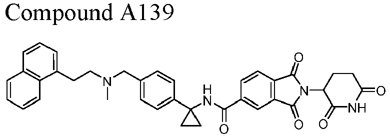

ESI-MS(M+H)⁺: 615.2.

¹H NMR (400 MHz, DMSO) δ 11.15 (s, 1H), 9.56 (s, 1H), 8.42 (s, 1H), 8.35 (dd, *J* = 7.8, 1.4 Hz, 1H), 8.04 (d, *J* = 7.8 Hz, 1H), 7.97 - 7.84 (m, 2H), 7.76 (d, *J* = 7.6 Hz, 1H), 7.54 - 7.34 (m, 4H), 7.27 - 7.09 (m, 4H), 5.20 (dd, *J* = 12.9, 5.4 Hz, 1H), 3.73 - 3.42 (m, 2H), 3.28 - 3.16 (m, 2H), 2.97 - 2.84 (m, 1H), 2.76 - 2.51 (m, 4H), 2.34 (s, 3H), 2.13 - 2.05 (m, 1H), 1.37 - 1.19 (m, 4H).

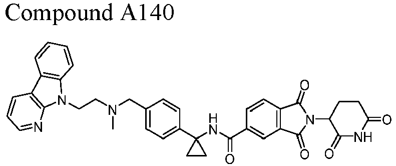

ESI-MS(M+H)⁺: 655.2.

¹H NMR (400 MHz, DMSO) δ 11.15 (s, 1H), 9.51 (s, 1H), 8.52 (dd, *J* = 7.6, 1.6 Hz, 1H), 8.45 - 8.38 (m, 2H), 8.34 (d, *J* = 7.8 Hz, 1H), 8.19 (d, *J* = 7.8 Hz, 1H), 8.04 (d, *J* = 7.8 Hz, 1H), 7.56 (d, *J* = 8.3 Hz, 1H), 7.47 (t, *J* = 7.2 Hz, 1H), 7.26 - 7.16 (m, 2H), 6.96 (d, *J* = 8.3 Hz, 2H), 6.87 (d, *J* = 8.2 Hz, 2H), 5.20 (dd, *J* = 13.0, 5.3 Hz, 1H), 4.60 (t, *J* = 6.6 Hz, 2H), 3.46 (s, 2H), 2.96 - 2.85 (m, 1H), 2.76 (t, *J* = 6.5 Hz, 2H), 2.70 - 2.55 (m, 2H), 2.24 (s, 3H), 2.15 - 2.05 (m, 1H), 1.31 - 1.16 (m, 4H).

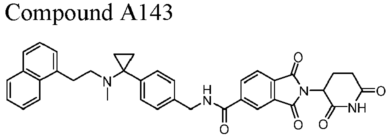

ESI-MS(M+H)⁺: 615.2.

¹H NMR (400 MHz, DMSO) δ 11.14 (s, 1H), 9.39 (t, *J* = 5.8 Hz, 1H), 8.40 - 8.30 (m, 2H), 8.03 - 7.97 (m, 2H), 7.94 - 7.87 (m, 1H), 7.76 (d, *J* = 8.0 Hz, 1H), 7.56 - 7.46 (m, 2H), 7.44 - 7.33 (m, 2H), 7.28 - 7.19 (m, 4H), 5.19 (dd, *J* = 12.8, 5.4 Hz, 1H), 4.47 (d, *J* = 5.8 Hz, 2H), 3.15 - 3.07 (m, 2H), 2.94 - 2.85 (m, 1H), 2.73 - 2.53 (m, 4H), 2.35 (s, 3H), 2.13 - 2.04(m, 1H), 0.81 (dd, *J* = 5.8, 3.9 Hz, 2H), 0.71 (dd, *J* = 6.0, 4.0 Hz, 2H).

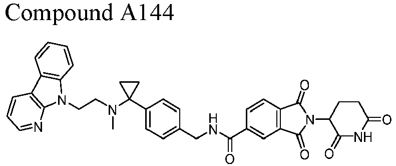

ESI-MS(M+H)⁺: 655.2.

¹H NMR (400 MHz, DMSO) δ 11.14 (s, 1H), 9.38 (t, *J* = 5.8 Hz, 1H), 8.52 (dd, *J* = 7.6, 1.6 Hz, 1H), 8.47 (dd, *J* = 4.8, 1.6 Hz, 1H), 8.40 - 8.31 (m, 2H), 8.19 (d, *J* = 7.7 Hz, 1H), 8.02 (d, *J* = 7.8 Hz, 1H), 7.64 (d, *J* = 8.2 Hz, 1H), 7.52 (t, *J* = 7.7 Hz, 1H), 7.30 - 7.14 (m, 6H), 5.19 (dd, *J* = 12.8, 5.4 Hz, 1H), 4.59 - 4.35 (m, 4H), 2.96 - 2.79 (m, 3H), 2.67 - 2.53 (m, 2H), 2.33 (s, 3H), 2.14 - 2.03 (m, 1H), 0.57 (q, *J* = 4.1 Hz, 2H), 0.43 (q, *J* = 3.7 Hz, 2H).

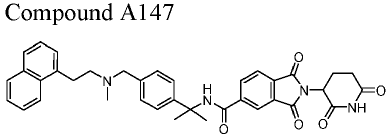

ESI-MS(M+H)⁺: 617.2.

¹H NMR (400 MHz, DMSO) δ 11.15 (s, 1H), 8.84 (s, 1H), 8.41 (s, 1H), 8.30 - 8.24 (m, 1H), 8.01 (d, *J* = 7.8 Hz, 1H), 7.97 - 7.92 (m, 1H), 7.91 - 7.87 (m, 1H), 7.75 (d, *J* = 7.4 Hz, 1H), 7.50 - 7.44 (m, 2H), 7.42 - 7.36 (m, 2H), 7.31 (d, *J* = 8.2 Hz, 2H), 7.20 (d, *J* = 8.2 Hz, 2H), 5.20 (dd, *J* = 12.9, 5.3 Hz, 1H), 3.54 (s, 2H), 3.25 - 3.17 (m, 2H), 2.99 - 2.82 (m, 1H), 2.71 - 2.56 (m, 4H), 2.30 (s, 3H), 2.15 - 2.05 (m, 1H), 1.69 (s, 6H).

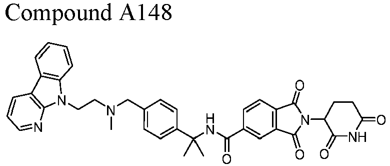

ESI-MS(M+H)⁺: 657.2.

¹H NMR (400 MHz, DMSO) δ 11.15 (s, 1H), 8.80 (s, 1H), 8.52 (dd, *J* = 7.6, 1.5 Hz, 1H), 8.45 - 8.36 (m, 2H), 8.26 (dd, *J* = 7.8, 1.2 Hz, 1H), 8.20 (d, *J* = 7.7 Hz, 1H), 8.01 (d, *J* = 7.8 Hz, 1H), 7.57 (d, *J* = 8.2 Hz, 1H), 7.46 (t, *J* = 7.4 Hz, 1H), 7.29 - 7.16 (m, 2H), 7.12 (d, *J* = 8.2 Hz, 2H), 6.88 (d, *J* = 8.2 Hz, 2H), 5.20 (dd, *J* = 12.9, 5.3 Hz, 1H), 4.60 (t, *J* = 6.5 Hz, 2H), 3.47 (s, 2H), 2.96 - 2.84 (m, 1H), 2.76 (t, *J* = 6.5 Hz, 2H), 2.67 - 2.53 (m, 2H), 2.26 (s, 3H), 2.14 - 2.05 (m, 1H), 1.64 (s, 6H).

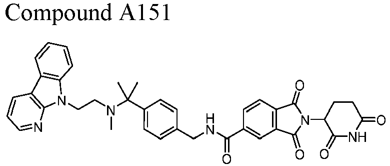

ESI-MS(M+H)⁺: 657.2.

¹H NMR (400 MHz, DMSO) δ 11.15 (s, 1H), 9.36 (t, *J* = 5.9 Hz, 1H), 8.49 (dd, *J* = 7.6, 1.5 Hz, 1H), 8.43 - 8.35 (m, 3H), 8.16 (d, *J* = 7.7 Hz, 1H), 8.06 (d, *J* = 7.6 Hz, 1H), 7.40 (t, *J* = 7.7 Hz, 1H), 7.27 (d, *J* = 8.1 Hz, 1H), 7.22 - 7.14 (m, 2H), 7.04 - 6.95 (m, 4H), 5.20 (dd, *J* = 12.9, 5.3 Hz, 1H), 4.48 (t, *J* = 6.5 Hz, 2H), 4.41 (d, *J* = 5.8 Hz, 2H), 2.96 - 2.83 (m, 1H), 2.70 - 2.53 (m, 4H), 2.31 (s, 3H), 2.13 - 2.05 (m, 1H), 1.05 (s, 6H).

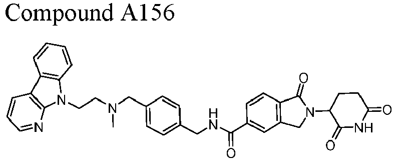

ESI-MS(M+H)⁺: 615.2.

¹H NMR (400 MHz, DMSO) δ 11.00 (s, 1H), 9.15 (t, *J* = 5.8 Hz, 1H), 8.52 (dd, *J* = 7.6, 1.6 Hz, 1H), 8.42 (dd, *J* = 4.8, 1.6 Hz, 1H), 8.20 (d, *J* = 7.8 Hz, 1H), 8.10 (s, 1H), 8.02 (d, *J* = 8.9 Hz, 1H), 7.83 (d, *J* = 8.0 Hz, 1H), 7.58 (d, *J* = 8.2 Hz, 1H), 7.47 (t, *J* = 7.2 Hz, 1H), 7.26 - 7.17 (m, 2H), 7.08 (d, *J* = 7.9 Hz, 2H), 6.94 (d, *J* = 7.9 Hz, 2H), 5.14 (dd, *J* = 13.3, 5.1 Hz, 1H), 4.61 (t, *J* = 6.5 Hz, 2H), 4.56 - 4.37 (m, 4H), 3.49 (s, 2H), 2.98 - 2.85 (m, 1H), 2.78 (t, *J* = 6.6 Hz, 2H), 2.68 - 2.57 (m, 1H), 2.47 - 2.37 (m, 1H), 2.24 (s, 3H), 2.07 - 1.99 (m, 1H).

### Example 6:

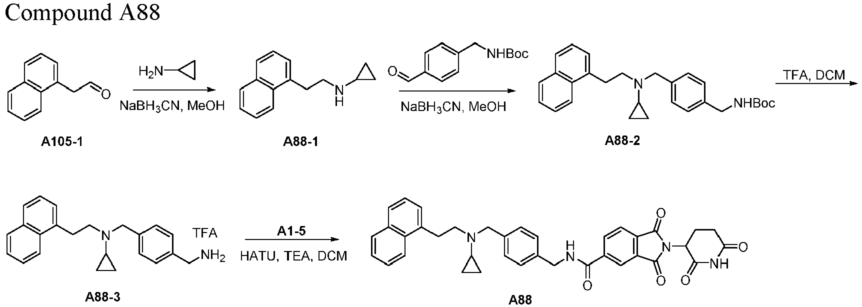

### Synthesis of compound A88-1

Compound A105-1 (170 mg, 1 mmol) and cyclopropylamine (57 mg, 1 mmol) were added to methanol (8 mL). The mixture was stirred at room temperature for 2 h, and then sodium cyanoborohydride (126 mg, 2 mmol) was added. The mixture was stirred overnight at room temperature, and then monitoring by TLC showed that the raw materials were completely reacted. Water (10 ml) was added, and the mixture was extracted with dichloromethane (20 mL*3). The organic phases were combined and then dried over anhydrous sodium sulfate. The solute was separated and purified by a thick preparative plate (developing solvent: DCM : MeOH = 20 : 1) to obtain compound A88-1 (74 mg, yield: 35%).

### Synthesis of compound A88-2

Compound A88-1 (74 mg, 0.35 mmol) and the compound tert-butyl 4-formylbenzylcarbamate (82 mg, 0.35 mmol) were added to methanol (6 mL). The mixture was stirred at room temperature for 2 h, and then sodium cyanoborohydride (44 mg, 0.7 mmol) was added. The mixture was stirred overnight at room temperature, and then monitoring by TLC showed that the raw materials were completely reacted. Water (10 ml) was added, and the mixture was extracted with dichloromethane (20 mL*3). The organic phases were combined and then dried over anhydrous sodium sulfate. The solute was separated and purified by a thick preparative plate (developing solvent: DCM : MeOH = 20 : 1) to obtain compound A88-2 (120 mg, yield: 80%).

### Synthesis of compound A88-3

Compound A88-2 (120 mg, 0.28 mmol) was added to dichloromethane (6 mL), and TFA (2 mL) was added at room temperature with stirring. The mixture was stirred at room temperature for 2 h, and then TLC showed that the reaction was completed. The reaction liquid was dried by rotary evaporation to obtain compound A88-3 (150 mg, crude), which was directly used in the next reaction.

### Synthesis of compound A88

Compound A88-3 (75 mg, 0.14 mmol, crude), compound A1-5 (42 mg, 0.14 mmol) and triethylamine (43 uL, 0.31 mmol) were added to dichloromethane (6 mL), and HATU (64 mg, 0.17 mmol) was added at room temperature with stirring. The mixture was reacted at room temperature for 1 h, and then TLC showed that the reaction was completed. Water (10 ml) was added, and the mixture was extracted with dichloromethane (20 mL*3). The organic phases were combined and then dried over anhydrous sodium sulfate. The solute was separated and purified by a thick preparative plate (developing solvent: DCM : MeOH= 10 : 1) and then purified by medium-pressure preparative chromatography to obtain compound A88 (30 mg, yield: 35%). ESI-MS(M+H)⁺: 615.2.

¹H NMR (400 MHz, DMSO) δ 11.14 (s, 1H), 9.43 (t, *J* = 5.9 Hz, 1H), 8.43 - 8.33 (m, 2H), 8.04 (d, *J* = 7.8 Hz, 1H), 7.85 (t, *J* = 8.7 Hz, 2H), 7.72 (d, *J* = 8.1 Hz, 1H), 7.45 - 7.34 (m, 3H), 7.29 (d, *J* = 10.6 Hz, 5H), 5.20 (dd, *J* = 12.9, 5.3 Hz, 1H), 4.52 (d, *J* = 5.8 Hz, 2H), 3.83 (s, 2H), 3.27 - 3.19 (m, 2H), 2.94 - 2.85 (m, 1H), 2.81 - 2.69 (m, 2H), 2.68 - 2.52 (m, 2H), 2.14 - 2.01 (m, 2H), 0.56 - 0.50 (m, 2H), 0.43 - 0.35 (m, 2H).

The synthesis methods for compounds A89-A97 and A233-A235 are the same as that for A88.

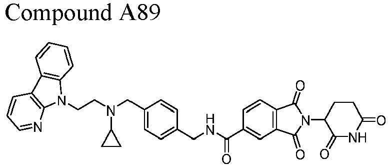

ESI-MS(M+H)⁺: 655.2.

¹H NMR (400 MHz, DMSO) δ 9.41 (t, *J* = 5.7 Hz, 1H), 8.54 - 8.43 (m, 2H), 8.41 - 8.33 (m, 2H), 8.17 (d, *J* = 7.7 Hz, 1H), 8.04 (d, *J* = 7.7 Hz, 1H), 7.48 - 7.38 (m, 2H), 7.24 - 7.16 (m, 6H), 5.19 (dd, *J* = 12.8, 5.3 Hz, 1H), 4.59 (t, *J* = 7.1 Hz, 2H), 4.49 (d, *J* = 5.8 Hz, 2H), 3.77 (s, 2H), 2.95 - 2.83 (m, 3H), 2.69 - 2.53 (m, 2H), 2.14 - 2.04 (m, 1H), 1.97 - 1.86 (m, 1H), 0.45 - 0.36 (m, 2H), 0.26 - 0.19 (m, 2H).

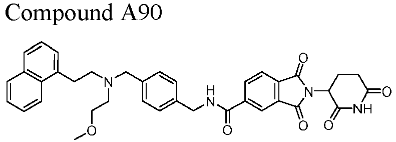

ESI-MS(M+H)⁺: 633.2.

¹H NMR (400 MHz, DMSO) δ 11.15 (s, 1H), 9.43 (t, *J* = 5.9 Hz, 1H), 8.44 - 8.35 (m, 2H), 8.04 (d, *J* = 7.8 Hz, 1H), 7.88 - 7.83 (m, 2H), 7.72 (d, *J* = 7.9 Hz, 1H), 7.45 - 7.36 (m, 3H), 7.34 - 7.27 (m, 5H), 5.20 (dd, *J* = 12.9, 5.4 Hz, 1H), 4.52 (d, *J* = 5.8 Hz, 2H), 3.72 (s, 2H), 3.44 (t, *J* = 5.9 Hz, 2H), 3.22 (s, 3H), 3.18 (dd, *J* = 9.2, 6.5 Hz, 2H), 2.95 - 2.85 (m, 1H), 2.77 - 2.73 (m, 4H), 2.66 - 2.52 (m, 2H), 2.13 - 2.07 (m, 1H).

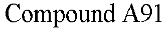

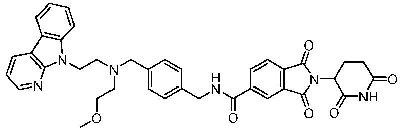

ESI-MS(M+H)⁺: 673.2.

¹H NMR (400 MHz, DMSO) δ 11.15 (s, 1H), 9.37 (t, *J* = 5.9 Hz, 1H), 8.50 (dd, *J* = 7.6, 1.6 Hz, 1H), 8.44 - 8.34 (m, 3H), 8.17 (d, *J* = 7.7 Hz, 1H), 8.04 (d, *J* = 7.8 Hz, 1H), 7.51 (d, *J* = 8.2 Hz, 1H), 7.46 - 7.40 (m, 1H), 7.25 - 7.16 (m, 2H), 7.10 (d, *J* = 8.0 Hz, 2H), 6.97 (d, *J* = 8.0 Hz, 2H), 5.20 (dd, *J* = 12.9, 5.4 Hz, 1H), 4.55 (t, *J* = 6.5 Hz, 2H), 4.45 (d, *J* = 5.7 Hz, 2H), 3.63 (s, 2H), 3.26 - 3.15 (m, 2H), 3.08 (s, 3H), 2.97 - 2.83 (m, 3H), 2.71 - 2.52 (m, 4H), 2.15 - 2.08 (m, 1H).

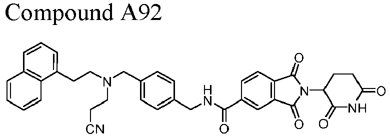

ESI-MS(M+H)⁺: 628.2.

¹H NMR (400 MHz, DMSO) δ 11.14 (s, 1H), 9.44 (t, *J* = 5.9 Hz, 1H), 8.43 - 8.34 (m, 2H), 8.05 (d, *J* = 7.8 Hz, 1H), 7.90 - 7.79 (m, 2H), 7.76 - 7.68 (m, 1H), 7.45 - 7.25 (m, 8H), 5.20 (dd, *J* = 12.9, 5.4 Hz, 1H), 4.52 (d, *J* = 5.8 Hz, 2H), 3.73 (s, 2H), 3.19 (dd, *J* = 9.3, 6.4 Hz, 2H), 2.96 - 2.84 (m, 3H), 2.78 - 2.67 (m, 4H), 2.66 - 2.52 (m, 2H), 2.14 - 2.06 (m, 1H).

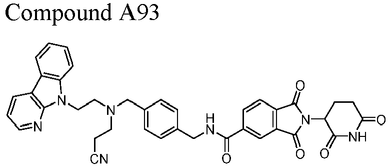

ESI-MS(M+H)⁺: 668.2.

¹H NMR (400 MHz, DMSO) δ 11.14 (s, 1H), 9.38 (t, *J* = 5.9 Hz, 1H), 8.51 (dd, *J* = 7.6, 1.6 Hz, 1H), 8.45 - 8.34 (m, 3H), 8.18 (d, *J* = 7.7 Hz, 1H), 8.05 (d, *J* = 7.8 Hz, 1H), 7.52 (d, *J* = 8.2 Hz, 1H), 7.47 - 7.39 (m, 1H), 7.26 - 7.18 (m, 2H), 7.11 (d, *J* = 8.0 Hz, 2H), 7.01 (d, *J* = 8.0 Hz, 2H), 5.20 (dd, *J* = 12.9, 5.4 Hz, 1H), 4.56 (t, *J* = 6.6 Hz, 2H), 4.45 (d, *J* = 5.7 Hz, 2H), 3.64 (s, 2H), 2.98 - 2.84 (m, 3H), 2.82 (t, *J* = 6.8 Hz, 2H), 2.66 - 2.52 (m, 4H), 2.14 - 2.07 (m, 1H).

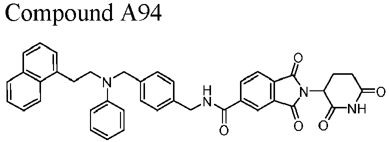

ESI-MS(M+H)⁺: 651.2.

¹H NMR (400 MHz, DMSO) δ 11.13 (s, 1H), 9.40 (t, *J* = 5.8 Hz, 1H), 8.36 - 8.32 (m, 2H), 8.02 - 8.00 (m, 2H), 7.91 (dd, *J* = 6.7, 2.7 Hz, 1H), 7.79 (dd, *J* = 6.7, 2.7 Hz, 1H), 7.54 - 7.45 (m, 2H), 7.43 - 7.40 (m, 2H), 7.28 (d, *J* = 8.1 Hz, 2H), 7.22 - 7.10 (m, 4H), 6.75 (d, *J* = 8.2 Hz, 2H), 6.61 (t, *J* = 7.2 Hz, 1H), 5.18 (dd, *J* = 12.8, 5.4 Hz, 1H), 4.48 (d, *J* = 8.7 Hz, 4H), 3.76 - 3.64 (m, 2H), 3.32 - 3.31 (m, 2H), 2.94 - 2.82 (m, 1H), 2.67 - 2.52 (m, 2H), 2.12 - 2.04 (m, 1H).

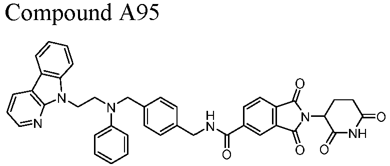

ESI-MS(M+H)⁺: 691.2.

¹H NMR (400 MHz, DMSO) δ 11.13 (s, 1H), 9.38 (t, *J* = 6.0 Hz, 1H), 8.56 - 8.48 (m, 2H), 8.39 - 8.28 (m, 2H), 8.19 (d, *J* = 7.7 Hz, 1H), 8.02 (d, *J* = 8.0 Hz, 1H), 7.53 - 7.44 (m, 2H), 7.28 - 7.20 (m, 4H), 7.13 - 7.03 (m, 4H), 6.77 (d, *J* = 8.1 Hz, 2H), 6.59 (t, *J* = 7.2 Hz, 1H), 5.18 (dd, *J* = 12.8, 5.4 Hz, 1H), 4.69 (t, *J* = 6.8 Hz, 2H), 4.48 - 4.38 (m, 4H), 3.85 (t, *J* = 6.8 Hz, 2H), 2.96 - 2.83 (m, 1H), 2.68 - 2.52 (m, 2H), 2.13 - 2.03 (m, 1H).

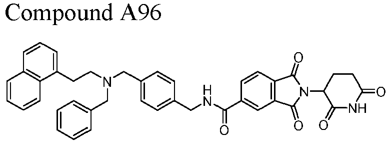

ESI-MS(M+H)⁺: 665.2.

¹H NMR (400 MHz, DMSO) δ 11.14 (s, 1H), 9.42 (t, *J* = 5.8 Hz, 1H), 8.46 - 8.31 (m, 2H), 8.04 (d, *J* = 7.8 Hz, 1H), 7.84 (d, *J* = 7.6 Hz, 1H), 7.70 (dd, *J* = 11.4, 8.5 Hz, 2H), 7.42 - 7.17 (m, 13H), 5.19 (dd, *J* = 12.9, 5.4 Hz, 1H), 4.51 (d, *J* = 5.7 Hz, 2H), 3.70 (s, 4H), 3.26 - 3.19 (m, 2H), 2.94 - 2.85 (m, 1H), 2.72 - 2.52 (m, 4H), 2.14 - 2.04 (m, 1H).

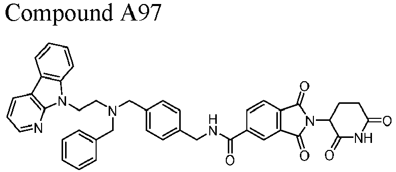

ESI-MS(M+H)⁺: 705.2.

¹H NMR (400 MHz, DMSO) δ 11.14 (s, 1H), 9.37 (t, *J* = 5.9 Hz, 1H), 8.52 (dd, *J* = 7.6, 1.6 Hz, 1H), 8.42 - 8.35 (m, 2H), 8.33 (dd, *J* = 4.8, 1.6 Hz, 1H), 8.20 (d, *J* = 7.7 Hz, 1H), 8.04 (d, *J* = 7.7 Hz, 1H), 7.46 - 7.31 (m, 2H), 7.25 - 7.14 (m, 2H), 7.13 - 7.03 (m, 5H), 7.01 - 6.93 (m, 4H), 5.20 (dd, *J* = 12.9, 5.4 Hz, 1H), 4.63 (t, *J* = 6.1 Hz, 2H), 4.43 (d, *J* = 5.7 Hz, 2H), 3.58 (s, 4H), 2.95 - 2.82 (m, 3H), 2.67 - 2.52 (m, 2H), 2.12 - 2.03 (m, 1H).

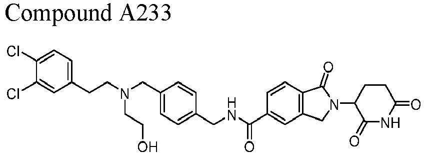

ESI-MS(M+H)⁺: 623.2.

¹H NMR (400 MHz, DMSO) δ 11.01 (s, 1H), 9.20 (t, *J* = 6.1 Hz, 1H), 8.10 (s, 1H), 8.01 (d, *J* = 7.7 Hz, 1H), 7.82 (d, *J* = 7.9 Hz, 1H), 7.49 - 7.46 (m, 2H), 7.25 - 7.14 (m, 5H), 5.14 (dd, *J* = 13.3, 5.1 Hz, 1H), 4.55 - 4.28 (m, 5H), 3.62 (s, 2H), 3.46 - 3.39 (m, 2H), 2.98 - 2.85 (m, 1H), 2.77 - 2.65 (m, 4H), 2.63 - 2.51 (m, 3H), 2.42 (dd, *J* = 12.9, 4.5 Hz, 1H), 2.06 - 1.98 (m, 1H).

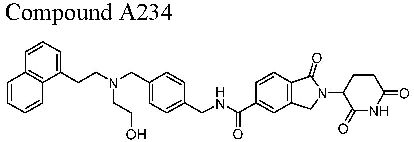

ESI-MS(M+H)⁺: 605.2.

¹H NMR (400 MHz, DMSO) δ 11.01 (s, 1H), 9.22 (t, *J* = 6.0 Hz, 1H), 8.11 (s, 1H), 8.03 (d, *J* = 8.1 Hz, 1H), 7.89 - 7.80 (m, 3H), 7.73 (d, *J* = 7.9 Hz, 1H), 7.45 - 7.36 (m, 3H), 7.35 - 7.24 (m, 5H), 5.14 (dd, *J* = 13.3, 5.1 Hz, 1H), 4.58 - 4.34 (m, 5H), 3.74 (s, 2H), 3.50 (t, *J* = 6.4 Hz, 2H), 3.23 - 3.14 (m, 2H), 2.98 - 2.83 (m, 1H), 2.81 - 2.72 (m, 2H), 2.71 - 2.56 (m, 3H), 2.46 - 2.32 (m, 1H), 2.06 - 2.00 (m, 1H).

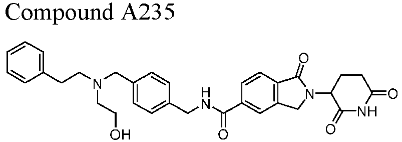

ESI-MS(M+H)⁺: 555.2.

¹H NMR (400 MHz, DMSO) δ 11.01 (s, 1H), 9.22 (s, 1H), 8.10 (s, 1H), 8.01 (d, *J* = 8.0 Hz, 1H), 7.82 (d, *J* = 7.9 Hz, 1H), 7.39 - 7.10 (m, 9H), 5.13 (dd, *J* = 13.2, 5.1 Hz, 1H), 4.60 - 4.32 (m, 4H), 3.69 - 3.33 (m, 6H), 2.98 - 2.86 (m, 1H), 2.83 - 2.53 (m, 5H), 2.46 - 2.31 (m, 1H), 2.09 - 1.96 (m, 1H).

### Example 7:

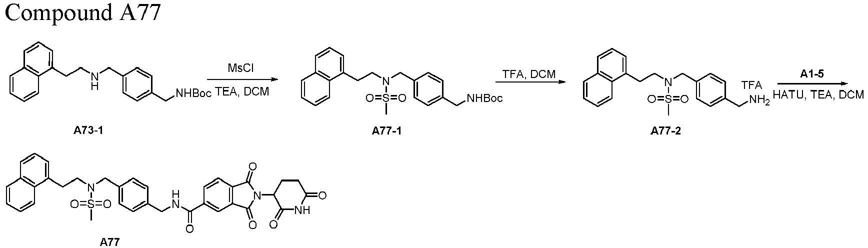

### Synthesis of compound A77-1

Compound A73-1 (100 mg, 0.25 mmol) was dissolved in dichloromethane (6 ml). Under ice bath, methanesulfonyl chloride (43 mg, 0.38 mmol) and triethylamine (50 mg, 0.5 mmol) were added. The reaction was stirred at room temperature for 1 h, and then monitoring by TLC showed that the reaction was completed. The reaction was quenched with water (20 mL). The aqueous phase was extracted with DCM (40 ml*3). The organic phases were combined and then dried by rotary evaporation. The solute was separated and purified by a thick preparative plate (DCM : MeOH = 15 : 1) to obtain A77-1 (120 mg, yield 100%) as a light yellow solid.

### Synthesis of compound A77-2

Compound A77-1 (80 mg, 0.17 mmol) was placed in a 50-ml round bottom flask, and 4 ml of (DCM : TFA = 3 : 1) solution was added. The reaction was stirred at room temperature for 2 h, and then monitoring by TLC showed that the reaction was completed. The solvent was removed by drying to obtain crude product A77-2 (150 mg, crude) as an oil, which was directly used in the next reaction.

### Synthesis of compound A77

Compound A77-2 (150 mg, 0.17 mmol) and compound A1-5 (52 mg, 0.17 mmol) were dissolved in dichloromethane (6 ml), and then HATU (84 mg, 0.22 mmol) and TEA (53 mg, 0.51 mmol) were added. The reaction was stirred at room temperature for 2 h, and then monitoring by TLC showed that the raw materials were completely reacted. Water was added, and then the mixture was extracted with dichloromethane (40 ml*3). The organic phases were combined and then dried over anhydrous sodium sulfate. The solvent was removed by drying. The solute was separated and purified by a thick preparative plate using a developing solvent (DCM : MeOH = 15 : 1) to obtain a crude, which was then purified by medium-pressure preparative chromatography to obtain compound A77 (13 mg, yield 12%).

ESI-MS(M+H)⁺: 653.2.

¹H NMR (400 MHz, DMSO) δ 11.14 (s, 1H), 9.47 (t, *J* = 5.9 Hz, 1H), 8.43 - 8.32 (m, 2H), 8.03 (d, *J* = 7.8 Hz, 1H), 7.89 - 7.84 (m, 1H), 7.77 - 7.72 (m, 2H), 7.46 - 7.35 (m, 7H), 7.26 (d, *J* = 6.6 Hz, 1H), 5.19 (dd, *J* = 12.9, 5.4 Hz, 1H), 4.55 (d, *J* = 5.8 Hz, 2H), 4.43 (s, 2H), 3.31 - 3.28 (m, 2H), 3.19 - 3.10 (m, 2H), 2.96 (s, 3H), 2.93 - 2.84 (m, 1H), 2.68 - 2.52 (m, 2H), 2.12 - 2.06 (m, 1H).

The synthesis methods for compounds A78-A82 and A232 are the same as that for A77.

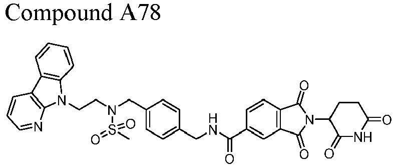

ESI-MS(M+H)⁺: 693.2.

¹H NMR (400 MHz, DMSO) δ 11.14 (s, 1H), 9.42 (t, *J* = 5.9 Hz, 1H), 8.49 (dd, *J* = 7.6, 1.4 Hz, 1H), 8.43 (dd, *J* = 4.8, 1.4 Hz, 1H), 8.39 - 8.35 (m, 2H), 8.16 (d, *J* = 7.8 Hz, 1H), 8.04 (d, *J* = 7.8 Hz, 1H), 7.47 (t, *J* = 7.7 Hz, 1H), 7.36 (d, *J* = 8.2 Hz, 1H), 7.27 - 7.14 (m, 6H), 5.19 (dd, *J* = 12.9, 5.4 Hz, 1H), 4.54 - 4.47 (m, 4H), 4.31 (s, 2H), 3.53 (t, *J* = 6.7 Hz, 2H), 2.95 - 2.85 (m, 1H), 2.83 (s, 3H), 2.67 - 2.52 (m, 2H), 2.12 - 2.07 (m, 1H).

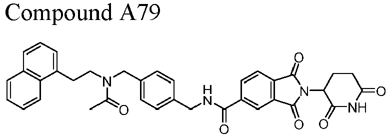

ESI-MS(M+H)⁺: 617.2.

¹H NMR (400 MHz, DMSO) δ 11.14 (s, 1H), 9.42 (q, *J* = 5.6 Hz, 1H), 8.41 - 8.30 (m, 2H), 8.15 - 7.99 (m, 2H), 7.89 (dd, *J* = 13.4, 5.4 Hz, 1H), 7.78 (dd, *J* = 14.1, 8.2 Hz, 1H), 7.57 - 7.38 (m, 3H), 7.36 - 7.30 (m, 3H), 7.24 - 7.12 (m, 2H), 5.19 (dd, *J* = 12.8, 5.3 Hz, 1H), 4.59 - 4.43 (m, 4H), 3.48 (dd, *J* = 14.2, 6.2 Hz, 2H), 3.31 - 3.26 (m, 1H), 3.22 - 3.14 (m, 1H), 2.96 - 2.83 (m, 1H), 2.67 - 2.52 (m, 2H), 2.13 - 1.86 (m, 4H).

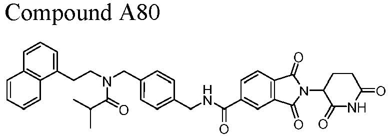

ESI-MS(M+H)⁺: 645.2.

¹H NMR (400 MHz, DMSO) δ 11.13 (s, 1H), 9.41 (d, *J* = 2.3 Hz, 1H), 8.41 - 8.32 (m, 2H), 8.20 - 7.98 (m, 2H), 7.90 (t, *J* = 8.4 Hz, 1H), 7.78 (dd, *J* = 12.2, 8.1 Hz, 1H), 7.58 - 7.46 (m, 2H), 7.45 - 7.25 (m, 4H), 7.15 (t, *J* = 8.4 Hz, 2H), 5.19 (dd, *J* = 12.8, 5.3 Hz, 1H), 4.57 - 4.48 (m, 4H), 3.56 - 3.43 (m, 2H), 3.31 - 3.27 (m, 1H), 3.25 - 3.14 (m, 1H), 2.96 - 2.51 (m, 4H), 2.13 - 2.03 (m, 1H), 1.02 (d, *J* = 6.6 Hz, 3H), 0.81 (d, *J* = 6.6 Hz, 3H).

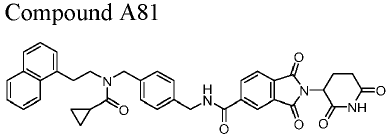

ESI-MS(M+H)⁺: 643.2.

¹H NMR (400 MHz, DMSO) δ 11.14 (s, 1H), 9.42 (q, *J* = 5.9 Hz, 1H), 8.41 - 8.31 (m, 2H), 8.21 - 7.95 (m, 2H), 7.90 (t, *J* = 8.4 Hz, 1H), 7.78 (dd, *J* = 12.4, 8.1 Hz, 1H), 7.56 - 7.45 (m, 2H), 7.45 - 7.28 (m, 4H), 7.24 (d, *J* = 8.1 Hz, 1H), 7.16 (d, *J* = 8.0 Hz, 1H), 5.19 (dd, *J* = 12.8, 5.4 Hz, 1H), 4.75 (s, 1H), 4.49 (d, *J* = 6.7 Hz, 3H), 3.70 (t, *J* = 7.3 Hz, 1H), 3.56 - 3.45 (m, 1H), 3.37 - 3.35 (m, 1H), 3.23 - 3.16 (m, 1H), 2.96 - 2.82 (m, 1H), 2.68 - 2.53 (m, 2H), 2.12 - 2.02 (m, 1H), 1.97 - 1.74 (m, 1H), 0.90 - 0.79 (m, 1H), 0.75 - 0.63 (m, 2H), 0.61 - 0.54 (m, 1H).

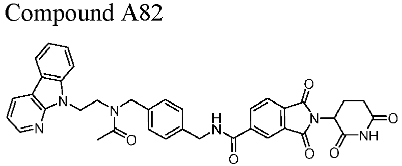

ESI-MS(M+H)⁺: 657.2.

¹H NMR (400 MHz, DMSO) δ 11.14 (s, 1H), 9.40 (d, *J* = 2.5 Hz, 1H), 8.59 - 8.43 (m, 2H), 8.42 - 8.30 (m, 2H), 8.21 (t, *J* = 7.0 Hz, 1H), 8.03 (d, *J* = 7.8 Hz, 1H), 7.67 - 7.58 (m, 1H), 7.56 - 7.50 (m, 1H), 7.31 - 7.22 (m, 4H), 7.14 (d, *J* = 8.0 Hz, 1H), 6.99 (d, *J* = 8.1 Hz, 1H), 5.19 (dd, *J* = 12.9, 5.4 Hz, 1H), 4.69 - 4.59 (m, 2H), 4.47 (t, *J* = 5.0 Hz, 2H), 4.45 (s, 1H), 4.20 (s, 1H), 3.70 - 3.61 (m, 2H), 2.96 - 2.81 (m, 1H), 2.69 - 2.52 (m, 2H), 2.13 - 2.05 (m, 1H), 1.88 - 1.67 (m, 3H).

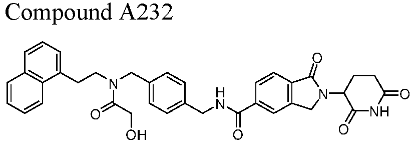

ESI-MS(M+H)⁺: 619.2.

¹H NMR (400 MHz, DMSO) δ 11.00 (s, 1H), 9.21 (d, *J* = 4.2 Hz, 1H), 8.14 (d, *J=* 7.9 Hz, 1H), 8.07 (s, 1H), 8.02 - 7.98 (m, 1H), 7.92 - 7.88 (m, 1H), 7.83 - 7.73 (m, 2H), 7.57 - 7.45 (m, 2H), 7.43 - 7.27 (m, 4H), 7.24 - 7.19 (m, 2H), 5.13 (dd, *J* = 13.2, 5.0 Hz, 1H), 4.62 (s, 1H), 4.53 - 4.32 (m, 5H), 4.16 (s, 1H), 3.96 (s, 1H), 3.53 - 3.44 (m, 1H), 3.30 - 3.13 (m, 3H), 3.00 - 2.84 (m, 1H), 2.66 - 2.55 (m, 1H), 2.47 - 2.27 (m, 1H), 2.05 - 1.93 (m, 1H).

### Example 8:

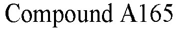

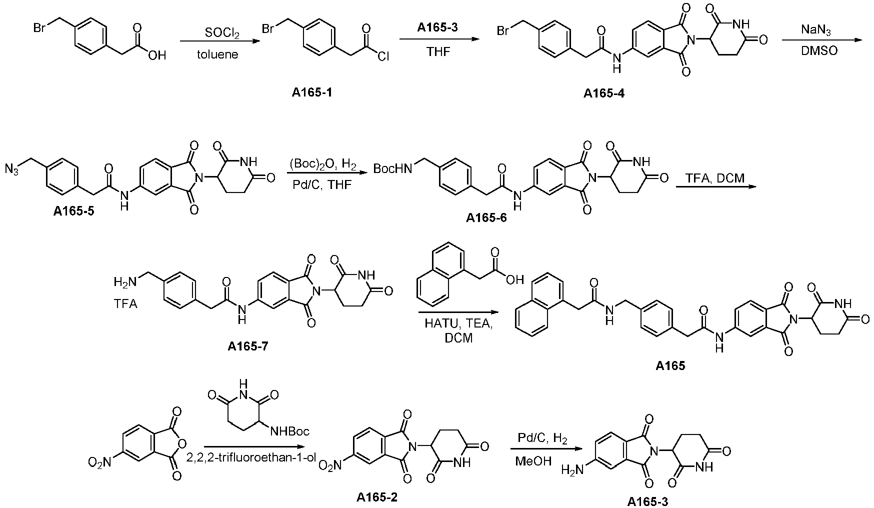

### Synthesis of compound A165-1

The compound 4-bromomethylphenylacetic acid (684 mg, 3.0 mmol) was dissolved in 20 mL of toluene, and thionyl chloride (1.07 g, 9.0 mmol) was added. The reaction was stirred at 90°C for 6 h, and then TLC showed that the reaction was completed. The solvent was dried by rotary evaporation to obtain compound A165-1 (750 mg, crude). Synthesis of compound A165-2

The compounds 4-nitrophthalic anhydride (1.0 g, 5.18 mmol) and 3-N-tertbutoxycarbonylamino-2,6-dioxopiperidine (1.18 g, 5.18 mmol) were dissolved in trifluoroethanol (10 ml). The mixture was reacted under microwave at 120°C for 2 h, cooled to room temperature and then further cooled to -20°C, with a blue solid precipitated. The precipitated solid was filtered, and the filter cake was washed with ethyl acetate (10 mL) to obtain compound A165-2 (1.56 g, yield 99%).

### Synthesis of compound A165-3

Compound A165-3 (1.56 g, 5.15 mmol) was dissolved in 25 mL of methanol, and Pd/C (150 mg, 5%) was added. The mixture was reacted overnight at room temperature under hydrogen atmosphere, and then TLC showed that the reaction was completed. The reaction liquid was subjected to suction filtration. The filtrate was dried by rotary evaporation to obtain compound A165-3 (650 mg, yield 46%).

### Synthesis of compound A165-4

Compound A165-3 (220 mg, 0.81 mmol) was dissolved in 10 mL of anhydrous tetrahydrofuran, and A165-1 (200 mg, 0.81 mmol) was added. The reaction was stirred at 80°C for 3 h, and then TLC showed that the reaction was completed. The reaction liquid was dried by rotary evaporation. The solute was purified by a preparative plate (mobile phase: petroleum ether : ethyl acetate = 1 : 3) to obtain compound A165-4 (350 mg, yield 89%).

### Synthesis of compound A165-5

Compound A165-4 (290 mg, 0.60 mmol) was dissolved in 10 mL of DMSO, and sodium azide (40 mg, 0.60 mmol) was added. The mixture was reacted at room temperature for 30 min, and then TLC showed that the reaction was completed. The mixture was diluted with water (20 mL). The aqueous phase was extracted with ethyl acetate (10 ml*3). The organic phases were combined and then washed with saturated brine (10 mL) to obtain compound A165-5 (270 mg, yield 99%).

### Synthesis of compound A165-6

Compound A165-5 (270 mg, 0.60 mmol) was dissolved in 10 mL of tetrahydrofuran, and (Boc)₂O (154 mg, 0.70 mmol) and Pd/C (30 mg, 5%) were added. The mixture was reacted overnight at room temperature under hydrogen atmosphere, and then TLC showed that the reaction was completed. The reaction liquid was filtered, and the filtrate was dried by rotary evaporation. The solute was purified by column chromatography (mobile phase: petroleum ether : ethyl acetate = 1 : 3) to obtain compound A165-6 (117 mg, yield 53%). ¹H NMR (400 MHz, DMSO) δ 11.10 (s, 1H), 10.80 (s, 1H), 8.23 (d, *J* = 1.4 Hz, 1H), 7.93 - 7.85 (m, 2H), 7.34 (dd, *J* = 13.0, 7.3 Hz, 1H), 7.28 (d, *J* = 8.0 Hz, 2H), 7.19 (d, *J* = 8.0 Hz, 2H), 5.11 (dd, *J* = 12.8, 5.4 Hz, 1H), 4.09 (d, *J* = 6.1 Hz, 2H), 3.71 (s, 2H), 2.94 - 2.81 (m, 1H), 2.64 - 2.53 (m, 2H), 2.10 - 2.00 (m, 1H), 1.36 (s, 9H).

### Synthesis of compound A165-7

Compound A165-6 (80 mg, 0.15 mmol) was dissolved in 6 mL of dichloromethane, and 2 mL of trifluoroacetic acid was added. The mixture was reacted at room temperature for 1 h, and then TLC showed that the reaction was completed. The solvent was dried by rotary evaporation to obtain compound A165-7 (120 mg, crude). Synthesis of compound A165

Compound A165-7 (120 mg, 0.15 mmol), naphthaleneacetic acid (32 mg, 0.17 mmol) and triethylamine (34 mg, 0.34 mmol) were dissolved in 5 mL of dichloromethane, and HATU (65 mg, 0.17 mmol) was added. The mixture was reacted at room temperature for 2 h, and then TLC showed that the reaction was completed. The reaction was quenched with water (20 mL). The aqueous phase was extracted with dichloromethane (10 mL*3). The organic phases were combined and dried by rotary evaporation. The solute was purified by a preparative plate (mobile phase: dichloromethane : methanol = 10 : 1) and then purified by medium-pressure preparative chromatography to obtain compound A165 (40 mg, 44%).

ESI-MS(M+H)⁺: 589.2.

¹H NMR (400 MHz, DMSO) δ 11.11 (s, 1H), 10.81 (s, 1H), 8.62 (t, *J* = 5.9 Hz, 1H), 8.23 (d, *J* = 1.4 Hz, 1H), 8.14 - 8.05 (m, 1H), 7.97 - 7.85 (m, 3H), 7.83 - 7.79 (m, 1H), 7.55 - 7.47 (m, 2H), 7.45 - 7.42 (m, 2H), 7.27 (d, *J* = 8.1 Hz, 2H), 7.19 (d, *J* = 8.1 Hz, 2H), 5.11 (dd, *J* = 12.8, 5.4 Hz, 1H), 4.26 (d, *J* = 5.8 Hz, 2H), 3.95 (s, 2H), 3.70 (s, 2H), 2.84 - 2.81 (m, 1H), 2.67 - 2.52 (m, 2H), 2.10 - 2.01 (m, 1H).

The synthesis method for compound A166 is the same as that for A165.

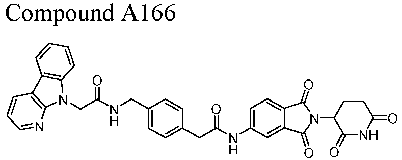

ESI-MS(M+H)⁺: 629.2.

¹H NMR (400 MHz, DMSO) δ 11.11 (s, 1H), 10.81 (s, 1H), 8.74 (t, *J* = 5.9 Hz, 1H), 8.54 (dd, *J* = 7.6, 1.6 Hz, 1H), 8.45 (dd, J= 4.8, 1.6 Hz, 1H), 8.24 - 8.19 (m, 2H), 7.94 - 7.86 (m, 2H), 7.56 (d, *J* = 8.2 Hz, 1H), 7.53 - 7.46 (m, 1H), 7.33 - 7.22 (m, 6H), 5.19 - 5.07 (m, 3H), 4.28 (d, *J* = 5.8 Hz, 2H), 3.72 (s, 2H), 2.95 - 2.80 (m, 1H), 2.66 - 2.53 (m, 2H), 2.07 - 1.99 (m, 1H).

### Example 9:

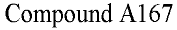

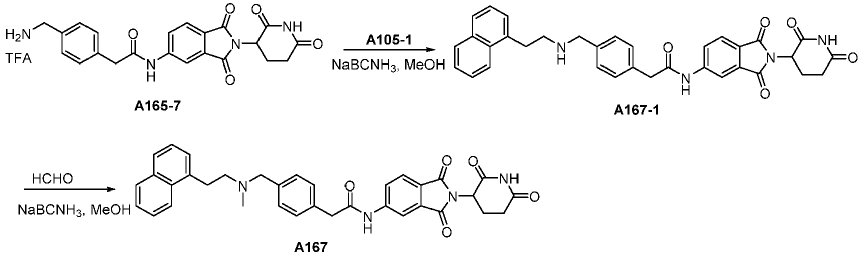

### Synthesis of compound A167-1

Compound A165-7 (180 mg, 0.20 mmol) was dissolved in 6 mL of methanol, and A105-1 (34 mg, 0.20 mmol) and sodium cyanoborohydride (15 mg, 0.23 mmol) were added. The mixture was reacted at room temperature for 5 h, and then TLC showed that the reaction was completed. The reaction was quenched with water (30 mL). The aqueous phase was extracted with dichloromethane (20 mL*3). The organic phases were combined and dried by rotary evaporation. The solute was purified by a preparative plate (mobile phase: dichloromethane : methanol = 20 : 1) to obtain compound A167-1 (45 mg, yield 39%).

### Synthesis of compound A167

Compound A167-1 (45 mg, 0.078 mmol) was dissolved in 5 mL of methanol, and paraformaldehyde (7 mg, 0.23 mmol) and sodium cyanoborohydride (15 mg, 0.23 mmol) were added. The mixture was reacted at room temperature for 5 h, and then TLC showed that the reaction was completed. The reaction was quenched with water (30 mL). The aqueous phase was extracted with dichloromethane (20 mL*3). The organic phases were combined and dried by rotary evaporation. The solute was purified by a preparative plate (mobile phase: dichloromethane : methanol = 20 : 1) and then purified by medium-pressure preparative chromatography to obtain compound A167 (10 mg, yield 22%). ESI-MS(M+H)⁺: 589.2.

¹H NMR (400 MHz, DMSO) δ 11.11 (s, 1H), 10.82 (s, 1H), 8.26 (d, *J =* 1.4 Hz, 1H), 7.95 - 7.85 (m, 4H), 7.74 (d, *J=* 7.5 Hz, 1H), 7.47 - 7.34 (m, 4H), 7.31 - 7.23 (m, 4H), 5.12 (dd, *J=* 12.9, 5.4 Hz, 1H), 3.71 (s, 2H), 3.56 (s, 2H), 3.25 - 3.18 (m, 2H), 2.94 - 2.82 (m, 1H), 2.69 - 2.51 (m, 4H), 2.32 (s, 3H), 2.07 - 1.99 (m, 1H).

The synthesis methods for compounds A168-170 and A204-A206 are the same as that for A167.

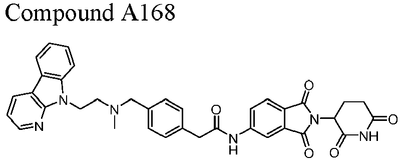

ESI-MS(M+H)⁺: 629.2.

¹H NMR (400 MHz, DMSO) δ 11.11 (s, 1H), 10.78 (s, 1H), 8.51 (dd, *J* = 7.6, 1.6 Hz, 1H), 8.41 (dd, *J* = 4.8, 1.6 Hz, 1H), 8.25 (d, *J* = 1.4 Hz, 1H), 8.19 (d, *J=* 7.7 Hz, 1H), 7.94 - 7.86 (m, 2H), 7.55 (d, *J=* 8.2 Hz, 1H), 7.49 - 7.41 (m, 1H), 7.24 - 7.18 (m, 2H), 7.09 (d, *J=* 8.0 Hz, 2H), 6.94 (d, *J=* 8.0 Hz, 2H), 5.12 (dd, *J* = 12.9, 5.4 Hz, 1H), 4.60 (t, *J* = 6.6 Hz, 2H), 3.64 (s, 2H), 3.48 (s, 2H), 2.95 - 2.82 (m, 1H), 2.81 - 2.73 (m, 2H), 2.64 - 2.51 (m, 2H), 2.24 (s, 3H), 2.08 - 2.00 (m, 1H).

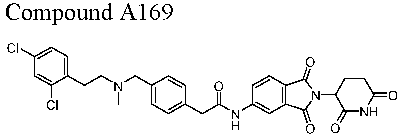

ESI-MS(M+H)⁺: 607.2.

¹H NMR (400 MHz, DMSO) δ 11.11 (s, 1H), 10.81 (s, 1H), 8.25 (d, *J=* 1.4 Hz, 1H), 7.93 - 7.86 (m, 2H), 7.50 (d, *J=* 1.9 Hz, 1H), 7.38 - 7.29 (m, 2H), 7.25 (d, *J* = 8.0 Hz, 2H), 7.17 (d, *J=* 8.0 Hz, 2H), 5.11 (dd, *J* = 12.8, 5.4 Hz, 1H), 3.70 (s, 2H), 3.49 (s, 2H), 2.94 - 2.82 (m, 3H), 2.65 - 2.51 (m, 4H), 2.20 (s, 3H), 2.08 - 2.00 (m, 1H).

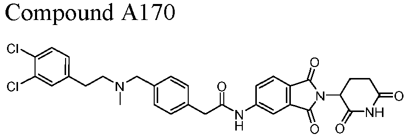

ESI-MS(M+H)⁺: 607.2.

¹H NMR (400 MHz, DMSO) δ 11.10 (s, 1H), 10.81 (s, 1H), 8.24 (d, *J* = 1.4 Hz, 1H), 7.93 - 7.86 (m, 2H), 7.50 - 7.48 (m, 2H), 7.25 (d, *J* = 8.0 Hz, 2H), 7.22 - 7.11 (m, 3H), 5.11 (dd, *J* = 12.8, 5.4 Hz, 1H), 3.70 (s, 2H), 3.48 (s, 2H), 2.98 - 2.81 (m, 1H), 2.76 (t, *J* = 7.1 Hz, 2H), 2.66 - 2.53 (m, 4H), 2.15 (s, 3H), 2.09 - 1.96 (m, 1H).

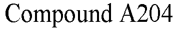

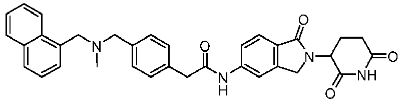

ESI-MS(M+H)⁺: 561.2
¹H NMR (400 MHz, DMSO) δ 10.98 (s, 1H), 10.50 (s, 1H), 8.27 - 8.22 (m, 1H), 7.97 (s, 1H), 7.94 - 7.89 (m, 1H), 7.84 (d, *J* = 7.8 Hz, 1H), 7.66 (d, *J* = 8.3 Hz, 1H), 7.61 (dd, *J* = 8.3, 1.6 Hz, 1H), 7.54 - 7.42 (m, 4H), 7.34 - 7.25 (m, 4H), 5.08 (dd, *J* = 13.3, 5.1 Hz, 1H), 4.41 (d, *J* = 17.4 Hz, 1H), 4.27 (d, *J=* 17.4 Hz, 1H), 3.91 (s, 2H), 3.68 (s, 2H), 3.56 (s, 2H), 2.95 - 2.85 (m, 1H), 2.64 - 2.56 (m, 1H), 2.41 - 2.33 (m, 1H), 2.08 (s, 3H), 2.02 - 1.95 (m, 1H).

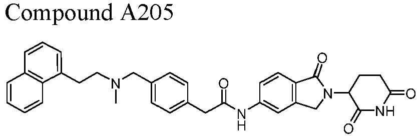

ESI-MS(M+H)⁺: 575.2
¹H NMR (400 MHz, DMSO) δ 10.99 (s, 1H), 10.52 (s, 1H), 7.99 (s, 1H), 7.91 - 7.85 (m, 2H), 7.74 (d, *J* = 7.6 Hz, 1H), 7.68 (d, *J* = 8.3 Hz, 1H), 7.62 (dd, *J* = 8.4, 1.5 Hz, 1H), 7.48 - 7.34 (m, 4H), 7.26 (q, *J* = 8.3 Hz, 4H), 5.09 (dd, *J* = 13.3, 5.1 Hz, 1H), 4.35 (dd, *J* = 55.0, 17.4 Hz, 2H), 3.67 (s, 2H), 3.55 (s, 2H), 3.20 (dd, *J* = 18.3, 10.3 Hz, 2H), 2.99 - 2.84 (m, 1H), 2.71 - 2.54 (m, 3H), 2.42 - 2.33 (m, 1H), 2.29 (s, 3H), 2.04 - 1.92 (m, 1H).

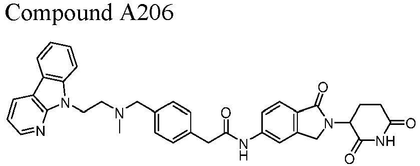

ESI-MS(M+H)⁺: 615.2
¹H NMR (400 MHz, DMSO) δ 10.99 (s, 1H), 10.57 (s, 1H), 8.54 (s, 1H), 8.44 (s, 1H), 8.22 (d, *J* = 7.3 Hz, 1H), 7.98 (s, 1H), 7.84 - 7.62 (m, 10H), 5.08 (dd, *J* = 13.3, 5.0 Hz, 1H), 4.97 - 4.45 (m, 2H), 4.35 (dd, *J* = 56.3, 17.4 Hz, 2H), 3.82 - 3.40 (m, 5H), 2.99 - 2.67 (m, 3H), 2.64 - 2.54 (m, 1H), 2.43 - 2.13 (m, 3H), 2.02 - 1.92 (m, 1H).

### Example 10:

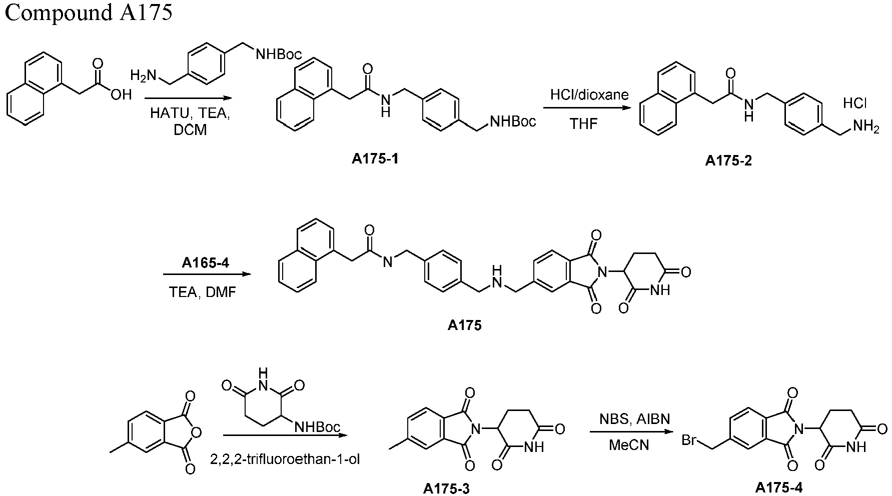

### Synthesis of compound A175-1

Naphthaleneacetic acid (2.71 g, 14.5 mmol) was dissolved in dichloromethane (50 mL), and then 1-(*N*-Boc-aminomethyl)-4-(aminomethyl)benzene (3.43 g, 14.5 mmol), triethylamine (1.75 g, 17.4 mmol) and HATU (6.61 g, 17.4 mmol) were successively added. The reaction was stirred at room temperature for 2 h, and then TLC showed that the raw materials were completely reacted. Water (50 mL) was added, and the mixture was extracted with dichloromethane (20 ml*3). The organic phases were combined. The solvent was dried by rotary evaporation. The solute was purified by column chromatography (mobile phase: dichloromethane : methanol = 30 : 1) to obtain compound A175-1 (4.19 g, yield 71%).

### Synthesis of compound A175-2

A175-1 (4.19 g, 10.4 mmol) was dissolved in tetrahydrofuran (20 mL), and HCl/dioxane (50 mL, 4 M) was added. The mixture was reacted at room temperature for 5 h, and then TLC showed that the reaction was completed. The solvent was dried by rotary evaporation to obtain compound A175-2 (3.95 g, crude).

### Synthesis of compound A175-3

4-methylphthalic anhydride (1.6 g, 10 mmol) and 3-*N-*tertbutoxycarbonylamino-2,6-dioxopiperidine (2.2 g, 10 mmol) were dissolved in trifluoroethanol (18 ml). The mixture was reacted under microwave at 120°C for 2 h, cooled to room temperature and then further cooled to -20°C, with a solid precipitated. The precipitated solid was filtered, and the filter cake was washed with ethyl acetate (10 mL) to obtain compound A175-3 (2.1 g, yield 77%). ¹H NMR (400 MHz, DMSO) δ 11.13 (s, 1H), 7.81 (d, *J=* 7.6 Hz, 1H), 7.76 (s, 1H), 7.69 (d, *J* = 7.7 Hz, 1H), 5.14 (dd, *J* = 12.9, 5.3 Hz, 1H), 2.89 (ddd, *J=* 17.7, 14.2, 5.3 Hz, 1H), 2.65 - 2.50 (m, 5H), 2.10 - 1.98 (m, 1H).

### Synthesis of compound A175-4

A175-3 (2.1 g, 7.70 mmol) was dissolved in 150 mL of acetonitrile, and NBS (1.50 g, 8.5 mmol) and AIBN (252 mg, 1.5 mmol) were added. The mixture was reacted overnight at 60°C, and then TLC showed that the reaction was completed. The reaction liquid was dried by rotary evaporation. The solute was purified by column chromatography (mobile phase: dichloromethane : methanol = 10 : 1) to obtain compound A175-4 (1.8 g, yield 67%). ¹H NMR (400 MHz, DMSO) δ 11.14 (s, 1H), 8.02 (s, 1H), 7.94 (q, *J* = 7.8 Hz, 2H), 5.16 (dd, *J=* 12.8, 5.3 Hz, 1H), 4.90 (s, 2H), 2.96 - 2.84 (m, 1H), 2.56 (s, 2H), 2.07 (dd, *J* = 10.8, 5.2 Hz, 1H)

### Synthesis of compound A175

A175-2 (40 mg, 0.13 mmol), A175-4 (45 mg, 0.13 mmol) and triethylamine (53 mg, 0.42 mmol) were dissolved in 5 mL of DMF. The mixture was reacted overnight at room temperature, and then TLC showed that the reaction was completed. The mixture was diluted with 20 mL of ethyl acetate. The organic phase was washed with saturated brine (10 mL*3) and then dried by rotary evaporation. The solute was purified by column chromatography (mobile phase: dichloromethane : methanol =20 : 1) to obtain compound A175 (17 mg, yield 29%). ESI-MS(M+H)⁺: 575.2.

¹H NMR (400 MHz, DMSO) δ 11.12 (s, 1H), 8.62 (t, *J=* 6.0 Hz, 1H), 8.14 - 8.07 (m, 1H), 7.94 - 7.78 (m, 5H), 7.56 - 7.48 (m, 2H), 7.47 - 7.42 (m, 2H), 7.26 (d, *J* = 8.0 Hz, 2H), 7.18 (d, *J* = 8.0 Hz, 2H), 5.15 (dd, *J* = 13.0, 5.3 Hz, 1H), 4.27 (d, *J* = 5.8 Hz, 2H), 3.96 (s, 2H), 3.84 (s, 2H), 3.65 (s, 2H), 2.95 - 2.83 (m, 1H), 2.70 - 2.52 (m, 2H), 2.12 - 1.99 (m, 1H).

The synthesis methods for compounds A176 and A186-A190 are the same as that for A175.

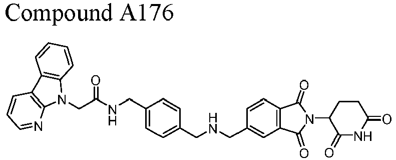

ESI-MS(M+H)⁺: 615.2.

¹H NMR (400 MHz, DMSO) δ 11.12 (s, 1H), 8.73 (t, *J* = 5.9 Hz, 1H), 8.55 (dd, *J=* 7.6, 1.5 Hz, 1H), 8.45 (dd, *J=* 4.8, 1.5 Hz, 1H), 8.21 (d, *J=* 7.7 Hz, 1H), 7.92 (s, 1H), 7.85 (dd, *J* = 18.2, 7.8 Hz, 2H), 7.57 (d, *J=* 8.2 Hz, 1H), 7.54 - 7.47 (m, 1H), 7.33 - 7.20 (m, 6H), 5.21 - 5.07 (m, 3H), 4.29 (d, *J=* 5.8 Hz, 2H), 3.85 (s, 2H), 3.67 (s, 2H), 2.95 - 2.83 (m, 1H), 2.67 - 2.50 (m, 2H), 2.11 - 2.05 (m, 1H).

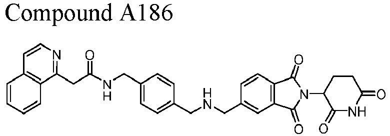

ESI-MS(M+H)⁺: 576.2.

¹H NMR (400 MHz, DMSO) δ 11.12 (s, 1H), 8.69 (t, *J* = 5.7 Hz, 1H), 8.41 (d, *J=* 5.7 Hz, 1H), 8.24 (d, *J=* 8.1 Hz, 1H), 8.00 - 7.90 (m, 2H), 7.88 - 7.77 (m, 2H), 7.72 - 7.66 (m, 2H), 7.65 (t, *J=* 7.7 Hz, 1H), 7.28 (d, *J=* 8.1 Hz, 2H), 7.22 (d, *J=* 8.1 Hz, 2H), 5.14 (dd, *J=* 13.0, 5.3 Hz, 1H), 4.28 (d, *J=* 5.9 Hz, 2H), 4.22 (s, 2H), 3.85 (s, 2H), 3.66 (s, 2H), 2.95 - 2.83 (m, 1H), 2.70 - 2.55 (m, 2H), 2.10 - 2.02 (m, 1H).

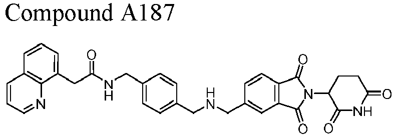

ESI-MS(M+H)⁺: 576.2.

¹H NMR (400 MHz, DMSO) δ 11.12 (s, 1H), 8.93 (dd, *J=* 4.2, 1.8 Hz, 1H), 8.47 (t, *J* = 5.9 Hz, 1H), 8.37 (dd, *J* = 8.3, 1.7 Hz, 1H), 7.95 - 7.79 (m, 4H), 7.68 (d, *J* = 6.0 Hz, 1H), 7.60 - 7.49 (m, 2H), 7.27 (d, *J* = 8.1 Hz, 2H), 7.22 (d, *J* = 8.1 Hz, 2H), 5.15 (dd, *J* = 12.9, 5.4 Hz, 1H), 4.27 (d, *J* = 6.0 Hz, 2H), 4.12 (s, 2H), 3.84 (s, 2H), 3.65 (s, 2H), 2.96 - 2.82 (m, 1H), 2.69 - 2.52 (m, 2H), 2.12 - 2.01 (m, 1H).

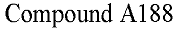

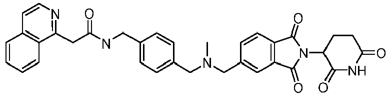

ESI-MS(M+H)⁺: 590.2.

¹H NMR (400 MHz, DMSO) δ 11.12 (s, 1H), 8.69 (t, *J=* 5.9 Hz, 1H), 8.41 (d, *J* = 5.7 Hz, 1H), 8.24 (d, *J* = 8.4 Hz, 1H), 7.96 (d, *J* = 8.2 Hz, 1H), 7.92 - 7.81 (m, 3H), 7.77 - 7.72 (m, 2H), 7.64 (t, *J=* 7.2 Hz, 1H), 7.31 (d, *J* = 8.0 Hz, 2H), 7.25 (d, *J=* 8.1 Hz, 2H), 5.15 (dd, *J* = 12.9, 5.3 Hz, 1H), 4.29 (d, *J* = 5.9 Hz, 2H), 4.22 (s, 2H), 3.67 (s, 2H), 3.52 (d, *J=* 13.7 Hz, 2H), 2.96 - 2.83 (m, 1H), 2.68 - 2.52 (m, 2H), 2.15 - 2.01 (m, 4H).

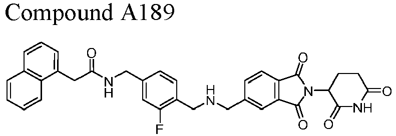

ESI-MS(M+H)⁺: 593.2.

¹H NMR (400 MHz, DMSO) δ 11.14 (s, 1H), 8.67 (t, *J* = 5.9 Hz, 1H), 8.14 - 8.07 (m, 1H), 7.96 - 7.89 (m, 2H), 7.89 - 7.78 (m, 3H), 7.55 - 7.48 (m, 2H), 7.46 (d, *J* = 5.4 Hz, 2H), 7.39 (t, *J* = 7.8 Hz, 1H), 7.04 (d, *J* = 7.8 Hz, 1H), 6.98 (d, *J* = 11.1 Hz, 1H), 5.15 (dd, *J* = 12.9, 5.4 Hz, 1H), 4.28 (d, *J* = 5.9 Hz, 2H), 3.98 (s, 2H), 3.86 (s, 2H), 3.67 (s, 2H), 2.94 - 2.83 (m, 1H), 2.67 - 2.52 (m, 2H), 2.12 - 2.02 (m, 1H).

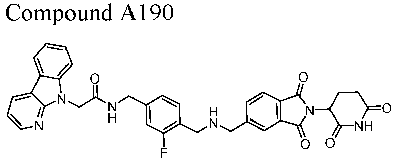

ESI-MS(M+H)⁺: 633.2.

¹H NMR (400 MHz, DMSO) δ 11.14 (s, 1H), 8.79 (t, *J* = 5.9 Hz, 1H), 8.56 (dd, *J* = 7.6, 1.3 Hz, 1H), 8.46 (dd, *J* = 4.8, 1.3 Hz, 1H), 8.22 (d, *J* = 7.6 Hz, 1H), 7.93 (s, 1H), 7.89 - 7.82 (m, 2H), 7.61 (d, *J* = 8.2 Hz, 1H), 7.51 (t, *J* = 7.6 Hz, 1H), 7.42 (t, *J* = 7.9 Hz, 1H), 7.33 - 7.24 (m, 2H), 7.11 - 7.03 (m, 2H), 5.22 - 5.10 (m, 3H), 4.30 (d, *J* = 5.9 Hz, 2H), 3.87 (s, 2H), 3.69 (s, 2H), 2.93 - 2.81 (m, 1H), 2.69 - 2.52 (m, 2H), 2.10 - 2.02 (m, 1H).

### Example 11:

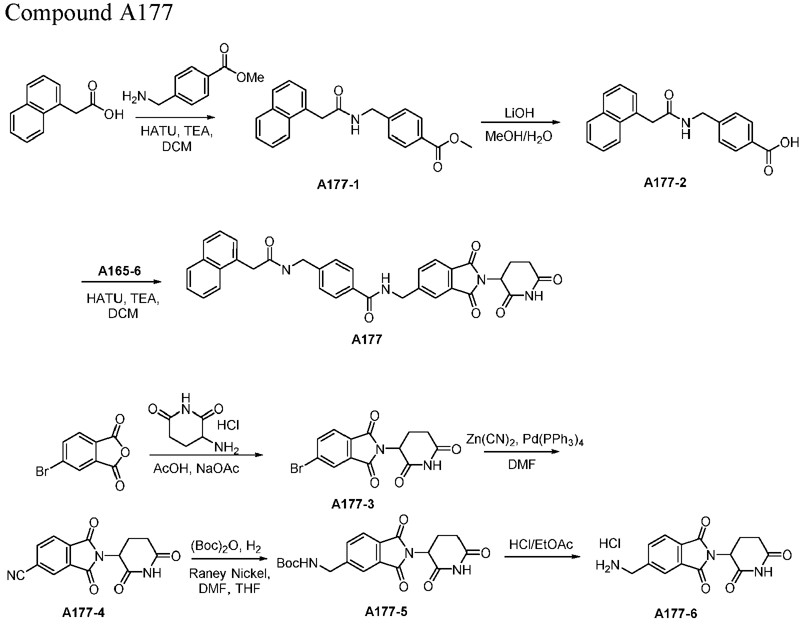

### Synthesis of compound A177-1

Naphthaleneacetic acid (100 mg, 0.54 mmol) was dissolved in dichloromethane (10 mL), and then methyl 4-aminomethylbenzoate (106 mg, 0.64 mmol), triethylamine (220 mg, 2.1 mmol) and HATU (350 mg, 0.80 mmol) were successively added. The reaction was stirred at room temperature for 2 h, and then TLC showed that the raw materials were completely reacted. Water (10 mL) was added, and the mixture was extracted with dichloromethane (10 ml*3). The organic phases were combined. The solvent was dried by rotary evaporation. The solute was purified by a preparative plate (mobile phase: dichloromethane : methanol = 30 : 1) to obtain compound A177-1 (150 mg, yield 83%).

### Synthesis of compound A177-2

Compound A177-1 (150 mg, 0.45 mmol) was dissolved in 12 mL of methanol/H₂O (3 : 1), and lithium hydroxide hydrate (80 mg, 1.8 mmol) was added. The mixture was reacted at 60°C for 2 h, and then TLC showed that the reaction was completed. The mixture was adjusted to pH 3-4 with dilute hydrochloric acid, and then the precipitated solid was subjected to suction filtration and dried to obtain compound A177-2 (130 mg, yield 90%).

### Synthesis of compound A177-3

4-bromophthalic anhydride (5 g, 22 mmol) was dissolved in 75 mL of glacial acetic acid, and 3-amino-2,6-piperidinedione (2.8 g, 21.8 mmol) and sodium acetate (3 g, 21.8 mmol) were added. The mixture was reacted at 120°C for 2 h, and then TLC showed that the reaction was completed. The reaction was quenched with water (20 mL) and then cooled to room temperature. The precipitated solid was subjected to suction filtration to obtain compound A177-3 (6.9 g, yield 93%).

### Synthesis of compound A177-4

A177-3 (4 g, 11.9 mmol) was dissolved in 130 mL of DMF, and zinc cyanide (2.52 g, 21.3 mmol) and tetrakis(triphenylphosphine)palladium (4.1 g, 3.56 mmol) were successively added. The mixture was reacted overnight at 120°C under nitrogen protection, and then TLC showed that the reaction was completed. The reaction liquid was filtered, and the filtrate was diluted with 400 mL of water. The precipitated solid was subjected to suction filtration. The filter cake was slurried with 100 mL of acetone and then subjected to suction filtration. The filter cake was rinsed with acetone to obtain compound A177-4 (4 g, crude).

### Synthesis of compound A177-5

A177-4 (4 g, 11.9 mmol) was dissolved in 70 mL of THF/DMF (1 : 1), and (Boc)₂O (3.08 g, 13.9 mmol) and Raney Ni (10.0 g) were added. The mixture was reacted overnight at room temperature under hydrogen atmosphere, and then TLC showed that the reaction was completed. The reaction liquid was filtered, and the filtrate was dried by rotary evaporation. The solute was separated by column chromatography (mobile phase: dichloromethane : methanol = 20 : 1) to obtain compound A177-5 (700 mg, yield 15%).

### Synthesis of compound A177-6

A177-5 (700 mg, 1.81 mmol) was dissolved in HCl/EtOAc (7 mL, 4 M). The mixture was reacted at room temperature for 30 min, and then TLC showed that the reaction was completed. The solvent was dried by rotary evaporation to obtain A177-6 (700 mg, crude).

### Synthesis of compound A177

A177-2 (30 mg, 0.09 mmol) was dissolved in dichloromethane (10 mL), and then A177-6 (37 mg, 0.11 mmol), triethylamine (38 mg, 0.38 mmol) and HATU (55 mg, 0.14 mmol) were successively added. The reaction was stirred at room temperature for 1 h, and then TLC showed that the raw materials were completely reacted. Water (10 mL) was added, and the mixture was extracted with dichloromethane (20 ml*3). The organic phases were combined. The solvent was dried by rotary evaporation. The solute was purified by a preparative plate (mobile phase: dichloromethane : methanol = 10 : 1) and then purified by medium-pressure preparative chromatography to obtain compound A177 (7 mg, yield 13%). ESI-MS(M+H)⁺: 589.2.

¹H NMR (400 MHz, DMSO) δ 11.12 (s, 1H), 9.18 (t, *J* = 5.9 Hz, 1H), 8.70 (t, *J* = 5.9 Hz, 1H), 8.15 - 8.07 (m, 1H), 7.97 - 7.86 (m, 2H), 7.86 - 7.75 (m, 5H), 7.58 - 7.49 (m, 2H), 7.49 - 7.40 (m, 2H), 7.33 (d, *J* = 8.3 Hz, 2H), 5.14 (dd, *J* = 12.9, 5.4 Hz, 1H), 4.64 (d, *J* = 5.8 Hz, 2H), 4.35 (d, *J* = 5.9 Hz, 2H), 3.99 (s, 2H), 2.95 - 2.84 (m, 1H), 2.67 - 2.52 (m, 2H), 2.11 - 1.99 (m, 1H).

The synthesis methods for compounds A178, A211, A218 and A219 are the same as that for A177.

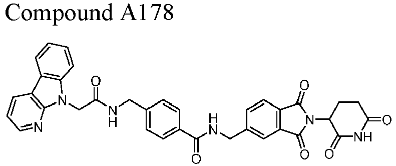

ESI-MS(M+H)⁺: 629.2.

¹H NMR (400 MHz, DMSO) δ 11.12 (s, 1H), 9.19 (t, *J=* 5.9 Hz, 1H), 8.81 (t, *J* = 6.0 Hz, 1H), 8.55 (dd, *J=* 7.6, 1.5 Hz, 1H), 8.47 (dd, *J=* 4.8, 1.5 Hz, 1H), 8.22 (d, *J=* 7.7 Hz, 1H), 7.91 - 7.80 (m, 5H), 7.60 (d, *J=* 8.2 Hz, 1H), 7.55 - 7.46 (m, 1H), 7.39 (d, *J=* 8.2 Hz, 2H), 7.32 - 7.21 (m, 2H), 5.20 (s, 2H), 5.15 (dd, *J* = 12.9, 5.4 Hz, 1H), 4.64 (d, *J* = 5.8 Hz, 2H), 4.37 (d, *J* = 5.8 Hz, 2H), 2.94 - 2.83 (m, 1H), 2.68 - 2.52 (m, 2H), 2.09 - 2.03 (m, 1H).

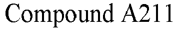

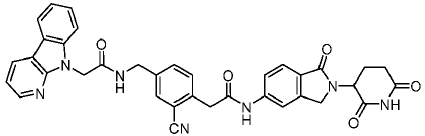

ESI-MS(M+H)⁺: 640.2
¹H NMR (400 MHz, DMSO) δ 10.98 (s, 1H), 10.66 (s, 1H), 8.83 (t, *J* = 6.0 Hz, 1H), 8.56 (d, *J=* 7.6 Hz, 1H), 8.49 (d, *J* = 4.0 Hz, 1H), 8.22 (d, *J=* 7.6 Hz, 1H), 7.96 (s, 1H), 7.72 (s, 1H), 7.68 (d, *J* = 8.3 Hz, 1H), 7.65 - 7.55 (m, 3H), 7.54 - 7.48 (m, 2H), 7.32 - 7.24 (m, 2H), 5.20 (s, 2H), 5.08 (dd, *J=* 13.3, 5.1 Hz, 1H), 4.45 - 4.26 (m, 4H), 3.98 (s, 2H), 2.98 - 2.84 (m, 1H), 2.70 - 2.57 (m, 1H), 2.42 - 2.33 (m, 1H), 2.03 - 1.98 (m, 1H).

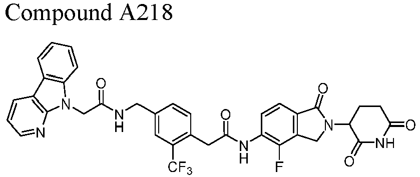

ESI-MS(M+H)⁺: 701.2
¹H NMR (400 MHz, DMSO) δ 11.02 (s, 1H), 10.31 (s, 1H), 8.85 (t, *J=* 6.1 Hz, 1H), 8.56 (dd, *J=* 7.6, 1.4 Hz, 1H), 8.45 (dd, *J=* 4.8, 1.3 Hz, 1H), 8.22 (d, *J=* 7.7 Hz, 1H), 8.04 (t, *J* = 7.5 Hz, 1H), 7.64 - 7.45 (m, 6H), 7.32 - 7.24 (m, 2H), 5.19 (s, 2H), 5.11 (dd, *J =* 13.3, 5.1 Hz, 1H), 4.62 - 4.31 (m, 4H), 4.03 (s, 2H), 2.97 - 2.83 (m, 1H), 2.67 - 2.55 (m, 1H), 2.47 - 2.35 (m, 1H), 2.05 - 1.95 (m, 1H).
The intermediates were synthesized as follows:

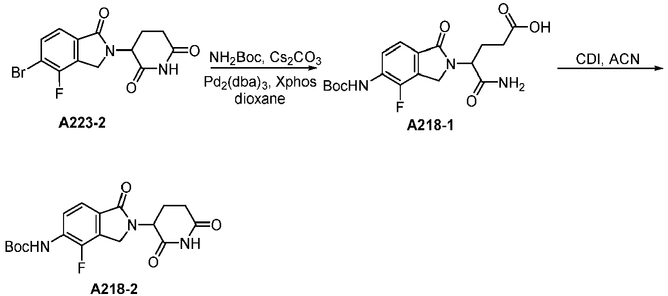

### Synthesis of compound A218-1

A223-2 (1.63 g, 7.68 mmol), BocNH₂ (2.97 g, 15.3 mmol), cesium carbonate (1.24 g, 3.84 mmol) and Pd₂(dba)₃ (1.24 g, 3.84 mmol) were dissolved in 40 mL of dioxane, and Xphos (1.24 g, 2.60 mmol) was added. The mixture was reacted at 100°C under nitrogen atmosphere for 4 h, and then TLC showed that the reaction was completed. The mixture was cooled to room temperature and diluted with 50 mL of dichloromethane. The mixture was filtered, and then the organic phase was washed with saturated brine (20 mL*2). The aqueous phase was adjusted to pH 3-4 with 1 N hydrochloric acid and extracted with dichloromethane/methanol (10 : 1, 60 ml*3). The organic phases were combined, dried over anhydrous sodium sulfate and dried by rotary evaporation to obtain A218-1 (1.4 g, yield 46%).

### Synthesis of compound A218-2

A218-1 (1.4 g, 3.54 mmol) was dissolved in 20 mL of acetonitrile, and CDI (2.64 g, 16.3 mmol) was added. The mixture was reacted at 80°C for 1 h, and then TLC showed that the reaction was completed. The solvent was dried by rotary evaporation, and then the solute was separated and purified by column chromatography (mobile phase: petroleum ether : ethyl acetate = 1 : 2) to obtain compound A218-2 (311 mg, yield 28%).

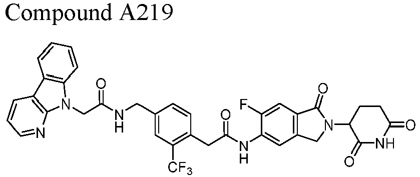

ESI-MS(M+H)⁺: 701.2
¹H NMR (400 MHz, DMSO) δ 11.00 (s, 1H), 10.29 (s, 1H), 8.85 (t, *J* = 6.0 Hz, 1H), 8.55 (d, *J=* 6.4 Hz, 1H), 8.45 (d, *J=* 4.8 Hz, 1H), 8.28 - 8.14 (m, 2H), 7.67 - 7.44 (m, 6H), 7.35 - 7.23 (m, 2H), 5.19 (s, 2H), 5.09 (dd, *J=* 13.2, 4.9 Hz, 1H), 4.44 - 4.23 (m, 4H), 4.05 (s, 2H), 2.96 - 2.82 (m, 1H), 2.69 - 2.58 (m, 1H), 2.41 - 2.29 (m, 1H), 2.03 - 1.99 (m, 1H).
The intermediates were synthesized as follows:

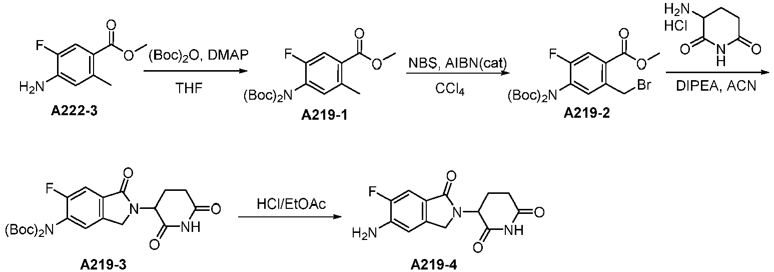

### Synthesis of compound A219-1

A222-3 (3.78 g, 20.63 mmol), (Boc)₂O (22.48 g, 103.2 mmol) and DMAP (504 mg, 4.13 mmol) were dissolved in 380 mL of DMF. The mixture was reacted overnight at room temperature, and then TLC showed that the reaction was completed. The reaction was quenched with 60 mL of water. The aqueous phase was extracted with ethyl acetate (80 mL*3). The organic phases were combined, washed with saturated brine (20 mL*3), dried over anhydrous sodium sulfate and dried by rotary evaporation. The solute was separated and purified by column chromatography (mobile phase: petroleum ether : ethyl acetate = 7 : 1) to obtain compound A219-1 (2.46 g, yield 31%).

### Synthesis of compound A219-2

Compound A219-1 (1 g, 2.61 mol), NBS (510 mg, 2.87 mol) and AIBN (128 mg, 0.78 mol) were dissolved in 10 mL of carbon tetrachloride. The mixture was reacted at 80°C for 1 h, and then TLC showed that the reaction was completed. The mixture was cooled to room temperature. The solvent was dried by rotary evaporation. The solute was separated and purified by column chromatography (mobile phase: petroleum ether : ethyl acetate = 10 : 1) to obtain compound A219-2 (960 mg, yield 75%).

### Synthesis of compound A219-3

Compound A219-2 (900 mg, 1.95 mmol), 3-amino-2,6-piperidinedione hydrochloride (385 mg, 2.34 mmol) and DIPEA (882 mg, 6.82 mmol) were dissolved in 30 mL of acetonitrile. The mixture was reacted overnight at 80°C, and then TLC showed that the reaction was completed. The mixture was cooled to room temperature. The solvent was dried by rotary evaporation. The mixture was slurried with methyl tert-butyl ether/water (20 mL/20 mL) and then filtered. The filter cake was separated and purified by column chromatography (mobile phase: dichloromethane : methanol = 30 : 1) to obtain compound A219-3 (1.20 g, yield 65%).

### Synthesis of compound A219-4

Compound A219-3 (1.0 g, 2.10 mmol) was dissolved in 4 M HCl/EtOAc (15 mL). The mixture was reacted at room temperature for 1 h, and then TLC showed that the reaction was completed. The solvent was dried by rotary evaporation to obtain compound A219-4 (980 mg, crude).

### Example 12:

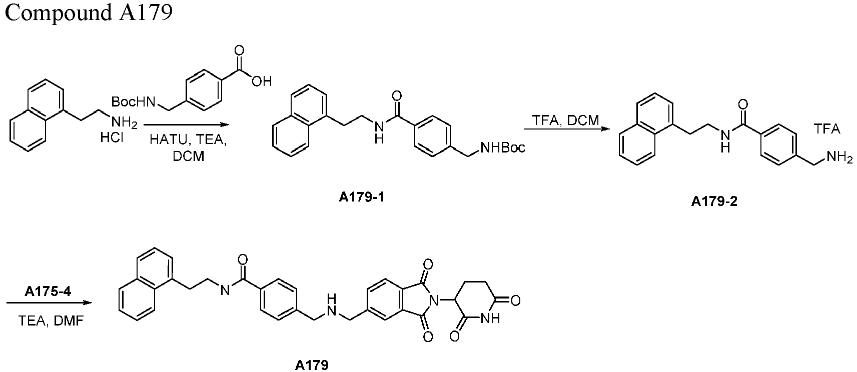

### Synthesis of compound A179-1

Naphthylethylamine hydrochloride (207 mg, 1.0 mmol) was dissolved in dichloromethane (15 mL), and then 4-[(tertbutoxycarbonylamino)methyl]benzoic acid (250 mg, 1.0 mmol), triethylamine (305 uL, 2.2 mmol) and HATU (418 mg, 1.1 mmol) were successively added. The reaction was stirred at room temperature for 1 h, and then TLC showed that the raw materials were completely reacted. Water (30 mL) was added, and the mixture was extracted with dichloromethane (20 ml*3). The organic phases were combined. The solvent was dried by rotary evaporation. The solute was purified by a preparative plate (mobile phase: dichloromethane : methanol = 30 : 1) to obtain compound A179-1 (144 mg, yield 36%).

### Synthesis of compound A179-2

A179-1 (144 mg, 0.356 mmol) was dissolved in 10 mL of dichloromethane, and 3 mL of trifluoroacetic acid was added. The mixture was reacted at room temperature for 1 h, and then TLC showed that the reaction was completed. The solvent was dried by rotary evaporation to obtain A179-2 (240 mg, crude).

### Synthesis of compound A179

A179-2 (240 mg, 0.36 mmol), A175-4 (100 mg, 0.28 mmol) and triethylamine (120 mg, 1.1 mmol) were dissolved in 5 mL of DMF. The mixture was reacted overnight at room temperature, and then TLC showed that the reaction was completed. The mixture was diluted with 20 mL of ethyl acetate. The organic phase was washed with saturated brine (10 mL*3) and then dried by rotary evaporation. The solute was purified by a preparative plate (mobile phase: dichloromethane : methanol =10 : 1) and then purified by medium-pressure preparative chromatography to obtain compound A179 (12 mg, yield 7%). ESI-MS(M+H)⁺: 575.2.

¹H NMR (400 MHz, DMSO) δ 11.12 (s, 1H), 8.65 (t, *J=* 5.6 Hz, 1H), 8.29 (d, *J=* 8.3 Hz, 1H), 7.93 (t, *J=* 3.5 Hz, 2H), 7.90 - 7.75 (m, 5H), 7.61 - 7.49 (m, 2H), 7.49 - 7.36 (m, 4H), 5.15 (dd, *J=* 12.9, 5.4 Hz, 1H), 3.87 (s, 2H), 3.74 (s, 2H), 3.57 (dd, *J=* 14.7, 6.1 Hz, 2H), 3.41 - 3.35 (m, 1H), 3.29 - 3.26 (m, 1H), 2.96 - 2.83 (m, 1H), 2.67 - 2.52 (m, 2H), 2.11 - 2.01 (m, 1H).

The synthesis method for compound A180 is the same as that for A179.

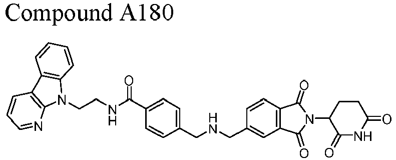

ESI-MS(M+H)⁺: 615.2.

¹H NMR (400 MHz, DMSO) δ 11.12 (s, 1H), 8.61 (t, *J* = 5.7 Hz, 1H), 8.54 (dd, *J* = 7.6, 1.5 Hz, 1H), 8.46 (dd, *J* = 4.8, 1.5 Hz, 1H), 8.20 (d, *J* = 7.7 Hz, 1H), 7.94 - 7.81 (m, 3H), 7.70 - 7.63 (m, 3H), 7.48 (t, *J=* 7.3 Hz, 1H), 7.37 (d, *J=* 8.2 Hz, 2H), 7.29 - 7.20 (m, 2H), 5.15 (dd, *J=* 12.9, 5.4 Hz, 1H), 4.65 (t, *J=* 6.3 Hz, 2H), 3.85 (s, 2H), 3.76 - 3.61 (m, 4H), 2.97 - 2.82 (m, 1H), 2.66 - 2.53 (m, 2H), 2.11 - 2.01 (m, 1H).

### Example 13:

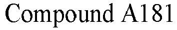

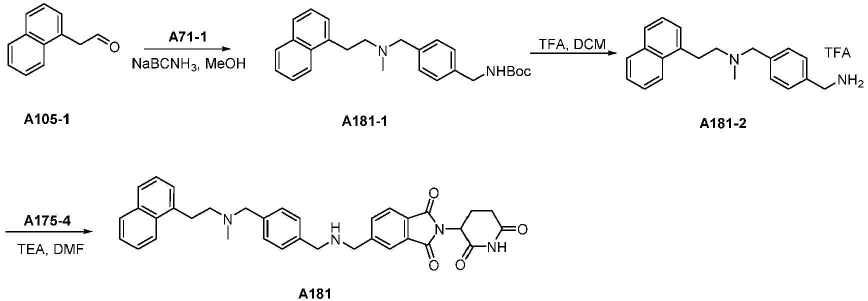

### Synthesis of compound A181-1

Compound A105-1 (68 mg, 0.40 mmol) was dissolved in 5 mL of methanol, and compound A71-1 (100 mg, 0.40 mmol) and sodium cyanoborohydride (60 mg, 0.8 mmol) were added. The mixture was reacted overnight at room temperature, and then TLC showed that the reaction was completed. The reaction was quenched with water (30 mL). The aqueous phase was extracted with dichloromethane (20 mL*3). The organic phases were combined and dried by rotary evaporation. The solute was purified by a preparative plate (mobile phase: dichloromethane : methanol = 10 : 1) to obtain compound A181-1 (90 mg, yield 47%).

### Synthesis of compound A181-2

Compound A181-1 (90 mg, 0.22 mmol) was dissolved in 4 mL of dichloromethane, and 1 mL of trifluoroacetic acid was added. The mixture was reacted at room temperature for 1 h, and then TLC showed that the reaction was completed. The solution was dried by rotary evaporation to obtain A181-2 (100 mg, crude).

### Synthesis of compound A181

A181-2 (100 mg, 0.22 mmol), A175-4 (77 mg, 0.22 mmol) and triethylamine (133 mg, 1.32 mmol) were dissolved in 5 mL of DMF. The mixture was reacted overnight at room temperature, and then TLC showed that the reaction was completed. The mixture was diluted with 20 mL of ethyl acetate. The organic phase was washed with saturated brine (10 mL*3) and then dried by rotary evaporation. The solute was purified by a preparative plate (mobile phase: dichloromethane : methanol = 10 : 1) and then purified by medium-pressure preparative chromatography to obtain compound A181 (80 mg, yield 63%). ESI-MS(M+H)+: 575.2.

¹H NMR (400 MHz, DMSO) δ 11.13 (s, 1H), 8.05 - 7.96 (m, 2H), 7.93 - 7.86 (m, 3H), 7.78 (d, J = 7.6 Hz, 1H), 7.55 - 7.30 (m, 8H), 5.16 (dd, J = 12.9, 5.4 Hz, 1H), 4.02 (s, 2H), 3.88 - 3.72 (m, 4H), 3.59 - 3.42 (m, 2H), 2.97 - 2.73 (m, 3H), 2.66 - 2.52 (m, 2H), 2.42 (s, 3H), 2.11 - 2.02 (m, 1H).

The synthesis methods for compounds A182-A185 are the same as that for A181.

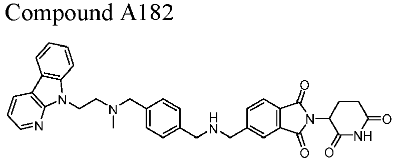

ESI-MS(M+H)⁺: 615.2.

¹H NMR (400 MHz, DMSO) δ 11.12 (s, 1H), 8.52 (dd, *J* = 7.6, 1.4 Hz, 1H), 8.42 (dd, *J* = 4.8, 1.4 Hz, 1H), 8.20 (d, *J=* 7.7 Hz, 1H), 7.93 - 7.84 (m, 2H), 7.81 (d, *J* = 7.7 Hz, 1H), 7.58 (d, *J* = 8.2 Hz, 1H), 7.47 (t, *J* = 7.7 Hz, 1H), 7.29 - 7.15 (m, 2H), 7.06 (d, *J=* 7.9 Hz, 2H), 6.90 (d, *J* = 7.9 Hz, 2H), 5.15 (dd, *J* = 12.9, 5.4 Hz, 1H), 4.60 (t, *J* = 6.5 Hz, 2H), 3.81 (s, 2H), 3.59 (s, 2H), 3.47 (s, 2H), 2.96 - 2.84 (m, 1H), 2.77 (t, *J* = 6.5 Hz, 2H), 2.66 - 2.52 (m, 2H), 2.24 (s, 3H), 2.09 - 2.01 (m, 1H).

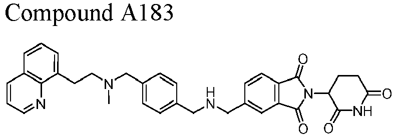

ESI-MS(M+H)⁺: 576.2.

¹H NMR (400 MHz, DMSO) δ 11.13 (s, 1H), 8.89 (dd, *J* = 4.1, 1.6 Hz, 1H), 8.36 - 8.29 (m, 1H), 7.92 (s, 1H), 7.84 (dd, *J=* 19.4, 7.9 Hz, 3H), 7.63 (d, *J=* 6.8 Hz, 1H), 7.56 - 7.46 (m, 2H), 7.21 (d, *J* = 7.8 Hz, 2H), 7.15 (d, *J* = 7.9 Hz, 2H), 5.15 (dd, *J* = 12.9, 5.3 Hz, 1H), 3.85 (s, 2H), 3.64 (s, 2H), 3.54 (s, 3H), 3.44 - 3.39 (m, 2H), 2.95 - 2.83 (m, 1H), 2.78 - 2.56 (m, 4H), 2.23 (s, 3H), 2.11 - 2.02 (m, 1H).

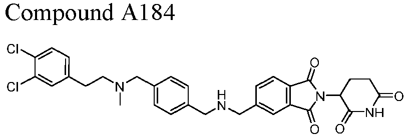

ESI-MS(M+H)⁺: 593.2.

¹H NMR (400 MHz, DMSO) δ 11.12 (s, 1H), 7.91 (s, 1H), 7.87 - 7.81 (m, 2H), 7.51 - 7.48 (m, 2H), 7.25 (d, *J=* 8.0 Hz, 2H), 7.20 (dd, *J* = 8.2, 2.0 Hz, 1H), 7.14 (d, *J* = 8.0 Hz, 2H), 5.14 (dd, *J=* 12.9, 5.4 Hz, 1H), 3.87 (s, 2H), 3.67 (s, 2H), 3.48 (s, 2H), 2.96 - 2.83 (m, 1H), 2.77 (t, *J=* 7.2 Hz, 2H), 2.65 - 2.52 (m, 4H), 2.15 (s, 3H), 2.10 - 2.04 (m, 1H).

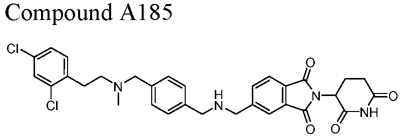

ESI-MS(M+H)⁺: 593.2.

¹H NMR (400 MHz, DMSO) δ 11.12 (s, 1H), 7.91 (s, 1H), 7.87 - 7.81 (m, 2H), 7.52 (d, *J* = 1.9 Hz, 1H), 7.41 - 7.31 (m, 2H), 7.24 (d, *J=* 8.0 Hz, 2H), 7.15 (d, *J=* 8.0 Hz, 2H), 5.14 (dd, *J* = 12.9, 5.4 Hz, 1H), 3.85 (s, 2H), 3.66 (s, 2H), 3.49 (s, 2H), 2.94 - 2.81(m, 3H), 2.65 - 2.51 (m, 4H), 2.19 (s, 3H), 2.11 - 2.01 (m, 1H).

### Example 14:

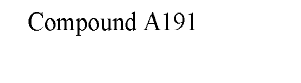

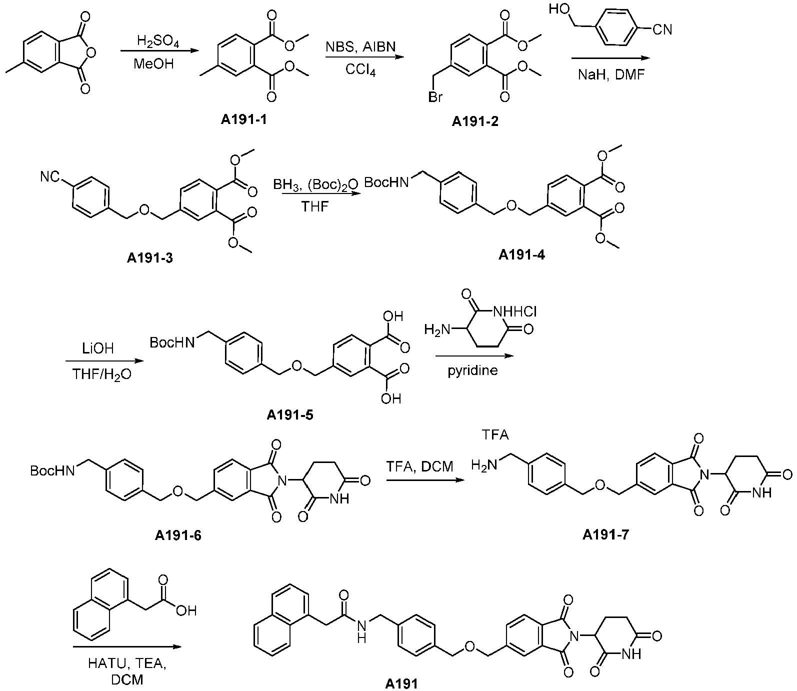

### Synthesis of compound A191-1

The compound 4-methylphthalic anhydride (20.0 g, 123.4 mmol) was dissolved in 200 mL of methanol, and concentrated sulfuric acid (12.0 g, 123.4 mmol) was added dropwise. The mixture was reacted overnight at 80°C, and then TLC showed that the reaction was completed. The reaction was quenched with ice water (200 mL). The aqueous phase was extracted with ethyl acetate (50 mL*3). The organic phases were combined and dried by rotary evaporation. The solute was separated and purified by column chromatography (mobile phase: PE : EA = 10 : 1) to obtain compound A191-1 (25.5 g, yield 99%).

### Synthesis of compound A191-2

A191-1 (25.5 g, 122.5 mmol) was dissolved in 550 mL of carbon tetrachloride, and NBS (21.4 g, 122.5 mmol) and AIBN (5.91 g, 36.7 mmol) were added. The mixture was reacted at 80°C for 1 h, and then TLC showed that the reaction was completed. The mixture was diluted with 150 mL of dichloromethane. The organic phase was washed with saturated aqueous sodium bicarbonate solution (30 mL*3) and dried by rotary evaporation. The solute was separated by column chromatography (mobile phase: petroleum ether : ethyl acetate = 9 : 1) to obtain compound A191-2 (29 g, yield 82%).

### Synthesis of compound A191-3

4-hydroxymethyl benzonitrile (13.3 g, 100.4 mmol) was dissolved in 200 mL of DMF. Under ice bath, sodium hydride (4.0 g, 100.4 mmol) was added in batches. The mixture was reacted under ice bath for 0.5 h, and then a solution of A191-2 (13.3 g, 50.2 mmol) in DMF (20 mL) was added. The mixture was reacted at 0°C for another 1 h, and then TLC showed that the reaction was completed. Under ice bath, the reaction was quenched by slowly adding ice water (200 mL) and extracted with ethyl acetate (60 mL*3). The organic phases were combined, washed with saturated brine (20 mL*3), dried over anhydrous sodium sulfate and concentrated. The solute was purified by column chromatography (developing solvent: PE : EA= 10 : 1) to obtain A191-3 (6.3 g, yield 37%).

### Synthesis of compound A191-4

A191-3 (6.3 g, 18.6 mmol) was dissolved in 60 mL of anhydrous tetrahydrofuran. Under ice bath, borane/THF solution (96 mL, 1 M, 96 mmol) was added dropwise. The mixture was reacted at 0°C for 1 h, and then TLC showed that the reaction was completed. The reaction was quenched with ice water (20 mL). The solution was adjusted to pH = 2-3 with 6 M dilute hydrochloric acid. (Boc)₂O (10.1 g, 46.5 mmol) was added, and the mixture was reacted at room temperature for 1 h. The aqueous phase was extracted with ethyl acetate (20 mL*3). The organic phases were combined and dried by rotary evaporation. The solute was separated by column chromatography (mobile phase: MeOH : DCM = 1 : 30) to obtain compound A191-4 (1.71 g, yield 20%).

### Synthesis of compound A191-5

Compound A191-4 (1.71 g, 3.9 mmol) was dissolved in 18 mL of tetrahydrofuran/H₂O (3 1 : 1), and lithium hydroxide hydrate (970 mg, 23.4 mmol) was added. The mixture was reacted at 50°C for 2 h, and then TLC showed that the reaction was completed. The mixture was adjusted to pH 3-4 with dilute hydrochloric acid, and then the aqueous phase was extracted with ethyl acetate (40 mL*3). The organic phases were combined and dried by rotary evaporation. The solute was separated by column chromatography (mobile phase: MeOH : DCM = 1 : 10) to obtain compound A191-5 (1.2 g, yield 75%).

### Synthesis of compound A191-6

Compound A191-5 (1.1 g, 2.6 mmol) was dissolved in 10 mL of pyridine, and 3-aminopiperidine-2,6-dione hydrochloride (430 mg, 2.6 mmol) was added. The mixture was reacted overnight at 110°C, and then TLC showed that the reaction was completed. The mixture was diluted with ethyl acetate (50 mL). The organic phase was washed successively with 0.5 M dilute hydrochloric acid (10 mL*2) and saturated brine (10 mL*2), and then dried by rotary evaporation. The solute was separated by column chromatography (mobile phase: MeOH : DCM = 1 : 10) to obtain compound A191-6 (633 mg, yield 47%). ¹H NMR (400 MHz, DMSO) δ 11.12 (s, 1H), 7.95 - 7.83 (m, 3H), 7.37 (d, *J* = 5.6 Hz, 1H), 7.33 (d, *J* = 7.8 Hz, 2H), 7.23 (d, *J* = 7.9 Hz, 2H), 5.15 (dd, *J=* 12.9, 5.3 Hz, 1H), 4.72 (s, 2H), 4.57 (s, 2H), 4.12 (d, *J* = 6.2 Hz, 2H), 2.90 (dd, *J* = 22.7, 8.6 Hz, 1H), 2.68 - 2.52 (m, 2H), 2.13 - 2.02 (m, 1H), 1.39 (s, 9H).

### Synthesis of compound A191-7

A191-6 (100 mg, 0.2 mmol) was dissolved in 10 mL of dichloromethane, and 3 mL of trifluoroacetic acid was added. The mixture was reacted at room temperature for 1 h, and then TLC showed that the reaction was completed. The solvent was dried by rotary evaporation to obtain A191-7 (120 mg, crude).

### Synthesis of compound A191

A191-7 (60 mg, 0.1 mmol), naphthaleneacetic acid (20 mg, 0.1 mmol) and triethylamine (30 mg, 0.3 mmol) were dissolved in 5 mL of dichloromethane, and HATU (51 mg, 0.15 mmol) was added. The mixture was reacted at room temperature for 2 h, and then TLC showed that the reaction was completed. The mixture was diluted with 20 mL of ethyl acetate. The organic phase was washed with saturated brine (10 mL*3) and then dried by rotary evaporation. The solute was purified by a preparative plate (mobile phase: dichloromethane : methanol = 10 : 1) and then purified by medium-pressure preparative chromatography to obtain compound A191 (15 mg, yield 26%). ESI-MS(M+H)⁺: 576.2.

¹H NMR (400 MHz, DMSO) δ 11.15 (s, 1H), 8.66 (t, *J=* 5.9 Hz, 1H), 8.14 - 8.07 (m, 1H), 7.92 (d, *J=* 8.0 Hz, 2H), 7.89 - 7.79 (m, 3H), 7.56 - 7.47 (m, 2H), 7.48 - 7.42 (m, 2H), 7.31 (d, *J=* 8.0 Hz, 2H), 7.24 (d, *J=* 8.0 Hz, 2H), 5.16 (dd, *J=* 12.9, 5.3 Hz, 1H), 4.71 (s, 2H), 4.57 (s, 2H), 4.29 (d, *J=* 5.9 Hz, 2H), 3.97 (s, 2H), 2.90 (ddd, *J=* 17.4, 14.1, 5.3 Hz, 1H), 2.67 - 2.53 (m, 2H), 2.08 - 2.02 (m, 1H).

The synthesis method for compound A192 is the same as that for A191.

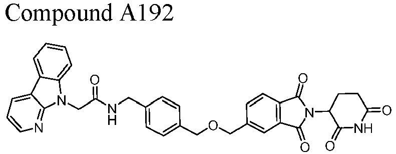

ESI-MS(M+H)⁺: 616.2.

¹H NMR (400 MHz, DMSO) δ 11.15 (s, 1H), 8.77 (t, *J* = 5.8 Hz, 1H), 8.55 (dd, *J* = 7.6, 1.3 Hz, 1H), 8.46 (dd, *J* = 4.8, 1.3 Hz, 1H), 8.21 (d, *J* = 7.7 Hz, 1H), 7.98 - 7.80 (m, 3H), 7.58 (d, *J=* 8.2 Hz, 1H), 7.50 (t, *J=* 7.6 Hz, 1H), 7.41 - 7.21 (m, 6H), 5.25 - 5.10 (m, 3H), 4.72 (s, 2H), 4.58 (s, 2H), 4.31 (d, *J=* 5.8 Hz, 2H), 2.95 - 2.79 (m, 1H), 2.69 - 2.53 (m, 2H), 2.11 - 2.02 (m, 1H).

### Example 15:

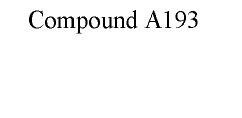

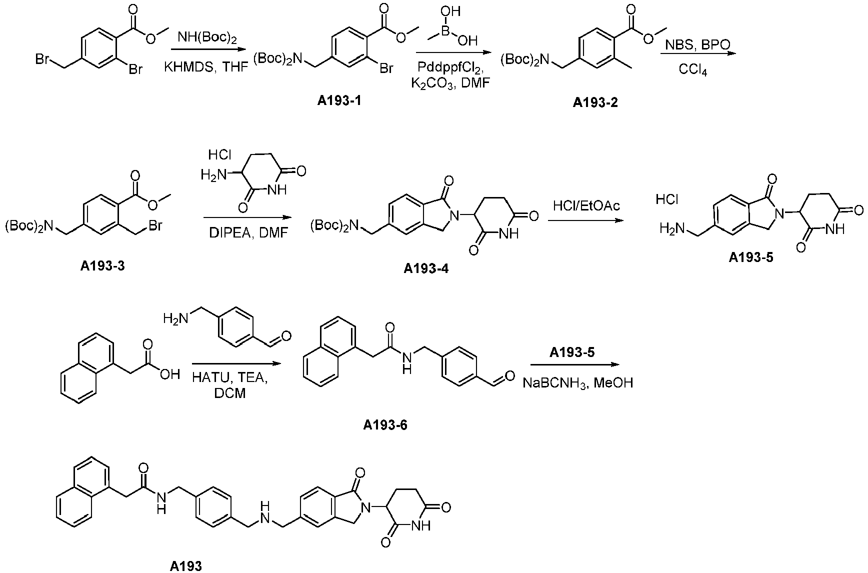

### Synthesis of compound A193-1

NH(Boc)₂ (5.0 g, 23 mmol) was dissolved in 80 mL of anhydrous tetrahydrofuran. Under ice bath, KHMDS (28 mL, 1 M, 28 mmol) was added. The mixture was reacted at 0°C for 0.5 h, and then a solution of methyl 2-bromo-4-bromomethylbenzoate (8.5 g, 27.6 mmol) in tetrahydrofuran (10 mL) was added. The mixture was reacted overnight at room temperature, and then TLC showed that the reaction was completed. The reaction was quenched with water (20 mL). The aqueous phase was extracted with ethyl acetate (50 mL*2). The organic phases were combined and dried by rotary evaporation. The solute was separated and purified by column chromatography (mobile phase: PE : EA =2 : 1) to obtain compound A193-1 (9.1 g, yield 89%). ¹H NMR (400 MHz, DMSO) δ 7.78 (d, *J=* 8.0 Hz, 1H), 7.57 (s, 1H), 7.32 (d, *J=* 8.0 Hz, 1H), 4.72 (s, 2H), 3.85 (s, 3H), 1.40 (s, 18H).

### Synthesis of compound A193-2

A193-1 (9.1 g, 20.5 mmol) was dissolved in 60 mL of DMF, and potassium carbonate (5.67 g, 41 mmol), methylboronic acid (6.1 g, 102 mmol) and PddppfCl₂ (1.5 g, 2 mmol) were successively added. The mixture was reacted at 90°C under nitrogen protection for 2 h, and then TLC showed that the reaction was completed. The reaction was quenched with water (100 mL). The aqueous phase was extracted with ethyl acetate (50 mL*2). The organic phases were combined, washed with saturated brine (20 mL*3) and then dried by rotary evaporation. The solute was separated and purified by column chromatography (mobile phase: PE : EA = 2 : 1) to obtain compound A193-2 (6.3 g, yield 81%). ¹H NMR (400 MHz, DMSO) δ 7.81 (d, *J=* 7.9 Hz, 1H), 7.17 - 7.10 (m, 2H), 4.70 (s, 2H), 3.81 (s, 3H), 2.50 (s, 3H), 1.40 (s, 18H).

### Synthesis of compound A193-3

A193-2 (6.3 g, 16.6 mmol) was dissolved in 100 mL of carbon tetrachloride, and NBS (2.96 g, 16.6 mmol) and BPO (400 mg, 1.6 mmol) were added. The mixture was reacted at 80°C for 2 h, and then TLC showed that the reaction was completed. The mixture was diluted with 130 mL of dichloromethane. The organic phase was washed with saturated aqueous sodium bicarbonate solution (30 mL*3) and dried by rotary evaporation. The solute was separated by column chromatography (mobile phase: petroleum ether : ethyl acetate =2 : 1) to obtain compound A193-3 (1.68 g, yield 22%). ¹H NMR (400 MHz, DMSO) δ 7.46 (d, *J* = 8.7 Hz, 1H), 7.41 (s, 1H), 7.29 (d, *J* = 8.2 Hz, 1H), 5.02 (s, 2H), 4.73 (s, 2H), 3.86 (s, 3H), 1.40 (s, 18H).

### Synthesis of compound A193-4

A193-3 (1.68 g, 3.7 mmol) was dissolved in 25 mL of DMF, and 3-aminopiperidine-2,6-dione hydrochloride (660 mg, 4 mmol) and DIPEA (1.4 g, 11 mmol) were added. The mixture was reacted overnight at 80°C, and then TLC showed that the reaction was completed. The mixture was diluted with ethyl acetate (100 mL). The organic phase was washed with saturated brine (20 mL*4) and then dried by rotary evaporation. The solute was separated by column chromatography (mobile phase: dichloromethane : methanol = 10 : 1) to obtain compound A193-4 (1.05 g, yield 60%). ¹H NMR (400 MHz, DMSO) δ 10.98 (s, 1H), 7.71 (d, *J=* 7.8 Hz, 1H), 7.44 (s, 1H), 7.36 (d, *J=* 7.9 Hz, 1H), 5.10 (dd, *J=* 13.3, 5.1 Hz, 1H), 4.80 (s, 2H), 4.39 (dd, *J=* 58.0, 17.4 Hz, 2H), 2.93 - 2.89 (m, 1H), 2.66 - 2.54 (m, 1H), 2.43 - 2.31 (m, 1H), 2.07 - 2.00 (m, 1H), 1.40 (s, 18H).

### Synthesis of compound A193-5

A193-4 (950 mg, 2 mmol) was dissolved in HCl/EtOAc (10 mL, 4 M). The mixture was reacted at room temperature for 30 min, and then TLC showed that the reaction was completed. The solvent was dried by rotary evaporation to obtain A193-5 (950 mg, crude).

### Synthesis of compound A193-6

4-(aminomethyl)benzaldehyde (88 mg, 0.64 mmol), naphthaleneacetic acid (100 mg, 0.1 mmol) and triethylamine (220 mg, 2.1 mmol) were dissolved in 15 mL of dichloromethane, and HATU (350 mg, 0.80 mmol) was added. The mixture was reacted at room temperature for 2 h, and then TLC showed that the reaction was completed. The mixture was diluted with 20 mL of ethyl acetate. The organic phase was washed with saturated brine (10 mL*3) and then dried by rotary evaporation. The solute was purified by a preparative plate (mobile phase: dichloromethane : methanol =30 : 1) to obtain compound A193-6 (140 mg, yield 86%).

### Synthesis of compound A193

A193-5 (70 mg, 0.23 mmol) and A193-6 (70 mg, 0.23 mmol) were dissolved in 8 mL of methanol, and sodium cyanoborohydride (30 mg, 0.46 mmol) was added. The mixture was reacted overnight at room temperature, and then TLC showed that the reaction was completed. The reaction was quenched with water (30 mL). The aqueous phase was extracted with dichloromethane (20 mL*3). The organic phases were combined and dried by rotary evaporation. The solute was purified by a preparative plate (mobile phase: dichloromethane : methanol = 10 : 1) and then purified by medium-pressure preparative chromatography to obtain compound A193 (4 mg, yield 3%).

ESI-MS(M+H)⁺: 561.2.

¹H NMR (400 MHz, DMSO) δ 11.00 (s, 1H), 8.67 (t, *J* = 5.9 Hz, 1H), 8.13 - 8.09 (m, 1H), 7.96 - 7.90 (m, 1H), 7.85 - 7.79 (m, 1H), 7.71 (d, *J* = 7.8 Hz, 1H), 7.62 (s, 1H), 7.56 - 7.49 (m, 3H), 7.45 (d, *J=* 5.2 Hz, 2H), 7.31 (d, *J=* 7.9 Hz, 2H), 7.21 (d, *J=* 7.9 Hz, 2H), 5.12 (dd, *J =* 13.3, 5.1 Hz, 1H), 4.50 - 4.24 (m, 4H), 3.97 (s, 2H), 3.91 (s, 2H), 3.79 (s, 2H), 2.97 - 2.87 (m, 1H), 2.67 - 2.51 (m, 1H), 2.44 - 2.32 (m, 1H), 2.05 - 1.98 (m, 1H).

The synthesis methods for compounds A194-A195 are the same as that for A193.

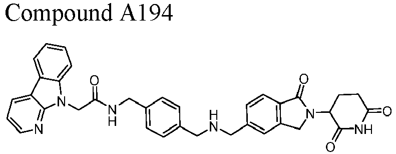

ESI-MS(M+H)⁺: 601.2.

¹H NMR (400 MHz, DMSO) δ 11.01 (s, 1H), 8.77 (t, *J=* 5.8 Hz, 1H), 8.56 (d, *J=* 7.6 Hz, 1H), 8.46 (d, *J=* 3.6 Hz, 1H), 8.22 (d, *J=* 7.7 Hz, 1H), 7.69 (d, *J=* 7.7 Hz, 1H), 7.59 (d, *J=* 7.7 Hz, 2H), 7.51 (t, *J=* 7.9 Hz, 2H), 7.34 - 7.23 (m, 6H), 5.22 - 5.08 (m, 3H), 4.52 - 4.27 (m, 4H), 3.95 - 3.64 (m, 4H), 3.00 - 2.86 (m, 1H), 2.69 - 2.56 (m, 1H), 2.46 - 2.31 (m, 1H), 2.06 - 1.96 (m, 1H).

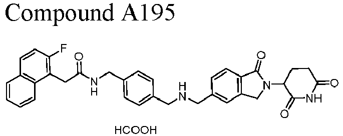

ESI-MS(M+H)⁺: 579.2.

¹H NMR (400 MHz, DMSO) δ 11.02 (s, 1H), 8.74 (t, *J* = 5.8 Hz, 1H), 8.05 (d, *J* = 8.4 Hz, 1H), 8.00 - 7.90 (m, 2H), 7.70 (d, *J* = 7.8 Hz, 1H), 7.62 - 7.54 (m, 2H), 7.49 (dd, *J =* 13.3, 6.9 Hz, 2H), 7.44 (t, *J* = 9.2 Hz, 1H), 7.30 (d, *J* = 7.8 Hz, 2H), 7.22 (d, *J =* 7.8 Hz, 2H), 5.13 (dd, *J=* 13.3, 5.1 Hz, 1H), 4.54 - 4.23 (m, 4H), 4.01 (s, 2H), 3.83 (s, 2H), 3.72 (s, 2H), 2.99 - 2.86 (m, 1H), 2.66 - 2.56 (m, 1H), 2.46 - 2.32 (m, 1H), 2.06 - 1.94 (m, 1H).

### Example 16:

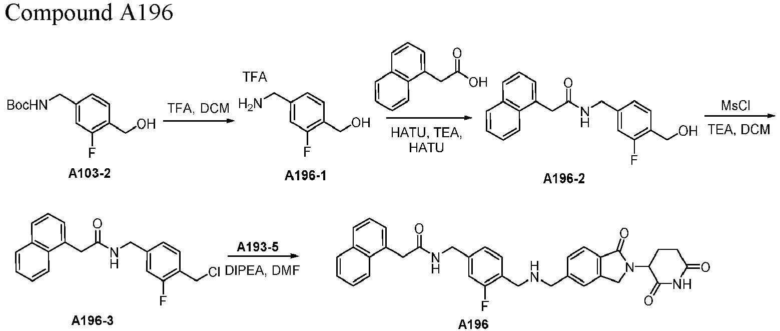

### Synthesis of compound A196-1

A103-2 (230 mg, 0.90 mmol) was dissolved in 10 mL of dichloromethane, and 3 mL of trifluoroacetic acid was added. The mixture was reacted at room temperature for 1 h, and then TLC showed that the reaction was completed. The solvent was dried by rotary evaporation to obtain A196-1 (240 mg, crude).

### Synthesis of compound A196-2

A196-1 (240 mg, 0.90 mmol), naphthaleneacetic acid (185 mg, 1.0 mmol) and triethylamine (370 mg, 3.6 mmol) were dissolved in 15 mL of dichloromethane, and HATU (520 mg, 1.4 mmol) was added. The mixture was reacted at room temperature for 2 h, and then TLC showed that the reaction was completed. The mixture was diluted with 20 mL of ethyl acetate. The organic phase was washed with saturated brine (10 mL*3) and then dried by rotary evaporation. The solute was purified by a preparative plate (mobile phase: dichloromethane : methanol = 20 : 1) to obtain compound A196-2 (280 mg, yield 96%).

### Synthesis of compound A196-3

A196-2 (280 mg, 0.87 mmol) was dissolved in 20 mL of dichloromethane. Under ice bath, methanesulfonyl chloride (360 mg, 3.6 mmol) was added dropwise. The mixture was reacted overnight at room temperature, and then TLC showed that the reaction was completed. The reaction liquid was diluted with dichloromethane (20 mL). The organic phase was washed with saturated brine (10 mL*3) and then dried by rotary evaporation. The solute was purified by a preparative plate (mobile phase: dichloromethane : methanol = 30 : 1) to obtain compound A196-3 (115 mg, yield 39%).

### Synthesis of compound A196

A196-3 (115 mg, 0.34 mmol) was dissolved in 9 mL of DMF, and A193-5 (125 mg, 0.4 mmol) and DIPEA (175 mg, 1.4 mmol) were added. The mixture was reacted overnight at room temperature, and then TLC showed that the reaction was completed. Water was added, and the mixture was extracted with ethyl acetate (30 mL*3). The organic phases were combined, and the solvent was removed by drying. The crude product was purified by medium-pressure preparative chromatography to obtain compound A196 (14 mg yield 7%). ESI-MS(M+H)⁺: 579.2.

¹H NMR (400 MHz, DMSO) δ 11.00 (s, 1H), 8.69 (t, *J* = 5.9 Hz, 1H), 8.16 - 8.04 (m, 1H), 7.96 - 7.89 (m, 1H), 7.85 - 7.79 (m, 1H), 7.67 (d, *J=* 7.8 Hz, 1H), 7.56 (d, *J* = 9.4 Hz, 1H), 7.54 - 7.45 (m, 5H), 7.41 (dd, *J* = 13.7, 5.8 Hz, 1H), 7.04 (d, *J* = 7.8 Hz, 1H), 6.98 (d, *J* = 11.2 Hz, 1H), 5.12 (dd, *J* = 13.3, 5.1 Hz, 1H), 4.47 - 4.23 (m, 4H), 3.98 (s, 2H), 3.79 (s, 2H), 3.68 (s, 2H), 2.98 - 2.87 (m, 1H), 2.65 - 2.53 (m, 1H), 2.45 - 2.33 (m, 1H), 2.04 - 1.95 (m, 1H).

### Example 17:

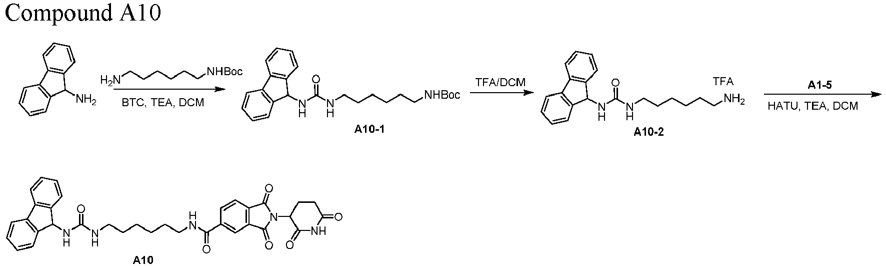

### Synthesis of compound A10-1

The compound 9-aminofluorene (651 mg, 3 mmol) was dissolved in dichloromethane (6 mL), and then triethylamine (303 mg, 3 mmol) was added. The mixture was stirred for several minutes, and triphosgene (355 mg, 1.2 mmol) was added under ice bath. The mixture was reacted at room temperature for 1 h, and then tert-butyl N-(6-aminohexyl)carbamate (712 mg, 3.3 mmol) and triethylamine (360 mg, 3.6 mmol) were added. The mixture was reacted at room temperature for 1 h, and then monitoring by TLC showed that the raw materials were completely reacted. Water (25 mL) was added, and then the mixture was extracted with dichloromethane (25 mL*3). The organic phases were combined, and the solvent was removed by drying. The solute was separated and purified by a thick preparative plate using a developing solvent (DCM : MeOH = 15 : 1) to obtain compound A10-1 (380 mg, yield 30%) as a yellow solid.

### Synthesis of compound A10-2

Compound A10-1 (380 mg, 0.9 mmol) was placed in a 50-mL round bottom flask, and 4 mL of (DCM : TFA = 3 : 1) solution was added. The mixture was reacted at room temperature for 1.5 h, and then monitoring by TLC showed that the raw materials were completely reacted. The solvent was removed by drying to obtain crude product A10-2 (450 mg) as an oil, which was directly used in the next reaction.

### Synthesis of compound A10

Compound A10-2 (155 mg, 0.48 mmol) and compound A1-5 (120 mg, 0.4 mmol) were dissolved in dichloromethane (8 mL), and then HATU (182 mg, 0.48 mmol) and triethylamine (121 mg, 1.2 mmol) were added. The mixture was reacted at room temperature for 2 h, and then monitoring by TLC showed that the raw materials were completely reacted. Water (20 mL) was added, and then the mixture was extracted with dichloromethane (20 mL *3). The organic phases were combined, and the solvent was removed by drying. The solute was separated and purified by a thick preparative plate (developing solvent: DCM : MeOH = 15 : 1) to obtain compound A10 (10 mg, yield 4%).

ESI-MS(M+H)+: 608.2.

¹H NMR (400 MHz, DMSO) δ 11.14 (s, 1H), 8.88 (t, *J=* 5.5 Hz, 1H), 8.40 - 8.29 (m, 2H), 8.02 (d, *J=* 7.7 Hz, 1H), 7.82 (d, *J=* 7.5 Hz, 2H), 7.52 (d, *J=* 7.4 Hz, 2H), 7.39 (t, *J=* 7.3 Hz, 2H), 7.31 (t, *J=* 7.4 Hz, 2H), 6.35 (d, *J=* 8.7 Hz, 1H), 5.86 (t, *J=* 5.6 Hz, 1H), 5.81 (d, *J* = 8.6 Hz, 1H), 5.19 (dd, *J* = 12.9, 5.4 Hz, 1H), 3.33 - 3.25 (m, 2H), 3.11 (dd, *J* = 12.7, 6.5 Hz, 2H), 2.94 - 2.85 (m, 1H), 2.68 - 2.53 (m, 2H), 2.12 - 2.05 (m, 1H), 1.63 - 1.50 (m, 2H), 1.49 - 1.40 (m, 2H), 1.39 - 1.30 (m, 4H).

The synthesis method for compound A12 is the same as that for A10.

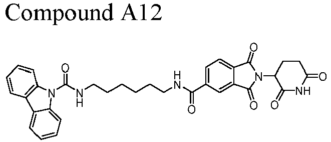

ESI-MS(M+H)⁺: 594.2.

¹H NMR (400 MHz, DMSO) δ 11.16 (s, 1H), 8.88 (t, *J=* 5.4 Hz, 1H), 8.37 - 8.31 (m, 3H), 8.17 (d, *J* = 7.6 Hz, 2H), 8.01 (d, *J* = 7.8 Hz, 1H), 7.90 (d, *J* = 8.3 Hz, 2H), 7.48 (dd, *J* = 11.4, 4.1 Hz, 2H), 7.32 (t, *J* = 7.4 Hz, 2H), 5.20 (dd, *J* = 12.9, 5.4 Hz, 1H), 3.39 (dd, *J* = 11.7, 5.5 Hz, 2H), 3.35 - 3.30 (m, 2H), 2.97 - 2.83 (m, 1H), 2.67 - 2.52 (m, 2H), 2.13 - 2.02 (m, 1H), 1.74 - 1.55 (m, 4H), 1.50 - 1.36 (m, 4H).

### Example 18:

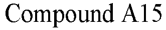

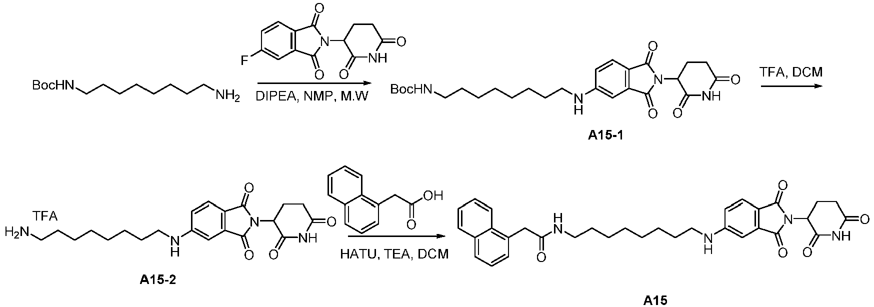

### Synthesis of compound A15-1

The compound tert-butyl *N*-(6-aminooctyl)carbamate (106 mg, 0.43 mmol) and the compound 2-(2,6-dioxo-piperidin-3-yl)-5-fluoro-isoindole-1,3-dione (100 mg, 0.36 mmol) were dissolved in NMP (4 mL), and DIPEA (185 mg, 1.44 mmol) was added. The mixture was reacted under microwave at 150°C for 3 h, and then TLC showed that the raw materials were completely reacted. Water (35 mL) was added, and the mixture was extracted with ethyl acetate (30 mL *3). The organic phases were combined and washed with saturated brine (20 mL). The solvent was removed by drying. The solute was separated and purified by a thick preparative plate (developing solvent: DCM : MeOH=10 : 1) to obtain compound A15-1 (170 mg, yield 94%).

### Synthesis of compound A15-2

Compound A15-1 (170 mg, 0.34 mmol) was placed in a 50-mL round bottom flask, and 4 mL of (DCM : TFA = 3 : 1) solution was added. The mixture was reacted at 25°C for 2 h. TLC showed that the raw materials were completely reacted. The solvent was removed by drying to obtain crude product A15-2 (200 mg, crude) as an oil, which was directly used in the next reaction.

### Synthesis of compound A15

The compound naphthaleneacetic acid (66 mg, 0.36 mmol) and compound A15-2 (200 mg, 0.38 mmol) were dissolved in dichloromethane (5 mL), and HATU (118 mg, 0.31 mmol) and triethylamine (79 mg, 0.78 mmol) were added. The mixture was reacted at room temperature for 2 h, and then monitoring by TLC showed that the raw materials were completely reacted. Water (25 mL) was added, and then the mixture was extracted with dichloromethane (30 mL *3). The organic phases were combined. The solvent was removed by drying. The solute was separated and purified by a thick preparative plate (developing solvent: DCM : MeOH = 10 : 1), and then purified by medium-pressure preparative chromatography to obtain compound A15 (20 mg, yield 9.7%).

ESI-MS(M+H)+: 569.2.

¹H NMR (400 MHz, DMSO) δ 11.07 (s, 1H), 8.13 - 8.05 (m, 2H), 7.92 - 7.89 (m, 1H), 7.80 (d, *J=* 7.5 Hz, 1H), 7.55 (d, *J=* 6.7 Hz, 1H), 7.53 - 7.47 (m, 2H), 7.46 - 7.38 (m, 2H), 7.10 (t, *J=* 5.2 Hz, 1H), 6.94 (s, 1H), 6.83 (dd, *J=* 8.4, 1.7 Hz, 1H), 5.03 (dd, *J=* 12.9, 5.4 Hz, 1H), 3.87 (s, 2H), 3.13 (dd, *J* = 12.4, 6.5 Hz, 2H), 3.05 (dd, *J=* 12.6, 6.6 Hz, 2H), 2.92 - 2.82 (m, 1H), 2.62 - 2.51 (m, 2H), 2.04 - 1.93 (m, 1H), 1.58 - 1.51 (m, 2H), 1.45 - 1.36 (m, 2H), 1.34 - 1.29 (m, 2H), 1.24 - 1.17 (m, 6H).

The synthesis methods for compounds A16 and A27-A28 are the same as that for A15.

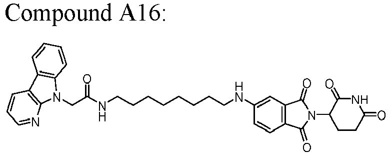

¹H NMR (400 MHz, DMSO) δ 11.05 (s, 1H), 8.53 (dd, *J=* 7.6, 1.6 Hz, 1H), 8.43 (dd, *J=* 4.8, 1.6 Hz, 1H), 8.25 - 8.15 (m, 2H), 7.58 - 7.46 (m, 3H), 7.30 - 7.22 (m, 2H), 7.09 (t, *J=* 5.3 Hz, 1H), 6.94 (d, *J=* 1.7 Hz, 1H), 6.83 (dd, *J* = 8.4, 2.0 Hz, 1H), 5.07 (s, 2H), 5.02 (dd, *J* = 12.8, 5.4 Hz, 1H), 3.14 (dd, *J* = 12.6, 6.6 Hz, 2H), 3.07 (dd, *J* = 12.7, 6.6 Hz, 2H), 2.92 - 2.82 (m, 1H), 2.62 - 2.51 (m, 2H), 2.01 - 1.95 (m, 1H), 1.61 - 1.51 (m, 2H), 1.47 - 1.31 (m, 4H), 1.30 - 1.23 (m, 6H).

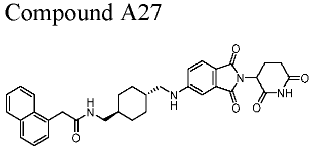

ESI-MS(M+H)⁺: 567.2.

¹H NMR (400 MHz, DMSO) δ 11.06 (s, 1H), 8.14 (t, *J=* 5.6 Hz, 1H), 8.11 - 8.05 (m, 1H), 7.94 - 7.87 (m, 1H), 7.80 (d, *J=* 7.5 Hz, 1H), 7.55 (d, *J=* 8.4 Hz, 1H), 7.52 - 7.47 (m, 2H), 7.47 - 7.36 (m, 2H), 7.15 (t, *J=* 5.4 Hz, 1H), 6.95 (s, 1H), 6.85 (d, *J=* 8.5 Hz, 1H), 5.03 (dd, *J=* 12.9, 5.4 Hz, 1H), 3.89 (s, 2H), 3.01 (t, *J* = 6.0 Hz, 2H), 2.92 (t, *J* = 6.1 Hz, 2H), 2.89 - 2.81 (m, 1H), 2.62 - 2.51 (m, 2H), 2.03 - 1.95 (m, 1H), 1.85 - 1.79 (m, 2H), 1.75 - 1.56 (m, 2H), 1.47 - 1.43 (m, 1H), 1.41 - 1.31 (m, 1H), 0.98 - 0.79 (m, 4H).

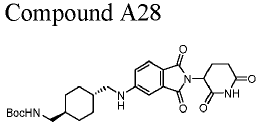

ESI-MS(M+H)⁺: 499.2.

¹H NMR (400 MHz, DMSO) δ 11.06 (s, 1H), 7.54 (d, *J* = 8.4 Hz, 1H), 7.14 (t, *J* = 5.3 Hz, 1H), 6.94 (s, 1H), 6.88 - 6.77 (m, 2H), 5.02 (dd, *J* = 12.9, 5.4 Hz, 1H), 3.01 (t, *J* = 5.9 Hz, 2H), 2.94 - 2.81 (m, 1H), 2.76 (t, *J* = 6.3 Hz, 2H), 2.63 - 2.51 (m, 2H), 2.04 - 1.93 (m, 1H), 1.81 (d, *J* = 11.2 Hz, 2H), 1.70 (d, *J* = 10.9 Hz, 2H), 1.51 (d, *J* = 9.6 Hz, 1H), 1.36 (s, 9H), 1.31 (s, 1H), 1.01 - 0.77 (m, 4H).

### Example 19:

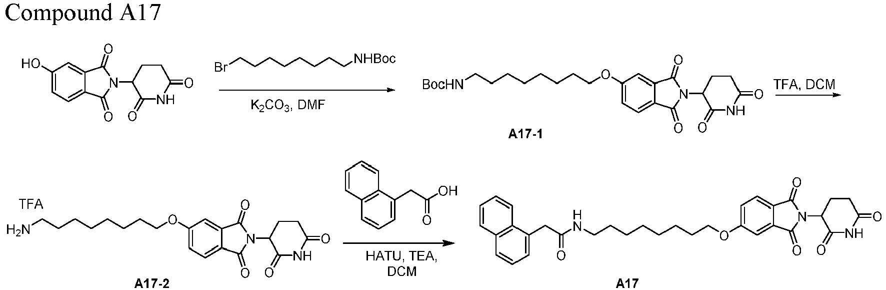

### Synthesis of compound A17-1

The compound 4-hydroxylthalidomide (50 mg, 0.18 mmol) and the compound tert-butyl (8-bromooctyl)carbamate (67 mg, 0.21 mmol) were dissolved in DMF (3 mL), and potassium carbonate (50 mg, 0.36 mmol) was added. The mixture was reacted at room temperature for 2 h. Monitoring by TLC showed that the raw materials were completely reacted, and then water (25 mL) was added. The mixture was extracted with ethyl acetate (30 mL *3). The organic phases were combined, washed with saturated brine (30 mL), dried over anhydrous sodium sulfate and concentrated to dryness. The solute was purified by a thick preparative plate (developing solvent: PE : EA = 1 : 1) to obtain compound A17-1 (40 mg, yield 44%).

### Synthesis of compound A17-2

Compound A17-1 (40 mg, 0.08 mmol) was dissolved in dichloromethane (3 mL), and TFA (1 mL) was added. The mixture was reacted at room temperature for 2 h. Monitoring by TLC showed that the raw materials were completely reacted, and then the solvent was removed by drying to obtain compound A17-2 (50 mg, crude) as an oil, which was directly used in the next reaction.

### Synthesis of compound A17

Compound A17-2 (30 mg, 0.08 mmol) and naphthaleneacetic acid (13 mg, 0.07 mmol) were dissolved in dichloromethane (4 mL), and HATU (53 mg, 0.14 mmol) and triethylamine (21 mg, 0.21 mmol) were added. The mixture was reacted at room temperature for 2 h, and then monitoring by TLC showed that the raw materials were completely reacted. Water (20 mL) was added, and the mixture was extracted with dichloromethane (20 mL*3). The organic phases were combined, washed with saturated brine (25 mL), dried over anhydrous sodium sulfate and concentrated to dryness. The solute was purified by a thick preparative plate (developing solvent: DCM : MeOH = 10 : 1), and then purified by medium-pressure preparative chromatography to obtain compound A17 (7 mg, yield 18%). ESI-MS(M+H)⁺: 570.2.

¹H NMR (400 MHz, DMSO) δ 11.11 (s, 1H), 8.08 (d, *J* = 6.4 Hz, 2H), 7.93 - 7.88 (m, 1H), 7.81 (t, *J=* 9.0 Hz, 2H), 7.55 - 7.46 (m, 2H), 7.46 - 7.40 (m, 3H), 7.34 (dd, *J* = 8.3, 2.1 Hz, 1H), 5.12 (dd, *J=* 12.9, 5.4 Hz, 1H), 4.15 (t, *J=* 6.4 Hz, 2H), 3.87 (s, 2H), 3.05 (q, *J=* 6.6 Hz, 2H), 2.94 - 2.82 (m, 1H), 2.65 - 2.51 (m, 2H), 2.08 - 2.00 (m, 1H), 1.81 - 1.68 (m, 2H), 1.45 - 1.33 (m, 4H), 1.30 - 1.17 (m, 6H).

The synthesis method for compound A18 is the same as that for A17.

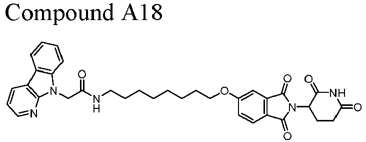

ESI-MS(M+H)⁺: 610.2.

¹H NMR (400 MHz, DMSO) δ 11.10 (s, 1H), 8.53 (dd, *J* = 7.6, 1.6 Hz, 1H), 8.43 (dd, *J* = 4.8, 1.5 Hz, 1H), 8.26 - 8.18 (m, 2H), 7.82 (d, *J* = 8.3 Hz, 1H), 7.53 - 7.46 (m, 2H), 7.41 (d, *J* = 2.1 Hz, 1H), 7.33 (dd, *J* = 8.3, 2.3 Hz, 1H), 7.30 - 7.21 (m, 2H), 5.11 (dd, *J* = 12.9, 5.4 Hz, 1H), 5.07 (s, 2H), 4.15 (t, *J* = 6.5 Hz, 2H), 3.07 (dd, *J* = 12.6, 6.6 Hz, 2H), 2.93 - 2.84 (m, 1H), 2.64 - 2.51 (m, 2H), 2.10 - 1.99 (m, 1H), 1.81 - 1.67 (m, 2H), 1.50 - 1.35 (m, 4H), 1.33 - 1.22 (m, 6H).

### Example 20:

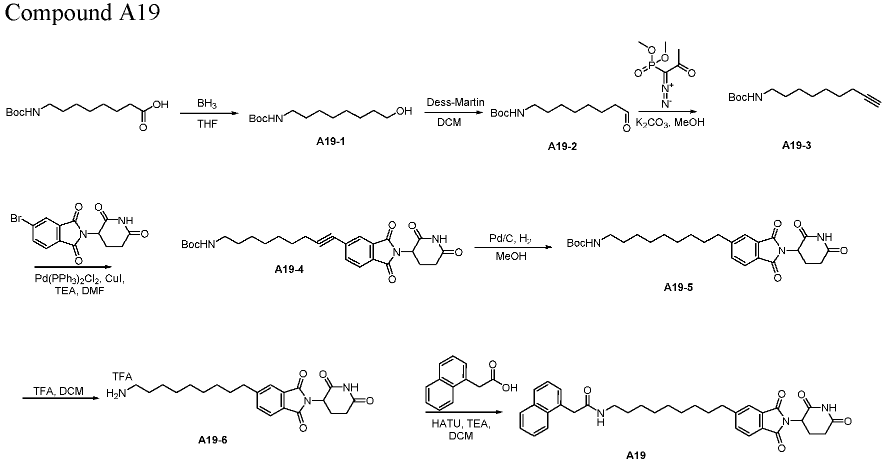

### Synthesis of compound A19-1

8-(Boc-amino)caprylic acid (9.9 g, 38.2 mmol) was dissolved in anhydrous THF (200 ml). Under argon protection, BH₂-BH₂ (114.59 ml, 114.59 mmol in THF) was added dropwise at -5°C. The mixture was reacted at -5°C to 0°C for 1 h, and then the reaction was quenched by adding sodium sulfate decahydrate and a little anhydrous sodium sulfate at low temperature. After the addition was completed, the mixture was stirred at room temperature for 30 min and then filtered over celite, and the filter cake was washed with THF for several times. The filtrate was dried by rotary evaporation and then purified by column chromatography (developing solvent: petroleum ether : ethyl acetate = 10 : 1) to obtain compound A19-1 (9.07 g, yield 97%).

### Synthesis of compound A19-2

A19-1 (4 g, 11.86 mmol) was dissolved in DCM (180 ml), and Dess-Martin reagent (18.8 g, 44.35 mmol) was added at 0°C. The mixture was reacted at 0°C for half an hour. After the reaction was completed, 170 ml of saturated sodium bicarbonate solution was added at low temperature, and then the mixture was extracted with dichloromethane (50 mL*2). The organic phases were combined and then dried by rotary evaporation. The filtrate was dried by rotary evaporation and then purified by column chromatography (developing solvent: petroleum ether : ethyl acetate = 10 : 1) to obtain compound A19-2 (6.5 g, yield 70%).

### Synthesis of compound A19-3

A19-2 (1 g, 4.11 mmol) and dimethyl (1-diazo-2-oxopropyl)phosphonate (1.18 g, 6.164 mmol) were dissolved in MeOH (15 ml), and K₂CO₃ (853 mg, 6.16 mmol) was added at room temperature. The mixture was stirred at room temperature for 4 h. The reaction was completed and then quenched with water (20 mL). The aqueous phase was extracted with ethyl acetate (20 mL*3). The organic phases were combined and then dried by rotary evaporation. The solute was purified by column chromatography (developing solvent: petroleum ether : ethyl acetate = 10 : 1) to obtain compound A19-3 (680 mg, yield 69%). ¹H NMR (400 MHz, CDCl₃) δ 4.50 (s, 1H), 3.15 - 3.01 (m, 2H), 2.17 (td, *J=* 7.0, 2.6 Hz, 2H), 1.93 (t, *J* = 2.6 Hz, 1H), 1.56 - 1.34 (m, 15H), 1.34 - 1.25 (m, 4H).

### Synthesis of compound A19-4

4-bromothalidomide (734 mg, 3.066 mmol) and intermediate A19-3 (679 mg, 3.986 mmol) were added to DMF (33 ml). CuI (41.5 mg, 0.306 mmol), triethylamine (1.1 g, 15.33 mmol) and PdCl₂ (PPh₃)₂ (153 mg, 0.31 mmol) were added at room temperature. The mixture was reacted overnight at 50°C under nitrogen protection. The reaction was completed and then diluted with 100 ml of water. The aqueous phase was extracted with ethyl acetate (20 mL*3). The organic phases were combined, washed with saturated brine (10 mL*2) and then dried by rotary evaporation. The solute was purified by column chromatography (developing solvent: petroleum ether : ethyl acetate = 1 : 2) to obtain compound A19-4 (720 mg, yield 67%).

### Synthesis of compound A19-5

A19-4 (730 mg, 3.07 mmol) was added to MeOH (12 ml), and Pd/C (150 mg, 5%) was added. The mixture was stirred overnight at room temperature under hydrogen atmosphere. The reaction was completed and then filtered over celite. The filter cake was washed with methanol for several times, and the filtrate was dried by rotary evaporation. The solute was purified by column chromatography (developing solvent: petroleum ether : ethyl acetate = 1 : 1) to obtain compound A19-5 (278 mg, yield 38%)

¹H NMR (400 MHz, DMSO) δ 11.12 (s, 1H), 7.82 (d, *J* = 7.6 Hz, 1H), 7.75 (s, 1H), 7.70 (d, *J* = 7.7 Hz, 1H), 6.72 (t, *J =* 5.5 Hz, 1H), 5.13 (dd, *J* = 12.9, 5.4 Hz, 1H), 2.94 - 2.84 (m, 3H), 2.76 (t, *J* = 7.5 Hz, 2H), 2.64 - 2.51 (m, 2H), 2.11 - 2.00 (m, 1H), 1.60 (s, 2H), 1.33 (d, *J* = 14.4 Hz, 11H), 1.30 - 1.15 (m, 10H).

### Synthesis of compound A19-6

A19-5 (90 mg, 0.18 mmol) was dissolved in 3 mL of dichloromethane, and 1 mL of trifluoroacetic acid was added. The mixture was reacted at room temperature for 1 h, and then TLC showed that the reaction was completed. The solvent was dried by rotary evaporation to obtain compound A19-6 (100 mg, crude).

### Synthesis of compound A19

Compound A19-6 (1000 mg, 0.18 mmol) and naphthaleneacetic acid (34 mg, 0.18 mmol) were dissolved in dichloromethane (4 mL), and HATU (03 mg, 0.27 mmol) and triethylamine (55 mg, 0.50 mmol) were added. The mixture was reacted at room temperature for 2 h, and then monitoring by TLC showed that the raw materials were completely reacted. Water (20 mL) was added, and the mixture was extracted with dichloromethane (20 mL*3). The organic phases were combined, washed with saturated brine (25 mL), dried over anhydrous sodium sulfate and concentrated to dryness. The solute was purified by a thick preparative plate (developing solvent: DCM : MeOH = 10 : 1), and then purified by medium-pressure preparative chromatography to obtain compound A19 (36 mg, yield 35%). ESI-MS(M+H)⁺: 568.2.

¹H NMR (400 MHz, DMSO) δ 11.11 (s, 1H), 8.11 - 8.04 (m, 2H), 7.91 - 7.88 (m, 1H), 7.84 - 7.74 (m, 3H), 7.70 (dd, *J =* 7.7, 1.2 Hz, 1H), 7.53 - 7.46 (m, 2H), 7.45 - 7.38 (m, 2H), 5.13 (dd, *J* = 12.9, 5.4 Hz, 1H), 3.86 (s, 2H), 3.04 (dd, *J* = 12.6, 6.7 Hz, 2H), 2.95 - 2.83 (m, 1H), 2.76 (t, *J* = 7.5 Hz, 2H), 2.64 - 2.51 (m, 2H), 2.07 - 2.01 (m, 1H), 167 - 1.54(m, 2H), 1.41 - 1.33 (m, 2H), 1.30 - 1.14 (m, 10H).

The synthesis method for compound A20 is the same as that for A19.

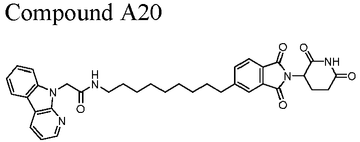

ESI-MS(M+H)⁺: 608.2.

¹H NMR (400 MHz, DMSO) δ 11.11 (s, 1H), 8.53 (dd, *J* = 7.6, 1.6 Hz, 1H), 8.43 (dd, *J* = 4.8, 1.6 Hz, 1H), 8.23 - 8.17 (m, 2H), 7.82 (d, *J* = 7.7 Hz, 1H), 7.76 (s, 1H), 7.69 (dd, *J* = 7.7, 1.2 Hz, 1H), 7.54 - 7.45 (m, 2H), 7.29 - 7.23 (m, 2H), 5.13 (dd, *J=* 12.9, 5.4 Hz, 1H), 5.07 (s, 2H), 3.06 (dd, *J* = 12.7, 6.7 Hz, 2H), 2.93 - 2.84 (m, 1H), 2.77 (t, *J=* 7.5 Hz, 2H), 2.65 - 2.51 (m, 2H), 2.10 - 2.00 (m, 1H), 1.66 - 1.54 (m, 2H), 1.44 - 1.33 (m, 2H), 1.32 - 1.16 (m, 10H).

### Example 21:

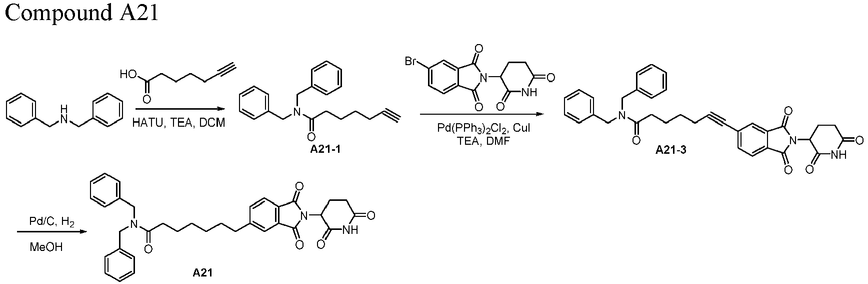

### Synthesis of compound A21-1

Dibenzylamine (400 mg, 2 mmol) and 6-heptynoic acid (277 mg, 2.2 mmol) were dissolved in dichloromethane (5 mL), and triethylamine (606 mg, 6 mmol) and HATU (912 mg, 2.4 mmol) were added. The mixture was reacted at room temperature for 1.5 h, and then TLC showed that the raw materials were completely reacted. Water (20 mL) was added, and the mixture was extracted with dichloromethane (20 mL*3) and dried. The solute was subjected to column purification using a developing solvent (PE : EA = 20 : 1-2 : 1) to obtain compound A21-1 (420 mg, yield 69%).

### Synthesis of compound A21-2

Compound A21-1 (305 mg, 1 mmol) and the compound 4-bromothalidomide (372 mg, 1.1 mmol) were dissolved in DMF (5 mL), and triethylamine (505 mg, 5 mmol), cuprous iodide (19 mg, 0.1 mmol) and Pd (PPh₃)₂Cl₂ (70 mg, 0.1 mmol) were added. The mixture was reacted at 55°C under nitrogen protection for 12 h, and then TLC showed that the raw materials were completely reacted. Water (20 mL) was added, and the mixture was extracted with ethyl acetate (20 mL*3) and dried. The solute was purified by a thick preparative plate (developing solvent: PE : EA = 1 : 1) to obtain compound A21-2 (220 mg, yield 39%).

### Synthesis of compound A21

Compound A21-2 (100 mg, 0.6 mmol) was dissolved in methanol (3 mL), and Pd/C (40 mg, 5%) was added. The mixture was reacted at room temperature under hydrogen protection for 36 h, and then TLC showed that the raw materials were completely reacted. The mixture was filtered, and the filtrate was dried. The solute was purified by a thick preparative plate (developing solvent: PE : EA = 1 : 1), and then purified by medium-pressure preparative chromatography to obtain compound A21 (10 mg, yield 10%). ESI-MS(M+H)⁺: 566.2.

¹H NMR (400 MHz, DMSO) δ 11.13 (s, 1H), 7.82 (d, *J* = 7.6 Hz, 1H), 7.75 (s, 1H), 7.68 (d, *J* = 7.7 Hz, 1H), 7.39 - 7.22 (m, 6H), 7.18 (dd, *J* = 14.3, 7.2 Hz, 4H), 5.14 (dd, *J=* 12.9, 5.4 Hz, 1H), 4.50 (s, 4H), 2.94 - 2.84 (m, 1H), 2.79 - 2.69 (m, 2H), 2.64 - 2.51 (m, 2H), 2.35 (t, *J=* 7.2 Hz, 2H), 2.07 - 1.99 (m, 1H), 1.64 - 1.46 (m, 4H), 1.35 - 1.15 (m, 4H).

### Example 22:

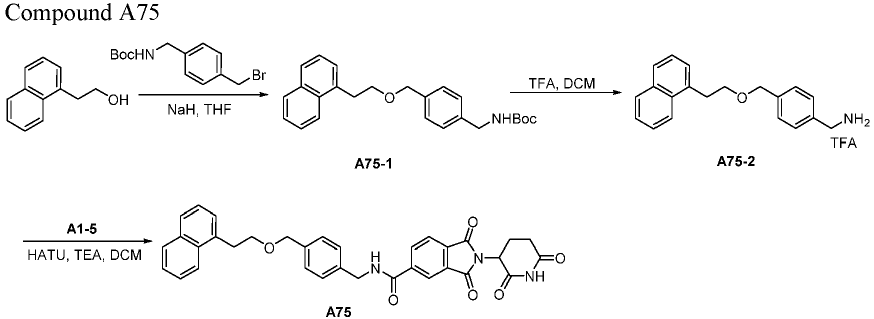

### Synthesis of compound A75-1

The compound 2-naphthaleneethanol (150 mg, 0.87 mmol) was dissolved in anhydrous THF (4 mL). Under ice bath, NaH (209 mg, 60%, 5.2 mmol) was added. 5 min after the addition, ice bath was removed, and the reaction was stirred at room temperature for one hour. A solution of the compound tert-butyl 4-(bromomethyl)benzylcarbamate (261 mg, 0.87 mmol) in THF (2 mL) was added dropwise, and then the reaction was stirred at room temperature for another hour. Monitoring by TLC showed that the raw materials were completely reacted, and then under ice bath, the mixture was diluted by slowly adding water (10 ml) and extracted with EA (10 mL*3). The organic phases were combined, washed with saturated brine (10 ml), dried over anhydrous sodium sulfate and concentrated to dryness. The solute was purified by a thick preparative plate (developing solvent: PE : EA = 5 : 1) to obtain compound A75-1 (90 mg, yield 26%).

### Synthesis of compound A75-2

Compound A75-1 (90 mg, 0.23 mmol) was dissolved in DCM (3 ml), and TFA (1 ml) was added with stirring. The mixture was reacted at room temperature for 1 h, and then monitoring by TLC showed that the raw materials were completely reacted. The solvent was removed by drying to obtain compound A75-2 (100 mg, crude), which was directly used in the next reaction.

### Synthesis of compound A75

Compound A75-2 (60 mg, 0.23 mmol) and compound A1-5 (74 mg, 0.25 mmol) were dissolved in dichloromethane (5 mL), and HATU (160 mg, 0.42 mmol) and TEA (63 mg, 0.63 mmol) were added. The reaction was stirred at room temperature for 2 h, and then monitoring by TLC showed that the raw materials were completely reacted. The mixture was diluted with water (10 ml) and extracted with dichloromethane (10 mL*3). The organic phases were combined, washed with saturated brine (10 ml), dried over anhydrous sodium sulfate and concentrated in vacuo to dryness. The crude was purified by a thick preparative plate (developing solvent: DCM : MeOH = 10 : 1), and then purified by medium-pressure preparative chromatography to obtain compound A75 (27 mg, yield 22%).

ESI-MS(M+H)⁺: 576.2.

¹H NMR (400 MHz, DMSO) δ 11.15 (s, 1H), 9.43 (t, *J=* 5.8 Hz, 1H), 8.43 - 8.32 (m, 2H), 8.07 - 8.02 (m, 2H), 7.93 - 7.86 (m, 1H), 7.81 - 7.73 (m, 1H), 7.54 - 7.46 (m, 2H), 7.45 - 7.37 (m, 2H), 7.33 - 7.26 (m, 2H), 7.24 (d, *J=* 8.1 Hz, 2H), 5.19 (dd, *J=* 12.8, 5.4 Hz, 1H), 4.56 - 4.41 (m, 4H), 3.74 (t, *J=* 7.0 Hz, 2H), 3.33 (t, *J=* 7.0 Hz, 2H), 2.94 - 2.84 (m, 1H), 2.69 - 2.53 (m, 2H), 2.16 - 2.06 (m, 1H).

The synthesis method for compound A76 is the same as that for A75.

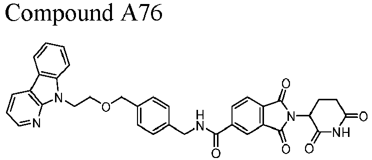

ESI-MS(M+H)⁺: 616.2.

¹H NMR (400 MHz, DMSO) δ 11.15 (s, 1H), 9.39 (t, *J* = 5.8 Hz, 1H), 8.52 (dd, *J* = 7.6, 1.6 Hz, 1H), 8.45 (dd, *J=* 4.8, 1.6 Hz, 1H), 8.40 - 8.32 (m, 2H), 8.19 (d, *J* = 7.7 Hz, 1H), 8.03 (d, *J* = 7.8 Hz, 1H), 7.71 (d, *J* = 8.3 Hz, 1H), 7.54 - 7.46 (m, 1H), 7.30 - 7.21 (m, 2H), 7.18 (d, *J=* 8.0 Hz, 2H), 7.04 (d, *J=* 8.1 Hz, 2H), 5.19 (dd, *J=* 12.8, 5.4 Hz, 1H), 4.68 (t, *J* = 5.5 Hz, 2H), 4.45 (d, *J* = 5.8 Hz, 2H), 4.41 (s, 2H), 3.86 (t, *J* = 5.5 Hz, 2H), 2.97 - 2.82 (m, 1H), 2.67 - 2.52 (m, 2H), 2.14 - 2.06 (m, 1H).
The corresponding intermediates were prepared as follows:

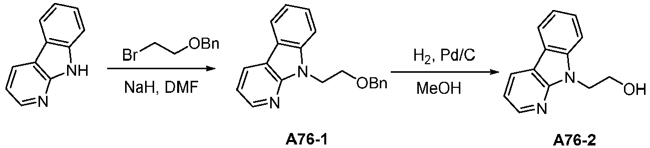

### Synthesis of compound A76-1

The compound 9H-pyrido[2,3-B]indole (1.68 g, 10 mmol) was dissolved in anhydrous DMF (15 mL). Under ice bath, sodium hydride (1 g, 60%, 25 mmol) was added. The mixture was reacted at room temperature for 0.5 h, and then a solution of the compound benzyl 2-bromoethyl ether (2.78 g, 13 mmol) in DMF (8 mL)was added. The mixture was reacted at room temperature for another 4 h, and then monitoring by TLC showed that the raw materials were completely reacted. Under ice bath, the reaction was quenched by slowly adding ice water (60 mL) and extracted with ethyl acetate (60 mL*3). The organic phases were combined, washed with saturated brine (80 mL), dried over anhydrous sodium sulfate and concentrated. The solute was purified by column chromatography (developing solvent: PE : EA = 100 : 1-15 : 1) to obtain compound A76-1 (1.1 g, yield 36%).

### Synthesis of compound A76-2

Compound A66-1 (1.1 g, 3.64 mmol) was dissolved in methanol (20 mL), and Pd/C (250 mg, 5%) was added. The mixture was reacted at room temperature under hydrogen protection for 18 h, and then monitoring by TLC showed that the raw materials were completely reacted. The mixture was filtered, and the filtrate was concentrated to dryness. The solute was purified by column chromatography (developing solvent: PE : EA = 40 : 1-2 : 1) to obtain compound A76-2 (550 mg, yield 71%).

### Example 23:

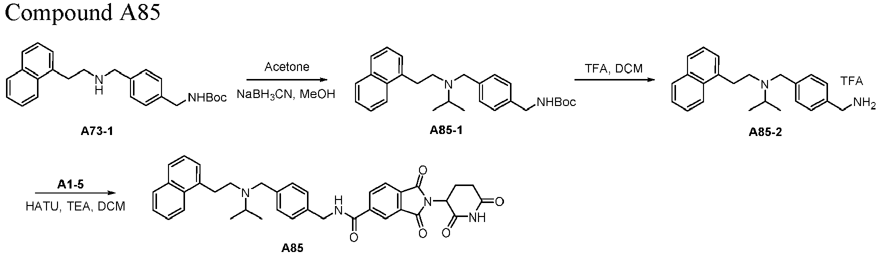

### Synthesis of compound A85-1

Compound A73-1 (40 mg, 0.10 mmol) and acetone (45 mg, 0.74 mmol) were dissolved in methanol (4 mL), and the mixture was reacted at room temperature for 2 h. Sodium cyanoborohydride (50 mg, 0.74 mmol) was further added. The mixture was reacted at room temperature for 16 h, and then monitoring by TLC showed that the raw materials were completely reacted. Water (20 mL) was added, and the mixture was extracted with dichloromethane (25 mL*3). The combined organic phase was washed with brine (30 mL), dried over anhydrous sodium sulfate and concentrated to dryness. The crude was purified by a thick preparative plate (DCM : MeOH = 10 : 1) to obtain compound A85-1 (25 mg, yield 57%).

### Synthesis of compound A85-2

Compound A85-1 (25 mg, 0.05 mmol) was dissolved in dichloromethane (2 mL), and TFA (0.8 mL) was added. The mixture was reacted at room temperature for 1 h, and then monitoring by TLC showed that the raw materials were completely reacted. The solvent was removed by drying to obtain compound A85-2 (25 mg, crude) as an oil, which was directly used in the next reaction.

### Synthesis of compound A85

Compound A85-2 (25 mg, 0.05 mmol) and compound A1-5 (14 mg, 0.046 mmol) were dissolved in dichloromethane (2 mL), and HATU (21 mg, 0.055 mmol) and triethylamine (14 uL, 0.1 mmol) were added. The mixture was reacted at room temperature for 2 h, and then monitoring by TLC showed that the raw materials were completely reacted. Water (20 ml) was added, and the mixture was extracted with dichloromethane (20 mL*3). The organic phases were combined, washed with saturated brine (25 ml), dried over anhydrous sodium sulfate and concentrated. The solute was purified by a thick preparative plate (developing solvent: DCM : MeOH = 10 : 1), and then purified by medium-pressure preparative chromatography to obtain compound A85 (18 mg, yield 64%).

ESI-MS(M+H)⁺: 617.2.

¹H NMR (400 MHz, DMSO) δ 11.15 (s, 1H), 9.44 (t, *J=* 5.9 Hz, 1H), 8.44 - 8.35 (m, 2H), 8.05 (d, *J=* 7.8 Hz, 1H), 7.86 - 7.82 (m, 1H), 7.77 - 7.69 (m, 2H), 7.43 - 7.34 (m, 3H), 7.33 - 7.27 (m, 5H), 5.19 (dd, *J=* 12.9, 5.3 Hz, 1H), 4.51 (d, *J=* 5.8 Hz, 2H), 3.63 (s, 2H), 3.13 - 2.82 (m, 4H), 2.70 - 2.52 (m, 4H), 2.13 - 2.04 (m, 1H), 0.97 (d, *J=* 6.6 Hz, 6H).

The synthesis methods for compounds A86-A87 and A230 are the same as that for A85.

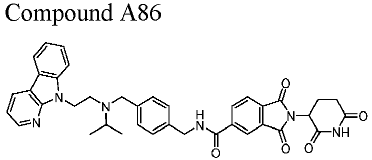

ESI-MS(M+H)⁺: 657.2.

¹H NMR (400 MHz, DMSO) δ 11.15 (s, 1H), 9.37 (t, *J* = 5.9 Hz, 1H), 8.52 - 8.46 (m, 1H), 8.43 - 8.33 (m, 3H), 8.16 (d, *J* = 7.7 Hz, 1H), 8.04 (d, *J* = 7.8 Hz, 1H), 7.45 - 7.35 (m, 2H), 7.23 - 7.15 (m, 2H), 7.08 (d, *J* = 8.0 Hz, 2H), 6.98 (d, *J* = 8.0 Hz, 2H), 5.19 (dd, *J* = 12.9, 5.4 Hz, 1H), 4.47 - 4.43 (m, 4H), 3.58 (s, 2H), 2.98 - 2.75 (m, 4H), 2.68 - 2.52 (m, 2H), 2.13 - 2.03 (m, 1H), 0.78 (d, *J* = 6.8 Hz, 6H).

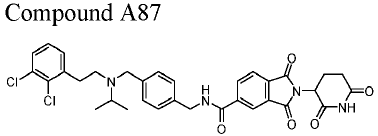

ESI-MS(M+H)⁺: 635.2.

¹H NMR (400 MHz, DMSO) δ 11.13 (s, 1H), 9.41 (t, *J* = 5.9 Hz, 1H), 8.40 - 8.35 (m, 2H), 8.04 (d, *J* = 7.8 Hz, 1H), 7.47 - 7.42 (m, 1H), 7.30 - 7.16 (m, 6H), 5.19 (dd, *J* = 12.9, 5.4 Hz, 1H), 4.49 (d, *J* = 5.8 Hz, 2H), 3.57 (s, 2H), 2.96 - 2.77 (m, 4H), 2.68 - 2.51 (m, 5H), 2.14 - 2.04 (m, 1H), 0.93 (d, *J* = 6.6 Hz, 6H).

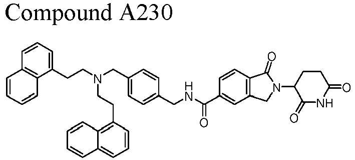

ESI-MS(M+H)⁺: 715.2.

¹H NMR (400 MHz, DMSO) δ 11.01 (s, 1H), 9.23 (t, *J* = 6.0 Hz, 1H), 8.11 (s, 1H), 8.03 (d, *J=* 7.9 Hz, 1H), 7.91 - 7.80 (m, 5H), 7.75 (d, *J=* 8.0 Hz, 2H), 7.47 - 7.36 (m, 6H), 7.35 - 7.23 (m, 6H), 5.14 (dd, *J=* 13.3, 5.0 Hz, 1H), 4.59 - 4.37 (m, 4H), 3.85 (s, 2H), 3.22 - 3.15 (m, 4H), 2.97 - 2.77 (m, 5H), 2.69 - 2.55 (m, 1H), 2.45 - 2.35 (m, 1H), 2.07 - 1.96 (m, 1H).

### Example 24:

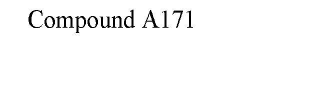

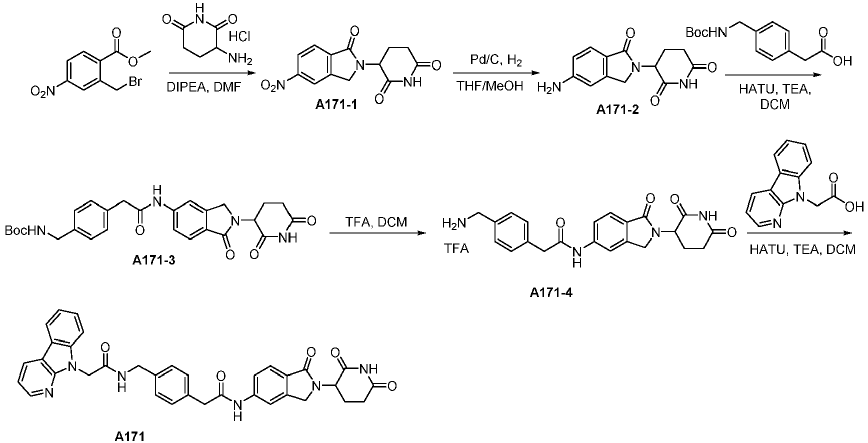

### Synthesis of compound A171-1

Methyl 2-(bromomethyl)-4-nitrobenzoate (1.6 g, 5.84 mmol) was dissolved in 15 mL of DMF, and 3-aminopiperidine-2,6-dione hydrochloride (1.0 g, 6.4 mmol) and DIPEA (1. g, 11.7 mmol) were added. The mixture was reacted overnight at 80°C, and then TLC showed that the reaction was completed. The mixture was diluted with ethyl acetate (100 mL). The organic phase was washed with saturated brine (20 mL*4) and then dried by rotary evaporation. The solute was separated by column chromatography (mobile phase: dichloromethane : methanol = 10 : 1) to obtain compound A171-1 (1.05 g, yield 59%).

### Synthesis of compound A171-2

A171-1 (1.05 g, 3.46 mmol) was dissolved in 30 mL of tetrahydrofuran/methanol (1 : 1), and Pd/C (300 mg, 5%) was added. The mixture was reacted overnight at room temperature under hydrogen atmosphere, and then TLC showed that the reaction was completed. The reaction liquid was filtered, and the filter cake was rinsed with THF. The filtrate was dried by rotary evaporation to obtain compound A171-2 (800 mg, yield 89%).

### Synthesis of compound A171-3

N-tertbutoxycarbonyl-(4-aminomethylphenyl)acetic acid (132 mg, 0.5 mmol) was dissolved in dichloromethane (5 mL), and then A171-2 (130 mg, 0.5 mmol), triethylamine (76 mg, 0.75 mmol) and HATU (228 mg, 0.6 mmol) were successively added. The reaction was stirred overnight at room temperature, and then TLC showed that the raw materials were completely reacted. Water (10 mL) was added, and the mixture was extracted with dichloromethane (20 ml*3). The organic phases were combined. The solvent was dried by rotary evaporation. The solute was purified by column chromatography (mobile phase: dichloromethane : methanol = 20 : 1) to obtain compound A171-3 (190 mg, yield 75%). ¹H NMR (400 MHz, DMSO) δ 10.95 (s, 1H), 10.50 (s, 1H), 7.96 (s, 1H), 7.66 - 7.58 (m, 2H), 7.35 (dd, *J=* 10.5, 4.4 Hz, 1H), 7.28 (d, *J* = 8.0 Hz, 2H), 7.18 (d, *J* = 8.0 Hz, 2H), 5.07 (dd, *J* = 13.3, 5.1 Hz, 1H), 4.41 (d, *J* = 17.3 Hz, 1H), 4.33 - 4.22 (m, 1H), 4.15 - 4.04 (m, 2H), 3.66 (s, 2H), 2.96 - 2.81 (m, 1H), 2.63 - 2.53 (m, 1H), 2.42 - 2.27 (m, 1H), 2.03 - 1.91 (m, 1H), 1.36 (s, 9H).

### Synthesis of compound A171-4

A171-3 (190 mg, 0.38 mmol) was dissolved in 5 mL of dichloromethane, and 1 mL of trifluoroacetic acid was added. The mixture was reacted at room temperature for 1 h, and then TLC showed that the reaction was completed. The solvent was dried by rotary evaporation to obtain compound A171-4 (250 mg, crude).

### Synthesis of compound A171

2-(9H-pyrido[2,3-b]indole-9-acetic acid (40 mg, 0.09 mmol) was dissolved in 6 mL of dichloromethane, and then HATU (38 mg, 0.10 mmol), TEA (20 mg, 0.20 mmol) and compound A171-4 (66 mg, 0.10 mmol) were successively added. The reaction was stirred at room temperature for 2 h, and then monitoring by TLC showed that the reaction was completed. The reaction was quenched with water (20 mL), and the aqueous phase was extracted with dichloromethane (40 mL*3). The organic phases were combined, dried and concentrated. The solute was separated by a thick preparative plate using a developing solvent (dichloromethane : methanol = 10 : 1) and then purified by medium-pressure preparative chromatography to obtain compound A171 (4 mg, 6%).

ESI-MS(M+H)⁺: 615.2.

¹H NMR (400 MHz, DMSO) δ 10.97 (s, 1H), 10.49 (s, 1H), 8.74 (t, *J* = 5.6 Hz, 1H), 8.54 (d, *J* = 6.8 Hz, 1H), 8.45 (d, *J* = 4.9 Hz, 1H), 8.21 (d, *J* = 7.7 Hz, 1H), 7.96 (s, 1H), 7.69 - 7.54 (m, 3H), 7.49 (t, *J* = 7.5 Hz, 1H), 7.32 - 7.21 (m, 6H), 5.16 (s, 2H), 5.07 (dd, *J* = 13.0, 5.0 Hz, 1H), 4.46 - 4.22 (m, 4H), 3.67 (s, 2H), 2.96 - 2.84 (m, 1H), 2.70 - 2.57 (m, 1H), 2.41 - 2.31 (m, 1H), 2.03 - 1.92 (m, 1H).

The synthesis methods for compounds A172-A173 and A201 are the same as that for A171.

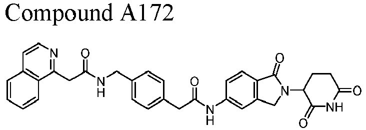

ESI-MS(M+H)⁺: 576.2.

¹H NMR (400 MHz, DMSO) δ 10.97 (s, 1H), 10.50 (s, 1H), 8.70 (t, *J* = 6.0 Hz, 1H), 8.41 (d, *J=* 5.7 Hz, 1H), 8.24 (d, *J=* 8.5 Hz, 1H), 7.99 - 7.93 (m, 2H), 7.78 - 7.71 (m, 2H), 7.68 - 7.58 (m, 3H), 7.29 (d, *J* = 8.1 Hz, 2H), 7.23 (d, *J* = 8.1 Hz, 2H), 5.08 (dd, *J* = 13.3, 5.1 Hz, 1H), 4.42 (d, *J* = 17.4 Hz, 1H), 4.30 - 4.25 (m, 3H), 4.22 (s, 2H), 3.67 (s, 2H), 2.97 - 2.84 (m, 1H), 2.68 - 2.55 (m, 1H), 2.42 - 2.30 (m, 1H), 2.03 - 1.96 (m, 1H).

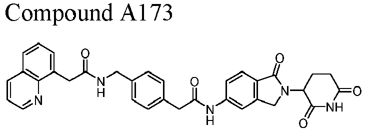

ESI-MS(M+H)⁺: 576.2.

¹H NMR (400 MHz, DMSO) δ 10.97 (s, 1H), 10.47 (s, 1H), 8.92 (dd, *J* = 4.2, 1.7 Hz, 1H), 8.47 (t, *J* = 6.0 Hz, 1H), 8.36 (dd, *J* = 8.3, 1.7 Hz, 1H), 7.96 (s, 1H), 7.90 - 7.84 (m, 1H), 7.69 - 7.63 (m, 2H), 7.62 - 7.58 (m, 1H), 7.58 - 7.50 (m, 2H), 7.28 - 7.21 (m, 4H), 5.07 (dd, *J* = 13.3, 5.1 Hz, 1H), 4.42 (d, *J* = 17.3 Hz, 1H), 4.31 - 4.24 (m, 3H), 4.12 (s, 2H), 3.66 (s, 2H), 2.97 - 2.84 (m, 1H), 2.68 - 2.54 (m, 1H), 2.43 - 2.29 (m, 1H), 2.03 - 1.92 (m, 1H).

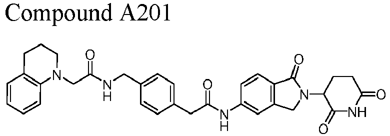

ESI-MS(M+H)⁺: 580.2

¹H NMR (400 MHz, DMSO) δ 10.97 (s, 1H), 10.48 (s, 1H), 8.37 (t, *J* = 5.7 Hz, 1H), 7.96 (s, 1H), 7.66 (d, *J* = 8.3 Hz, 1H), 7.61 (d, *J* = 8.1 Hz, 1H), 7.27 (d, *J* = 7.9 Hz, 2H), 7.19 (d, *J* = 7.9 Hz, 2H), 6.98 - 6.84 (m, 2H), 6.50 (t, *J* = 7.2 Hz, 1H), 6.38 (d, *J* = 8.2 Hz, 1H), 5.08 (dd, *J* = 13.2, 4.9 Hz, 1H), 4.42 (d, *J* = 17.3 Hz, 1H), 4.35 - 4.20 (m, 3H), 3.84 (s, 2H), 3.66 (s, 2H), 3.35 (s, 2H), 2.97 - 2.85 (m, 1H), 2.70 (t, *J* = 6.2 Hz, 2H), 2.60 (d, *J* = 16.8 Hz, 1H), 2.42-2.32 (m, 1H), 2.05 - 1.86 (m, 3H).

### Example 25:

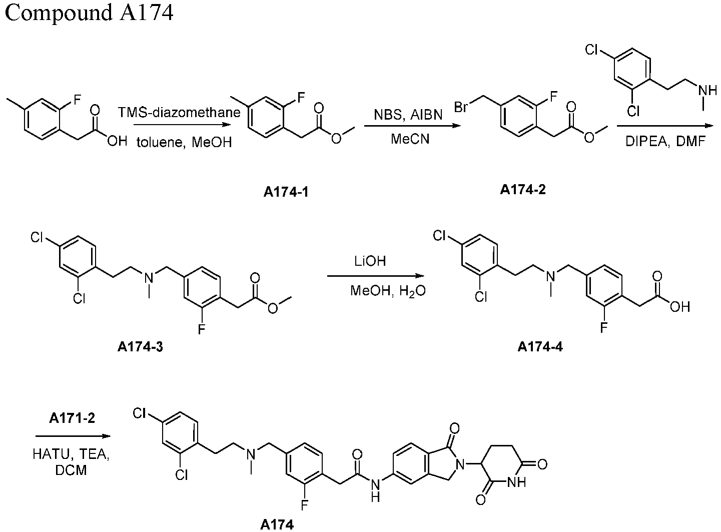

### Synthesis of compound A174-1

2-fluoro-4-methylphenylacetic acid (200 mg, 1.19 mmol) was dissolved in 5 mL of toluene/methanol (1 : 4). Under ice bath, TMSCH₂N₂ (2.4 mL, 2 M, 4.8 mmol) was added dropwise. The reaction was stirred at 0°C for 1.5 h, and then TLC showed that the reaction was completed. The solvent was dried by rotary evaporation to obtain compound A174-1 (220 mg, crude).

### Synthesis of compound A174-2

A174-1 (220 mg, 1.19) was dissolved in 6 mL of acetonitrile, and NBS (233 mg, 1.31 mmol) and AIBN (39 mg, 0.24 mmol) were added. The reaction was stirred at 60°C for 2 h, and then TLC showed that the reaction was completed. The solvent was dried by rotary evaporation. The solute was separated by a thick preparative plate using a developing solvent (petroleum ether : ethyl acetate = 5 : 1) to obtain compound A174-1 (178 mg, 58%). ¹H NMR (400 MHz, DMSO) δ 7.36 - 7.23 (m, 3H), 4.69 (s, 2H), 3.73 (s, 2H), 3.62 (s, 3H).

### Synthesis of compound A174-3

A174-2 (85 mg, 0.33 mmol) and amine (67 mg, 0.33 mol) were dissolved in 5 mL of DMF, and DIPEA (128 mg, 0.99 mmol) was added. The mixture was reacted at room temperature for 3 h, and then TLC showed that the reaction was completed. The mixture was diluted with ethyl acetate (20 mL). The organic layer was washed with saturated brine (10 mL*3) and then dried by rotary evaporation. The solute was separated by a thick preparative plate using a developing solvent (dichloromethane : methanol = 10 : 1) to obtain compound A174-3 (93 mg, 74%).

### Synthesis of compound A174-4

Compound A174-3 (93 mg, 0.24 mmol) was dissolved in 4 mL of methanol/H₂O (3 : 1), and lithium hydroxide hydrate (20 mg, 0.48 mmol) was added. The mixture was reacted overnight at room temperature, and then TLC showed that the reaction was completed. The mixture was adjusted to pH 3-4 with dilute hydrochloric acid, and then the aqueous phase was extracted with ethyl acetate (40 mL*3). The organic phases were combined and dried by rotary evaporation to obtain compound A174-4 (75 mg, yield 84%).

### Synthesis of compound A174

Compound A174-4 (75 mg, 0.20 mmol) and compound A171-2 (58 mg, 0.22 mmol) were dissolved in DCM (5 mL), and HATU (85 mg, 0.22 mmol) and TEA (45 mg, 0.45 mmol) were added. The mixture was reacted overnight at room temperature, and then monitoring by TLC showed that the raw materials were completely reacted. The mixture was diluted with water (10 ml) and extracted with DCM (10 mL*3). The organic phases were combined, washed with saturated brine (10 ml), dried over anhydrous sodium sulfate and concentrated in vacuo to dryness. The crude was purified by a thick preparative plate (developing solvent: DCM : MeOH = 10 : 1), and then purified by medium-pressure preparative chromatography to obtain compound A174 (6 mg, yield 5%). ESI-MS(M+H)⁺: 611.2.

NMR (400 MHz, DMSO) δ 10.97 (s, 1H), 10.53 (s, 1H), 7.97 (s, 1H), 7.69 - 7.62 (m, 1H), 7.60 (dd, *J* = 8.4, 1.4 Hz, 1H), 7.50 (d, *J* = 8.2 Hz, 2H), 7.29 (t, *J* = 7.8 Hz, 1H), 7.20 (dd, *J* = 8.2, 1.9 Hz, 1H), 7.00 (d, *J* = 7.8 Hz, 1H), 6.95 (d, *J* = 11.0 Hz, 1H), 5.08 (dd, *J* = 13.3, 5.1 Hz, 1H), 4.42 (d, *J* = 17.4 Hz, 1H), 4.28 (d, *J* = 17.4 Hz, 1H), 3.75 (s, 2H), 3.50 (s, 2H), 2.98 - 2.85 (m, 1H), 2.77 (t, *J* = 7.1 Hz, 2H), 2.63 - 2.53 (m, 3H), 2.43 - 2.31 (m, 1H), 2.18 (s, 3H), 2.04 - 1.93 (m, 1H).

### Example 26

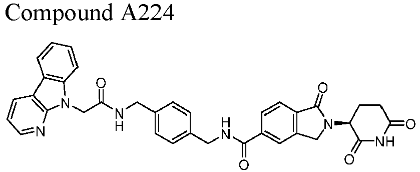

ESI-MS(M+H)⁺: 615.2

NMR (400 MHz, DMSO) δ 11.03 (s, 1H), 9.24 (t, *J* = 6.0 Hz, 1H), 8.76 (t, *J* = 5.8 Hz, 1H), 8.55 (d, *J* = 7.6 Hz, 1H), 8.46 (d, *J* = 4.8 Hz, 1H), 8.22 (d, *J* = 7.7 Hz, 1H), 8.10 (s, 1H), 8.02 (d, *J* = 8.1 Hz, 1H), 7.83 (d, *J* = 7.9 Hz, 1H), 7.58 (d, *J* = 8.1 Hz, 1H), 7.50 (t, *J* = 7.6 Hz, 1H), 7.32 - 7.24 (m, 6H), 5.21 - 5.10 (m, 3H), 4.56 - 4.38 (m, 4H), 4.29 (d, *J* = 5.7 Hz, 2H), 2.99 - 2.85 (m, 1H), 2.70 - 2.57 (m, 1H), 2.46 - 2.36 (m, 1H), 2.07 - 1.97 (m, 1H).

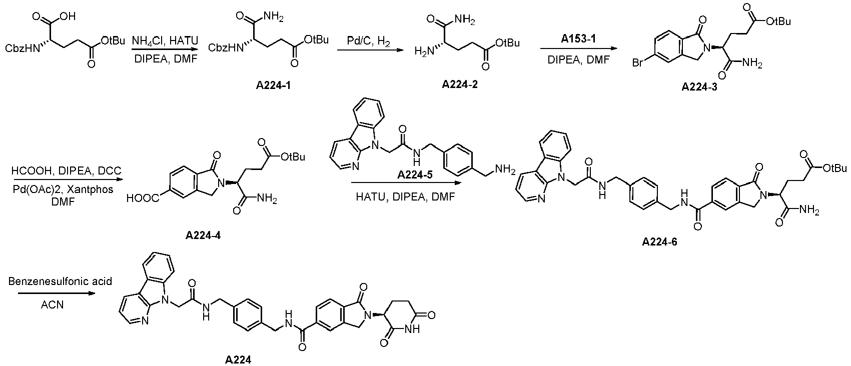

### Synthesis of compound A224-1

5-tert-butyl N-carbobenzoxy-L-glutamate (8.0 g, 0.023 mol), NH4Cl (6.34 g, 0.118 mol) and DIEA (30.6 g, 0.237 mol) were successively added to DMF (80 ml). Under ice bath, HATU (13.52 g, 0.035 mol) was added in batches. After the addition was completed, the mixture was warmed to room temperature and reacted for 1 h, and then TLC showed that the reaction was completed. To the reaction liquid, ice water (160 ml) was added, with a white solid precipitated; and water was further added to about 300 ml, with a large amount of solids precipitated. The resulting solid was filtered, and the filter cake was rinsed with water and then dried to obtain A224-1 (6.56 g, yield 82%).

### Synthesis of compound A224-2

A224-1 (6.56 g, 19.50 mmol) and Pd/C (1.3 g) were dissolved in MeOH (65 ml). The mixture was stirred at room temperature under hydrogen atmosphere for 3 h, and then TLC showed that the reaction was completed. The reaction liquid was filtered over celite, and the filter cake was rinsed with methanol. The filtrate was dried by rotary evaporation to obtain A224-2 (3.82 g, crude), which was directly used in the next reaction. Synthesis of compound A224-3

A224-2 (3.80 g, 18.80 mmol), A153-1 (6.36 g, 20.65 mmol) and DIPEA (14.57 g, 112.74 mmol) were successively added to DMF (40 mL). The reaction was stirred overnight at 80°C, and then TLC showed that the reaction was completed. The reaction was cooled to room temperature and quenched with ice water (80 mL). The aqueous phase was extracted with ethyl acetate (100 ml*3). The organic phases were combined, washed with saturated brine (80 ml*3), dried over anhydrous sodium sulfate and dried by rotary evaporation. The resulting crude was slurried with water (20 mL) at room temperature for half an hour and then filtered. The filter cake was dried to obtain A224-3 (5.1 g, yield 68%).

### Synthesis of compound A224-4

A224-3 (2.08 g, 5.24 mmol), formic acid (846 mg, 18.33 mmol), palladium acetate (70 mg, 0.31 mmol), Xantphos (180 mg, 0.31 mmol), DCC (216 mg, 1.05 mmol) and DIPEA (1.36 g, 10.52 mmol) were successively added to DMF (20 mL). The mixture was reacted at 80°C under nitrogen atmosphere for 2 h. TLC showed that the reaction was completed. The reaction was cooled to room temperature, quenched with ice water (30 mL) and adjusted to pH 3-4 with 1 N hydrochloric acid. The aqueous phase was extracted with ethyl acetate (30 ml*3). The organic phases were combined, washed with saturated brine (30 ml*3), dried over anhydrous sodium sulfate and dried by rotary evaporation. The resulting crude was slurried with methyl tert-butyl ether (10 mL) at room temperature for half an hour and then filtered, and the filter cake was dried to obtain A224-4 (1.22 g, yield 64%).

### Synthesis of compound A224-6

A224-4 (440 mg, 1.21 mmol), A224-5 (536 mg, 1.21 mmol) and DIPEA (942 mg, 7.28 mmol) were dissolved in 5 mL of DMF. Under ice bath, HATU (554 mg, 1.45 mmol) was added in batches. The mixture was reacted at room temperature for 2 h, and then TLC showed that the reaction was completed. The reaction was quenched with ice water (10 mL). The aqueous phase was extracted with ethyl acetate (20 ml*3). The organic phases were combined, washed with saturated brine (15 ml*3), dried over anhydrous sodium sulfate and dried by rotary evaporation. The solute was separated by column chromatography (mobile phase: dichloromethane : methanol = 30 : 1 - 15 : 1) to obtain compound A224-6 (500 mg, yield 60%).

### Synthesis of compound A224

A224-6 (500 mg, 0.73 mmol) and benzenesulfonic acid (287 mg, 1.81 mmol) were dissolved in 5 mL of acetonitrile. The mixture was reacted overnight at 60°C, and then TLC showed that the reaction was completed. The mixture was cooled to room temperature. The solvent was dried by rotary evaporation, and the solute was purified by medium-pressure preparative chromatography to obtain compound A224 (93 mg, yield 12%).

The synthesis method for compound A225 is the same as that for A224

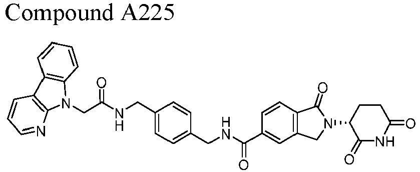

ESI-MS(M+H)⁺: 615.2

NMR (400 MHz, DMSO) δ 11.01 (s, 1H), 9.22 (t, *J* = 5.9 Hz, 1H), 8.74 (t, *J* = 6.0 Hz, 1H), 8.54 (dd, *J* = 7.6, 1.5 Hz, 1H), 8.45 (dd, *J* = 4.8, 1.5 Hz, 1H), 8.21 (d, *J* = 7.7 Hz, 1H), 8.09 (s, 1H), 8.01 (d, *J* = 8.1 Hz, 1H), 7.82 (d, *J* = 7.9 Hz, 1H), 7.56 (d, *J* = 8.2 Hz, 1H), 7.49 (t, *J* = 7.1 Hz, 1H), 7.32 - 7.22 (m, 6H), 5.20 - 5.10 (m, 3H), 4.55 - 4.37 (m, 4H), 4.28 (d, *J* = 5.8 Hz, 2H), 2.97 - 2.85 (m, 1H), 2.69 - 2.56 (m, 1H), 2.46 - 2.35 (m, 1H), 2.08 - 1.99 (m, 1H).

### Example 27

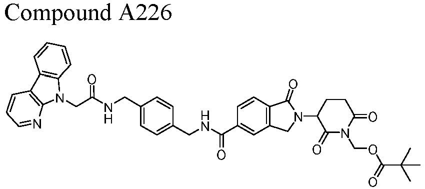

ESI-MS(M+H)⁺: 729.2

¹H NMR (400 MHz, DMSO) δ 9.21 (t, *J* = 6.0 Hz, 1H), 8.74 (t, *J* = 5.9 Hz, 1H), 8.54 (dd, *J* = 7.6, 1.6 Hz, 1H), 8.44 (dd, *J* = 4.8, 1.6 Hz, 1H), 8.20 (d, *J* = 7.7 Hz, 1H), 8.10 (s, 1H), 8.02 (d, *J* = 8.0 Hz, 1H), 7.84 (d, *J* = 7.9 Hz, 1H), 7.56 (d, *J* = 8.2 Hz, 1H), 7.53 - 7.45 (m, 1H), 7.32 - 7.21 (m, 6H), 5.63 (q, *J* = 9.6 Hz, 2H), 5.33 (dd, *J* = 13.3, 5.1 Hz, 1H), 5.16 (s, 2H), 4.56 (d, *J* = 17.5 Hz, 1H), 4.48 (d, *J* = 5.8 Hz, 2H), 4.36 (d, *J* = 17.5 Hz, 1H), 4.28 (d, *J* = 5.9 Hz, 2H), 3.16 - 3.05 (m, 1H), 2.91 - 2.80 (m, 1H), 2.47 - 2.35 (m, 1H), 2.14 - 2.05 (m, 1H), 1.11 (s, 9H).

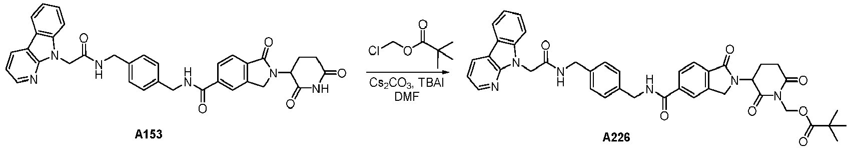

### Synthesis of compound A226

A153 (300 mg, 0.49 mmol), cesium carbonate (81 mg, 0.54 mmol) and tetrabutylammonium iodide (90 mg, 0.24 mmol) were dissolved in 6 mL of DMF. Chloromethyl pivalate (81 mg, 0.54 mmol) was added, and the mixture was reacted overnight at room temperature. TLC showed that the reaction was completed. The reaction was quenched with ice water (10 mL). The aqueous phase was extracted with ethyl acetate (20 ml*3). The organic phases were combined, washed with saturated brine (15 ml*3), dried over anhydrous sodium sulfate and dried by rotary evaporation. The solute was purified by column chromatography (mobile phase: dichloromethane : methanol = 10 : 1) to obtain A226 (80 mg, yield 22%).

### Example 28

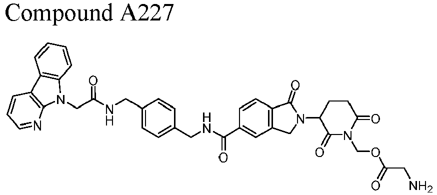

ESI-MS(M+H)⁺: 702.2

¹H NMR (400 MHz, DMSO) δ 9.23 (t, *J* = 5.9 Hz, 1H), 8.75 (t, *J* = 5.8 Hz, 1H), 8.54 (dd, *J* = 7.6, 1.6 Hz, 1H), 8.45 (dd, *J* = 4.8, 1.6 Hz, 1H), 8.21 (d, *J* = 7.7 Hz, 1H), 8.11 (s, 1H), 8.03 (d, *J* = 8.0 Hz, 1H), 7.84 (d, *J* = 8.0 Hz, 1H), 7.56 (d, *J* = 8.2 Hz, 1H), 7.53 - 7.47 (m, 1H), 7.33 - 7.21 (m, 6H), 5.78 (dd, *J* = 26.9, 9.6 Hz, 2H), 5.30 (dd, *J* = 13.4, 5.0 Hz, 1H), 5.16 (s, 2H), 4.59 - 4.43 (m, 4H), 4.28 (d, *J* = 5.8 Hz, 2H), 3.80 (s, 2H), 3.17 - 3.06 (m, 1H), 2.92 - 2.83 (m, 1H), 2.45 - 2.40 (m, 1H), 2.16 - 2.07 (m, 1H).

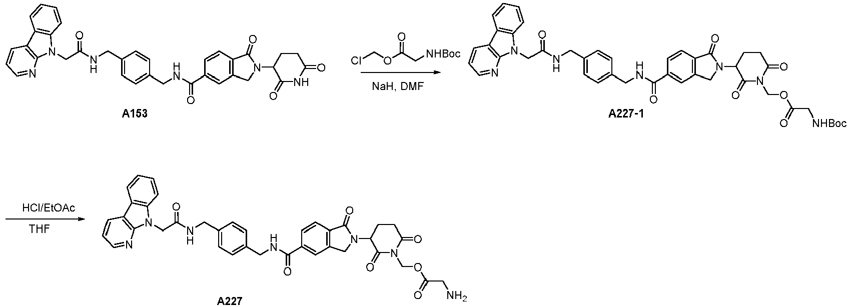

### Synthesis of compound A227-1

A153 (300 mg, 0.49 mmol) was dissolved in 4 mL of DMF. At 0°C, NaH (24 mg, 60%, 0.60 mmol) was added. The mixture was reacted for 10 min, and then N-boc-glycine chloromethyl ester (130 mg, 0.59 mmol) was added. After the addition was completed, the mixture was warmed to room temperature and reacted for 2 h. TLC showed that the reaction was completed. The reaction was quenched with ice water (10 mL). The aqueous phase was extracted with ethyl acetate (20 ml*3). The organic phases were combined, washed with saturated brine (15 ml*3), dried over anhydrous sodium sulfate and dried by rotary evaporation. The solute was purified by column chromatography (mobile phase: dichloromethane : methanol = 10 : 1) to obtain A227-1 (41 mg, yield 10%). Synthesis of compound A227

A227-1 (41 mg, 0.05 mmol) was dissolved in 4 mL of THF, and HCl/EtOAc (2 M, 2 mL) was added. The mixture was reacted at room temperature for 2 h, and then TLC showed that the reaction was completed. The solution was dried by rotary evaporation, and the solute was purified by medium-pressure preparative chromatography to obtain A227 (25 mg, yield 68%).
The synthesis method for compound A228 is the same as that for A227

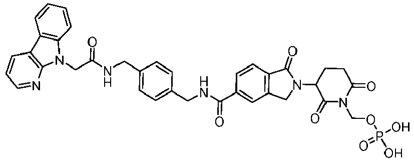

ESI-MS(M+H)⁺: 725.2

¹H NMR (400 MHz, DMSO) δ 9.23 (t, *J* = 5.9 Hz, 1H), 8.73 (t, *J* = 5.9 Hz, 1H), 8.54 (dd, *J* = 7.6, 1.5 Hz, 1H), 8.44 (dd, *J* = 4.8, 1.4 Hz, 1H), 8.20 (d, *J* = 7.8 Hz, 1H), 8.09 (s, 1H), 8.00 (d, *J* = 7.9 Hz, 1H), 7.83 (d, *J* = 7.9 Hz, 1H), 7.56 (d, *J* = 8.2 Hz, 1H), 7.51 - 7.47 (m, 1H), 7.34 - 7.16 (m, 6H), 5.48 - 5.29 (m, 1H), 5.29 - 5.21 (m, 1H), 5.15 (s, 2H), 4.60 - 4.37 (m, 4H), 4.27 (d, *J* = 5.8 Hz, 2H), 3.05 - 2.98 (m, 1H), 2.85 - 2.73 (m, 1H), 2.44 - 2.35 (m, 1H), 2.05 - 1.95 (m, 1H).

### Activity test examples

### I. Activity test 1: Ability of compounds to degrade c-Myc protein in HL60 cells:

### 1. Cell plating

HL60 cells in the exponential growth phase were centrifuged, diluted with fresh culture mediums to 6 × 10⁴ cells/100 µL and plated into a 96-well plate at 100 µL per well.

### 2. Drug addition to cells

Preparation of test compounds: the compounds were weighed and diluted with DMSO to a 10 mM stock solution for later use. According to test requirements, drugs were diluted with cell culture mediums, with ten-fold dilutions starting from 10 µM, to achieve 3 concentration gradients: 10 µM, 1 µM and 100 nM; and finally, 1 µL of the drugs (DMSO content: 0.01%) were added to each well. For the blank control group, DMSO was also diluted with cell culture mediums, and finally, 1 µL of the control reagent (DMSO content: 0.01%) was added to each well.

### 3. Effect of drugs on c-Myc in cells as determined by ELISA

After 24 h of drug action, the culture mediums were removed, and the cells were washed once with ice-cold 1X PBS. PBS was removed, and 100 µL of ice-cold 1X Cell Lysis Buffer plus 1 mM phenylmethylsulfonyl fluoride (PMSF) was added to each well. The plate was incubated on ice for 20 min and centrifuged at 4000 RPM at 4°C for 10 min for later use.

### 4. Reagent and antibody preparation in ELISA kit

### 1X wash buffer

1). 16 mL of ELISA wash buffer concentrate (25X) was diluted with 384 mL of deionized water or distilled water, labeled as 1X Wash Buffer.
2). The concentrate and 1X Wash Buffer were stored in the refrigerator. The diluted buffer was used within 14 days.

### Cell extraction buffer

The cell extraction buffer contains 10 mM Tris (pH 7.4), 100 mM NaCl, 1 mM EDTA, 1 mM EGTA, 1 mM NaF, 20 mM Na₄P₂O₇, 2 mM Na₃VO₄, 1% Triton^{™}X-100, 10% glycerol, 0.1% SDS and 0.5% deoxycholate. Immediately before use, PMSF (0.3 M stock solution in DMSO) was added to the buffer to a final concentration of 1 mM.

Standard curve configuration: The standard liquid was diluted using glass or plastic tubes.
1). Huc-Myc (total) standard liquid (Invitrogen, KHO2041) was reconstituted with a standard dilution to 1000 pg/mL. Refer to the standard vial label for instructions. The contents were gently swirled or mixed and then allowed to stand for 10 minutes to ensure complete reconstitution, labeled as 1,000 pg/mL human c-Myc. The standard liquid was used within 1 hour after reconstitution.
2). 250 µL of standard dilution buffers were added to 7 test tubes respectively labeled 500, 250, 125, 62.5, 31.25, 15.63 and 0 pg/mL.
3). 250 µL of the 1000 pg/ml standard stock solution was added to the first EP tube to a concentration of 500 pg/mL, vortexed and mixed evenly. 250 µL of the resulting mixture was further pipetted and added to the second EP tube to a concentration of 250 pg/mL. Serial dilutions were performed until the sixth EP tube with a concentration of 15.63 pg/mL. Dilution was not performed on the last EP tube.
4). The remaining reconstituted standard liquid should be discarded, or aliquoted and frozen at -80°C for further use. Standards can be frozen and thawed only once without losing immunoreactivity.

1X anti-rabbit IgG HRP solution: 1X anti-rabbit IgG HRP solution was prepared within 15 minutes of use. The anti-rabbit IgG HRP (100X) contained 50% viscous glycerol. To ensure accurate dilutions, the following operations were carried out: 1. For each 8-well test strip used in the test, 10 µL of the anti-rabbit IgG HRP (100X) solution was pipetted, wherein the pipette tip was wiped with clean absorbent paper to remove excess solutions, and then the solution was dispensed into a test tube containing 1 mL of HRP diluent and mixed thoroughly. 2. The unused anti-rabbit IgG HRP (100X) was placed in the refrigerator for storage.

### 5. ELISA

5.1
50 µL of the standard liquid was added to the appropriate wells and not added to the blank control well. Except for the blank control well, 50 µL of Hu c-Myc (total) detection antibody solution was added to each well and mixed by tapping the side face of the plate. The plate was covered with a lid and incubated at room temperature for 3 hours. The solution was aspirated thoroughly, and the wells were washed 4 times with 1X Wash Buffer.

5.2
Except for the blank control well, 100 µL of 1X anti-rabbit IgG HRP solution was added to each well. The plate was covered with a lid and incubated at room temperature for 30 minutes. The solution was aspirated thoroughly, and the wells were washed 4 times with 1X Wash Buffer.

5.3
100 µL of Stabilized Chromogen was added to each well. The substrate solution turned blue. The plate was incubated in the dark at room temperature for 30 minutes.

5.4
100 µL of stop solution was added to each well and mixed by tapping the side face of the plate. The solution in the wells changed from blue to yellow and was detected. 6. Result analysis:

"Tsample" represented the data obtained from test results; "Blank" represented the data from the blank group (culture medium group without adding cells); and "Control" represented the data from the control group (cell group only with DMSO and without adding drugs). Degradation rate% = (1-(Tsample-Blank)/(Control-Blank)) * 100%.

### II. Activity test 2: Anti-proliferation ability of compounds on HL60 cells:

### 1. Cell plating

HL60 cells in the logarithmic growth phase were counted and plated evenly in a 96-well transparent bottom white plate at 20000 cells per well and 100 µL per well.

### 2. Drug addition to cells

A certain amount of the compounds was weighed and diluted with DMSO to a 10 mM stock solution. The culture mediums corresponding to the cells were taken, and the drugs were diluted to the concentration required by the test, shaken gently and mixed evenly. 50 µL of the gradient dilution of the diluted compound was added to each well of the plated cells, and 50 µL of 0.1% DMSO was added to the control group. The plate was shaken gently, mixed evenly and incubated in a cell culture incubator for 3 days.

### 3. CTG detection

3.1 The CellTiter-Glo^{™} (CTG) reagent was taken in advance from the -20°C refrigerator and equilibrated to room temperature in the dark (white bottle+brown bottle, 1:1 mixing evenly into the brown bottle, for later use).

3.2 The 96-well plate was taken out and inspected under a microscope for growth situation.

3.3 The bottom of the 96-well plate was tagged with white paper, and then the plate was wrapped with tin foil paper, shaken at 37°C at 300 r for 10 min and mixed evenly.

3.4 50 ul of CTG detection reagent was added to each well, Luminescence signal was detected using a microplate reader, and the inhibition rate was calculated. IC₅₀ values of the compounds were calculated by XLfit software to evaluate the proliferation inhibitory activity on HL60 cells.

The activity test results were as shown in Table 1.

**Table 1 Activity test results**

| Compound | HL60 cells Protein degradation rate (1 µM)¹ | HL60 cells Proliferation inhibitory activity (nM) | Compound | HL60 cells Protein degradation rate (1 µM)¹ | HL60 cells Proliferation inhibitory activity (nM) |
|---|---|---|---|---|---|
| A1 | B | 334.9 ± 83.1 | A115 | A | 8.6 ± 1.8 |
| A2 | B | 50.7 ± 11.4 | A116 | A | 5.5 ± 3.3 |
| A3 | B | 145.1.9 ± 75.7 | A117 | A | 13.8 ± 4.0 |
| A4 | C | 220.7 ± 54.5 | A118 | A | 18.2 ± 11.4 |
| A5 | B | 391.3 ± 4.7 | A119 | A | 44.4 ± 0.3 |
| A6 | C | 3587 ± 2223 | A120 | A | 17.8 ± 4.9 |
| A7 | B | 319.6 ± 38.0 | A121 | A | 6.3 ± 3.0 |
| A8 | B | 69.8 ± 8.8 | A122 | A | 5.9 ± 3.9 |
| A9 | B | 569.7 ± 38.0 | A123 | A | 35.2 ± 9.3 |
| A10 | C | 1213 ± 4 | A124 | C | 1104 ± 295 |
| A11 | C | 1235 ± 490 | A125 | A | 26.1 ± 2.0 |
| A12 | B | 1057 ± 62 | A126 | A | 8.5 ± 0.4 |
| A13 | C | 1272 ± 51 | A127 | A | 3.6 ± 0.1 |
| A14 | B | 225.5 ± 72.0 | A128 | C | 2138 ± 788 |
| A15 | A | 16.3 ± 3.3 | A129 | A | 36.1 ± 13.3 |
| A16 | A | 21.2 ± 6.1 | A130 | A | 4.7 ± 2.3 |
| A17 | B | 3589 ± 1181 | A131 | A | 112.4 ± 16.5 |
| A18 | B | 1223 ± 217 | A132 | C | 4210 ± 659 |
| A19 | A | 584.9 ± 79.8 | A133 | C | 5020 ± 23 |
| A20 | A | 490.0 ± 64.9 | A134 | A | 334.4 ± 83.5 |
| A21 | C | 177.9 ± 68.4 | A135 | B | 1706 ± 120 |
| A22 | B | 29.2 ± 14.4 | A136 | A | 211.1 ± 45.9 |
| A23 | B | 55.4 ± 6.7 | A137 | A | 239.1 ± 35.0 |
| A24 | B | 103.1 ± 53.2 | A138 | A | 12.4 ± 4.4 |
| A25 | B | 270.4 ± 126.0 | A139 | A | 22.5 ± 14.3 |
| A26 | B | 2821 ± 1064 | A140 | A | 11.0 ± 1.7 |
| A27 | C | 6805 ± 1536 | A141 | A | 6.5 ± 0.3 |
| A28 | A | 5055 ± 137 | A142 | A | 6.1 ± 0.9 |
| A29 | A | 30.1 ± 21.1 | A143 | A | 22.9 ± 3.0 |
| A30 | A | 18.0 ± 11.5 | A144 | A | 18.4 ± 7.4 |
| A31 | A | 1362 | A145 | A | 117.7 ± 1.1 |
| A32 | A | 433.0 ± 165.8 | A146 | A | 14.7 ± 4.0 |
| A33 | A | 9.1 ± 0.3 | A147 | A | 16.7 ± 9.5 |
| A34 | A | 18.0 ± 6.0 | A148 | A | 12.0 ± 8.1 |
| A35 | A | 10.9 ± 4.8 | A149 | A | 7.6 ± 3.0 |
| A36 | B | 13.4 ± 3.3 | A150 | A | 7.2 ± 1.0 |
| A37 | B | 313.6 | A151 | A | 11.3 ± 4.7 |
| A38 | B | 66.2 ± 14.6 | A152 | A | 0.6 ± 0.3 |
| A39 | A | 978.7 ± 80.2 | A153 | A | 0.5 ± 0.3 |
| A40 | A | 306.1 ± 73.8 | A154 | A | 1.2 ± 0.1 |
| A41 | A | 154.6 ± 8.9 | A155 | A | 3.0 ± 0.6 |
| A42 | A | 3641 ± 895 | A156 | A | <3 |
| A43 | A | 282.5 ± 27.5 | A157 | A | 0.5 ± 0.1 |
| A44 | B | 5400 ± 683 | A158 | B | 3.3 ± 0.3 |
| A45 | A | 61.6 ± 21.0 | A159 | A | 2.2 ± 0.8 |
| A46 | A | 50.2 ± 14.0 | A160 | A | 0.4 ± 0.3 |
| A47 | A | 1044 ± 171 | A161 | A | 0.9 ± 0.1 |
| A48 | A | 3579 ± 29 | A162 | A | 1.9 ± 0.2 |
| A49 | A | 6815 ± 1628 | A163 | A | 1.3 ± 0.3 |
| A50 | C | 10400 ± 235 | A164 | A | 2.4 ± 0.3 |
| A51 | A | 335.9 ± 30.0 | A165 | A | 27.9 ± 19.5 |
| A52 | B | 8060 ± 637 | A166 | A | 1.7 ± 0.3 |
| A53 | A | 1452 ± 280 | A167 | A | 2.1 ± 1.1 |
| A54 | A | 2326 ± 518 | A168 | A | 2.6 ± 0.7 |
| A55 | A | 181.1 ± 32.8 | A169 | A | 1.9 ± 0.6 |
| A56 | A | 666.4 ± 60.2 | A170 | A | 4.0 ± 1.7 |
| A57 | A | 214.2 ±4.7 | A171 | A | 0.3 ± 0.2 |
| A58 | A | 285.2 ± 10.7 | A172 | A | <3 |
| A59 | A | 172.8 ± 61.3 | A173 | A | <3 |
| A60 | C | 48.5 ± 6.0 | A174 | A | <3 |
| A61 | A | 108.3 ± 6.5 | A175 | A | 14.4 ± 6.4 |
| A62 | A | 320.0 ± 17.6 | A176 | A | 2.1 ± 0.6 |
| A63 | A | 702.1 ± 33.8 | A177 | A | 9.5 |
| A64 | A | 2021 | A178 | A | 16.9 |
| A65 | A | 472.7 ± 93.3 | A179 | A | 14.0 ± 4.1 |
| A66 | A | 1847 ± 1226 | A180 | A | 133.3 ± 48.4 |
| A67 | A | 1067 ± 617 | A181 | A | 2.0 ± 1.0 |
| A68 | A | 1000 ± 480 | A182 | A | 1.1 ± 0.8 |
| A69 | A | 709.6 ± 40.1 | A183 | A | 16.7 ± 2.5 |
| A70 | A | 373.8 ± 51.1 | A184 | A | 11.9 ± 5.5 |
| A71 | A | 24.3 ± 8.5 | A185 | A | 7.5 ± 1.1 |
| A72 | A | 45.6 ± 3.0 | A186 | A | 32.1 ± 1.3 |
| A73 | A | 59.3 ± 5.7 | A187 | A | 9.2 ± 0.5 |
| A74 | A | 5.5 ± 2.3 | A188 | B | 279.0 ± 33.8 |
| A75 | A | 2733 ± 614 | A189 | A | 5.6 ± 1.0 |
| A76 | A | 431.6 ± 138.3 | A190 | A | 1.7 ± 0.6 |
| A77 | A | 73.4 | A191 | A | 16.9 ± 2.2 |
| A78 | A | 4.6 | A192 | A | 7.7 ± 2.1 |
| A79 | A | 21.7 | A193 | A | 0.6 ± 0.1 |
| A80 | A | <3 | A194 | A | 0.14 ± 0.01 |
| A81 | A | <3 | A195 | A | 1.4 ± 0.01 |
| A82 | A | <3 | A196 | A | 0.5 ± 0.1 |
| A83 | B | 27.2 ± 7.6 | A197 | / | 6.4 ±1.3 |
| A84 | A | 13.1 ± 6.2 | A198 | / | 17.8 ± 1.9 |
| A85 | A | 21.3 ± 10.9 | A199 | A | 3.3 ± 0.6 |
| A86 | A | 24.4 ± 10.4 | A200 | A | 2.5 ± 1.0 |
| A87 | A | 11.5 | A201 | A | 2.0 ± 0.9 |
| A88 | A | 83.1 | A202 | / | 6.4 ± 2.7 |
| A89 | A | 81.1 ± 36.9 | A203 | A | 6.6 ± 0.8 |
| A90 | A | <3 | A204 | / | 1.7 ± 0.7 |
| A91 | A | 12.0 ± 4.4 | A205 | A | 0.2 ± 0.1 |
| A92 | A | 440.0 ± 120.5 | A206 | / | 0.5 ± 0.2 |
| A93 | A | 170.6 ± 60.4 | A207 | / | 0.2 |
| A94 | A | 289.3 ± 48.3 | A208 | A | 0.5 |
| A95 | A | 43.6 ± 44.0 | A209 | / | 0.9 |
| A96 | A | 960 ± 121 | A210 | / | 3.6 ± 1.8 |
| A97 | A | 169.4 ± 50.7 | A211 | / | 13.3 ± 1 |
| A98 | A | 11.2 ± 1.3 | A212 | / | 1.5 ± 0.1 |
| A99 | A | 19.8 ± 4.8 | A213 | / | 5.4 ± 1.9 |
| A100 | A | 0.9 | A214 | / | 5.2 ± 1.4 |
| A101 | A | 0.4 | A215 | / | 186 ± 67 |
| A102 | A | 5.4 | A216 | / | 3.5 ± 1.1 |
| A103 | A | 5.8 ± 2.5 | A217 | / | 0.6 |
| A104 | A | 2.5 ± 0.7 | A218 | / | 3.3 ± 0.8 |
| A105 | A | 8.3 ± 1.9 | A219 | / | 7.8 ± 2.0 |
| A106 | A | 4.2 | A220 | A | 1.2 ± 0.1 |
| A107 | A | 3.4 | A221 | A | 4.9 ± 0.8 |
| A108 | A | 2.7 ± 1.8 | A222 | / | 0.6 ± 0.2 |
| A109 | A | 5.5 ± 4.2 | A223 | / | 0.7 ± 0.4 |
| A110 | A | 4.4 | A224 | A | 1.7 ± 1.3 |
| A111 | A | 57.6 ± 25.9 | A225 | A | 1.3 ± 0.2 |
| A112 | A | 3.0 ± 0.1 | A226 | / | 1 |
| A113 | A | 4.4 ± 3.2 | A227 | B | 0.6 ± 0.1 |
| A114 | A | 6.5 ± 0.3 | A228 | A | 5.0 ± 0.2 |
| A229 | / | < 3.04 | A230 | / | 11.8 ± 1.2 |
| A231 | / | < 3.04 | A232 | / | 0.54 |
| A233 | / | < 3.04 | A234 | / | 0.71 ± 0.04 |
| A235 | / | / | A236 | / | 6.3 ± 1.2 |

| | | | | | |
|---|---|---|---|---|---|
| 1: Activity data A represents the degradation rate of 60%-90%, B represents the degradation rate of 30%-60%, and C represents the degradation rate of 10%-30%. "j" represents that the corresponding test has not been performed. | | | | | |

### III. Activity test 3: Test of the ability of compounds (taking A153 as an example) to degrade tumorigenic proteins in various tumor cells:

### 1. Test materials

Cell culture conditions: All cells in the examples of the present invention were purchased from the Shanghai Cell Bank of the Chinese Academy of Sciences. Among them, human acute promyelocytic leukemia cells (HL60 cells) were cultured in the culture medium containing RPMI-1640 (meilunbio, MA0215) + 20% FBS (Manufacturer: BI, Cat. No. 04-001-1ACS)+1% penicillin/streptomycin (Manufacturer: Hyclone, Cat. No. SH40003.01). The small cell lung cancer cells (H69 cells), human B lymphoma cells (Ramos cells), MM1S myeloma cells (MM1S cells) and human prostate cancer cells (22RV1 cells) were cultured in the culture medium containing RPMI-1640+10% FBS +1% penicillin/streptomycin. The above-mentioned cells were all cultured at 5% CO₂ and 37% humidity. When the coverage rate reached about 80%, the cells were passaged in a ratio of 1 : 3.

### 2. Cell drug treatment and protein extraction

### 2.1 Cell treatment

The designated cells in the exponential growth phase were digested and plated into a 6-well plate at 1 × 10⁶ cells per well. After one day of growth, the cells were cultured by adding the compounds of the present invention. After 24 h of drug treatment, the protein was extracted.

### 2.2 Cell protein extraction

In the 6-well plate, the culture mediums were removed, and the cells were washed once with PBS, digested with trypsin, and centrifuged and collected separately into 1.5 mL centrifuge tubes. 100 µL of RIPA lysis buffer (containing 100 µM PMSF) was added to each tube, mixed thoroughly, allowed to stand on ice for 30 min and then centrifuged at 12000 rpm at 4°C using a centrifuge for 20 min. The supernatant was used for Western blotting (WB) experiments. The sample can be stored at -80°C.

### 2.3 Determination of protein concentration

By using the BCA protein concentration determination kit (from Thermo Fisher, Cat. No. 23225), the BSA standard determination solution and the supernatant in 2.2 to be tested (the supernatant can be diluted and then tested) were prepared according to the table below. Samples were added to the 96-well plate. Each well was supplemented with PBS to 20 µl, and then 200 µl of BCA working solution (prepared according to the kit) was added respectively and mixed evenly. The plate was placed at 60°C for 10 min, and then the absorbance at 562 nm was detected. After the reading was recorded, a standard curve was plotted using concentration gradients of the standards, and the absorbance of the samples was substituted to calculate the protein concentration of the samples.

**Table A Preparation of protein quantitative standards**

| No. | 0 | 1 | 2 | 3 | 4 | 5 | 6 | 7 | 8 |
|---|---|---|---|---|---|---|---|---|---|
| | 1 mg/ml BSA standard solution (µl) | | | | | | | 5 mg/ml BSA standard solution (µl) | |
| BSA standard solution (µl) | 0 | 0.5 | 2.5 | 5.0 | 10 | 15 | 20 | 6 | 8 |
| PBS solution (µl) | 20 | 19.5 | 17.5 | 15 | 10 | 5 | 0 | 14 | 12 |
| BSA final concentration | 0 | 25 | 125 | 250 | 500 | 750 | 1000 | 1500 | 2000 |
| (µg/ml) | | | | | | | | | |
| Total volume (µl) | 20 µl | | | | | | | | |

### 3 Standard procedure for Western blotting experiment

3.1 Protein denaturation: RIPA protein buffer (Solarbio, Cat. No. R0010) and 5× Loading Buffer (SDS, glycerol, bromophenol blue, TRIS) (purchased from Solarbio, Cat. No. P1040) were added, and the protein samples were denatured at 100°C for 5 min.

3.2 Sample loading and electrophoresis: 10% 15-well precast gel (Genscript, Cat. No. M00666) and the corresponding electrophoresis buffer (Genscript, Cat. No. M00138) were used. The same mass of protein sample and protein ladder (purchased from thermo, Cat. No. 26617) were loaded into each well, and electrophoresis at 200 V was performed for 30 min.

3.3 Blocking: The gel was excised to remove the excess part. Transferring to PVDF membrane (Millipore, Cat. No. ISEQ00010) was performed by the wet transfer method (before use, it was necessary to activate the PVDF membrane with methanol for 1 min) at 300 mA for 2 h, during which a lot of heat was generated, and thus cooling with an ice box was required.

3.4 Blocking: After the transferring, the PVDF membrane was placed in 5% (w/w) skim milk and blocked with shaking at room temperature for 1 h.

3.5 Incubating with primary antibody: The PVDF membrane was cut according to the molecular weight indicated on the marker and placed in c-myc (purchased from abcam, Cat. No. ab32072), N-myc (purchased from abcam, Cat. No. ab24193), GSPT1 (purchased from abcam, Cat. No. ab234433), CK1α (purchased from abcam, Cat. No. ab206652), IKZF2 (purchased from proteintech, Cat. No. 13554-1-AP), β-actin (purchased from CST, Cat. No. #3700), GAPDH (purchased from CST, Cat. No. #97166) primary antibodies. The antibodies were diluted with TBST buffer (a solution prepared from TRIS, KCL and NaCl and adjusted to pH 7.4 with hydrochloric acid, and Tween 20 was added) at a ratio of 1 : 1000, and blocked overnight on a shaker at 4°C.

3.6 Incubating with secondary antibody: After incubated with the primary antibodies, the PVDF membrane was washed 3 times with TBST on a shaker, 10 min each time. After washed, the membrane was respectively placed in anti-mouse IgG, HRP-linked antibody (purchased from CST, Cat. No. #7076) or anti-rabbit IgG, HRP-linked antibody (purchased from CST, Cat. No. #7074) and incubated on a shaker at room temperature for 2 h.

3.7 Washing membrane and exposure: After incubated with the secondary antibodies, the membrane was washed 3 times with TBST on a shaker, 10 minutes each time. After washed, the membrane was subjected to enhanced chemiluminescence (ECL) for visualizing the intensity of chemoluminescence.

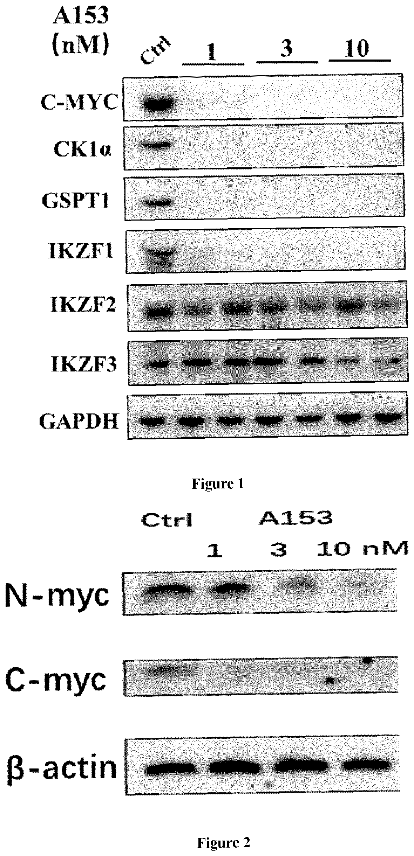

According to the above method, the ability to degrade proteins in the above cells was tested respectively, and the results were as shown in FIGs. 1-3. In the figures, all Crtl groups represent DMSO blank groups without adding the compounds to be tested.

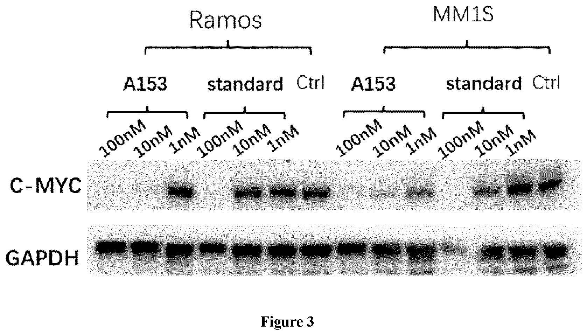

It can be seen from the test results that the compounds of the present invention have strong ability to degrade various proteins in various cells (HL60, H69, Ramos, MM1S and 22RV1). The test results in FIG. 1 show that (in HL60 cell system), the compounds of the present invention can achieve the degradation of c-Myc, CK1α, GSPT1, IKZF1, IKZF2 and IKZF3 at nM (i.e., 1 nmol/L), and can achieve basically complete degradation of c-Myc, CK1α, GSPT1 and IKZF1 at 1 nM (low concentration). The test results in FIG. 2 show that using different experimental verifications (for β-actin and GAPDH), the compounds of the present invention can achieve basically complete degradation of c-Myc in HL60 cells at 1 nM, and can also achieve basically complete degradation of N-myc at 10 nM. The test results in FIG. 3 show that the compounds of the present invention have better degradation ability than other c-Myc inhibitors (the standard in FIG. 3 is the A10 molecule in patent WO 2021004391 A1).

In summary, the compounds of the present invention have excellent ability of degrading tumorigenic proteins in various tumor cells, for example, degrading any one or more of proteins comprising c-Myc, N-myc, GSPT1, CK1α, IKZF (1/2/3), AR, AR-V7, etc., and therefore can be used to prevent, alleviate or treat diseases related to high expression of any one (e.g., c-Myc) or more of the above proteins (c-Myc), such as cancer, cardiovascular and cerebrovascular diseases and viral infection-related diseases. The compounds have precise and significant effects on degrading c-Myc, N-myc, GSPT1, CK1α, IKZF (1/2/3), AR and AR-V7 proteins.

The content of the present invention merely exemplifies some specific embodiments set forth thereby, and the technical features recited in one or more technical solutions may be combined with any one or more technical solutions, wherein the technical solutions obtained by combination also fall within the scope of protection of the present application, as if the technical solutions obtained by combination have been specifically recited in the content disclosed by the present invention.

## Claims

1. A compound represented by formula (I), and a pharmaceutically acceptable salt, solvent compound, stereoisomer, isotope and prodrug thereof: 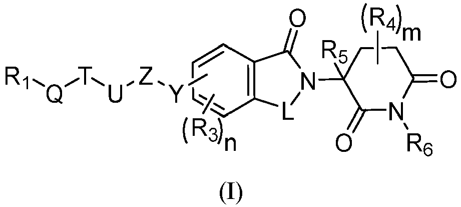 wherein,
R₁ is selected from RₐC(=O)-, RₐCH₂C(=O)-, RₐCH₂CH₂C(=O)-, Rₐ-, RₐCH₂-, RₐCH₂CH₂-, RₐNHC(=O)-, RₐCH₂NHC(=O)-, RₐCH₂CH₂NHC(=O)-, RₐOC(=O)-, RₐCH₂OC(=O)-, RₐCH₂CH₂OC(=O)-, RₐS(=O)₂-, RₐCH₂S(=O)₂- or RₐCH₂CH₂S(=O)₂-, wherein the "CH₂" is optionally substituted with one or more C₁-C₄ alkyl, or is optionally substituted with -CH₂CH₂-, and the "NH" is optionally substituted with R_{b}-, R_{b}C(=O)- or R_{b}S(=O)₂-;
Q is selected from -NR₂- or -O-;
R₂ is selected from hydrogen, R_{b}-, R_{b}C(=O)- or R_{b}S(=O)₂-;
Rₐ and R_{b} are each independently selected from C₁-C₈ alkyl, C₃-C₁₀ cycloalkyl, a C₃-C₁₀ bridged cyclic group, -NR₁₁R₁₂, C₃-C₁₀ heterocyclyl optionally containing O, S, SO₂, N or NHC(=O)R₂₂, aryl, heteroaryl, fused arylcycloalkyl, fused heteroarylheterocyclyl, arylcycloalkyl, aryl-heterocyclyl, cycloalkyl-heterocyclyl or heterocyclyl-heterocyclyl, wherein a group selected from one of -O-, -S-, -C(=O)-, -S(=O)-, -S(=O)₂-, -NH-, - NHC(=O)-, -NHC(=O)NH- or -NHS(=O)₂- may be inserted between any two C-C of the C₁-C₈ alkyl, and Rₐ or R_{b} may be optionally substituted with one or more R₉;
T, U and Z are each independently selected from a chemical bond, carbonyl, C₁-C₆ alkylene, C₃-C₁₀ cycloalkylene, arylene, heteroarylene or heterocyclylene, wherein the alkylene, cycloalkylene, arylene, heteroarylene or heterocyclylene may be optionally substituted with one or more R₉;
Y is selected from a chemical bond, -NHC(=O)-, -C(=O)NH-, -C(=O)NHCH₂-, - NHC(=O)CH₂-, -O-, -OCH₂-, -OCH₂CH₂-, -NH-, -NHCH₂- or -NHCH₂CH₂-, wherein the "CH₂" is optionally substituted with one or more C₁-C₄ alkyl, or is optionally substituted with -CH₂CH₂-, and the "NH" is optionally substituted with R_{b}-, R_{b}C(=O)- or R_{b}S(=O)₂-;
L is selected from -(CR₇R₈)ₒ- or -C(=O)-;
R₃-R₅ and R₇-R₉ are each independently selected from hydrogen, halogen, hydroxyl, cyano, amino, nitro, -R₂₁NHC(=O)R₂₂, -R₂₁C(=O)OR₂₂, C₁-C₆ alkyl, C₁-C₆ alkoxy, C₁-C₆ alkylthio, C₁-C₆ alkylamino, C₃-C₈ cycloalkyl, C₂-C₈ alkenyl, C₂-C₈ alkynyl, aryl, 5- to 6-membered heteroaryl containing 1-3 heteroatoms or 3- to 10-membered heterocyclyl containing 1-3 heteroatoms, wherein the alkyl, alkoxy, alkylamino, alkylthio, cycloalkyl, aryl, heteroaryl or heterocyclyl is optionally substituted with 1-3 groups each selected from halogen, cyano, C₁-C₃ alkyl or C₁-C₃ alkoxy; when there are multiple R₃-R₅ and R₇-R₉, any two adjacent ones may be combined to form a ring;
R₆ is selected from hydrogen, C₁-C₆ alkyl, C₃-C₈ cycloalkyl, C₂-C₈ alkenyl, C₂-C₈ alkynyl, aryl, 5- to 6-membered heteroaryl containing 1-3 heteroatoms or 3- to 10-membered heterocyclyl containing 1-3 heteroatoms, wherein the alkyl, cycloalkyl, aryl, heteroaryl or heterocyclyl is optionally substituted with 1-3 groups each selected from halogen, hydroxyl, cyano, amino, nitro, -C(=O)OR₂₃, -OC(=O)R₂₃, -NHC(=O)R₂₃, - C(=O)NHR₂₃, C₁-C₃ alkyl, C₁-C₃ alkoxy or -OP(=O)(OM)₂;
M is independently selected from hydrogen or C₁-C₄ alkyl;
R₁₁ and R₁₂ are each independently selected from hydrogen, C₁-C₄ alkyl or aryl;
R₂₁ is selected from C₁-C₄ alkyl;
R₂₂ is selected from hydrogen, amino, C₁-C₄ alkyl, C₁-C₄ alkoxy, allyl or benzyl, wherein the C₁-C₄ alkyl, C₁-C₄ alkoxy, allyl or benzyl is optionally substituted with aryl or -C(=O)OR₂₁;
R₂₃ is selected from hydrogen or C₁-C₄ alkyl, wherein the C₁-C₄ alkyl is optionally substituted with 1-3 groups each selected from halogen, hydroxyl or amino;
n is selected from 0, 1, 2 or 3;
m is selected from 0, 1, 2, 3 or 4;
o is selected from 1 or 2.

2. A compound represented by formula (I), and a pharmaceutically acceptable salt, solvent compound, stereoisomer, isotope and prodrug thereof: 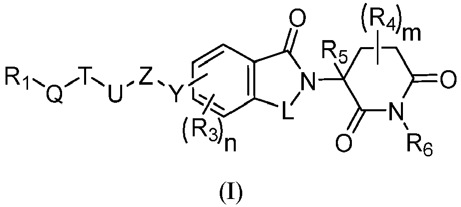 wherein,
R₁ is selected from RₐC(=O)-, RₐCH₂C(=O)-, RₐCH₂CH₂C(=O)-, Rₐ-, RₐCH₂-, RₐCH₂CH₂-, RaNHC(=O)-, RₐCH₂NHC(=O)-, RₐCH₂CH₂NHC(=O)-, RaOC(=O)-, RₐCH₂OC(=O)-, RₐCH₂CH₂OC(=O)-, RₐS(=O)₂-, RₐCH₂S(=O)₂- or RₐCH₂CH₂S(=O)₂-, wherein the "CH₂" is optionally substituted with one or more C₁-C₄ alkyl, or is optionally substituted with -CH₂CH₂-, and the "NH" is optionally substituted with C₁-C₄ alkyl;
Q is selected from -NR₂- or -O-;
R₂ is selected from hydrogen, R_{b}-, R_{b}C(=O)- or R_{b}S(=O)₂-;
Rₐ and R_{b} are each independently selected from C₁-C₈ alkyl, C₃-C₁₀ cycloalkyl, a C₃-C₁₀ bridged cyclic group, -NR₁₁R₁₂, C₃-C₁₀ heterocyclyl optionally containing O, S, SO₂, N or NHC(=O)R₂₂, aryl, heteroaryl, fused arylcycloalkyl, fused heteroarylheterocyclyl, arylcycloalkyl, aryl-heterocyclyl, cycloalkyl-heterocyclyl or heterocyclyl-heterocyclyl, and Rₐ or R_{b} may be optionally substituted with one or more R₉;
T, U and Z are each independently selected from a chemical bond, carbonyl, C₁-C₆ alkylene, C₃-C₁₀ cycloalkylene, arylene, heteroarylene or heterocyclylene, wherein the alkylene, cycloalkylene, arylene, heteroarylene or heterocyclylene may be optionally substituted with one or more R₉;
Y is selected from a chemical bond, -NHC(=O)-, -C(=O)NH-, -C(=O)NHCH₂-, - NHC(=O)CH₂-, -O-, -OCH₂-, -OCH₂CH₂-, -NH-, -NHCH₂- or -NHCH₂CH₂-, wherein the "CH₂" is optionally substituted with one or more C₁-C₄ alkyl, or is optionally substituted with -CH₂CH₂-, and the "NH" is optionally substituted with C₁-C₄ alkyl;
L is selected from -(CR₇R₈)ₒ- or -C(=O)-;
R₃-R₅ and R₇-R₉ are each independently selected from hydrogen, halogen, hydroxyl, cyano, amino, -R₂₁NHC(=O)R₂₂, -R₂₁C(=O)OR₂₂, C₁-C₆ alkyl, C₁-C₆ alkoxy, C₁-C₆ alkylthio, C₁-C₆ alkylamino, C₃-C₈ cycloalkyl, C₂-C₈ alkenyl, C₂-C₈ alkynyl, aryl, 5- to 6-membered heteroaryl containing 1-3 heteroatoms or 3- to 10-membered heterocyclyl containing 1-3 heteroatoms, wherein the alkyl, alkoxy, alkylamino, alkylthio, cycloalkyl, aryl, heteroaryl or heterocyclyl is optionally substituted with 1-3 groups each selected from halogen, cyano, C₁-C₃ alkyl or C₁-C₃ alkoxy; when there are multiple R₃-R₅ and R₇-R₉, any two adjacent ones may be combined to form a ring;
R₆ is selected from hydrogen, C₁-C₆ alkyl, C₃-C₈ cycloalkyl, C₂-C₈ alkenyl, C₂-C₈ alkynyl, aryl, 5- to 6-membered heteroaryl containing 1-3 heteroatoms or 3- to 10-membered heterocyclyl containing 1-3 heteroatoms, wherein the alkyl, cycloalkyl, aryl, heteroaryl or heterocyclyl is optionally substituted with 1-3 groups each selected from halogen, cyano, C₁-C₃ alkyl or C₁-C₃ alkoxy;
R₁₁ and R₁₂ are each independently selected from hydrogen, C₁-C₄ alkyl or aryl;
R₂₁ is selected from C₁-C₄ alkyl;
R₂₂ is selected from hydrogen, amino, C₁-C₄ alkyl, C₁-C₄ alkoxy, allyl or benzyl, wherein the C₁-C₄ alkyl, C₁-C₄ alkoxy, allyl or benzyl is optionally substituted with aryl or -C(=O)OR₂₁;
n is selected from 0, 1, 2 or 3;
m is selected from 0, 1, 2, 3 or 4;
o is selected from 1 or 2.

3. The compound and the pharmaceutically acceptable salt, solvent compound, stereoisomer, isotope and prodrug thereof according to claim 1 or 2, wherein, the R₁ is selected from RₐC(=O)-, RₐCH₂C(=O)-, RₐCH₂CH₂C(=O)-, Rₐ-, RₐCH₂-, RₐCH₂CH₂-, RₐNHC(=O)-, RₐCH₂NHC(=O)-, RₐCH₂CH₂NHC(=O)-, RₐOC(=O)- or RₐCH₂OC(=O)- or RₐCH₂CH₂OC(=O)-, wherein the "CH₂" is optionally substituted with one or more C₁-C₄ alkyl, or is optionally substituted with -CH₂CH₂-, the "NH" is optionally substituted with R_{b}-, R_{b}C(=O)- or R_{b}S(=O)₂-, the R_{b} is selected from C₁-C₄ alkyl, the C₁-C₄ alkyl is optionally substituted with one or more R₉, the Rₐ is selected from C₁-C₈ alkyl, -NR₁₁R₁₂, aryl or heteroaryl, and Rₐ may be optionally substituted with one or more R₉;
preferably, the R₁ is selected from RₐC(=O)-, RₐCH₂C(=O)-, RₐCH₂CH₂C(=O)-, Rₐ-, RₐCH₂-, RₐCH₂CH₂-, RₐNHC(=O)-, RₐCH₂NHC(=O)-, RₐCH₂CH₂NHC(=O)-, RₐOC(=O)- or RₐCH₂OC(=O)-, or RₐCH₂CH₂OC(=O)-, wherein the "CH₂" is optionally substituted with one or more C₁-C₄ alkyl, or is optionally substituted with -CH₂CH₂-, the "NH" is optionally substituted with C₁-C₄ alkyl, the Rₐ is selected from C₁-C₈ alkyl, -NR₁₁R₁₂, aryl or heteroaryl, and Rₐ may be optionally substituted with one or more R₉.

4. The compound and the pharmaceutically acceptable salt, solvent compound, stereoisomer, isotope and prodrug thereof according to any one of claims 1-3, wherein, the Rₐ is selected from C₂-C₆ alkyl, carbazolyl, 1-azacarbazolyl, 2-azacarbazolyl, 1,8-diazacarbazolyl, indolyl, phenoxazinyl, naphthyl, fluorenyl, diphenylamino, dibenzylamino, tert-butyl, quinolyl, isoquinolyl, phenyl, 2,3-indolinyl, 7-azaindolyl, 2,3-dihydro-7-azaindolyl, naphthyridinyl, tetrahydronaphthyridinyl, tetrahydroquinolyl, pyrimidyl or triazolyl, and Rₐ may be optionally substituted with one or more R₉, wherein the R₉ is selected from hydrogen, halogen, C₁-C₆ alkoxy, cyano, C₁-C₆ alkyl, or C₁-C₆ alkyl, phenyl or naphthyl optionally substituted with 1-3 groups selected from halogen or hydroxyl;
preferably, the Rₐ is selected from C₂-C₆ alkyl, carbazolyl, 1-azacarbazolyl, 2-azacarbazolyl, 1,8-diazacarbazolyl, indolyl, phenoxazinyl, naphthyl, fluorenyl, diphenylamino, dibenzylamino, tert-butyl, quinolyl, isoquinolyl or phenyl, and Rₐ may be optionally substituted with one or more R₉, wherein the R₉ is selected from hydrogen, halogen, C₁-C₆ alkoxy, cyano, C₁-C₆ alkyl, or C₁-C₆ alkyl optionally substituted with 1-3 groups selected from halogen.

5. The compound and the pharmaceutically acceptable salt, solvent compound, stereoisomer, isotope and prodrug thereof according to any one of claims 1-4, wherein, the Rₐ is selected from tert-butyl, carbazol-1-yl, 1-azacarbazol-9-yl, 2-azacarbazol-9-yl, 1,8-diazacarbazol-9-yl, indol-1-yl, phenoxazin-10-yl, naphthalen-1-yl, naphthalen-2-yl, fluoren-9-yl, diphenylamino, dibenzylamino, tert-butyl, quinolin-4-yl, quinolin-5-yl, quinolin-8-yl, isoquinolin-1-yl, isoquinolin-4-yl, isoquinolin-5-yl, isoquinolin-8-yl, phenyl, 2,3-dihydro-indol-1-yl, 7-azaindol-1-yl, 2,3-dihydro-7-azaindol-1-yl, 1,2,3,4-tetrahydro-1,8-naphthyridin-1-yl, 1,2,3,4-tetrahydroquinolin-1-yl, pyrimidin-2-yl, pyrimidin-4-yl, pyrimidin-5-yl, 1,2,3-triazol-1-yl, 1,2,4-triazol-1-yl or 1,3,4-triazol-1-yl, and Rₐ may be optionally substituted with one or more R₉, wherein the R₉ is selected from F, Cl, Br, methoxy, ethoxy, cyano, methyl, ethyl, trifluoromethyl, hydroxymethyl, phenyl, naphthalen-1-yl or naphthalen-2-yl;
preferably, the Rₐ is selected from tert-butyl, carbazol-1-yl, 1-azacarbazol-9-yl, 2-azacarbazol-9-yl, 1,8-diazacarbazol-9-yl, indol-1-yl, phenoxazin-10-yl, naphthalen-1-yl, naphthalen-2-yl, fluoren-9-yl, diphenylamino, dibenzylamino, tert-butyl, quinolin-4-yl, quinolin-5-yl, quinolin-8-yl, isoquinolin-1-yl, isoquinolin-4-yl, isoquinolin-5-yl, isoquinolin-8-yl or phenyl, and Rₐ may be optionally substituted with one or more R₉, wherein the R₉ is selected from F, Cl, Br, methoxy, ethoxy, cyano, methyl, ethyl or trifluoromethyl.

6. The compound and the pharmaceutically acceptable salt, solvent compound, stereoisomer, isotope and prodrug thereof according to any one of claims 1-5, wherein, the Rₐ is selected from the following groups: 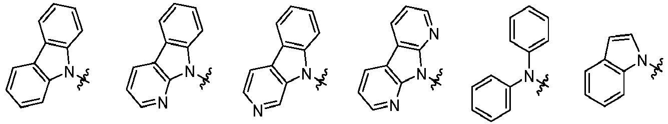 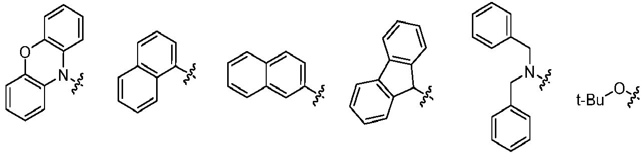 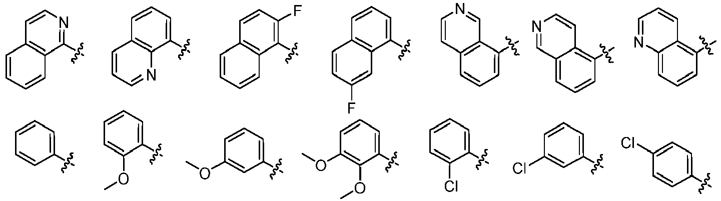 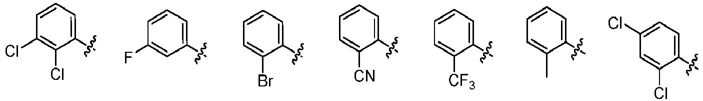 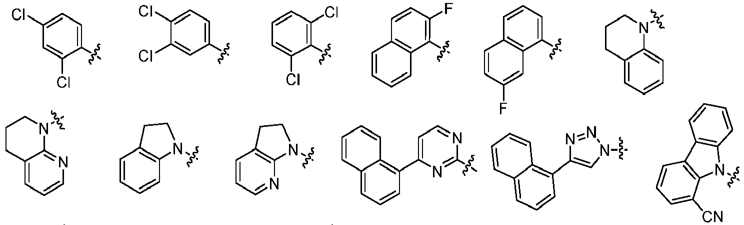 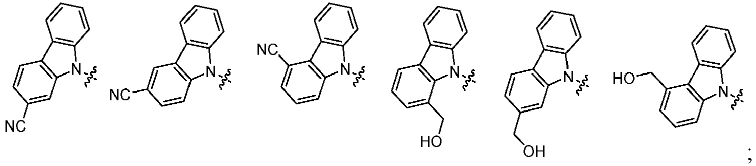 preferably, the Rₐ is selected from the following groups:

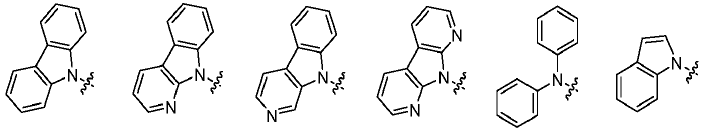

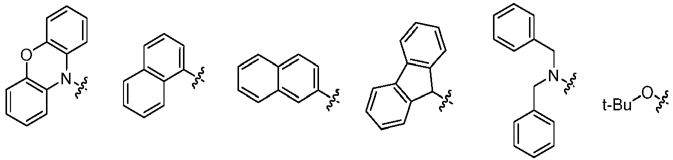

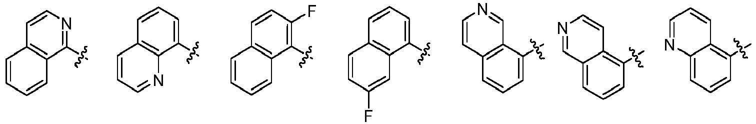

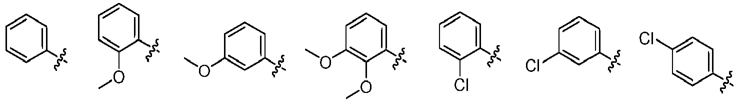

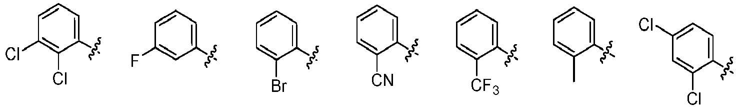

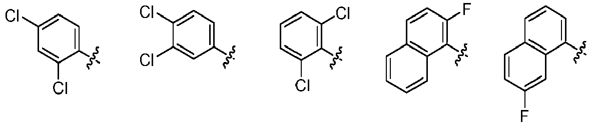

7. The compound and the pharmaceutically acceptable salt, solvent compound, stereoisomer, isotope and prodrug thereof according to claim 1, wherein, the Q is selected from -NR₂-; the R₂ is selected from hydrogen, C₁-C₈ alkyl, C₃-C₁₀ cycloalkyl, aryl, C₁-C₈ alkyl C(=O)- or C₁-C₈ alkyl S(=O)₂-, wherein the C₁-C₈ alkyl, C₃-C₁₀ cycloalkyl, aryl and the alkyl portion of the C₁-C₈ alkyl C(=O)- or C₁-C₈ alkyl S(=O)₂- are optionally substituted with one or more R₉, and the R₉ is selected from hydrogen, halogen, hydroxyl, amino, cyano, carboxyl, C₁-C₆ alkyl, C₁-C₆ alkoxy or aryl;
preferably, the Q is selected from -NR₂-; the R₂ is selected from hydrogen, C₁-C₈ alkyl, C₃-C₁₀ cycloalkyl, aryl, C₁-C₈ alkyl C(=O)- or C₁-C₈ alkyl S(=O)₂-, wherein the C₁-C₈ alkyl, C₃-C₁₀ cycloalkyl, aryl and the alkyl portion of the C₁-C₈ alkyl C(=O)- or C₁-C₈ alkyl S(=O)₂- are optionally substituted with one or more R₉, and the R₉ is selected from hydrogen, halogen, cyano, C₁-C₆ alkyl, C₁-C₆ alkoxy or aryl.

8. The compound and the pharmaceutically acceptable salt, solvent compound, stereoisomer, isotope and prodrug thereof according to claim 1, wherein, the Q is selected from -NH-, -O-, -N(CH₃)-, -N(SO₂CH₃)-, -N(COCH₃)-, -N(CO-isopropyl)-, -N(CO-cyclopropyl)-, -N(isopropyl)-, -N(cyclopropyl)-, -N(2-methoxyethyl)-, -N(2-cyanoethyl)-, - N(phenyl)-, -N(benzyl)-, -N(1-naphthylmethyl)-, -N(2-naphthylethyl)-, -N(CH₂OH)-, - N(CH₂CH₂OH)-, -N(CH₂CH₂CH₂OH)-, -N(COCH₂OH)-, -N(COCH₂CH₂OH)-, - N(COCH₂CH₂CH₂OH)-, -N(CH₂NH₂)-, -N(CH₂CH₂NH₂)-, -N(COCH₂CH₂CH₂NH₂)-, - N(COCH₂NH₂)-, -N(COCH₂CH₂NH₂)- or -N(COCH₂CH₂CH₂NH₂)-;
preferably, the Q is selected from -NH-, -O-, -N(CH₃)-, -N(SO₂CH₃)-, -N(COCH₃)-, - N(CO-isopropyl)-, -N(CO-cyclopropyl)-, -N(isopropyl)-, -N(cyclopropyl)-, -N(2-methoxyethyl)-, -N(2-cyanoethyl)-, -N(phenyl)- or -N(benzyl)-.

9. The compound and the pharmaceutically acceptable salt, solvent compound, stereoisomer, isotope and prodrug thereof according to claim 1, wherein, the T and Z are each independently selected from a chemical bond, carbonyl, C₁-C₆ alkylene or C₃-C₁₀ cycloalkylene, wherein the C₁-C₆ alkylene or C₃-C₁₀ cycloalkylene may be optionally substituted with one or more R₉, wherein the R₉ is selected from hydrogen, C₁-C₆ alkyl or C₃-C₈ cycloalkyl; preferably, the T and Z are each independently selected from a chemical bond, carbonyl, methylene, 1,2-ethylene, 1,1-cyclopropylene or 2,2-propylene.

10. The compound and the pharmaceutically acceptable salt, solvent compound, stereoisomer, isotope and prodrug thereof according to claim 1, wherein, the U is selected from C₁-C₆ alkylene, C₃-C₁₀ cycloalkylene, arylene or heteroarylene, wherein the alkylene, cycloalkylene, arylene or heteroarylene may be optionally substituted with one or more R₉; preferably, the U is selected from C₂-C₆ alkylene, C₅-C₆ cycloalkylene, C₆-C₁₀ arylene or 5- to 6-membered monocyclic heteroarylene, wherein the alkylene, cycloalkylene, arylene or heteroarylene may be optionally substituted with one or more R₉.

11. The compound and the pharmaceutically acceptable salt, solvent compound, stereoisomer, isotope and prodrug thereof according to claim 1 or 10, wherein, the U is selected from 1,2-ethylene, 1,3-propylene, 1,4-butylene, 1,5-pentylene, 1,6-hexylene, 1,3-cyclopentylene, 1,3-cyclohexylene, 1,4-cyclohexylene, 1,3-phenylene, 1,4-phenylene, 2,5-pyridylene, 2,5-pyrimidinylene, 2,5-thiazolylene or 2,4-thiazolylene, wherein the 1,3-phenylene, 1,4-phenylene, 2,5-pyridylene, 2,5-pyrimidinylene, 2,5-thiazolylene or 2,4-thiazolylene may be optionally substituted with one or more R₉.

12. The compound and the pharmaceutically acceptable salt, solvent compound, stereoisomer, isotope and prodrug thereof according to claim 10 or 11, wherein, the R₉ is selected from hydrogen, halogen, cyano, C₁-C₆ alkyl or C₁-C₆ alkoxy; preferably, the R₉ is selected from hydrogen, halogen, C₁-C₆ alkyl or C₁-C₆ alkoxy; also preferably, the R₉ is selected from F, Cl, Br, cyano, methyl, methoxy or trifluoromethyl; most preferably, the R₉ is selected from F, Cl, Br, methyl, methoxy or trifluoromethyl.

13. The compound and the pharmaceutically acceptable salt, solvent compound, stereoisomer, isotope and prodrug thereof according to any one of claims 1 and 10-12, wherein, the U is selected from the following groups: 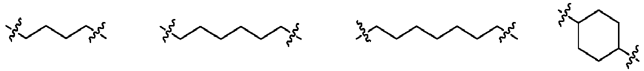 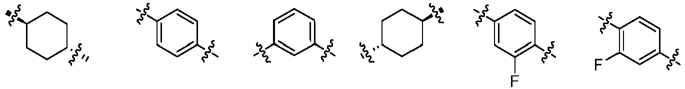 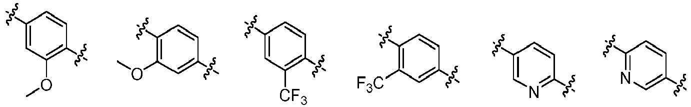 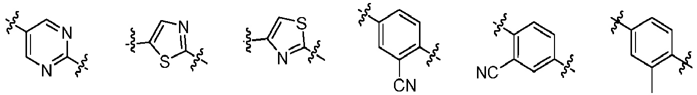 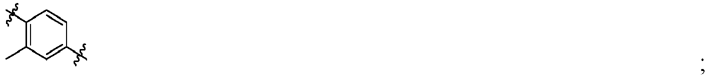 preferably, the U is selected from the following groups:

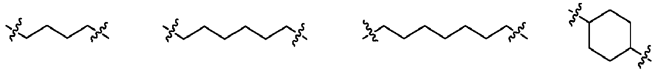

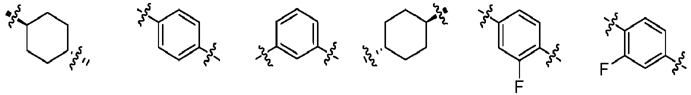

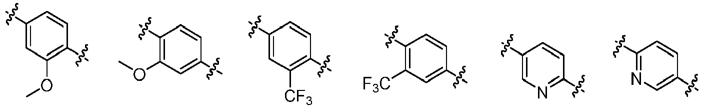

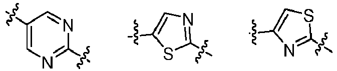

14. The compound and the pharmaceutically acceptable salt, solvent compound, stereoisomer, isotope and prodrug thereof according to any one of claims 1 and 9-13, wherein, the -T-U-Z- together form a group selected from: 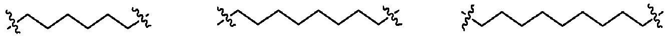 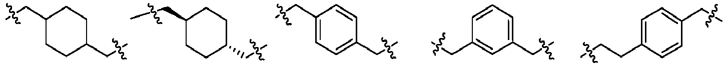 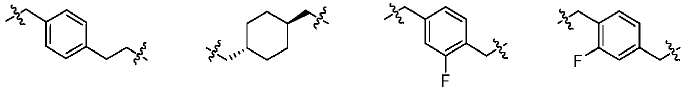 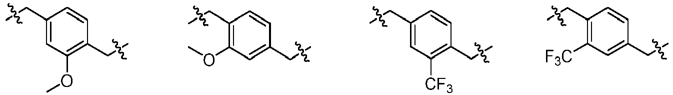 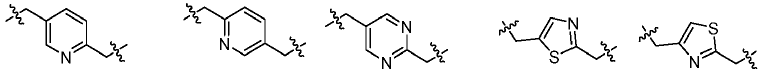 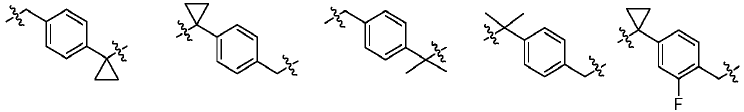 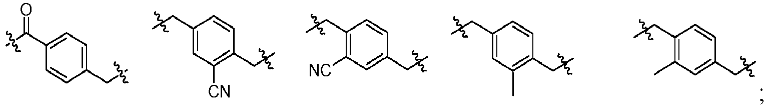 preferably, the -T-U-Z- together form a group selected from:

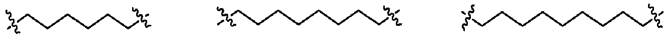

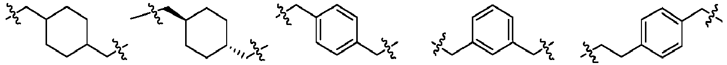

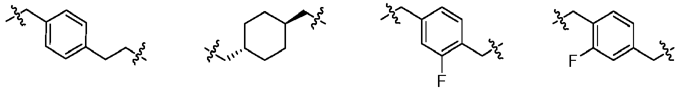

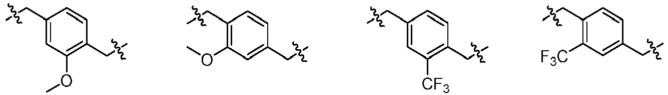

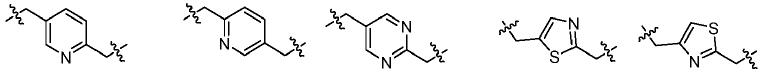

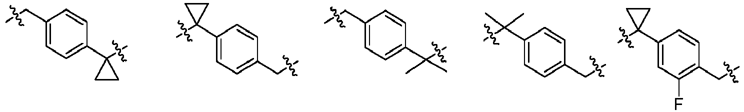

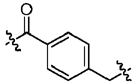

15. The compound and the pharmaceutically acceptable salt, solvent compound, stereoisomer, isotope and prodrug thereof according to claim 1, wherein, the Y is selected from a chemical bond, -NHC(=O)-, -C(=O)NH-, -C(=O)NHCH₂-, -NHC(=O)CH₂-, -O-, - OCH₂-, -OCH₂CH₂-, -NH-, -NHCH₂- or -N(CH₃)CH₂-, wherein the "NH" is optionally substituted with R_{b}-, R_{b}C(=O)- or R_{b}S(=O)₂-, the R_{b} is selected from C₁-C₄ alkyl, and the C₁-C₄ alkyl is optionally substituted with one or more R₉;
preferably, the Y is selected from a chemical bond, -NHC(=O)-, -C(=O)NH-, - C(=O)NHCH₂-, -NHC(=O)CH₂-, -O-, -OCH₂-, -OCH₂CH₂-, -NH-, -NHCH₂-, - N(CH₃)CH₂-, -N(CH₂OH)CH₂-, -N(CH₂CH₂OH)CH₂-, -N(CH₂CH₂CH₂OH)CH₂-, - N(COCH₂OH)CH₂-, -N(COCH₂CH₂OH)CH₂-, -N(COCH₂CH₂CH₂OH)CH₂-, - N(CH₂NH₂)CH₂-, -N(CH₂CH₂NH₂)CH₂-, -N(COCH₂CH₂CH₂NH₂)CH₂-, - N(COCH₂NH₂)CH₂-, -N(COCH₂CH₂NH₂)CH₂- or -N(COCH₂CH₂CH₂NH₂)CH₂-;
more preferably, the Y is selected from a chemical bond, -NHC(=O)-, -C(=O)NH-, - C(=O)NHCH₂-, -NHC(=O)CH₂-, -O-, -OCH₂-, -OCH₂CH₂-, -NH-, -NHCH₂- or - N(CH₃)CH₂-; more preferably, the Y is selected from -NHC(=O)-, -C(=O)NH- or - NHCH₂-.

16. The compound and the pharmaceutically acceptable salt, solvent compound, stereoisomer, isotope and prodrug thereof according to claim 1, wherein, the L is selected from -CH₂-, -CH₂CH₂- or -C(=O)-; preferably, the L is selected from -CH₂- or -C(=O)-.

17. The compound and the pharmaceutically acceptable salt, solvent compound, stereoisomer, isotope and prodrug thereof according to claim 1, wherein, the R₆ is selected from hydrogen, C₁-C₆ alkyl or C₃-C₈ cycloalkyl, wherein the alkyl or cycloalkyl is optionally substituted with 1-3 groups each selected from halogen, hydroxyl, cyano, amino, -C(=O)OR₂₃, -OC(=O)R₂₃, -NHC(=O)R₂₃, -C(=O)NHR₂₃ or -OP(=O)(OM)₂.

18. The compound and the pharmaceutically acceptable salt, solvent compound, stereoisomer, isotope and prodrug thereof according to claim 1, wherein, the R₆ is selected from hydrogen, C₁-C₄ alkyl, C₁-C₄ haloalkyl, -CH₂OC(=O)R₂₃, -CH₂CH₂OC(=O)R₂₃, - CH₂C(=O)OR₂₃, -CH₂CH₂C(=O)OR₂₃, -CH₂OP(=O)(OH)₂ or -CH₂CH₂OP(=O)(OH)₂; R₂₃ is selected from hydrogen or C₁-C₄ alkyl, wherein the C₁-C₄ alkyl is optionally substituted with 1-3 groups each selected from hydroxyl or amino.

19. The compound and the pharmaceutically acceptable salt, solvent compound, stereoisomer, isotope and prodrug thereof according to any one of the preceding claims, wherein, the compound represented by formula (I) is a compound represented by formula (I-1) or formula (I-2): 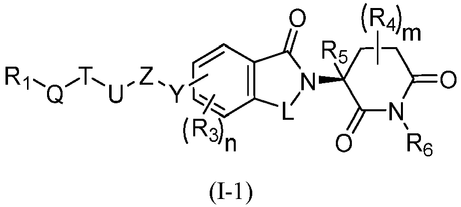 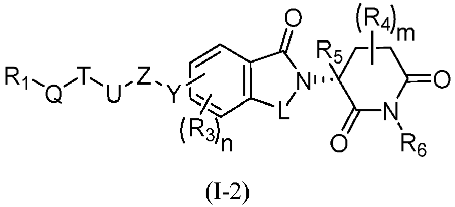 wherein R₁, R₃-R₆, Q, T, U, Z, Y, L, n and m are defined as above.

20. The compound and the pharmaceutically acceptable salt, solvent compound, stereoisomer, isotope and prodrug thereof according to any one of the preceding claims, wherein, the compound represented by formula (I) is a compound represented by formula (II) or formula (III): 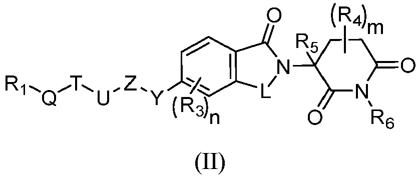 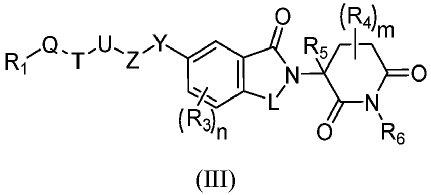 wherein R₁, R₃-R₆, Q, T, U, Z, Y, L, n and m are defined as above.

21. The compound and the pharmaceutically acceptable salt, solvent compound, stereoisomer, isotope and prodrug thereof according to any one of the preceding claims, wherein, the compound represented by formula (I) is a compound represented by formula (IV) or formula (V): 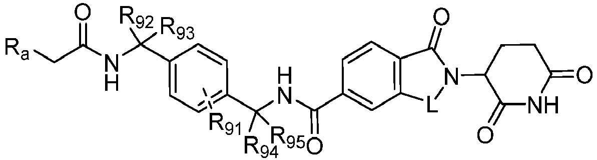 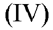 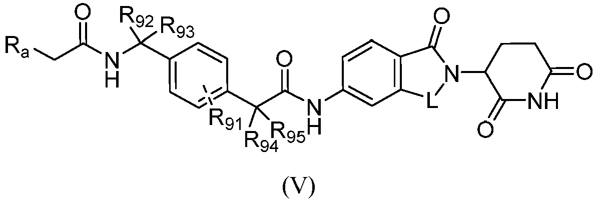
wherein the "NH" in the RₐCH₂C(=O)NH-, -C(R₉₄)(R₉₅)NHC(=O)- or - C(R₉₄)(R₉₅)C(=O)NH- is optionally substituted with R_{b}-, R_{b}C(=O)- or R_{b}S(=O)₂-;
Rₐ, R_{b} and L are defined as above;
R₉₁-R₉₅ are each independently selected from hydrogen, halogen, hydroxyl, cyano, amino, -R₂₁NHC(=O)R₂₂, -R₂₁C(=O)OR₂₂, C₁-C₆ alkyl, C₁-C₆ alkoxy, C₁-C₆ alkylthio, C₁-C₆ alkylamino, C₃-C₈ cycloalkyl, C₂-C₈ alkenyl, C₂-C₈ alkynyl, aryl, 5- to 6-membered heteroaryl containing 1-3 heteroatoms or 3- to 10-membered heterocyclyl containing 1-3 heteroatoms, wherein the alkyl, alkoxy, alkylamino, alkylthio, cycloalkyl, aryl, heteroaryl or heterocyclyl is optionally substituted with 1-3 groups each selected from halogen, cyano, C₁-C₃ alkyl or C₁-C₃ alkoxy; any two adjacent ones of R₉₂-R₉₄ may be combined to form a ring.

22. The compound and the pharmaceutically acceptable salt, solvent compound, stereoisomer, isotope and prodrug thereof according to claim 21, wherein, the R₉₁ is selected from hydrogen, halogen, hydroxyl, cyano, amino, C₁-C₆ alkyl, C₁-C₆ alkoxy, C₁-C₆ alkylthio, C₁-C₆ alkylamino, C₃-C₈ cycloalkyl, aryl, 5- to 6-membered heteroaryl containing 1-3 heteroatoms or 3- to 10-membered heterocyclyl containing 1-3 heteroatoms, wherein the alkyl, alkoxy, alkylamino, alkylthio, cycloalkyl, aryl, heteroaryl or heterocyclyl is optionally substituted with 1-3 groups each selected from halogen, cyano, C₁-C₃ alkyl or C₁-C₃ alkoxy; the R₉₂-R₉₄ are each independently selected from hydrogen or C₁-C₆ alkyl, or any two adjacent ones of R₉₂-R₉₄ may be combined to form cycloalkyl; preferably, the R₉₂-R₉₄ are each independently selected from hydrogen, methyl or ethyl, or R₉₂ and R₉₃ together form -CH₂CH₂-, or R₉₄ and R₉₅ together form -CH₂CH₂-.

23. The compound and the pharmaceutically acceptable salt, solvent compound, stereoisomer, isotope and prodrug thereof according to any one of claims 1-19, wherein, the compound represented by formula (I) is a compound represented by formula (IV') or formula (V'): 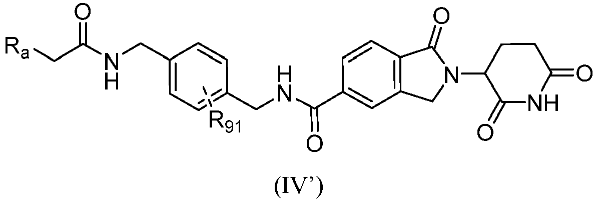 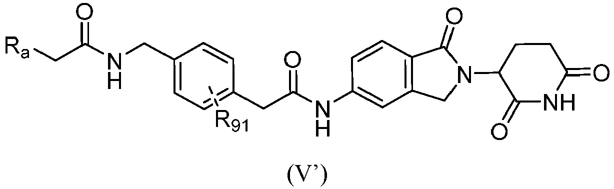
wherein the "NH" in the RₐCH₂C(=O)NH-, -CH₂NHC(=O)- or -CH₂C(=O)NH- is optionally substituted with R_{b}-, R_{b}C(=O)- or R_{b}S(=O)₂-;
Rₐ, R_{b} and R₉₁ are defined as above.

24. The compound and the pharmaceutically acceptable salt, solvent compound, stereoisomer, isotope and prodrug thereof according to any one of claims 1-19, wherein, the compound represented by formula (I) is a compound represented by formula (VI): 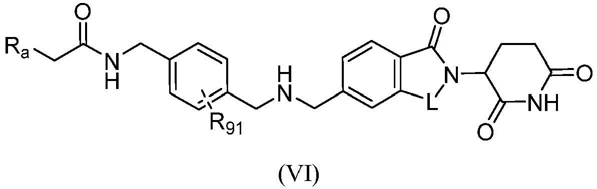
wherein the "NH" in the RₐCH₂C(=O)NH- or -CH₂NHCH₂- is optionally substituted with R_{b}-, R_{b}C(=O)- or R_{b}S(=O)₂-;
Rₐ, R_{b}, R₉₁ and L are defined as above.

25. The compound and the pharmaceutically acceptable salt, solvent compound, stereoisomer, isotope and prodrug thereof according to any one of claims 1-19, wherein, the compound represented by formula (I) is a compound represented by formula (VI'): 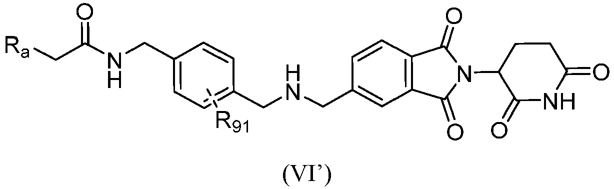
wherein the "NH" in the RₐCH₂C(=O)NH- or -CH₂NHCH₂- is optionally substituted with R_{b}-, R_{b}C(=O)- or R_{b}S(=O)₂-;
Rₐ, R_{b} and R₉₁ are defined as above.

26. The compound and the pharmaceutically acceptable salt, solvent compound, stereoisomer, isotope and prodrug thereof according to any one of claims 1-19, wherein, the compound represented by formula (I) is a compound represented by formula (VII): 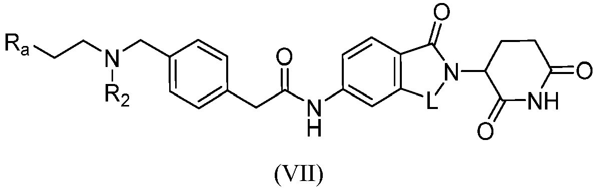
wherein the "NH" in the -CH₂C(=O)NH- is optionally substituted with R_{b}-, R_{b}C(=O)- or R_{b}S(=O)₂-;
Rₐ, R_{b}, R₂ and L are defined as above.

27. The compound and the pharmaceutically acceptable salt, solvent compound, stereoisomer, isotope and prodrug thereof according to claim 26, wherein, the R₂ is selected from hydrogen, C₁-C₈ alkyl, C₃-C₁₀ cycloalkyl, aryl, C₁-C₈ alkyl C(=O)- or C₁-C₈ alkyl S(=O)₂-, wherein the C₁-C₈ alkyl, C₃-C₁₀ cycloalkyl, aryl and the alkyl portion of the C₁-C₈ alkyl C(=O)- or C₁-C₈ alkyl S(=O)₂- are optionally substituted with one or more R₉, and the R₉ is selected from hydrogen, halogen, hydroxyl, amino, cyano, C₁-C₆ alkyl, C₁-C₆ alkoxy or aryl; preferably, the R₂ is selected from hydrogen, methyl, ethyl, isopropyl, cyclopropyl, -SO₂CH₃, -CO cyclopropyl, 2-methoxyethyl, 2-cyanoethyl, 2-hydroxyethyl, 2-aminoethyl, phenyl, benzyl, 1-naphthylmethyl or 2-naphthylethyl; preferably, the R₂ is selected from hydrogen, C₁-C₈ alkyl, C₃-C₁₀ cycloalkyl, aryl, C₁-C₈ alkyl C(=O)- or C₁-C₈ alkyl S(=O)₂-, wherein the C₁-C₈ alkyl, C₃-C₁₀ cycloalkyl, aryl and the alkyl portion of the C₁-C₈ alkyl C(=O)- or C₁-C₈ alkyl S(=O)₂- are optionally substituted with one or more R₉, and the R₉ is selected from hydrogen, halogen, cyano, C₁-C₆ alkyl, C₁-C₆ alkoxy or aryl; preferably, the R₂ is selected from hydrogen, methyl, ethyl, isopropyl, cyclopropyl, - SO₂CH₃, -CO cyclopropyl, 2-methoxyethyl, 2-cyanoethyl, phenyl or benzyl.

28. The compound and the pharmaceutically acceptable salt, solvent compound, stereoisomer, isotope and prodrug thereof according to any one of claims 1-19, wherein, the compound represented by formula (I) is a compound represented by formula (VII') and a pharmaceutically acceptable salt, solvent compound, stereoisomer, isotope and prodrug thereof: 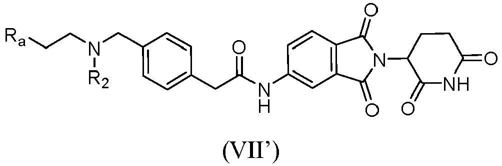
wherein the "NH" in the -CH₂C(=O)NH- is optionally substituted with R_{b}-, R_{b}C(=O)- or R_{b}S(=O)₂-;
Rₐ, R_{b} and R₂ are defined as above.

29. The compound and the pharmaceutically acceptable salt, solvent compound, stereoisomer, isotope and prodrug thereof according to claim 1, wherein the compound has the following structures:
| | | | |
|---|---|---|---|
| **A1** | 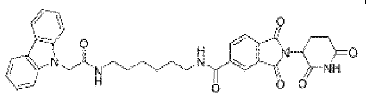 | **A118** | 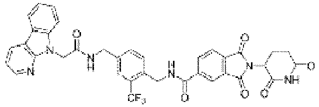 |
| **A2** | 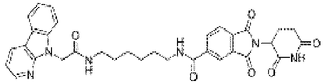 | **A119** | 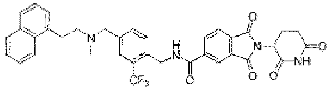 |
| **A3** | 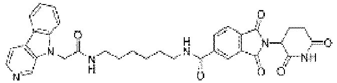 | **A120** | 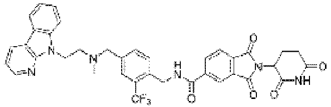 |
| **A4** | 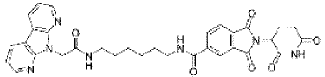 | **A121** | 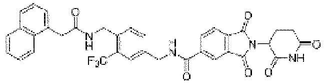 |
| **A5** | 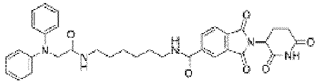 | **A122** | 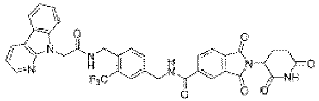 |
| **A6** | 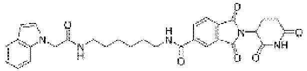 | **A123** | 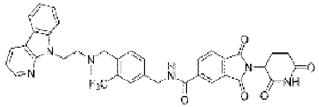 |
| **A7** | 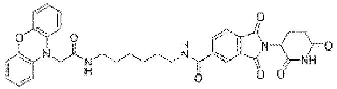 | **A124** | 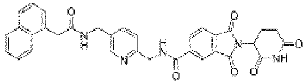 |
| **A8** | 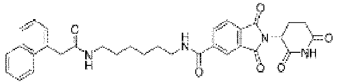 | **A125** | 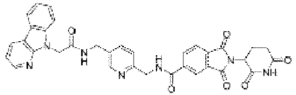 |
| **A9** | 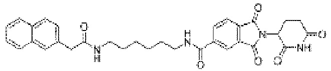 | **A126** | 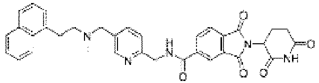 |
| **A10** | 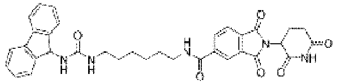 | **A127** | 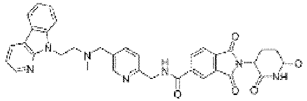 |
| **A11** | 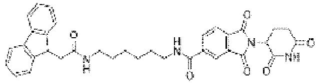 | **A128** | 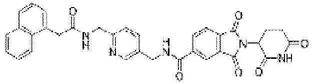 |
| **A12** | 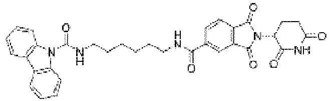 | **A129** | 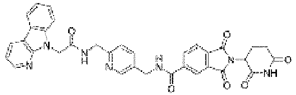 |
| **A13** | 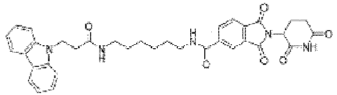 | **A130** | 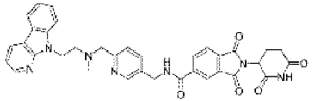 |
| **A14** | 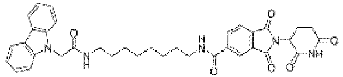 | **A131** | 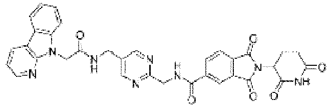 |
| **A15** | 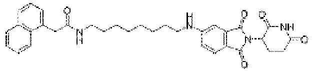 | **A132** | 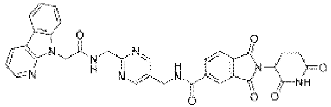 |
| **A16** | 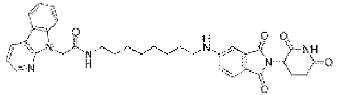 | **A133** | 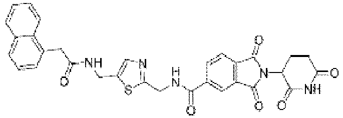 |
| **A17** | 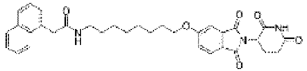 | **A134** | 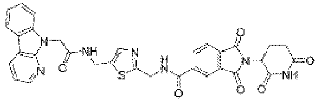 |
| **A18** | 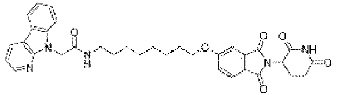 | **A135** | 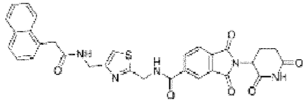 |
| **A19** | 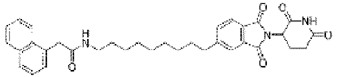 | **A136** | 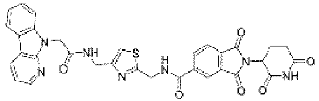 |
| **A20** | 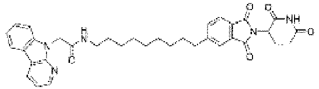 | **A137** | 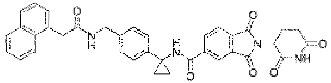 |
| **A21** | 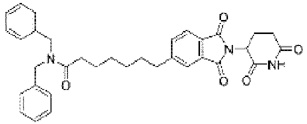 | **A138** | 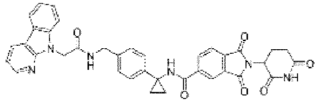 |
| **A22** | 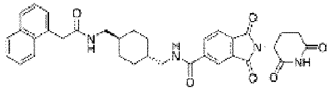 | **A139** | 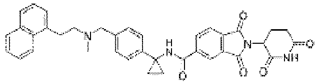 |
| **A23** | 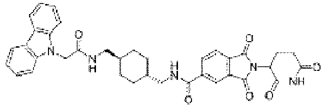 | **A140** | 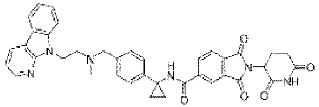 |
| **A24** | 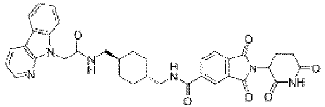 | **A141** | 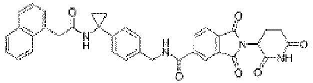 |
| **A25** | 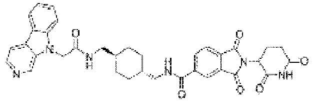 | **A142** | 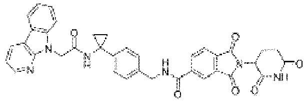 |
| **A26** | 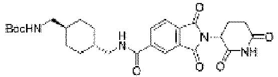 | **A143** | 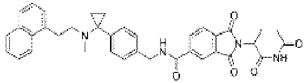 |
| **A27** | 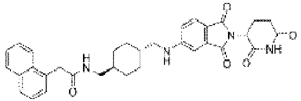 | **A144** | 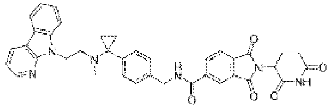 |
| **A28** | 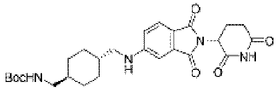 | **A145** | 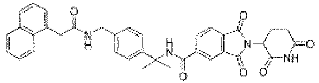 |
| **A29** | 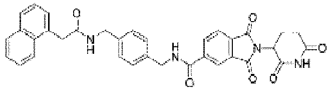 | **A146** | 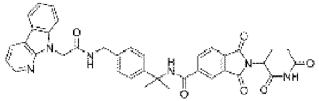 |
| **A30** | 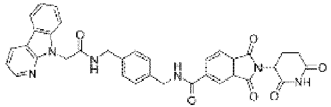 | **A147** | 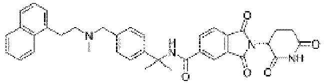 |
| **A31** | 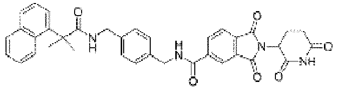 | **A148** | 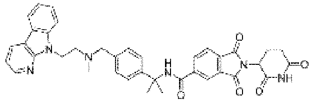 |
| **A32** | 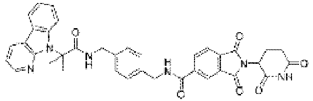 | **A149** | 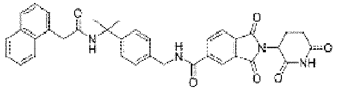 |
| **A33** | 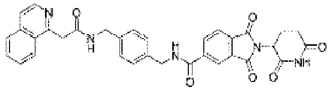 | **A150** | 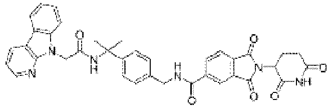 |
| **A34** | 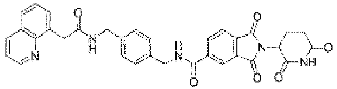 | **A151** | 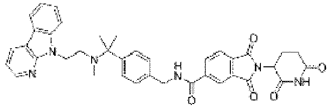 |
| **A35** | 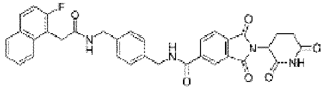 | **A152** | 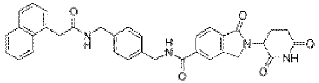 |
| **A36** | 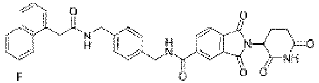 | **A153** | 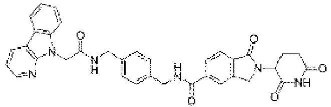 |
| **A37** | 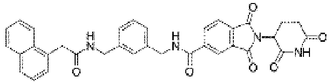 | **A154** | 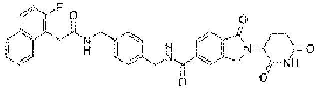 |
| **A38** | 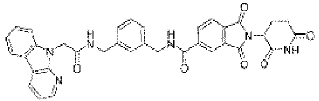 | **A155** | 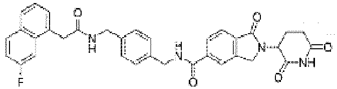 |
| **A39** | 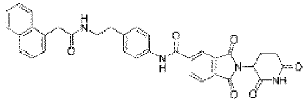 | **A156** | 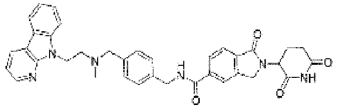 |
| **A40** | 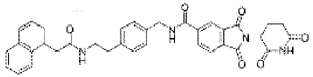 | **A157** | 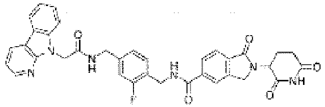 |
| **A41** | 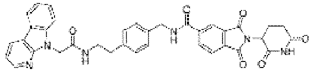 | **A158** | 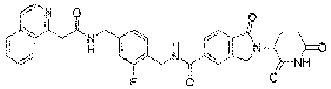 |
| **A42** | 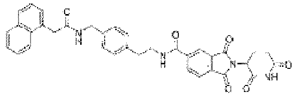 | **A159** | 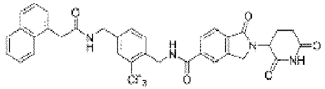 |
| **A43** | 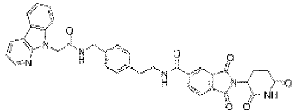 | **A160** | 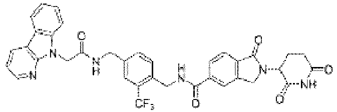 |
| **A44** | 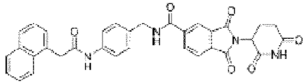 | **A161** | 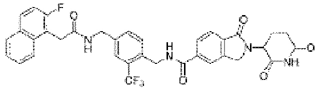 |
| **A45** | 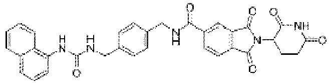 | **A162** | 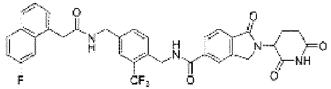 |
| **A46** | 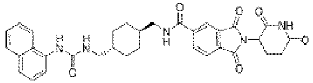 | **A163** | 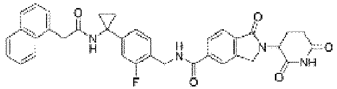 |
| **A47** | 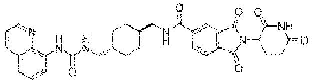 | **A164** | 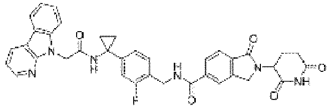 |
| **A48** | 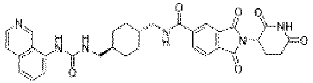 | **A165** | 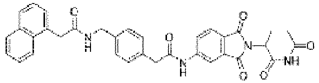 |
| **A49** | 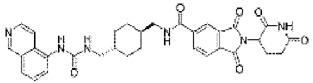 | **A166** | 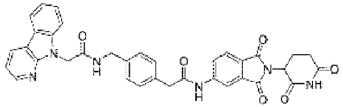 |
| **A50** | 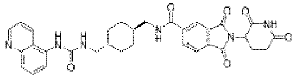 | **A167** | 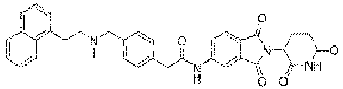 |
| **A51** | 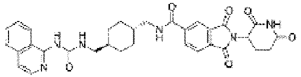 | **A168** | 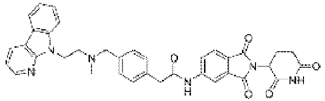 |
| **A52** | 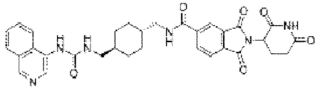 | **A169** | 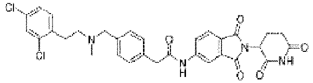 |
| **A53** | 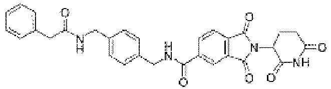 | **A170** | 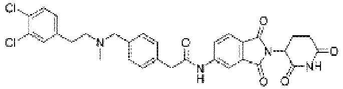 |
| **A54** | 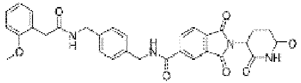 | **A171** | 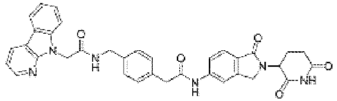 |
| **A55** | 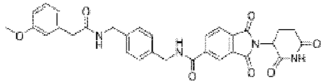 | **A172** | 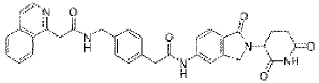 |
| **A56** | 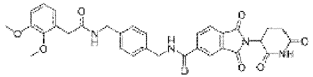 | **A173** | 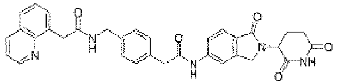 |
| **A57** | 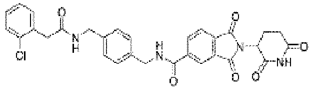 | **A174** | 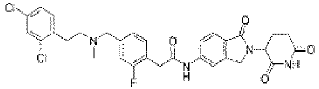 |
| **A58** | 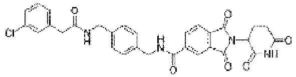 | **A175** | 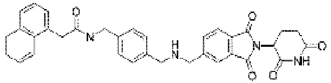 |
| **A59** | 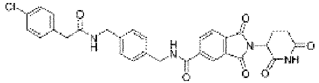 | **A176** | 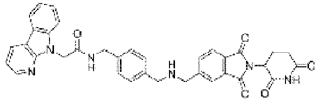 |
| **A60** | 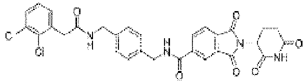 | **A177** | 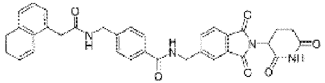 |
| **A61** | 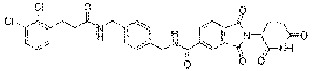 | **A178** | 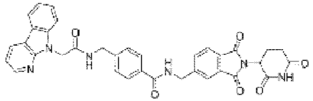 |
| **A62** | 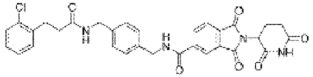 | **A179** | 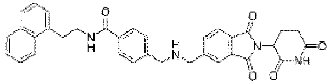 |
| **A63** | 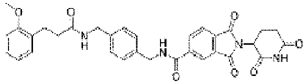 | **A180** | 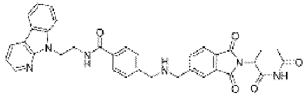 |
| **A64** | 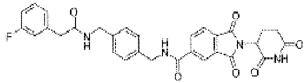 | **A181** | 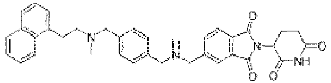 |
| **A65** | 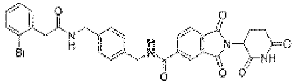 | **A182** | 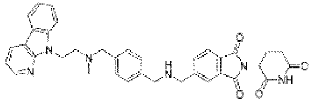 |
| **A66** | 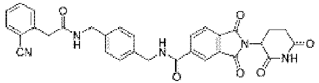 | **A183** | 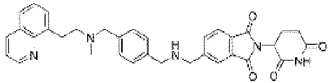 |
| **A67** | 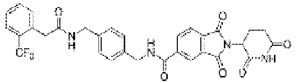 | **A184** | 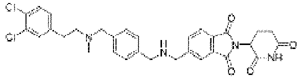 |
| **A68** | 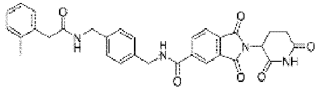 | **A185** | 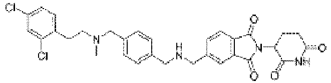 |
| **A69** | 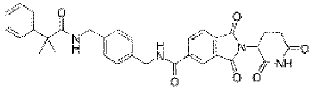 | **A186** | 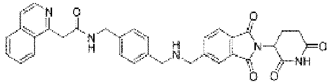 |
| **A70** | 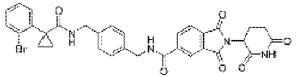 | **A187** | 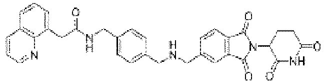 |
| **A71** | 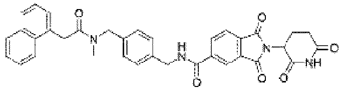 | **A188** | 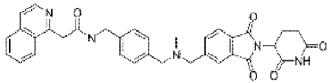 |
| **A72** | 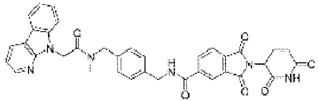 | **A189** | 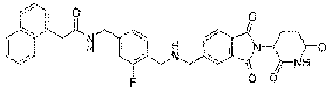 |
| **A73** | 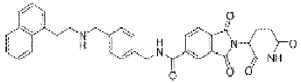 | **A190** | 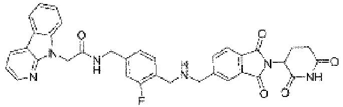 |
| **A74** | 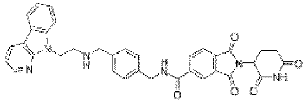 | **A191** | 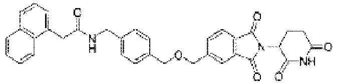 |
| **A75** | 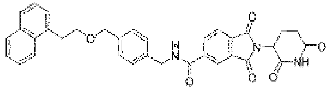 | **A192** | 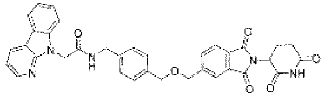 |
| **A76** | 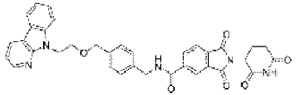 | **A193** | 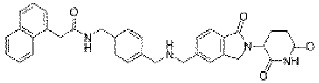 |
| **A77** | 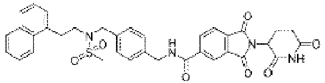 | **A194** | 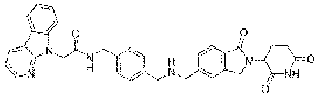 |
| **A78** | 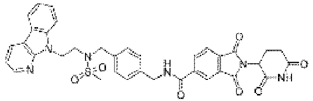 | **A195** | 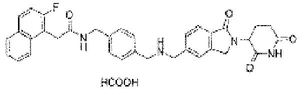 |
| **A79** | 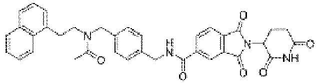 | **A196** | 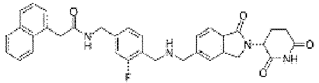 |
| **A80** | 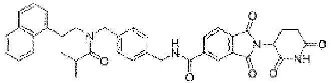 | **A197** | 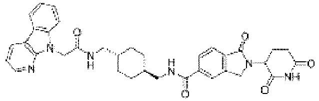 |
| **A81** | 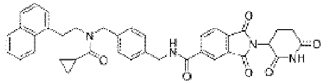 | **A198** | 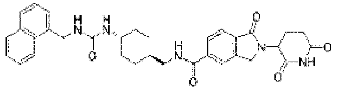 |
| **A82** | 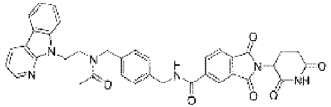 | **A199** | 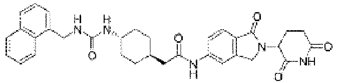 |
| **A83** | 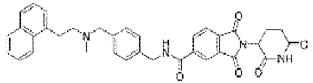 | **A200** | 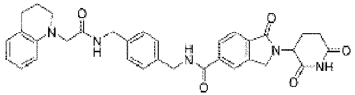 |
| **A84** | 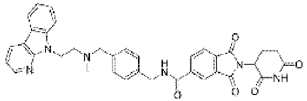 | **A201** | 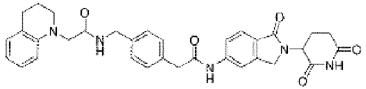 |
| **A85** | 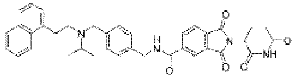 | **A202** | 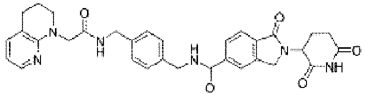 |
| **A86** | 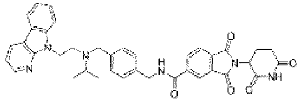 | **A203** | 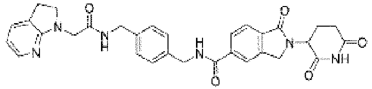 |
| **A87** | 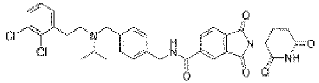 | **A204** | 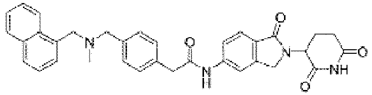 |
| **A88** | 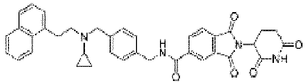 | **A205** | 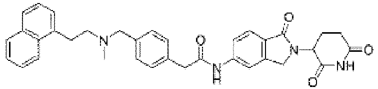 |
| **A89** | 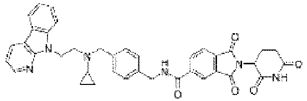 | **A206** | 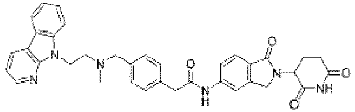 |
| **A90** | 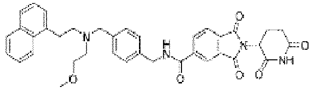 | **A207** | 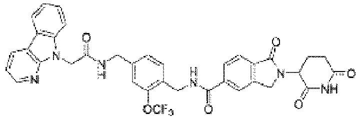 |
| **A91** | 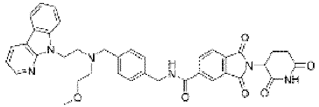 | **A208** | 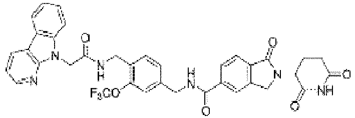 |
| **A92** | 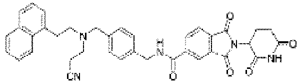 | **A209** | 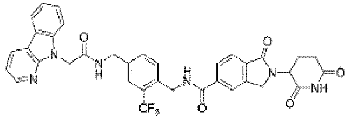 |
| **A93** | 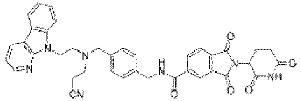 | **A210** | 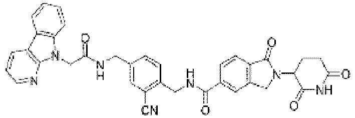 |
| **A94** | 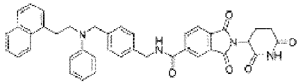 | **A211** | 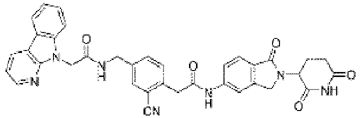 |
| **A95** | 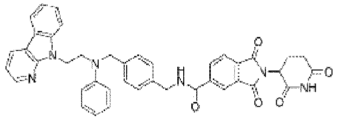 | **A212** | 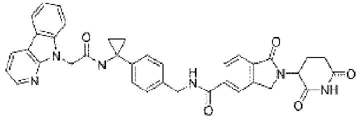 |
| **A96** | 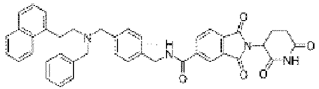 | **A213** | 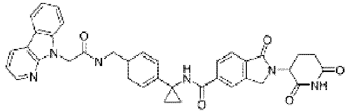 |
| **A97** | 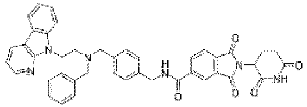 | **A214** | 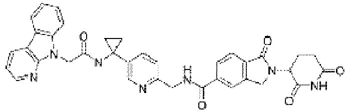 |
| **A98** | 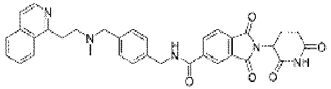 | **A215** | 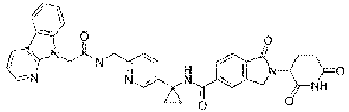 |
| **A99** | 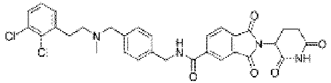 | **A216** | 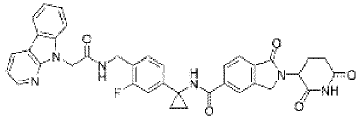 |
| **A100** | 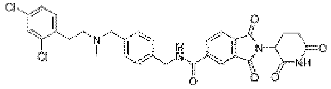 | **A217** | 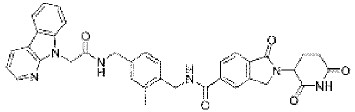 |
| **A101** | 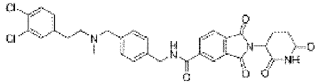 | **A218** | 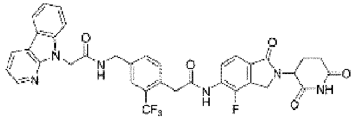 |
| **A102** | 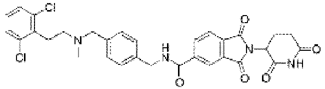 | **A219** | 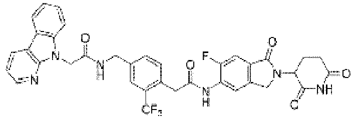 |
| **A103** | 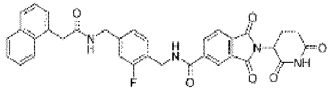 | **A220** | 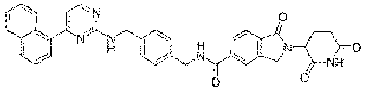 |
| **A104** | 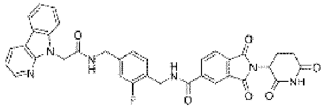 | **A221** | 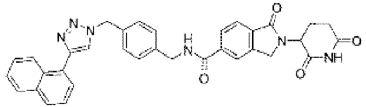 |
| **A105** | 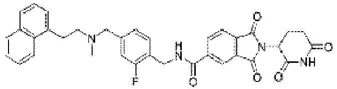 | **A222** | 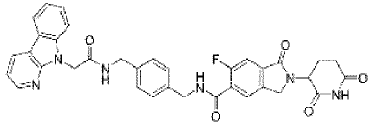 |
| **A106** | 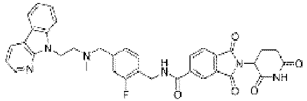 | **A223** | 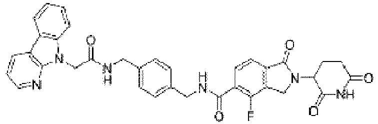 |
| **A107** | 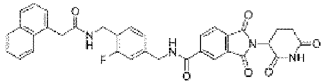 | **A224** | 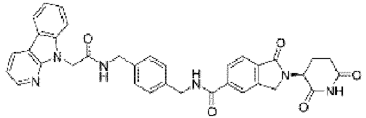 |
| **A108** | 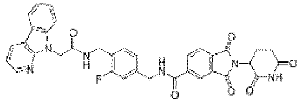 | **A225** | 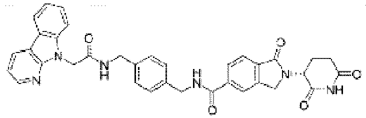 |
| **A109** | 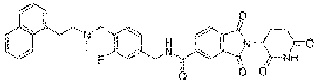 | **A226** | 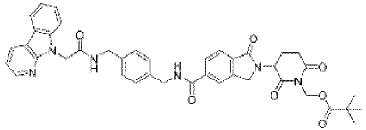 |
| **A110** | 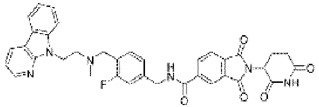 | **A227** | 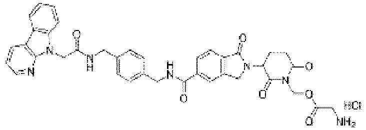 |
| **A111** | 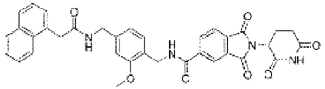 | **A228** | 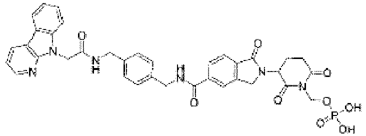 |
| **A112** | 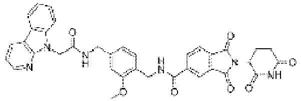 | **A229** | 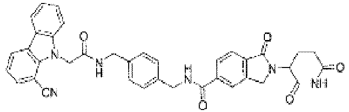 |
| **A113** | 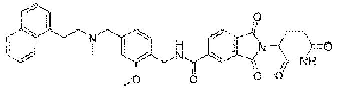 | **A230** | 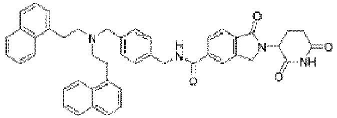 |
| **A114** | 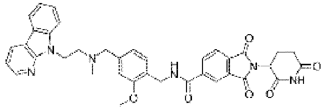 | **A231** | 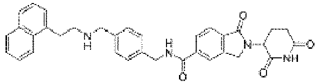 |
| **A115** | 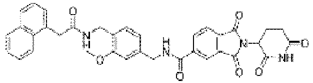 | **A232** | 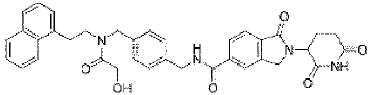 |
| **A116** | 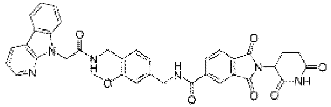 | **A233** | 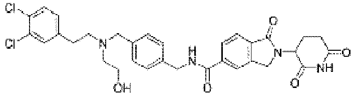 |
| **A117** | 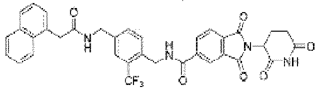 | **A234** | 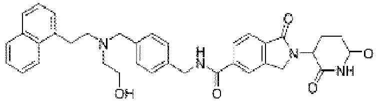 |
| **A235** | 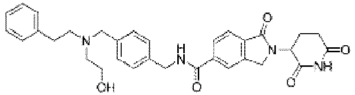 | **A236** | 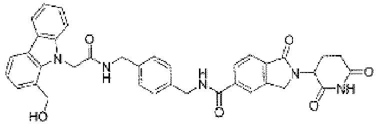 |

30. A pharmaceutical composition, wherein the pharmaceutical composition comprises a pharmaceutically acceptable carrier, and the compound and the pharmaceutically acceptable salt, solvent compound, stereoisomer, isotope and prodrug thereof according to any one of claims 1-29.

31. The pharmaceutical composition according to claim 30, wherein the pharmaceutical composition may further comprise an MYC inhibitor, a DNA methyltransferase inhibitor or a Bcl-2 selective inhibitor.

32. The pharmaceutical composition according to claim 31, wherein the MYC inhibitor is any one or more of OMO-103, APTO-253, PLX-51107, DCR-M1711, Oncomyc-NG, INX-3280, PU-27, GSK-3179106, cholesterol butyrate and NSC-165563; the DNA methyltransferase inhibitor is any one or more of 5-azacytidine, RG108, SGI-1027, GSK3685032, CM272, Bobcat339 hydrochloride, Decitabine (NSC 127716), Thioguanine (NSC 752), 2'-Deoxy-5-Fluorocytidine, Procainamide HCl or Zebularine (NSC 309132); the Bcl-2 selective inhibitor is any one or more of Venetoclax (ABT-199), S55746, BDA-366, Obatoclax Mesylate (GX15-070), HA14-1 or APG-2575 (CAS No. 2180923-05-9).

33. The pharmaceutical composition according to claim 31, wherein the pharmaceutical composition comprises a Bcl-2 selective inhibitor, and the Bcl-2 selective inhibitor is Venetoclax (ABT-199), Obatoclax Mesylate (GX15-070) or APG-2575 (CAS No. 2180923-05-9).

34. Use of the compound and the pharmaceutically acceptable salt, solvent compound, stereoisomer, isotope and prodrug thereof according to any one of claims 1-29 and the pharmaceutical composition according to any one of claims 30-33 in the preparation of a protein degradation agent for any one of or at least two of c-Myc, N-myc, GSPT1, CK1α, IKZF (1/2/3), AR and AR-V7.

35. Use of the compound and the pharmaceutically acceptable salt, solvent compound, stereoisomer, isotope and prodrug thereof according to any one of claims 1-29 and the pharmaceutical composition according to any one of claims 30-33 in the preparation of a drug for treating a disease related to dysregulation of proteins comprising any one of or at least two of c-Myc, N-myc, GSPT1, CK1α, IKZF (1/2/3), AR and AR-V7.

36. Use of the compound and the pharmaceutically acceptable salt, solvent compound, stereoisomer, isotope and prodrug thereof according to any one of claims 1-29 and the pharmaceutical composition according to any one of claims 30-33 in the treatment of a disease related to dysregulation of proteins comprising any one of or at least two of c-Myc, N-myc, GSPT1, CK1α, IKZF (1/2/3), AR and AR-V7.

37. The use according to claim 35 or 36, wherein the dysregulation of proteins is selected from an overexpression of proteins.

38. The use according to claim 35 or 36, wherein the disease related to dysregulation of proteins is selected from cancer, a cardiovascular and cerebrovascular disease, and a viral infection-related disease.

39. The use according to claim 38, wherein the cancer is selected from leukemia, lymphoma, malignant glioma, medulloblastoma, melanoma, multiple myeloma, myelodysplastic syndrome, liver cancer, lung cancer, kidney cancer, pancreatic cancer, oral cancer, gastric cancer, esophageal cancer, laryngeal cancer, nasopharyngeal cancer, skin cancer, breast cancer, colon cancer, rectal cancer, cervical cancer, ovarian cancer, prostate cancer, rhabdomyosarcoma, osteoblastic sarcoma or chondrosarcoma; the viral infection-related disease is selected from HIV, hepatitis B, hepatitis C, hepatitis A, influenza, epidemic encephalitis B or herpes; the leukemia includes chronic lymphocytic leukemia (CLL), chronic granulocytic leukemia (CML), acute myeloid leukemia (AML) or acute non-lymphocytic leukemia (ANLL).

40. Use of the compound and the pharmaceutically acceptable salt, solvent compound, stereoisomer, isotope and prodrug thereof according to any one of claims 1-29 and the pharmaceutical composition according to any one of claims 30-33 in the preparation of a drug for treating acute myeloid leukemia (AML).

41. The use according to claim 40, wherein the pharmaceutical composition comprises a Bcl-2 selective inhibitor, and the Bcl-2 selective inhibitor is Venetoclax (ABT-199), Obatoclax Mesylate (GX15-070) or APG-2575 (CAS No. 2180923-05-9).

42. Use of the compound and the pharmaceutically acceptable salt, solvent compound, stereoisomer, isotope and prodrug thereof according to any one of claims 1-29 in the preparation of a proteolysis targeting chimera (PROTAC) or an antibody-drug conjugate (ADC).
